# EUROPEAN PATENT APPLICATION

(11) **EP 4 516 344 A1**
(43) Date of publication of application: **05.03.2025**
(21) Application number: 23830521.3
(22) Date of filing: 30.06.2023
(51) Int. Cl.: A61M 16/00, F16K 17/38, A61M 16/20, F16K 5/08, F16L 55/10

(54) **FIREPROOF DEVICE, OXYGEN THERAPY INSTRUMENT, AND VENTILATION THERAPY SYSTEM**

(30) Priority: 30.06.2022 CN 202210761499; 30.06.2022 CN 202221696322 U; 30.06.2022 CN 202221696321 U; 30.06.2022 CN 202210761502; 30.06.2022 CN 202210761495; 30.06.2022 CN 202221696132 U; 01.07.2022 CN 202210769833; 29.12.2022 CN 202223598765 U; 29.12.2022 CN 202223598962 U; 30.12.2022 CN 202211733732; 30.12.2022 CN 202223599609 U; 27.06.2023 CN 202321658850 U
(71) Applicant: BMC (Tianjin) Medical Co., Ltd., Tianjin 301700 (CN)
(72) Inventor: LIU, Zhen, Tianjin 301700 (CN); HE, Long, Tianjin 301700 (CN); WANG, Yajie, Tianjin 301700 (CN); ZHOU, Mingzhao, Tianjin 301700 (CN); ZHUANG, Zhi, Tianjin 301700 (CN)
(74) Representative: Grünecker Patent- und Rechtsanwälte PartG mbB
(86) International application number: PCT/CN2023/105040
(87) International publication number: WO 2024/002359

(57) **Abstract**

A fire protection device, an oxygen therapy instrument and a ventilation treatment system, relate to the technical field of medical devices. The fire protection device includes a housing (10), a valve body (20), a torsion spring (30), and a meltable member (40), a fluid passage (101) is provided in the housing (10), the fluid passage (101) is provided with a first opening (102) and a second opening (103), the first opening (102) and the second opening (103) are used for connecting with a pipeline of an oxygen therapy instrument or a patient end, respectively; the valve body (20) is located in the fluid passage (101) and is rotatably connected to the housing (10); the valve body (20) is provided with an accommodating cavity (201), and the fluid passage (101) and the accommodating cavity (201) are two spaces independent from each other; the torsion spring (30) is embedded in the accommodating cavity (201) so as to drive the relative rotation of the valve body (20) and the housing (10); and the meltable member (40) is disposed on an inner wall of the fluid passage (101). When the meltable member (40) is in a non-molten state, the meltable member (40) supports the valve body (20) to be in a first position, both the first opening (102) and the second opening (103) are in an open state; and when the meltable member (40) is in a molten state, the torsion spring (30) drives the valve body (20) to rotate to a second position, at least one of the first opening (102) and the second opening (103) is in a closed state.

## Description

### CROSS REFERENCE TO THE RELATED APPLICATIONS

The present disclosure claims the priority of the Chinese patent application filed on June 30, 2022 before the China National Intellectual Property Administration with the application number of 202210761502.0, and the title of "Fire Protection Device, Oxygen Therapy Instrument And Ventilation Treatment System"; the present disclosure claims the priority of the Chinese patent application filed on June 30, 2022 before the China National Intellectual Property Administration with the application number of 202210761499.2, and the title of "Fire Protection Device, Oxygen Therapy Instrument And Ventilation Treatment System"; the present disclosure claims the priority of the Chinese patent application filed on June 30, 2022 before the China National Intellectual Property Administration with the application number of 202221696322.0, and the title of "Fire Protection Device, Oxygen Therapy Instrument And Ventilation Treatment System"; the present disclosure claims the priority of the Chinese patent application filed on June 30, 2022 before the China National Intellectual Property Administration with the application number of 202221696321.6, and the title of "Fire Protection Device, Oxygen Therapy Instrument And Ventilation Treatment System"; the present disclosure claims the priority of the Chinese patent application filed on June 30, 2022 before the China National Intellectual Property Administration with the application number of 202210761495.4, and the title of "Fire Protection Device And Ventilation Treatment Apparatus"; the present disclosure claims the priority of the Chinese patent application filed on July 01, 2022 before the China National Intellectual Property Administration with the application number of 202210769833.9, and the title of "Fire Protection Device And ventilation Treatment Apparatus"; the present disclosure claims the priority of the Chinese patent application filed on December 30, 2022 before the China National Intellectual Property Administration with the application number of 202211733732.2, and the title of "Fire Protection Device And Ventilation Treatment Apparatus"; the present disclosure claims the priority of the Chinese patent application filed on December 30, 2022 before the China National Intellectual Property Administration with the application number of 202223599609.4, and the title of "Fire Protection Device And Ventilation Treatment Apparatus"; the present disclosure claims the priority of the Chinese patent application filed on December 29, 2022 before the China National Intellectual Property Administration with the application number of 202223598962.0, and the title of "Fire Protection Device And Ventilation Treatment Apparatus"; the present disclosure claims the priority of the Chinese patent application filed on June 30, 2022 before the China National Intellectual Property Administration with the application number of202221696132.9, and the title of "fire Damper And Ventilation Apparatus"; the present disclosure claims the priority of Chinese patent application filed on December 29, 2022 before the China National Intellectual Property Administration with the application number of 202223598765.9, and the title of "Automatic Fire Protection Device Applied To Oxygen Therapy Apparatus"; the present disclosure claims the priority of the Chinese patent application filed on June 27, 2023 before the China National Intellectual Property Administration with the application number of 202321658850.1, and the title of "Fire Protection Device And Ventilation Treatment Apparatus", which are incorporated herein in them entirety by reference.

### TECHNICAL FIELD

The present disclosure relates to the technical field of medical devices and more particularly, to a fire protection device, an oxygen therapy instrument and a ventilation treatment system.

### BACKGROUND

At present, the oxygen therapy has been widely used in clinical treatment and hospital rescue. When patients cannot meet their needs by inhaling oxygen themselves, external oxygen supply is usually required. External instruments can be used to supply oxygen to the patients by invasive or non-invasive methods. Instruments used to supply oxygen to a patient are collectively referred to as oxygen therapy instruments.

Due to the combustion-supporting characteristic of oxygen, it is usually necessary to provide a fire protection device on the oxygen therapy instrument. When oxygen leakage occurs and causes fire, the oxygen pathway is timely cut off by the fire protection device, so as to reduce the spread of fire and reduce the loss. In the prior art, a support having a relatively low melting point is generally provided at an opening of the fire protection device. A sealing valve is supported by the support. After the support is melted by heat, the sealing valve is driven by a spring to move to the opening and is engaged with the opening to block a gas path.

The existing fire protection device communicates with the oxygen therapy instrument and the oxygen pipeline at the patient end. A metal spring is provided inside the fire protection device. When the metal spring is in an oxygen-rich environment, the phenomenon of oxidation failure is easy to occur. When a fire occurs, there is still a risk of oxygen leakage, with a low safety factor.

When the oxygen therapy instrument is normally used, in order to ensure that oxygen can pass through the fire protection device, it is necessary to open a small hole on the support. The design of opening holes has a greater impact on the smooth gas path, and the stability is not strong. It is easy to block the gas path in non-fire conditions, thus affecting the treatment effect.

With the fire protection device in the related art, when the sealing valve moves inside the fire protection device, the sealing valve will be deflected, there is a problem in that the sealing valve cannot be accurately engaged with the opening and the stability of the fire protection device is poor. Furthermore, the slow melting speed of the support results in that the sealing valve cannot move to the opening in time, and cannot rapidly block the gas path, thus easily causing the spread of fire.

The ventilation apparatus communicates with a respiratory airway of a user via a pipeline to provide oxygen to the user continuously via the pipeline. Due to the combustion-supporting characteristic of oxygen, the oxygen produced by the ventilation apparatus can cause the fire to continue to expand when the user side catches fire.

In a medical or home environment, external oxygen is often required when the patients cannot meet their needs by inhaling oxygen themselves. This method of supplying oxygen to the patient by means of an external instrument, either in an invasive manner or a non-invasive manner, is known as oxygen therapy. Instruments used to provide oxygen to the patients are collectively referred to as oxygen generators. The oxygen generators are typically connected by flexible plastic pipe to a respiratory mask or nasal intubation that is worn to a face of a patient in need of ventilation treatment (such as oxygen therapy).

Oxygen as a combustion-supporting gas, if exposed to open fire (such as smoking, etc.), will certainly promote fire, which is very easy to cause fire. Most oxygen generators are configured for continuously delivering oxygen to the respiratory mask or nasal intubation at a determined rate according to the needs of the patient. Even if the respiratory mask or nasal intubation is removed, it does not cause the delivery of oxygen to cease. In this case, it is easy to establish an oxygen rich environment around the patient, thus preparing the surrounding environment for a catastrophic fire based on ignition. However, the oxygen concentration output by the oxygen generator is generally greater than 90%. Once the flexible plastic pipe which is outputting oxygen is accidentally ignited, the flame will gradually burn towards the generator body along with the oxygen output pipe since there is a continuous high-concentration oxygen outflow from the flexible plastic pipe, and finally the oxygen generator is ignited to generate fire. When the oxygen generator is used in the home care environment, due to the lack of corresponding supervision conditions, the above-mentioned fire hazards will be aggravated in the home environment. If the output path of oxygen cannot be timely closed, the fire will become more severe, which will aggravate the difficulty of fire rescue.

In order to block the output path of oxygen in the event of a fire, in the related art, a method of providing a fire-protection isolation device is generally used. When the fire-protection isolation device in the related art is in normal operation, a gas (such as oxygen) flows out through a small hole of a meltable nose component after passing through a central hole of surrounding the compression spring. In the event of a fire, the meltable nose component is melted at an elevated temperature, thus allowing the compression spring to urge the poppet valve to close so as to block the output path of oxygen. However, the compression springs in such fire-protection isolation devices are typically made of metallic materials, after the compression springs made of the metallic materials are exposed to oxygen for a long time, particularly in humid environments, there will inevitably be chemical reactions such as oxidation and thus causing the failure of the compression springs, which would greatly reduce the useful life of the fire-protection isolation device while affecting the health of the patients.

### SUMMARY

The present disclosure provides a fire protection device, an oxygen therapy instrument and a ventilation treatment system, aiming to solve the problems in the related art that oxidation failure is easy to occur since a metal spring inside the fire protection device is in an oxygen-rich environment, and there is still a risk of oxygen leakage when a fire occurs, with a low safety.

In a first aspect, embodiments of the present disclosure disclose a fire protection device, including a housing, a valve body, a torsion spring, and a meltable member;
a fluid passage is provided in the housing, the fluid passage is provided with a first opening and a second opening, and the first opening and the second opening are used for communicating with a pipeline of an oxygen therapy instrument or a patient end, respectively;
the valve body is located in the fluid passage and is rotatably connected to the housing;
the valve body is provided with an accommodating cavity, and the fluid passage and the accommodating cavity are two spaces independent from each other;
the torsion spring is embedded in the accommodating cavity so as to drive the relative rotation of the valve body and the housing;
the meltable member is disposed on an inner wall of the fluid passage;
when the meltable member is in a non-molten state, the meltable member supports the valve body to be in a first position, and both the first opening and the second opening are in an open state; and
when the meltable member is in a molten state, the torsion spring drives the valve body to rotate to a second position, and at least one of the first opening and the second opening is in a closed state.

Optionally, the valve body includes a mounting portion, a first connecting portion and a first sealing portion;
the mounting portion includes an inner shaft sleeve and an outer shaft sleeve, and the accommodating cavity is located between the inner shaft sleeve and the outer shaft sleeve;
a rotating shaft is provided in the fluid passage, and the inner shaft sleeve is sleeved on the rotating shaft and is rotatably connected to the rotating shaft;
one end of the first connecting portion is connected to a side wall of the outer shaft sleeve, and the other end of the first connecting portion is connected to the first sealing portion; and
when the valve body is in the second position, the first sealing portion is engaged with the first opening, so that the first opening is in the closed state.

Optionally, the valve body further includes a second connecting portion and a second sealing portion;
one end of the second connecting portion is connected to a side wall of the outer shaft sleeve, and the other end of the second connecting portion is connected to the second sealing portion; and
when the valve body is in the second position, the second sealing portion is engaged with the second opening, so that the second opening is in the closed state.

Optionally, the first connecting portion and/or the second connecting portion are provided with a notch for passage of a fluid.

Optionally, the inner wall of the fluid passage is provided with at least one limiting portion, and the limiting portion is located on a rotational path of the valve body; and
when the valve body is in the second position, the valve body abuts against the limiting portion.

Optionally, the housing is provided with a first pipeline joint and a second pipeline joint;
the first pipeline joint is provided with a first through-hole, and the first through-hole communicates with the first opening;
the second pipeline joint is provided with a second through-hole, and the second through-hole communicates with the second opening; and
the first pipeline joint and the second pipeline joint are used for connecting with the pipeline of the oxygen therapy instrument or the patient end, respectively.

Optionally, an outer side wall of the first pipeline joint and/or the second pipeline joint is provided with at least one clamping portion for being clamped with the pipeline of the oxygen therapy instrument or the patient end.

Optionally, the meltable member has an extension passing through the first through-hole or the second through-hole.

In a second aspect, embodiments of the present disclosure also disclose an oxygen therapy instrument including the above-described fire protection device.

**In** a third aspect, embodiments of the present disclosure also disclose a ventilation treatment system including the above-described oxygen therapy instrument.

**In** an embodiment of the present disclosure, the fire protection device includes the housing, the valve body, the torsion spring, and the meltable member, wherein the housing has a fluid passage therein, the fluid passage is provided with a first opening and a second opening, and the first opening and the second opening are used for communicating with the pipeline of the oxygen therapy instrument or the patient end, respectively; the valve body is located in the fluid passage and is rotatably connected to the housing; the valve body is provided with the accommodating cavity, and the fluid passage and the accommodating cavity are two spaces independent from each other; the torsion spring is embedded in the accommodating cavity so as to drive the relative rotation of the valve body and the housing; and the meltable member is disposed on the inner wall of the fluid passage. When the meltable member is in the non-molten state the meltable member supports the valve body to be in the first position, both the first opening and the second opening are in the open state, and the fluid passage normally transmits oxygen. When a fire occurs, the meltable member will be in a molten state as the temperature reaches the melting point, the meltable member is insufficient to support the elastic force applied to the valve body by the torsion spring, the torsion spring releases the stored elastic potential energy, the valve body rotates from the first position to the second position under the drive of the torsion spring, so that at least one of the first opening and the second opening is in the closed state, thereby cutting off the oxygen passage and preventing the oxygen from continuously leaking out to cause the fire to spread. In addition, the accommodating cavity in which the torsion spring is located and the fluid passage are independent from each other, so that the problem of oxidation of the torsion spring is avoided, and the durability and safety factor of the device are improved.

In a fourth aspect, embodiments of the present disclosure disclose a fire protection device including a housing, an elastic member, a valve body, and a meltable member;
an accommodating cavity and a gas passage are provided in the housing, and the gas passage is used for communicating with a pipeline of an oxygen therapy instrument or a patient end;
the valve body separates the accommodating cavity and the gas passage into mutually independent spaces;
the elastic member is disposed in the accommodating cavity, the valve body is at least partially located in the gas passage, and the elastic member abuts against the housing and the valve body;
the meltable member is disposed between the valve body and the gas passage, and a gap is located between the meltable member and the gas passage;
when the meltable member is in a non-molten state, the meltable member supports the valve body to be in a first position, with the gas passage in an open state; and
when the meltable member is in a molten state, the elastic member drives the valve body to be in a second position, with the gas passage in a closed state.

Optionally, the accommodating cavity has an opening, the valve body is provided at the opening of the accommodating cavity, and the valve body is slidably connected to the accommodating cavity;
when the meltable member is in the non-molten state, the meltable member supports the valve body to be in the first position, with the gas passage in the open state; and
when the meltable member is in the molten state, the elastic member drives the valve body to slide to the second position, with the gas passage in the closed state.

Optionally, the housing includes a first housing and a second housing;
the first housing is rotatably connected to the second housing;
the first housing is provided with a first through-hole and a second through-hole;
the second housing is provided with a third through-hole and a fourth through-hole;
the valve body is fixedly connected to the first housing, and the valve body is provided with a fifth through-hole;
the meltable member is clamped with the valve body and the second housing, respectively, and the meltable member is provided with a sixth through-hole;
the elastic members abut against the valve body and the second housing, respectively;
when the meltable member is in the non-molten state, the meltable member supports the valve body to be in the first position, and the first through-hole, the second through-hole, the third through-hole, the fourth through-hole, the fifth through-hole and the sixth through-hole are in communication together to form the gas passage, and the gas passage is in the open state; and
when the meltable member is in the molten state, the elastic member drives the first housing and the second housing to rotate relative to each other; the valve body is in the second position; the first through-hole, the second through-hole and the fifth through-hole attach to the wall of the second housing, respectively, and the gas passage is in the closed state.

Optionally, a first protrusion and a second protrusion are provided in the second housing;
the third through-hole is disposed in the first protrusion, and the fourth through-hole is disposed in the second protrusion;
the meltable member is at least partially embedded in the third through-hole or the fourth through-hole.

Optionally, the first protrusion is provided with a first extension, and the second protrusion is provided with a second extension;
when the valve body is in the second position, both ends of the fifth through-hole attach to the first extension and the second extension, respectively.

Optionally, one end of the valve body is provided with a third extension and the other end of the valve body is provided with a fourth extension;
when the valve body is in the second position, the third through-hole attaches to the third extension and the fourth through-hole attaches to the fourth extension.

Optionally, the second housing is provided with at least one limiting portion located on a movement path of the valve body; and
when the valve body is in the second position, the valve body abuts against the limiting portion.

Optionally, the first housing is provided with a first connecting portion and a second connecting portion;
the first through-hole is provided at the first connecting portion, and the second through-hole is provided at the second connecting portion;
the first connecting portion and the second connecting portion are used for communicating with the pipeline of the oxygen therapy instrument or the patient end, respectively.

Optionally, the gas passage includes a first pipeline interface, a second pipeline interface and a communicating portion;
the communicating portion has an inner cavity;
the first pipeline interface and the second pipeline interface are located at both ends of the communicating portion, respectively, and communicate with the inner cavity to form the gas passage; and
the first pipeline interface and the second pipeline interface are symmetrically arranged.

Optionally, the meltable member is located in the inner cavity, and the meltable member is in clearance fit with a cavity wall of the inner cavity.

In a fifth aspect, embodiments of the present disclosure also disclose an oxygen therapy instrument including the above-described fire protection device.

In a sixth aspect, embodiments of the present disclosure also disclose a ventilation treatment system including the above-described oxygen therapy instrument.

In an embodiment of the present disclosure, a fire protection device includes the housing, the elastic member, the valve body, and the meltable member; the housing has the accommodating cavity therein, and the gas passage for communicating with the pipeline of the oxygen therapy instrument or the patient end; the valve body separates the accommodating cavity and the gas passage into mutually independent spaces; the elastic member is disposed in the accommodating cavity, the valve body is at least partially located in the gas passage, and the elastic member abuts against the housing and the valve body, respectively; the meltable member is disposed between the valve body and the gas passage, and the gap is located between the meltable member and the gas passage; when the meltable member is in a non-molten state, the meltable member supports the valve body to be in the first position, with the gas passage in the open state; and when the meltable member is in a molten state, the elastic member drives the valve body to be in the second position, with the gas passage in the closed state. When a fire occurs, the meltable member will be in the molten state when the temperature reaches the melting point. The meltable member is insufficient to support the elastic force applied to the valve body by the elastic member. The elastic member releases the stored elastic potential energy. The valve body is switched from the first position to the second position under the drive of the elastic member to encroach on the space of the gas passage, thereby cutting off the oxygen passage and avoiding the spread of the fire caused by the continuous leakage of oxygen. Furthermore, the accommodating cavity where the elastic member is located and the gas passage are independent from each other, so that the problem of oxidation of the elastic member is avoided, and the durability and safety factor of the device are improved.

In a seventh aspect, embodiments of the present disclosure disclose a fire protection device including a housing, a first elastic valve body, and a first meltable member;
an accommodating cavity is provided in the housing, two sides of the accommodating cavity are provided with a first opening and a second opening, and the first opening and the second opening are used for communicating with a pipeline of an oxygen therapy instrument or a patient end;
the first elastic valve body is positioned within the accommodating cavity, and the first elastic valve body is disposed opposite to the first opening, wherein the first elastic valve body has oxidation resistant characteristics;
the first meltable member is located within and connected to the housing, the first meltable member abuts against a side of the first elastic valve body close to the first opening;
when the first meltable member is in a non-molten state, the first meltable member compresses the first elastic valve body, with a gap between the first opening and the first elastic valve body for gas flow; and
when the first meltable member is in a molten state, the first elastic valve body releases at least part of elastic potential energy, and the first elastic valve body abuts against a first end surface of the accommodating cavity, so that the first opening is in a closed state, wherein the first opening is disposed at the first end surface.

Optionally, the housing includes a first connecting portion and a second connecting portion;
the first connecting portion has a first through-hole communicating with the first opening;
the second connecting portion has a second through-hole communicating with the second opening;
the first meltable member is located in the first through-hole, one end of the first meltable member extends to form at least two reinforcing ribs, and the reinforcing ribs are connected to the hole wall of the first through-hole; and
the other end of the first meltable member abuts against the side of the first elastic valve body close to the first opening.

Optionally, the first meltable member is located between the first elastic valve body and the first end surface, and the first meltable member abuts against the side of the first elastic valve body close to the first opening.

Optionally, a quantity of the first meltable members is two and two of the first meltable members are symmetrically disposed on the first end surface.

Optionally, the fire protection device further includes a support;
the support is located in the accommodating cavity and is connected to a cavity wall of the accommodating cavity; and
the support abuts against a side of the first elastic valve body away from the first opening.

Optionally, a first positioning portion is disposed at an end of the support close to the first elastic valve body, and a second positioning portion is disposed at a side of the first elastic valve body away from the first opening, the first positioning portion and the second positioning portion are in positioning fit.

Optionally, an outer side wall of the first connecting portion and/or an outer side wall of the second connecting portion is provided with at least one clamping portion for being clamped with the pipeline of the oxygen therapy instrument or the patient end.

Optionally, the fire protection device further includes a second elastic valve body and a second meltable member;
the second elastic valve body is disposed opposite to the second opening;
the second meltable member is located within and connected to the housing, the second meltable member abuts against a side of the second elastic valve body close to the second opening, wherein the second elastic valve body has oxidation resistant characteristics;
the support is located between the first elastic valve body and the second elastic valve body, and abuts against the first elastic valve body and the second elastic valve body, respectively;
when the second meltable member is in a non-molten state, the second meltable member compresses the second elastic valve body, with a gap between the second opening and the second elastic valve body for gas flow;
when the second meltable member is in a molten state, the second elastic valve body releases at least part of the elastic potential energy, and the second elastic valve body abuts against the second end surface of the accommodating cavity so that the second opening is in a closed state, wherein the second opening is disposed at the second end surface.

In an eighth aspect, embodiments of the present disclosure also disclose an oxygen therapy instrument including the above-described fire protection device.

In a ninth aspect, embodiments of the present disclosure also disclose a ventilation treatment system including the above-described oxygen therapy instrument.

In an embodiment of the present disclosure, the fire protection device includes the housing, the first elastic valve body, and the first meltable member; the accommodating cavity is provided in the housing, two sides of the accommodating cavity are provided with the first opening and the second opening, and the first opening and the second opening are used for communicating with the pipeline of the oxygen therapy instrument or the patient end; and the first elastic valve body is positioned within the accommodating cavity, and the first elastic valve body is disposed opposite to the first opening, wherein the first elastic valve body has oxidation resistant characteristics. The first meltable member is located within and connected to the housing, the first meltable member abuts against the side of the first elastic valve body close to the first opening. When the first meltable member is in the non-molten state, the first meltable member compresses the first elastic valve body, and the thickness of the first elastic valve body decreases, with a gap between the first opening and the first elastic valve body for gas flow. **In** the event of a fire, when the temperature of the meltable member reaches the melting point, the meltable member will be in the molten state; the first elastic valve body releases at least part of the elastic potential energy, and the first elastic valve body abuts against the first end surface of the accommodating cavity, so that the first opening is in a closed state, thereby cutting off the passage of oxygen, and preventing oxygen from continuously leaking out and causing the fire to spread, wherein the first opening is disposed at the first end surface. Since the first elastic valve body has oxidation resistance characteristics, the problem of oxidation of the first elastic valve body is avoided, and the durability and safety factor of the device are improved.

The present disclosure also provides a fire protection device, an oxygen therapy instrument and a ventilation treatment system, aiming to solve the problems in the related art that transmitting oxygen by opening a small hole on a support, has a great influence on the conduction of a gas path and is not stable, is easy to block the gas path in a non-fire situation, and affects the treatment effect.

In a first aspect, embodiments of the present disclosure disclose a fire protection device including a housing, a first movable rod, a first meltable member, a first valve body, and a first elastic member;
a fluid passage is provided in the housing, the fluid passage is provided with a first narrowed portion and a second narrowed portion, and the first movable rod, the first meltable member, the first valve body and the first elastic member are all disposed in the fluid passage;
the first meltable member is disposed at one end of the fluid passage and is connected to an inner wall of the fluid passage;
the first movable rod is slidably connected to the inner wall of the fluid passage, and the first meltable member is disposed on the first movable rod;
the first valve body passes through the first narrowed portion and is slidably connected to the inner wall of the fluid passage, one end of the first valve body is connected to the first movable rod, the other end of the first valve body is provided with the first elastic member, and the first elastic member abuts against the housing;
the first valve body is provided with a first sealing member close to the first narrowed portion;
when the first valve body is in an open state, the first meltable member supports the first movable rod and the first valve body is in a first position, the first sealing member is in clearance fit with the first narrowed portion, and the fluid passage is in an open state; and
when the first elastic member drives the first movable rod and the first valve body slides to a second position, the first valve body is in a closed state, the first sealing member is engaged with the first narrowed portion, and the fluid passage is in a closed state.

Optionally, the first meltable member is provided with a first clamping portion and the first movable rod is provided with a first clamping-fitting portion, and the first meltable member is clamped with the first movable rod.

Optionally, the side wall of the first movable rod is provided with at least one first guide portion and the inner wall of the fluid passage is provided with a first chute, and the first guide portion is in sliding fit with the first chute.

Optionally, the side wall of the first valve body is provided with at least one second guide portion and the inner wall of the fluid passage is provided with a second chute, and the second guide portion is in sliding fit with the second chute.

Optionally, the other end of the first valve body is provided with a groove body in which the first elastic member is at least partially located; and
one end of the first elastic member abuts against a groove bottom of the groove body, and the other end of the first elastic member abuts against the housing.

Optionally, a mounting shaft is disposed in the groove body, and the mounting shaft is provided coaxially with the groove body; and
the first elastic member is sleeved on the mounting shaft.

Optionally, the housing further includes a baffle disposed within the fluid passage and connected to an inner wall of the fluid passage; and
the first elastic member abuts against the baffle.

Optionally, the fire protection device further includes a second movable rod, a second meltable member, a second valve body, and a second elastic member;
the second movable rod, the second meltable member, the second valve body, and the second elastic member are all disposed in the fluid passage;
the second meltable member is disposed at the other end of the fluid passage and is connected to the inner wall of the fluid passage;
the second movable rod is slidably connected to the inner wall of the fluid passage, and the second meltable member is disposed on the second movable rod;
the second valve body passes through the second narrowed portion and is slidably connected to the inner wall of the fluid passage, one end of the second valve body is connected to the second movable rod, the other end of the second valve body is provided with the second elastic member, and the second elastic member abuts against the housing;
the second valve body is provided with a second sealing member close to the second narrowed portion;
when the second valve body is in an open state, the second meltable member supports the second movable rod and the second valve body is in a third position, the second sealing member is in clearance fit with the second narrowed portion, and the fluid passage is in an open state;
when the second elastic member drives the second movable rod and the second valve body to slide to a fourth position, the second valve body is in a closed state, the second sealing member is engaged with the second narrowed portion, and the fluid passage is in a closed state.

Optionally, the second meltable member is provided with a second clamping portion, the second movable rod is provided with a second clamping-fitting portion, and the second meltable member is clamped with the second movable rod.

Optionally, the housing is provided with a first pipeline joint and a second pipeline joint, the first pipeline joint and the second pipeline joint are used for connecting with a pipeline of an oxygen therapy instrument or a patient end, respectively; and
an outer side wall of the first pipeline joint and/or the second pipeline joint is provided with at least one third clamping portion for being clamped with the pipeline of the oxygen therapy instrument or the patient end.

Optionally, the first movable rod and the first valve body are in an integrated structure.

Optionally, the first movable rod and the first valve body are in a split structure.

In a second aspect, embodiments of the present disclosure also disclose an oxygen therapy instrument including the above-described fire protection device.

In a third aspect, embodiments of the present disclosure also disclose a ventilation treatment system including the above-described oxygen therapy instrument.

In an embodiment of the present disclosure, the fire protection device includes the housing, the first movable rod, the first meltable member, the first valve body, and the first elastic member; the fluid passage is provided in the housing, the fluid passage is provided with a first narrowed portion and a second narrowed portion, the first movable rod, the first meltable member, the first valve body and the first elastic member are all provided in the fluid passage; the first meltable member is disposed at one end of the fluid passage and is connected to an inner wall of the fluid passage; the first movable rod is slidably connected to the inner wall of the fluid passage, and the first meltable member is disposed on the first movable rod; the first valve body passes through the first narrowed portion and is slidably connected to the inner wall of the fluid passage; one end of the first valve body is connected to the first movable rod, and the other end of the first valve body is provided with the first elastic member, the first elastic member abuts against the housing; the first valve body is provided with the first sealing member close to the first narrowed portion. When the first valve body is in the open state, the first meltable member supports the first movable rod and the first valve body is in the first position, the first sealing member is in clearance fit with the first narrowed portion, and the fluid passage is in the open state, so that the normal transmission of oxygen can be ensured without forming holes on the first meltable member, thereby improving the stability of the fire protection device. In the event of a fire, the first meltable member melts and is insufficient to support the elastic force applied to the first elastic member on the first valve body and the first movable rod. The first valve body and the first movable rod slide from the first position to the second position under the drive of the first elastic member, and the first valve body is in the closed state. The first sealing member is engaged with the first narrowed portion, and the fluid passage is in the closed state, so as to cut off the oxygen passage, and prevent the oxygen from continuously leaking out to cause the fire to spread.

The present disclosure also provides a fire protection device and a ventilation treatment apparatus, and also aims to solve the problems in the related art that the fire protection device is prone to aging failure, there is still a risk of oxygen leakage in the event of a fire, and the safety factor is low.

**In** a first aspect, embodiments of the present disclosure disclose a fire protection device, including a housing, a first positioning member, and a first sealing member;
a fluid passage is provided in the housing, a first opening and a second opening are provided on two opposite sides of the fluid passage, and the first opening and the second opening are used for communicating with a pipeline of an oxygen therapy instrument or a patient end, respectively;
the first positioning member is disposed in the fluid passage and is connected to the housing;
the first sealing member is sleeved on the first positioning member, and the first sealing member has oxidation resistance characteristics;
at a first temperature, the first sealing member is in clearance fit with an inner wall of the fluid passage, and the fluid passage is in an open state;
at a second temperature, the first sealing member increases in volume and attaches to the inner wall of the fluid passage, and the fluid passage is in a closed state;
wherein the second temperature is higher than the first temperature.

Optionally, the first sealing member is a thermally induced shape memory plastic;
at the second temperature, the first sealing member is expanded by heat and attaches to the inner wall of the fluid passage, and the fluid passage is in a closed state.

Optionally, the inner wall of the fluid passage is provided with at least one annular protrusion; and
at the second temperature, the annular protrusion abuts against the first sealing member and the fluid passage is in a closed state.

Optionally, the inner wall of the fluid passage is provided with a first protruding structure and a second protruding structure;
the first protruding structure and the second protruding structure are located at both ends of the first positioning member, respectively; and
the first protruding structure is provided with a first positioning portion, the second protruding structure is provided with a second positioning portion, and both ends of the first positioning member are clamped with the first positioning portion and the second positioning portion, respectively.

Optionally, the inner wall of the fluid passage is provided with a mounting portion, and the mounting portion is provided with an accommodating groove; and
one end of the first positioning member is embedded in the accommodating groove.

Optionally, one end of the first positioning member is provided with a clamping portion, a groove wall of the accommodating groove is provided with a clamping-fitting portion, and one end of the first positioning member is clamped with the accommodating groove.

Optionally, one end of the first positioning member is provided with an external thread, the groove wall of the accommodating groove is provided with an internal thread, and one end of the first positioning member is threadedly connected with the accommodating groove.

Optionally, one end of the first positioning member is in interference fit with the accommodating groove.

Optionally, the mounting portion is provided with at least one connecting rib by which the mounting portion is connected to the inner wall of the fluid passage.

Optionally, the fire protection device further includes a meltable member;
the first sealing member is an elastomer and the meltable member wraps the first sealing member;
when the meltable member is in a non-molten state, the meltable member compresses the first sealing member, the meltable member is in clearance fit with the inner wall of the fluid passage, and the fluid passage is in an open state; and
when the meltable member is in a molten state, the first sealing member releases at least a part of elastic potential energy, the first sealing member increases in volume and attaches to the inner wall of the fluid passage, and the fluid passage is in a closed state.

Optionally, the fire protection device further includes a second positioning member and a second sealing member;
the second positioning member is disposed in the fluid passage and is connected to the housing, the first positioning member is close to the first opening, and the second positioning member is close to the second opening;
the second sealing member is sleeved on the second positioning member, and the second sealing member has oxidation resistance characteristics;
at a first temperature, the first sealing member and the second sealing member are in clearance fit with the inner wall of the fluid passage, respectively, and the fluid passage is in an open state;
at a second temperature, the first sealing member and the second sealing member increase in volume and attach to the inner wall of the fluid passage, respectively, and the fluid passage is in a closed state.

In a second aspect, embodiments of the present disclosure also disclose a ventilation treatment apparatus including a fire protection device as described above.

In an embodiment of the present disclosure, the fire protection device includes the housing, the first positioning member, and the first sealing member; the fluid passage is provided in the housing, the first opening and the second opening are formed on two opposite sides of the fluid passage, the first opening and the second opening are used for communicating with the pipeline of the oxygen therapy instrument or the patient end, respectively; the first positioning member is disposed in the fluid passage and is connected to the housing; the first sealing member is sleeved on the first positioning member, and the first sealing member has oxidation resistant characteristics. When a fire does not occur, the fire protection device is at a first temperature, the first sealing member is in clearance fit with the inner wall of the fluid passage, and the fluid passage is in an open state. In the event of a fire, the fire protection device is at a second temperature. The first sealing member increases in volume and attaches to the inner wall of the fluid passage, and the fluid passage is in a closed state, so as to cut off the oxygen passage and prevent the oxygen from continuously leaking out and causing the fire to spread, wherein the second temperature is higher than the first temperature. Since the first sealing member has oxidation resistance characteristics, the problem of oxidation of the first sealing member is avoided, and the durability and safety factor of the device are improved.

The present disclosure also provides a fire protection device and a ventilation treatment apparatus, aiming to solve the problem in the related art that a sealing valve is deflected when it moves within the fire protection device, resulting in failure of the sealing valve to be accurately engaged with an opening, and poor stability of the fire protection device.

In a first aspect, embodiments of the present disclosure disclose a fire protection device including a housing and a valve body;
a fluid passage is provided in the housing, the fluid passage is provided with a first narrowed portion and a second narrowed portion, and the valve body is disposed in the fluid passage;
the valve body is slidably connected to the inner wall of the fluid passage, the outer side wall of the valve body is provided with at least one guide portion, and the guide portion is in sliding fit with the inner wall of the fluid passage;
the valve body has a first position and a second position relative to the fluid passage;
when the valve body is in the first position, the valve body is in clearance fit with the first narrowed portion and the second narrowed portion, respectively, and the fluid passage is in an open state; and
when the valve body is in the second position, the valve body is engaged with the first narrowed portion and/or the second narrowed portion, and the fluid passage is in a closed state.

Optionally, the fire protection device further includes a first meltable member and a first elastic member;
the valve body includes a first end and a second end that are opposite to each other, the first end is close to the first narrowed portion and the second end is close to the second narrowed portion;
the housing is provided with a fixing portion close to the first narrowed portion;
the first end of the valve body is provided with a first groove body, and the first elastic member is at least partially located in the first groove body;
one end of the first elastic member abuts against a groove bottom of the first groove body, and the other end of the first elastic member abuts against an end surface of the fixing portion;
the first meltable member is disposed at a first end of the valve body and is engaged with the fixing portion to support the valve body to be in the first position; and
the first meltable member melts, the first elastic member drives the valve body to slide to the second position, the second end of the valve body is engaged with the second narrowed portion, and the fluid passage is in a closed state.

Optionally, a first mounting shaft is provided in the first groove body, and the first mounting shaft is provided coaxially with the first groove body; and
the first elastic member is sleeved on the first mounting shaft and the first meltable member is connected to the first mounting shaft.

Optionally, the second end of the valve body is provided with a first sealing member which at least partially wraps the second end; and
when the valve body is in the second position, the first sealing member is engaged with the second narrowed portion, and the fluid passage is in the closed state.

Optionally, the fire protection device further includes a second meltable member and a second elastic member;
the valve body includes a first end and a second end opposite to each other, the first end is close to the first narrowed portion and the second end is close to the second narrowed portion;
the second end of the valve body is provided with a second groove body, and the second elastic member is at least partially located in the second groove body;
one end of the second elastic member abuts against a groove bottom of the second groove body, and the other end of the second elastic member abuts against the housing;
the second meltable member is disposed at the first narrowed portion and is clamped with the first narrowed portion, and the second meltable member is provided with a through-hole for passing a fluid;
the first end of the valve body abuts against the second meltable member to support the valve body to be in the first position; and
the second meltable member melts, the second elastic member drives the valve body to slide to the second position, and the first end of the valve body is engaged with the first narrowed portion, and the fluid passage is in the closed state.

Optionally, a second mounting shaft is disposed in the second groove body, and the second mounting shaft is provided coaxially with the second groove body; and
the second elastic member is sleeved on the second mounting shaft.

Optionally, the first end of the valve body is provided with a second sealing member which at least partially wraps the first end;
when the valve body is in the second position, the second sealing member is engaged with the first narrowed portion, and the fluid passage is in the closed state.

Optionally, the first narrowed portion is provided with a first positioning structure, the second meltable member is provided with a second positioning structure, and the first positioning structure and the second positioning structure are in positioning fit.

Optionally, the first narrowed portion is provided with a first positioning structure, and the second sealing member is provided with a third positioning structure, the first positioning structure and the third positioning structure are in positioning fit.

Optionally, the outer side wall of the valve body is provided with at least one positioning rib;
the inner wall of the fluid passage is provided with at least one positioning groove, and the positioning rib is in sliding fit with the positioning groove.

In a second aspect, embodiments of the present disclosure also disclose a ventilation treatment apparatus including a fire protection device as described above.

In an embodiment of the present disclosure, the fire protection device includes the housing and the valve body; the fluid passage is provided in the housing, the fluid passage is provided with the first narrowed portion and the second narrowed portion, and the valve body is provided in the fluid passage; the valve body is slidably connected to the inner wall of the fluid passage, the outer side wall of the valve body is provided with at least one guide portion, and the guide portion is in sliding fit with the inner wall of the fluid passage; the valve body has the first position and the second position relative to the fluid passage; when the valve body is in the first position, the valve body is in clearance fit with the first narrowed portion and the second narrowed portion respectively, and the fluid passage is in the open state; when the valve body is in the second position, the valve body is engaged with the first narrowed portion and/or the second narrowed portion, and the fluid passage is in the closed state, thereby cutting off the oxygen passage and avoiding the continuous leakage of oxygen which could cause the spread of fire. By providing the guide portion on the outer side wall of the valve body, the guide portion may act as a limit when the valve body slides relative to the fluid passage, thus avoiding the problem of deflection and jamming of the valve body, ensuring that the valve body may be accurately engaged with the first narrowed portion and/or the second narrowed portion, and improving the stability of the fire protection device.

The present disclosure also provides a fire protection device and a ventilation treatment apparatus, which aim to solve the problem in the related art that the melting speed of a support is slow, which results in that a sealing valve cannot move to an opening in time and cannot rapidly block a gas path, and thus easily causing the spread of fire.

In a first aspect, embodiments of the present disclosure disclose a fire protection device, including a housing, a base, a valve body, an elastic member, and a meltable collar;
the housing is provided with a fluid passage for communicating with a pipeline of an oxygen therapy instrument or a patient end;
the base is positioned within the fluid passage, the valve body is connected to the base, and the elastic member is disposed between the base and the valve body;
a sealing portion is provided at an end of the valve body away from the base, at least one narrowed portion is provided in the fluid passage, and the sealing portion is provided opposite to the narrowed portion;
the meltable collar is nested on a whole including the base, the elastic member, and at least a portion of the valve body in a direction of elastic deformation of the elastic member, to compress the elastic member, such that a gap is provided between the sealing portion and the narrowed portion for passing the fluid; and
after the meltable collar is fused, the elastic member releases at least part of the elastic potential energy and pushes against the valve body, so that the sealing portion snaps with the narrowed portion to block the fluid passage.

Optionally, the valve body further includes a bearing portion connected to the base, and the sealing portion is disposed at an end of the bearing portion away from the base; and
a raised first support structure is disposed on a peripheral side of the bearing portion; the elastic member is sleeved on the bearing portion, one end of the elastic member abuts against the base, and the other end of the elastic member abuts against the first support structure.

Optionally, the bearing portion is provided with a hollowed-out structure along its length direction, and the hollowed-out structure is for the passage of fluid.

Optionally, the valve body further includes a connecting portion disposed between the bearing portion and the sealing portion, and the connecting portion is connected to the bearing portion and the sealing portion, respectively; and
the connecting portion is provided with a clamping structure for clamping and positioning the meltable collar.

Optionally, a peripheral side of the sealing portion extends to form a raised guide portion which is in sliding fit with the inner wall of the fluid passage.

Optionally, the base is provided with a vent hole; and
an edge of the base is provided with at least one first positioning groove which is used for clamping and positioning the meltable collar.

Optionally, the valve body includes a first valve body and a second valve body which are respectively located at both sides of the base and are respectively connected to the base; and
the elastic member includes a first elastic member and a second elastic member, the first elastic member is disposed between the base and the first valve body, and the second elastic member is disposed between the base and the second valve body;
a first narrowed portion and a second narrowed portion are disposed in the fluid passage, a first sealing portion is disposed at an end of the first valve body away from the base, the first sealing portion is provided opposite to the first narrowed portion, a second sealing portion is provided at an end of the second valve body away from the base, and the second sealing portion is provided opposite to the second narrowed portion;
the meltable collar is nested on a whole including the base, the first elastic member, the second elastic member, the first valve body, and the second valve body, along a direction of elastic deformation of the first elastic member and the second elastic member, to compress the first elastic member and the second elastic member, such that gaps are provided between the first sealing portion and the first narrowed portion and between the second sealing portion and the second narrowed portion for passing the fluid; and
after the meltable collar is fused, the first elastic member releases at least part of the elastic potential energy and pushes against the first valve body, to snap-fit the first sealing portion with the first narrowed portion, and the second elastic member releases at least part of the elastic potential energy and pushes against the second valve body, to snap-fit the second sealing portion with the second narrowed portion to block the fluid passage.

Optionally, the first valve body includes a first mounting sleeve, the second valve body includes a second mounting sleeve, the first sealing portion is provided at an end of the first mounting sleeve away from the base, and the second sealing portion is provided at an end of the second mounting sleeve away from the base;
the base is provided with a raised first positioning portion facing towards the first valve body, and the base is provided with a raised second positioning portion facing towards the second valve body;
the first positioning portion is at least partially embedded in the first mounting sleeve, and the second positioning portion is at least partially embedded in the second mounting sleeve;
a raised second support structure is disposed on a circumferential side of the first mounting sleeve, the first elastic member is sleeved on an outer side wall of the first mounting sleeve, one end of the first elastic member abuts against the base, and the other end of the first elastic member abuts against the second support structure; and
a raised third support structure is disposed on a circumferential side of the second mounting sleeve, the second elastic member is sleeved on an outer side wall of the second mounting sleeve, one end of the second elastic member abuts against the base, and the other end of the second elastic member abuts against the third support structure.

Optionally, the peripheral side of the base is provided with a raised flange, and two opposite end surfaces of the flange extend towards the first valve body and the second valve body, respectively, to form a first limiting portion and a second limiting portion;
the second support structure extends towards the base to form a third limiting portion, and the third support structure extends towards the base to form a fourth limiting portion;
a first limiting space is formed among the first limiting portion, the third limiting portion and the outer side wall of the first mounting sleeve, and the first elastic member is embedded in the first limiting space; and
a second limiting space is formed among the second limiting portion, the fourth limiting portion and the outer side wall of the second mounting sleeve, and the second elastic member is embedded in the second limiting space.

Optionally, a second positioning groove is disposed on the first sealing portion and a third positioning groove is disposed on the second sealing portion, and the second positioning groove and the third positioning groove are used for clamping and positioning the meltable collar.

In a second aspect, embodiments of the present disclosure also disclose a ventilation treatment apparatus including the fire protection device as described above.

In the embodiments of the present disclosure, the base, the valve body, the elastic member and the meltable collar together constitute a valve body trigger system. When the meltable collar is not fused, under the constraint of the meltable collar, the elastic member is in a compressed state, a gap is provided between the sealing portion of the valve body and the narrowed portion of the fluid passage for the fluid to pass through, and oxygen can pass through normally. After the meltable collar is fused, the elastic member releases at least part of the elastic potential energy and pushes against the valve body, causing the sealing portion to snap-fit with the narrowed portion to block the fluid passage. Since the meltable collar itself is easily fused when heated, the fusing speed is further accelerated under the elastic force of the elastic member, thereby increasing the triggering speed of the valve body, reducing the risk of fire spreading due to the failure of triggering in time, thereby improving the safety factor of the fire protection device.

The present disclosure also provides a fire protection device and a ventilation treatment apparatus, which aim to solve the problems in the related art that the fusing speed of a support is slow, resulting in that a sealing valve cannot move to an opening in time and cannot rapidly block a gas path, and thus easily causing the spread of fire.

In a first aspect, embodiments of the present disclosure disclose a fire protection device, including a housing, an inner shell, a valve body, and an elastic member;
the housing has a fluid passage for communicating with a pipeline of an oxygen therapy instrument or a patient end;
the inner shell is provided in the fluid passage and is sealed with an inner wall of the fluid passage, and the inner shell is provided with a through-hole for the fluid to pass through;
the valve body is slidably connected to an inner wall of the fluid passage and has a first position and a second position relative to the fluid passage;
in the first position, a gap is provided between the valve body and the through-hole for the passage of fluid;
in the second position, the valve body abuts against a circumferential side of the through-hole, so that the fluid passage is in a blocked state;
the elastic member is provided between the valve body and an inner wall of the fluid passage;
the inner walls at both ends of the fluid passage are provided with at least one raised support portion, respectively, one end of the valve body is disposed opposite to the through-hole, and the other end of the valve body is clamped with the support portion, to support the valve body to be in the first position, and the elastic member is in a compressed state; and
after at least one of the support portion and the valve body are melted, the elastic member releases at least part of the elastic potential energy to drive the valve body to switch from the first position to the second position.

Optionally, the support portion is disposed on an inner wall of the fluid passage close to the opening.

Optionally, the valve body includes a clamping member and a sealing member, the clamping member and the sealing member are of a split type structure, and the clamping member and the sealing member are slidably connected with the inner wall of the fluid passage, respectively;
the elastic member is disposed between the clamping member and the inner wall of the fluid passage;
one end of the clamping member is used for being clamped with the support portion, and the other end of the clamping member is used for pushing against the sealing member, so that the sealing member abuts against the peripheral side of the through-hole; and
an assembly gap is provided between the clamping member and the sealing member for the passage of fluid.

Optionally, one end of the clamping member close to the sealing member is provided with a raised first guide portion, and the first guide portion is in sliding fit with the inner wall of the fluid passage;
the elastic member is sleeved on the clamping member, one end of the elastic member abuts against the inner wall of the fluid passage, and the other end of the elastic member abuts against the first guide portion; and
the elastic member is compressed by the inner wall of the fluid passage and the first guide portion when the clamping member is clamped with the support portion.

Optionally, the fluid passage includes a main body and an opening portion provided at both ends of the main body, wherein an inner diameter of the opening portion is less than an inner diameter of the main body;
a junction of the main body and the opening portion is narrowed to form a shoulder;
the support portion is disposed on an inner wall of the opening portion; and
one end of the elastic member abuts against the shoulder, and the other end of the elastic member abuts against the first guide portion.

Optionally, the clamping member is provided with a raised second guide portion which is in sliding fit with the inner wall of the opening portion.

Optionally, a side of the sealing member facing towards the clamping member has an end surface, a peripheral side of the end surface extends towards the clamping member to form a third guide portion, and the third guide portion is in sliding fit with an inner wall of the fluid passage.

Optionally, the third guide portion and the end surface enclose and form a limiting groove; and
the clamping member extends towards one end of the sealing member to form a limiting portion, and the limiting portion is at least partially embedded in the limiting groove.

Optionally, the outer side wall of the sealing member extends to form a raised fourth guide portion which is in sliding fit with the inner wall of the fluid passage.

Optionally, an end of the clamping member facing towards the sealing member is provided with a groove, and an end of the sealing member facing towards the clamping member is provided with a boss at least partially embedded in the groove.

Optionally, a sealing structure is provided in the assembly gap of the housing and the inner shell.

Optionally, a quantity of the support portions is two or more, and the support portions are provided at intervals along a peripheral direction of the inner wall of the opening portion.

Optionally, one end of the valve body close to the support portion is provided with a fusible portion;
the valve body is clamped with the support portion to support the valve body to be in the first position when the support portion and the fusible portion are in a non-molten state; and
the elastic member releases at least part of the elastic potential energy to drive the valve body to be in the second position when the support and/or the fusible portion are in a molten state.

Optionally, the valve body includes a first valve body and a second valve body;
the first valve body and the second valve body are symmetrically arranged at both sides of the through-hole; and
the fluid passage is in a blocked state when at least one of the first valve body and the second valve body abuts against the peripheral side of the through-hole.

Optionally, an elastic support is disposed between the first valve body and the second valve body, and the elastic support passes through the through-hole; and
both ends of the elastic support abut against the first valve body and the second valve body, respectively;
wherein the elastic force of the elastic support is less than the elastic force of the elastic member.

Optionally, one end of the first valve body facing towards the second valve body is provided with a first mounting groove, and one end of the second valve body facing towards the first valve body is provided with a second mounting groove, and the first mounting groove is arranged opposite to the second mounting groove; and
the elastic support is at least partially embedded in the first mounting groove and the second mounting groove.

In a second aspect, embodiments of the present disclosure disclose a fire protection device, including a housing and a valve body;
the housing has a fluid passage for communicating with a pipeline of an oxygen therapy instrument or a patient end;
the valve body is disposed within the fluid passage, and the valve body has a first position and a second position relative to the fluid passage;
in the first position, a gap is provided between the valve body and the fluid passage for passing the gas;
in the second position, the valve body blocks the fluid passage;
the inner walls at both ends of the fluid passage are provided with at least one raised meltable support portion, respectively;
the valve body is clamped with the meltable support portion to support the valve body to be in the first position when the meltable support portion is in a non-molten state; and
the valve body is switched from the first position to the second position when the meltable support portion is in a molten state.

Optionally, the fire protection device further includes an elastic member;
the elastic member is provided between the valve body and an inner wall of the fluid passage;
the elastic member is in a compressed state when the valve body is in the first position; and
the elastic member is used for providing an elastic driving force when the valve body is switched from the first position to the second position.

Optionally, the valve body includes a clamping member and a sealing member, the clamping member and the sealing member are of a split type structure, and the clamping member and the sealing member are slidably connected with the inner wall of the fluid passage, respectively;
the elastic member is disposed between the clamping member and the inner wall of the fluid passage;
one end of the clamping member is used for being clamped with the meltable support portion, and the other end of the clamping member is used for pushing against the sealing member;
an assembly gap is provided between the clamping member and the sealing member for the passage of a fluid.

Optionally, one end of the clamping member close to the sealing member is provided with a raised first guide portion, and the first guide portion is in sliding fit with the inner wall of the fluid passage;
the elastic member is sleeved on the clamping member, one end of the elastic member abuts against the inner wall of the fluid passage, and the other end of the elastic member abuts against the first guide portion; and
the elastic member is compressed by the inner wall of the fluid passage and the first guide portion when the clamping member is clamped with the meltable support portion.

Optionally, the fluid passage includes a main body and an opening portion provided at both ends of the main body, wherein an inner diameter of the opening portion is less than an inner diameter of the main body;
a junction of the main body and the opening portion is narrowed to form a shoulder;
the meltable support portion is disposed on an inner wall of the opening portion;
one end of the elastic member abuts against the shoulder, and the other end of the elastic member abuts against the first guide portion.

Optionally, the clamping member is provided with a raised second guide portion which is in sliding fit with the inner wall of the opening portion.

Optionally, a side of the sealing member facing towards the clamping member has an end surface, and a peripheral side of the end surface extends towards the clamping member to form a third guide portion, and the third guide portion is in sliding fit with an inner wall of the fluid passage.

Optionally, the third guide portion and the end surface enclose and form a limiting groove; and
the clamping member extends towards one end of the sealing member to form a limiting portion, and the limiting portion is at least partially embedded in the limiting groove.

Optionally, the outer side wall of the sealing member extends to form a raised fourth guide portion which is in sliding fit with the inner wall of the fluid passage.

Optionally, an end of the clamping member facing towards the sealing member is provided with a groove, and an end of the sealing member facing towards the clamping member is provided with a boss at least partially embedded in the groove.

In a third aspect, embodiments of the present disclosure disclose a fire protection device, including a housing and a valve body;
the housing has a fluid passage for communicating with a pipeline of an oxygen therapy instrument or a patient end;
the valve body is disposed within the fluid passage, and the valve body has a first position and a second position relative to the fluid passage;
in the first position, a gap is provided between the valve body and the fluid passage for passing the gas;
in the second position, the valve body blocks the fluid passage;
the inner walls of both ends of the fluid passage are provided with at least one raised support portion, respectively;
one end of the valve body close to the support portion is provided with a fusible portion;
the valve body is clamped with the support portion to support the valve body to be in the first position when the fusible portion is in a non-molten state; and
the valve body is switched from the first position to the second position when the fusible portion is in a molten state.

Optionally, an outer diameter of the fusible portion is less than an outer diameter of the valve body in other locations.

Optionally, the fire protection device further includes an elastic member;
the elastic member is provided between the valve body and an inner wall of the fluid passage;
the elastic member is in a compressed state when the valve body is in the first position;
the elastic member is used for providing an elastic driving force when the valve body is switched from the first position to the second position.

Optionally, the valve body includes a clamping member and a sealing member, the clamping member and the sealing member are of a split type structure, and the clamping member and the sealing member are slidably connected with the inner wall of the fluid passage, respectively;
the fusible portion is provided at one end of the clamping member close to the support portion, and the elastic member is disposed between the clamping member and the inner wall of the fluid passage;
one end of the clamping member is used for being clamped with the support portion, and the other end of the clamping member is used for pushing against the sealing member; and
an assembly gap is provided between the clamping member and the sealing member for the passage of a fluid.

Optionally, one end of the clamping member close to the sealing member is provided with a raised first guide portion, and the first guide portion is in sliding fit with the inner wall of the fluid passage;
the elastic member is sleeved on the clamping member, one end of the elastic member abuts against the inner wall of the fluid passage, and the other end of the elastic member abuts against the first guide portion; and
the elastic member is compressed by the inner wall of the fluid passage and the first guide portion when the clamping member is clamped with the support portion.

Optionally, the fluid passage includes a main body and an opening portion provided at both ends of the main body, wherein an inner diameter of the opening portion is less than an inner diameter of the main body;
a junction of the main body and the opening portion is narrowed to form a shoulder;
the support portion is disposed on an inner wall of the opening portion;
one end of the elastic member abuts against the shoulder, and the other end of the elastic member abuts against the first guide portion.

Optionally, the clamping member is provided with a raised second guide portion which is in sliding fit with the inner wall of the opening portion.

Optionally, a side of the sealing member facing towards the clamping member has an end surface, and a peripheral side of the end surface extends towards the clamping member to form a third guide portion, and the third guide portion is in sliding fit with an inner wall of the fluid passage.

Optionally, the third guide portion and the end surface enclose and form a limiting groove; and
the clamping member extends towards one end of the sealing member to form a limiting portion, and the limiting portion is at least partially embedded in the limiting groove.

Optionally, the outer side wall of the sealing member extends to form a raised fourth guide portion which is in sliding fit with the inner wall of the fluid passage.

Optionally, an end of the clamping member facing towards the sealing member is provided with a groove, and an end of the sealing member facing towards the clamping member is provided with a boss at least partially embedded in the groove.

In a fourth aspect, embodiments of the present disclosure also disclose a ventilation treatment apparatus including a fire protection device as described above.

In the embodiments of the present disclosure, the inner wall of the fluid passage is provided with a support portion, the valve body is clamped with the meltable support portion so that the valve body is in the first position, the gap is provided between the valve body and the through-hole of the inner shell for the fluid to pass through, and the elastic member is in the compressed state; after melting of at least one of the support portion and the valve body, the elastic member releases at least part of the elastic potential energy, to drive the valve body to abut against the peripheral side of the through-hole, so that the fluid passage is in the blocked state. A triggering system including the support portion, the elastic member and the valve body, when any one of the valve body and the support portion melts, can move the valve body to the second position in time and abut against the peripheral side of the through-hole, thereby blocking the gas path, increasing the triggering speed of the valve body, reducing the risk of fire spreading due to the triggering not being performed in time, and thus improving the safety factor of the fire protection device.

The present disclosure also provides a fire protection device and a ventilation treatment apparatus, which aim to solve the problems in the related art that the melting speed of a support is slow, resulting in that a sealing valve cannot move to an opening in time and cannot rapidly block a gas path, and thus easily causing the spread of fire.

In a first aspect, embodiments of the present disclosure disclose a fire protection device including a housing, a sealing member, a valve body, and an elastic member;
a fluid passage is provided in the housing, and the fluid passage is used for communicating with a pipeline of an oxygen therapy instrument or a patient end;
the sealing member is connected to an inner wall of the fluid passage, and the sealing member is provided with a notch for passage of a fluid;
the valve body is slidably connected to the inner wall of the fluid passage, and the elastic member is provided between the valve body and the inner wall of the fluid passage;
the valve body is provided with a fusible portion, the inner wall of the fluid passage is provided with at least one raised meltable support portion, one end of the valve body is arranged opposite to the notch, and the other end of the valve body is used for being clamped with the meltable support portion;
the valve body has a first position and a second position relative to the fluid passage;
when the meltable support portion and the fusible portion are in a non-molten state, the valve body is clamped with the meltable support portion, to support the valve body to be in the first position, a gap is provided between the valve body and the sealing member for passage of a fluid, and the elastic member is in a compressed state; and
when the meltable support portion and/or the fusible portion are in a molten state, the elastic member releases at least part of the elastic potential energy to drive the valve body to be in the second position, and the valve body is snap-fit with the notch of the sealing member, so that the fluid passage is in a blocked state.

Optionally, the valve body is an integrated structure.

Optionally, the notch has a first side and a second side that are opposite to each other; and
at least one side of the notch is provided with the valve body, and when the at least one side of the notch is snap-fit with the valve body, the fluid passage is in the blocked state.

Optionally, the valve body includes a clamping portion, a connecting portion, and a sealing portion, and the clamping portion and the sealing portion are connected by the connecting portion;
the clamping portion is configured for being clamped with the meltable support portion, to support the valve body to be in the first position;
the sealing portion is disposed opposite to the notch, and the sealing portion is used for being snap-fit with the notch when the valve body is in the second position; and
the fusible portion is disposed at the connecting portion.

Optionally, the elastic member is sleeved on the connecting portion, one end of the elastic member abuts against the inner wall of the fluid passage, and the other end of the elastic member abuts against the sealing portion;
the elastic member is compressed by the inner wall of the fluid passage and the sealing portion when the clamping portion is clamped with the meltable support portion; and
when the meltable support and/or the fusible portion are in a molten state, the elastic member releases at least part of the elastic potential energy to drive the sealing portion to be snap-fitted with the notch, so that the fluid passage is in a blocked state.

Optionally, the fluid passage includes a main body and an opening portion provided at both ends of the main body, wherein an inner diameter of the opening portion is less than an inner diameter of the main body;
a junction of the main body and the opening portion is narrowed to form a shoulder;
the meltable support portion is disposed on an inner wall of the opening portion, and the sealing member is connected to the inner wall of the main body; and
one end of the elastic member abuts against the shoulder, and the other end of the elastic member abuts against the sealing portion.

Optionally, the sealing portion is provided with a raised first guide portion which is in sliding fit with an inner wall of the main body; and
one end of the elastic member abuts against the shoulder, and the other end of the elastic member abuts against the first guide portion.

Optionally, the connecting portion is provided with a raised second guide portion which is in sliding fit with the inner wall of the opening portion.

Optionally, a quantity of the meltable support portions is two or more, and the meltable supports are provided at intervals along a peripheral direction of the inner wall of the opening portion.

Optionally, the housing includes a first housing and a second housing;
the first housing and/or the second housing is provided with a mounting engagement groove;
when the first housing and the second housing are assembled, the sealing member is in interference fit with the mounting engagement groove.

Optionally, the mounting engagement groove is provided with an expanded portion; and
an edge of the sealing member extends to form an embedded portion which is at least partially embedded into the expanded portion.

Optionally, the fire protection device includes two valve bodies arranged symmetrically in the fluid passage;
the two valve bodies are located on both sides of the sealing member, respectively;
the elastic member is provided between each of the valve bodies and the inner wall of the fluid passage; and
the meltable support portions are provided in the opening portions at both ends of the main body, respectively.

In a second aspect, embodiments of the present disclosure also disclose a ventilation treatment apparatus including a fire protection device as described above.

In the embodiments of the present disclosure, the valve body is provided with the fusible portion, the inner wall of the fluid passage is provided with the meltable support portion. When the meltable support portion and the fusible portion are in the non-molten state, the valve body is clamped with the meltable support portion, so as to support the valve body in a first position, the gap is provided between the valve body and the sealing member for passage of a fluid, and the elastic member is in a compressed state. When the meltable support and/or the fusible portion are in the molten state, the elastic member releases at least part of the elastic potential energy to drive the valve body to be in the second position, and the valve body is snap-fit with the notch of the sealing member, so that the fluid passage in the blocked state. By using the combination of the fusible portion and the meltable support portion, when any one of the fusible portion and the meltable support portion is melted, the valve body can be moved to the second position in time and snap-fit with the notch of the sealing member, thereby blocking the gas path, increasing the triggering speed of the valve body, reducing the risk of fire spreading due to the failure of triggering in time, and thus improving the safety factor of the fire protection device.

Embodiments of the present disclosure also provide a fire damper and a ventilation apparatus for blocking the delivery of oxygen in the event of a fire at a user side.

In a first aspect, the present disclosure provides a fire damper for being applied in a ventilation apparatus, including a housing and a sealing member; a penetrating airflow passage is provided in the housing, the airflow passage is provided with a first partition separating the airflow passage, and the first partition is provided with a first vent hole; the sealing member is located in the airflow passage, and a first gap is provided between the sealing member and the first vent hole before the sealing member is heated, so that gas flows from one end of the airflow passage to the other end through the first vent hole and the first gap; and the sealing member includes a heat-shrinkable material layer, such that the sealing member is shrunk after being heated and blocks the first vent hole.

Optionally, the first vent hole faces towards the sealing member, and a projection of the sealing member on the first vent hole completely covers the first vent hole, such that the sealing member is attached to the first partition and covers the first vent hole after being shrunk by heat.

Optionally, the first partition includes a first vent pipe, the first vent hole is disposed on a sidewall of the first vent pipe, and the sealing member has a tubular shape and is sleeved outside the first vent pipe.

Optionally, the first vent pipe divides the airflow passage into a first passage and a second passage, the sealing member is located within the second passage, and a second gap is provided between an outer wall of the sealing member and an inner wall of the second passage.

Optionally, the sealing member has a tubular shape, one end of the sealing member is connected to a peripheral edge of the first vent hole, and the other end of the sealing member is in an open state before being heated, and is in a closed state after being shrunk by heat.

Optionally, the sealing member further includes a hot melt layer inside the heat-shrinkable material layer.

Optionally, a wall thickness of an end of the hot melt layer away from the first vent hole is greater than a wall thickness of an end of the hot melt layer close to the first vent hole.

Optionally, the hot melt layer is provided with a plurality of protrusions facing towards an interior of the sealing member.

Optionally, a second partition is further provided in the airflow passage, a second vent hole is disposed on the second partition, the first partition and the second partition are arranged at intervals along an axial direction of the airflow passage, and the sealing member blocks the second vent hole after being shrunk by heat.

In a second aspect, the present disclosure provides a ventilation apparatus including the fire damper as described.

The present disclosure provides a fire damper. The first partition is disposed in the housing, and the first partition divides the airflow passage in the housing into two portions communicated through the first vent hole. The first gap is provided between the sealing member and the first vent hole before the sealing member is heated, so as to allow normal communication between the two portions of the airflow passage. A heat-shrinkable material layer is included in the sealing member, so that the sealing member, after being heated, may block the first vent hole and prevent communication between the two portions of the airflow passage, thereby cutting off the oxygen transmission path. The present disclosure provides the fire damper that includes the housing and the sealing member, has a small quantity of parts, and has a simple structure, as compared with fire dampers in the related art.

The present disclosure provides an automatic fire protection device applied to an oxygen therapy apparatus, which is capable of preventing occurrence of a failure phenomenon contacting with oxygen for a long time, thereby improving the service life of the automatic fire protection device.

The present disclosure provides an automatic fire protection device applied to an oxygen therapy apparatus, including:
a housing having a gas passage; and a sealing member provided inside the housing, wherein the sealing member is made of an elastic material, and the sealing member includes a sealing portion;
wherein when the sealing member is in a first state, a gap is provided between the sealing portion and the gas outlet end of the gas passage, so that the gas passage is opened; when the sealing member is in a second state, the sealing portion is attached to the gas outlet end of the gas passage, so that the gas passage is closed.

In an embodiment, the sealing member further includes a first connector and a second connector respectively disposed on the sealing portion, wherein the first connector and the second connector are respectively fixed on both sides of the gas outlet end of the gas passage. When the sealing member is in the first state, the first connector and the second connector are both fixed in the housing and are both in a stretched state, so that the sealing portion is away from the gas passage to form the gap with the gas outlet end of the gas passage. When the sealing member is in a second state, the first connector is fixed in the housing and is in the stretched state, and the second connector is disconnected and is in a natural state, so that the sealing portion is close to the gas passage and is attached to the gas outlet end of the gas passage.

In an embodiment, the second connector is fixed in the housing by a fixing structure, the fixing structure is configured such that it is capable of being fused after being heated above a predetermined temperature, and the second connector is converted from the stretched state to the natural state.

In an embodiment, a mounting arm for mounting the sealing member is further disposed in the housing, and the first connector and the second connector are fixedly connected to both ends of the mounting arm, respectively;
wherein the second connector is connected to the mounting arm via the fixing structure to be fixed in the housing, the fixing structure is capable of being fused after being heated above the preset temperature, so that the second connector is disconnected from the mounting arm, and the second connector is converted from the stretched state to the natural state.

In an embodiment, the first connector is disposed on a side wall of the sealing portion, and the second connector is disposed on an end side of the sealing portion, wherein when the first connector and the second connector are both in the natural state, an extending direction of the first connector is perpendicular to an extending direction of the second connector; and
stretching extension directions of the first connector and the second connector are opposite when the sealing member is in the first state.

In an embodiment, the first connector and the second connector are disposed on corresponding sides of the sealing portion, and the first connector is located on a side of the sealing portion away from the second connector; wherein when both the first connector and the second connector are in a natural state, the first connector and the second connector extend in opposite directions, respectively.

In an embodiment, the mounting arm includes:
an adapter block, wherein an accommodating hole is disposed in the adapter block, and the gas passage is penetratingly disposed in the accommodating hole; a mounting post disposed on a side wall of the adapter block, wherein the mounting post is used for being connected with the first connector; and
a connecting plate extending in an axial direction of the accommodating hole, wherein a trigger post is disposed at a side of the connecting plate away from the adapter block, and the trigger post is used for being connected with the second connector;
wherein the sealing member is in a first state in the housing when the first connector is connected to the mounting post and the second connector is connected to the trigger post; the sealing member is in a second state in the housing when the first connector is connected to the mounting post and the second connector is disconnected from the trigger post.

In an embodiment, the fixing structure includes a connecting ring at an end of the second connector and the trigger post, and the connecting ring is capable of being matched with the trigger post;
wherein the connecting ring and/or the trigger post is capable of being fused after being heated above the preset temperature.

In an embodiment, the mounting post includes:
a connecting post extending in a radial direction of the accommodating hole on a side wall of the adapter block; and
a stop circular truncated cone disposed on the connecting post; and a fixing post disposed at an end of the stop circular truncated cone for being connected with an inner wall of the housing to fix the mounting arm in the housing.

In an embodiment, the first connector is provided with a first connecting hole penetrating a thickness direction thereof, and an inner diameter of the first connecting hole is less than a maximum outer diameter of the stop circular truncated cone.

In an embodiment, the mounting posts are symmetrically disposed on the side wall of the adapter block, and a quantity and distribution of the first connectors are the same as a quantity and distribution of the mounting posts, respectively, and quantities of both the first connectors and the mounting posts are at least two.

In an embodiment, the connecting plate includes:
a recessed portion, wherein one end of the recessed portion is connected to a side wall of the adapter block, and the recessed portion is used for receiving the sealing portion; and
an extension plate connected to the other end of the recessed portion, wherein the extension plate extends in the axial direction of the accommodating hole, and the trigger post is disposed at a side of the extension plate away from the recessed portion.

In an embodiment, the housing includes a first housing and a second housing, wherein the first housing and the second housing are connected to form a sealed chamber therein, and the gas passage extends from an inner wall side of the chamber;
a first nozzle is disposed at a side of the first housing, and a second nozzle is disposed at a side of the second housing opposite to the first housing;
wherein the first nozzle, the gas passage, the chamber, and the second nozzle are in fluid communication when the sealing member is in the first state; when the sealing member is in the second state, fluid entering the gas passage through the first nozzle is isolated from the chamber outside the gas passage.

In an embodiment, an inner wall of the chamber is provided with a connecting groove for being connected with the fixing post.

In an embodiment, the trigger post is disposed in the chamber or in the second nozzle. In an embodiment, the outer walls of the first nozzle and the second nozzle are each provided with an anti-detachment portion.

In an embodiment, the first housing and the second housing are connected by means of a sealing snap, welding or a threaded connection.

The advantages of the present disclosure compared to the related art are as follows.
(1) Since the sealing member is made of an elastic non-metallic material, even if it is in contact with oxygen for a long time, a chemical reaction such as an oxidation reaction does not occur, so that the service life of the automatic fire protection device applied to the oxygen therapy apparatus can be improved and it can avoid affecting the health of the user.
(2) The small quantity of parts in the interior of the housing and its simple structure make the performance of the automatic fire protection device applied to the oxygen therapy apparatus more stable.

It is an object of the present disclosure to provide a fire protection device and a ventilation treatment apparatus, which can solve the problem that if a flame appears during the use of an oxygen therapy instrument in the related art, the flame will gradually burn to a fuselage along with an oxygen pipe, easily causing the spread of fire, and finally igniting the oxygen generator to generate a serious fire.

In order to solve the above technical problem, the present disclosure is achieved as follows.

In a first aspect, the present disclosure provides a fire protection device including a housing, a first valve body, a second valve body and an elastic member, wherein a fluid passage is provided in the housing; one end of the housing has a first opening, and a second end of the housing has a second opening; the first opening and the second opening are both in communication with the fluid passage, and the first opening and the second opening are used for communicating with a pipeline of an oxygen therapy instrument or a pipeline of an oxygen supply end;
the first valve body and the second valve body are both located in the fluid passage and spaced apart in the direction from the first opening to the second opening, a first limiting rib is fixed on a hole wall of the first opening, a second limiting rib is fixed on a hole wall of the second opening; a first end of the first valve body abuts against the first limiting rib, a first end of the second valve body abuts against the second limiting rib; the elastic member is located between the first valve body and the second valve body, two ends of the elastic member abut against the second end of the first valve body and the second end of the second valve body, respectively, the elastic member is in a compressed state; and gaps for the circulation of a gas is provided between a peripheral portion of the first valve body and a passage wall of the fluid passage and between a peripheral portion of the second valve body and the passage wall of the fluid passage;
when the first limiting rib is in a molten state, the elastic member is elongated, the first valve body is in a first position, and the first valve body is in sealed connection with the passage wall of the fluid passage, to block the fluid passage; when the second limiting rib is in the molten state, the elastic member is elongated, the second valve body is in a second position, and the second valve body is in sealed connection with the passage wall of the fluid passage to block the fluid passage.

Optionally, the first valve body includes a first mounting rod and a first mounting seat; an axial direction of the first mounting rod is consistent with an extension and retraction direction of the elastic member; one end of the first mounting rod is connected to the first mounting seat, the other end of the first mounting rod abuts against the first limiting rib, and one end of the elastic member abuts against the first mounting seat;
the second valve body includes a second mounting rod and a second mounting seat, an axial direction of the second mounting rod is consistent with the extension and retraction direction of the elastic member, one end of the second mounting rod is connected to the second mounting seat, the other end of the second mounting rod abuts against the second limiting rib, and the other end of the elastic member abuts against the second mounting seat;
when the first limiting rib is in the molten state, the first mounting seat is in sealed connection with the passage wall of the fluid passage; and
when the second limiting rib is in the molten state, the second mounting seat is in sealed connection with the passage wall of the fluid passage.

Optionally, one end of the first mounting seat away from the first mounting rod is provided with a first mounting cavity, one end of the second mounting seat away from the second mounting rod is provided with a second mounting cavity, the first mounting cavity is opposite to the second mounting cavity; one end of the elastic member abuts against a cavity bottom of the first mounting cavity, and the other end of the elastic member abuts against a cavity bottom of the second mounting cavity.

Optionally, a first guide rod is connected to the cavity bottom of the first mounting cavity, and/or a second guide rod is connected to the cavity bottom of the second mounting cavity, the elastic member is sleeved on the first guide rod and/or the second guide rod, and the first guide rod and the second guide rod are used for guiding the extension and retraction of the elastic member.

Optionally, a first positioning portion is disposed on a peripheral portion of the first mounting seat; a first positioning groove is disposed on the inner wall of the fluid passage; an extension direction of the first positioning groove is consistent with the extension and retraction direction of the elastic member; and the first positioning portion is embedded in the first positioning groove which is used for limiting a movement direction of the first mounting seat when the first limiting rib is in the molten state; and/or
a second positioning portion is disposed on a peripheral portion of the second mounting seat; a second positioning groove is disposed on an inner wall of the fluid passage; an extension direction of the second positioning groove is consistent with the extension and retraction direction of the elastic member; and the second positioning portion is embedded in the second positioning groove which is used for limiting a movement direction of the second mounting seat when the second limiting rib is in the molten state.

Optionally, a first mounting groove is disposed on the inner wall of the fluid passage and extends along a circumferential direction of the fluid passage and surrounds the fluid passage; a first sealing member is provided in the first mounting groove, and a portion of the first sealing member extends outside the first mounting groove; the first sealing member is opposite to the first valve body, and the first sealing member is used to abut against the first valve body when the first limiting rib is in the molten state, so that the first valve body is in sealing connection with the passage wall of the fluid passage; and/or

a second mounting groove is disposed on the inner wall of the fluid passage and extends along the circumferential direction of the fluid passage and surrounds the fluid passage; a second sealing member is provided in the second mounting groove, and a portion of the second sealing member extends outside the second mounting groove; the second sealing member is opposite to the second valve body, and the second sealing member is used to abut against the second valve body when the second limiting rib is in the molten state, so that the second valve body is in sealing connection with the passage wall of the fluid passage.

Optionally, the passage wall of the fluid passage has a first blocking platform and a second blocking platform along the circumferential direction of the fluid passage, and the first blocking platform and the second blocking platform are spaced apart along the direction from the first opening to the second opening; the first valve body and the second valve body are located between the first blocking platform and the second blocking platform; and in the direction from the first opening to the second opening, a projection of the first blocking platform has an overlapping portion with a projection of the first valve body, and a projection of the second blocking platform has an overlapping portion with a projection of the second valve body;
the first blocking platform has a first surface facing towards the first valve body in a direction from the first opening to the second opening, the first mounting groove is disposed on the first surface and extends in the circumferential direction of the fluid passage, and a projection of the first mounting groove is located within the projection of the first valve body in the direction from the first opening to the second opening; and/or
the second blocking platform has a second surface facing towards the second valve body in the direction from the first opening to the second opening, the second mounting groove is disposed on the second surface and extends in the circumferential direction of the fluid passage, and a projection of the second mounting groove is located within the projection of the second valve body in the direction from the first opening to the second opening.

Optionally, the housing includes a first sub-housing and a second sub-housing, the first sub-housing is disposed opposite to the second sub-housing, a first passage is disposed inside the first sub-housing, and a second passage is disposed inside the second sub-housing, the first sub-housing is connected to the second sub-housing, the first passage communicates with the second passage to form the fluid passage, the first valve body is disposed in the first sub-housing, and the second valve body is disposed in the second sub-housing.

Optionally, a first joint is connected to the first opening, and the first joint is in communication with the fluid passage;
a second joint is connected to the second opening, and the second joint is in communication with the fluid passage; and
the first joint and the second joint are used for communicating with the pipeline of the oxygen therapy instrument or the oxygen supply end.

In a second aspect, the present disclosure provides a ventilation treatment apparatus, specifically including an oxygen therapy instrument, an oxygen supply end and the fire protection device as described in any of the first aspects above; the first opening is connected to the oxygen therapy instrument, and the second opening is connected to the oxygen supply end; or, the second opening is connected to the oxygen therapy instrument, and the first opening is connected to the oxygen supply end.

In the present disclosure, the fire protection device includes the housing, the first valve body, the second valve body, and the elastic member, wherein the housing has the fluid passage therein; one end of the housing has the first opening, and the second end of the housing has the second opening. The first opening and the second opening are in communication with the fluid passage; therefore, the first opening, the fluid passage, and the second opening can form a passage for the circulation of the gas. Herein, the first opening and the second opening are used for communicating with the pipeline of the oxygen therapy instrument or the pipeline of the oxygen supply end, so that one end of the fluid passage can be connected to the pipeline of the oxygen therapy instrument through the first opening and the second opening, and the other end of the fluid passage can be connected to the pipeline of the oxygen supply end; and the oxygen therapy instrument can deliver oxygen to the patient through the oxygen supply end. The first limiting rib is fixed on the hole wall of the first opening; the second limiting rib is fixed on the hole wall of the second opening; the first end of the first valve body abuts against the first limiting rib; the second end of the second valve body abuts against the second limiting rib; the elastic member is connected between the second end of the first valve body and the second end of the second valve body, so that the first valve body and the second valve body can be distributed at intervals along the direction from the first opening to the second opening. Since the elastic member is in a compressed state, and the extension and retraction direction of the elastic member is consistent with the direction of extension of the fluid passage, the elastic member can exert a force on the first valve body and the second valve body, so that the first valve body has a tendency to move in a direction towards the first opening, and the second valve body has a tendency to move towards the second opening. At this time, the first limiting rib can block the first valve body, and the second limiting rib can block the second valve body. Since the gaps for the circulation of the gas is provided between the peripheral portion of the first valve body and the passage wall of the fluid passage and between the peripheral portion of the second valve body and the passage wall of the fluid passage, in normal use, the gas can flow through the gaps, so that the oxygen therapy instrument can be normally used. In the case where the first limiting rib is in the molten state, the first limiting rib loses a blocking effect on the first valve body; at this time, the elastic member is elongated, namely, the elastic member may release elastic potential energy, and the first valve body moves to a first position in the direction towards the first opening under the elastic force of the elastic member, so that the first valve body is in sealing connection with the passage wall of the fluid passage, thereby blocking the fluid passage and preventing oxygen from continuing to flow in the fluid passage. When the second limiting rib is in the molten state, the second limiting rib loses a blocking effect on the second valve body; at this time, the elastic member is elongated, namely, the elastic member may release elastic potential energy, and the second valve body moves to a second position in the direction towards the second opening under the elastic force of the elastic member, so that the second valve body is in sealing connection with the passage wall of the fluid passage, thereby blocking the fluid passage and preventing oxygen from continuing to flow in the fluid passage.

That is, in the embodiment of the present disclosure, if a flame is induced due to improper operation during the oxygen therapy, the first limiting rib or the second limiting rib burns in the molten state. At this time, the elastic member disposed between the first valve body and the second valve body may push the first valve body or the second valve body to move, so that the first valve body or the second valve body is in sealing connection with the fluid passage, thereby blocking the fluid passage. The fire protection device may prevent the oxygen from continuing to flow in the fluid passage, avoid the problem that the flame with the oxygen pipe gradually burns to the fuselage, which easily causes the spread of fire and finally ignites the oxygen generator to have serious fire.

The above description is merely an overview of the technical solutions of the present disclosure, which can be carried out in accordance with the contents of the specification in order to make the technical means of the present disclosure more clearly understood. The detailed description of the present disclosure will be described below to make the above and other objects, features and advantages of the present disclosure more apparent .

### BRIEF DESCRIPTION OF THE DRAWINGS

In order to more clearly illustrate the technical solutions in the embodiments of the present disclosure, the drawings to be used in the description of the embodiments will be briefly introduced below. Obviously, the drawings in the description below are only some embodiments of the present disclosure. It will be apparent to those skilled in the art to obtain other drawings according to these drawings without involving any inventive effort.
FIG. 1 shows a schematic diagram showing an explosion structure of a first type of a fire protection device according to an embodiment of the present disclosure;
FIG. 2 shows a structural schematic diagram of a first type of a fire protection device in an open state according to an embodiment of the present disclosure;
FIG. 3 shows a structural schematic diagram of a first type of fire protection device in a closed state according to an embodiment of the present disclosure;
FIG. 4 shows a first structural schematic diagram of a first type of a valve body according to an embodiment of the present disclosure;
FIG. 5 shows a second structural schematic diagram of a first type of a valve body according to an embodiment of the present disclosure;
FIG. 6 shows a first structural schematic diagram of a first type of a housing according to an embodiment of the present disclosure;
FIG. 7 shows a second structural schematic diagram of a first type of a housing according to an embodiment of the present disclosure;
FIG. 8 shows a first structural schematic diagram of a second type of a fire protection device according to an embodiment of the present disclosure;
FIG. 9 shows a second structural schematic diagram of a second type of a fire protection device according to an embodiment of the present disclosure;
FIG. 10 shows a third structural schematic diagram of a second type of a fire protection device according to an embodiment of the present disclosure;
FIG. 11 shows a structural schematic diagram of a first housing according to an embodiment of the present disclosure;
FIG. 12 shows an assembled structural schematic diagram of a first housing and a second housing according to an embodiment of the present disclosure;
FIG. 13 shows a first structural schematic diagram of a second housing according to an embodiment of the present disclosure;
FIG. 14 shows a second structural schematic diagram of a second housing according to an embodiment of the present disclosure;
FIG. 15 shows a structural schematic diagram of a gas passage in an open state according to an embodiment of the present disclosure;
FIG. 16 shows a structural schematic diagram of a gas passage in a closed state according to an embodiment of the present disclosure;
FIG. 17 shows a first structural schematic diagram of a third type of a fire protection device in an open state according to an embodiment of the present disclosure;
FIG. 18 shows a second structural schematic diagram of a third type of a fire protection device in an open state according to an embodiment of the present disclosure;
FIG. 19 shows a first structural schematic diagram of a third type of a fire protection device in a closed state according to an embodiment of the present disclosure;
FIG. 20 shows a second structural schematic diagram of a third type of a fire protection device in a closed state according to an embodiment of the present disclosure;
FIG. 21 shows a structural schematic diagram of a third type of a fire protection device according to an embodiment of the present disclosure;
FIG. 22 shows an assembled structural schematic diagram of a support and a first elastic valve body according to an embodiment of the present disclosure;
FIG. 23 shows a third structural schematic diagram of a third type of a fire protection device in an open state according to an embodiment of the present disclosure;
FIG. 24 shows a fourth structural schematic diagram of a third type of a fire protection device in an open state according to an embodiment of the present disclosure;
FIG. 25 shows a third structural schematic diagram of a third type of a fire protection device in a closed state according to an embodiment of the present disclosure;
FIG. 26 shows a fourth structural schematic diagram of a third type of a fire protection device in a closed state according to an embodiment of the present disclosure;
FIG. 27 shows a fifth structural schematic diagram of a third type of a fire protection device in an open state according to an embodiment of the present disclosure;
FIG. 28 shows a sixth structural schematic diagram of a third type of a fire protection device in an open state according to an embodiment of the present disclosure;
FIG. 29 shows a first structural schematic diagram of a fourth type of a fire protection device according to an embodiment of the present disclosure;
FIG. 30 shows a second structural schematic diagram of a fourth type of a fire protection device according to an embodiment of the present disclosure;
FIG. 31 shows a third structural schematic diagram of a fourth type of a fire protection device according to an embodiment of the present disclosure;
FIG. 32 shows a fourth structural schematic diagram of a fourth type of a fire protection device according to an embodiment of the present disclosure;
FIG. 33 shows a first structural schematic diagram of a fifth type of a fire protection device in an open state according to an embodiment of the present disclosure;
FIG. 34 shows a first structural schematic diagram of a fifth type of a fire protection device in a closed state according to an embodiment of the present disclosure;
FIG. 35 shows a second structural schematic diagram of a fifth type of a fire protection device in an open state according to an embodiment of the present disclosure;
FIG. 36 shows a third structural schematic diagram of a fifth type of a fire protection device in an open state according to an embodiment of the present disclosure;
FIG. 37 shows a fourth structural schematic diagram of a fifth type of a fire protection device in an open state according to an embodiment of the present disclosure;
FIG. 38 shows a first assembled structural schematic diagram of a first positioning member and a second type of a housing according to an embodiment of the present disclosure;
FIG. 39 shows a second assembled structural schematic diagram of a first positioning member and a second type of a housing according to an embodiment of the present disclosure;
FIG. 40 shows a structural schematic diagram of a fifth type of a fire protection device according to an embodiment of the present disclosure;
FIG. 41 shows a fifth structural schematic diagram of a fifth type of a fire protection device in an open state according to an embodiment of the present disclosure;
FIG. 42 shows a second structural schematic diagram of a fifth type of a fire protection device in a closed state according to an embodiment of the present disclosure;
FIG. 43 shows a sixth structural schematic diagram of a fifth type of a fire protection device in an open state according to an embodiment of the present disclosure;
FIG. 44 shows a seventh structural schematic diagram of a fifth type of a fire protection device in an open state according to an embodiment of the present disclosure;
FIG. 45 shows a third structural schematic diagram of a fifth type of a fire protection device in a closed state according to an embodiment of the present disclosure;
FIG. 46 shows a first structural schematic diagram of a sixth type of a fire protection device in which a fluid passage is in an open state according to an embodiment of the present disclosure;
FIG. 47 shows a second structural schematic diagram of a sixth type of a fire protection device in which a fluid passage is in an open state according to an embodiment of the present disclosure;
FIG. 48 shows a first structural schematic diagram of a sixth type of a fire protection device in which a fluid passage is in a closed state according to an embodiment of the present disclosure;
FIG. 49 shows a second structural schematic diagram of a sixth type of a fire protection device in which a fluid passage is in a closed state according to an embodiment of the present disclosure;
FIG. 50 shows a first structural schematic diagram of a second type of a valve body according to an embodiment of the present disclosure;
FIG. 51 shows a structural schematic diagram of a third type of a housing according to an embodiment of the present disclosure;
FIG. 52 shows a third structural schematic diagram of a sixth type of a fire protection device in which a fluid passage is in an opened state according to an embodiment of the present disclosure;
FIG. 53 shows a fourth structural schematic diagram of a sixth type of a fire protection device in which a fluid passage is in an opened state according to an embodiment of the present disclosure;
FIG. 54 shows a third structural schematic diagram of a sixth type of a fire protection device in which a fluid passage is in a closed state according to an embodiment of the present disclosure;
FIG. 55 shows a fourth structural schematic diagram of a sixth type of a fire protection device in which a fluid passage is in a closed state according to an embodiment of the present disclosure;
FIG. 56 shows a second structural schematic diagram of a second type of a valve body according to an embodiment of the present disclosure;
FIG. 57 shows a first structural schematic diagram of a seventh type of a fire protection device according to an embodiment of the present disclosure;
FIG. 58 shows a structural schematic diagram of a third type of a valve body according to an embodiment of the present disclosure;
FIG. 59 shows a first structural schematic diagram of a base according to an embodiment of the present disclosure;
FIG. 60 shows a first assembled structural schematic diagram of a third type of a valve body according to an embodiment of the present disclosure;
FIG. 61 shows a second structural schematic diagram of a seventh type of a fire protection device according to an embodiment of the present disclosure;
FIG. 62 shows a second assembled structural schematic diagram of a third type of a valve body according to an embodiment of the present disclosure;
FIG. 63 shows a third structural schematic diagram of a seventh type of a fire protection device according to an embodiment of the present disclosure;
FIG. 64 shows a structural schematic diagram of a first valve body according to an embodiment of the present disclosure;
FIG. 65 shows a second structural schematic diagram of a base according to an embodiment of the present disclosure;
FIG. 66 shows an assembled structural schematic diagram of a first valve body according to an embodiment of the present disclosure;
FIG. 67 shows an assembled structural schematic diagram of a first valve body, a second valve body and a base according to an embodiment of the present disclosure;
FIG. 68 shows a first structural schematic diagram of an eighth type of a fire protection device according to an embodiment of the present disclosure;
FIG. 69 shows a second structural schematic diagram of an eighth type of a fire protection device according to an embodiment of the present disclosure;
FIG. 70 shows a third structural schematic diagram of an eighth type of a fire protection device according to an embodiment of the present disclosure;
FIG. 71 shows a fourth structural schematic diagram of an eighth type of a fire protection device according to an embodiment of the present disclosure;
FIG. 72 shows a fifth structural schematic diagram of an eighth type of a fire protection device according to an embodiment of the present disclosure;
FIG. 73 shows a first structural schematic diagram of a ninth type of a fire protection device according to an embodiment of the present disclosure;
FIG. 74 shows a second structural schematic diagram of a ninth type of a fire protection device according to an embodiment of the present disclosure;
FIG. 75 shows a third structural schematic diagram of a ninth type of a fire protection device according to an embodiment of the present disclosure;
FIG. 76 is a sectional view of a fire damper in the related art;
FIG. 77 is an axonometric view of a fire damper according to an embodiment of the present disclosure;
FIG. 78 is a first sectional view of a fire damper according to an embodiment of the present disclosure;
FIG. 79 is a second sectional view of a fire damper according to an embodiment of the present disclosure;
FIG. 80 is a first sectional view of another fire damper according to an embodiment of the present disclosure;
FIG. 81 is a second sectional view of another fire damper according to an embodiment of the present disclosure;
FIG. 82 is a first sectional view of another fire damper according to an embodiment of the present disclosure;
FIG. 83 is a second sectional view of another fire damper according to an embodiment of the present disclosure;
FIG. 84 is a sectional view of another fire damper according to an embodiment of the present disclosure;
FIG. 85 is a partial enlarged diagram of a fast-plug joint in FIG. 84.
FIG. 86 is a first sectional view of a fire damper according to an embodiment of the present disclosure;
FIG. 87 is a second sectional view of a fire damper according to an embodiment of the present disclosure;
FIG. 88 is a sectional view of another fire damper according to an embodiment of the present disclosure;
FIG. 89 is a sectional view of another fire damper according to an embodiment of the present disclosure;
FIG. 90 is a sectional view of another fire damper according to an embodiment of the present disclosure;
FIG. 91 is a structural schematic diagram of a fire-protection isolation device in the related art;
FIG. 92 is a three-dimensional structural schematic diagram of an automatic fire protection device applied to an oxygen therapy apparatus according to an embodiment of the present disclosure;
FIG. 93 is a three-dimensional sectional view of the automatic fire protection device of FIG. 92, and which shows that a sealing member is in a first state, and a trigger post and a connecting ring are connected in the chamber;
FIG. 94 is a sectional view of the automatic fire protection device of FIG. 92
FIG. 95 is a three-dimensional structural schematic diagram of the sealing member shown in FIG. 93, where the sealing member is in an unstretched state;
FIG. 96 is a three-dimensional structural schematic diagram of the sealing member shown in FIG. 93, where the sealing member is in a stretched state;
FIG. 97 is a three-dimensional structural schematic diagram of a mounting arm shown in FIG. 96;
FIG. 98 is a structural schematic diagram of the sealing member shown in FIG. 93 mounted on a mounting arm after being stretched;
FIG. 99 is a three-dimensional sectional view of the automatic fire protection device of FIG. 92, where the sealing member is in a second state;
FIG. 100 is a three-dimensional sectional view of an automatic fire protection device applied to an oxygen therapy apparatus in another embodiment of the present disclosure, where a trigger post and a connecting ring are shown connected in a second nozzle;
FIG. 101 is a cross-sectional view of a tenth type of a fire protection device according to an embodiment of the present disclosure;
FIG. 102 is a schematic diagram of a first limiting rib in a molten state of a tenth type of a fire protection device according to an embodiment of the present disclosure;
FIG. 103 is a side view of a tenth type of a fire protection device according to an embodiment of the present disclosure;
FIG. 104 is a schematic diagram of a tenth type of a fire protection device according to an embodiment of the present disclosure;
FIG. 105 is a cross-sectional view of an eleventh type of a fire protection device according to an embodiment of the present disclosure;
FIG. 106 is a structural diagram of a first valve body according to another embodiment of the present disclosure;
FIG. 107 is a first side view of a first valve body according to another embodiment of the present disclosure; and
FIG. 108 is a second side view of a first valve body according to another embodiment of the present disclosure.

### description of reference numerals

10-housing; 20-valve body; 30-torsion spring; 40-meltable member; 101-fluid passage; 102-first opening; 103-second opening; 104-rotating shaft; 105-limiting portion; 106-first pipeline joint; 107-first through-hole; 108-second pipeline joint; 109-second through-hole; 110-clamping portion; 201-accommodating cavity; 202-mounting portion; 203-first connecting portion; 204-first sealing portion; 205-second connecting portion; 206-second sealing portion; 207-notch; 401-extension; 2021-inner shaft sleeve; 2022-outer shaft sleeve.
220-elastic member; 230-valve body; 240-meltable member; 2101-accommodating cavity; 2102-gas passage; 2103-first housing; 2104-second housing; 2105-first through-hole; 2106-second through-hole; 2107-third through-hole; 2108-fourth through-hole; 2109-first protrusion; 2110-second protrusion; 2111-first extension; 2112-second extension; 2113-limiting portion; 2114-first connecting portion; 2115-second connecting portion; 2301-fifth through-hole; 2302-third extension; 2303-fourth extension; 2401-sixth through-hole.
320-first elastic valve body; 330-first meltable member; 340-support; 350-second elastic valve body; 360-second meltable member; 3101-accommodating cavity; 3102-first opening; 3103-second opening; 3104-first connecting portion; 3105-second connecting portion; 3106-first through-hole; 3107-second through-hole; 3108-reinforcing rib; 3109-clamping portion; 3201-second positioning portion; 3401-first positioning portion.
420-first movable rod; 430-first meltable member; 440-first valve body; 450-first elastic member; 460-second movable rod; 470-second meltable member; 480-second valve body; 490-second elastic member; 4101-fluid passage; 4102-first narrowed portion; 4103-second narrowed portion; 4104-baffle; 4105-first pipeline joint; 4106-second pipeline joint; 4107-third clamping portion; 4201-first clamping-fitting portion; 4202-first guide portion; 4301-first clamping portion; 4401-first sealing member; 4402-second guide portion; 4403-groove body; 4404-mounting shaft; 4601-second clamping-fitting portion; 4701-second clamping portion; 4801-second sealing member.
520-first positioning member; 530-first sealing member; 540-meltable member; 550-second positioning member; 560-second sealing member; 5101-fluid passage; 5102-first opening; 5103-second opening; 5104-annular protrusion; 5105-first protruding structure; 5106-second protruding structure; 5107-first positioning portion; 5108-second positioning portion; 5109-mounting portion; 5110-accommodating groove; 5111-connecting rib.
620-valve body; 630-guide portion; 640-first meltable member; 650-first elastic member; 660-second meltable member; 670-second elastic member; 6101-fluid passage; 6102-first narrowed portion; 6103-second narrowed portion; 6104-fixing portion; 6105-positioning groove; 6201-first groove body; 6202-first mounting shaft; 6203-first sealing member; 6204-second groove body; 6205-second mounting shaft; 6206-second sealing member; 6207-positioning rib.
7101-fluid passage; 7102-narrowed portion; 720-base; 7201-vent hole; 7202-first positioning groove; 7203-first positioning portion; 7204-second positioning portion; 7205-flange; 7206-first limiting portion; 7207-second limiting portion; 730-valve body; 7301-sealing portion; 73011-guide portion; 7302-bearing portion; 73021-first support structure; 73022-hollowed-out structure; 7303-connecting portion; 7303 1-clamping structure; 7304-first valve body; 73041-first sealing portion; 73042-first mounting sleeve; 73043-second support structure; 73044-third limiting portion; 73045-second positioning groove; 7305-second valve body; 73051-second sealing portion; 73052-second mounting sleeve; 73053-third support structure; 73054-fourth limiting portion; 73055-third positioning groove; 740-elastic member; 7401-first elastic member; 7402-second elastic member; 750-meltable collar.
8101-main body; 8102-opening portion; 8103-shoulder; 820-inner shell; 8201-through-hole; 830-valve body; 8301-fusible portion; 8302-clamping member; 83021-first guide portion; 83022-second guide portion; 83023-limiting portion; 83024-groove; 8303-sealing member; 83031-third guide portion; 83032-limiting groove; 83033-fourth guide portion; 83034-boss; 840-elastic member; 850-meltable support portion; 860-sealing structure; 870-elastic support; 880-first mounting groove; 890-second mounting groove; 8100-support portion.
9101-fluid passage; 9102-first housing; 9103-second housing; 9104-mounting engagement groove; 9105-expanded portion; 91011-main body; 91012-opening portion; 91013-shoulder; 920-sealing member; 9201-embedded portion; 930-valve body; 9301-fusible portion; 9302-clamping portion; 9303-connecting portion; 9304-sealing portion; 9305-first guide portion; 9306-second guide portion; 940-elastic member; 950-meltable support portion.
1-spring; 2-piston; 3-first limiting ball; 4-second limiting ball; 5-airflow passage;
10a-first housing; 10b-second housing;
11-first joint; 12-second joint; 13-first vent pipe; 14-first vent hole; 15-second vent pipe; 16-second vent hole; 17-fixing portion; 18-second gap;
120-sealing member; 121-hot melt layer; 1211-thin end; 1212-thick end; 1213-protrusion; 122-heat-shrinkable material layer;
131-outer shell; 132-pressing member; 133-reed.
1-10, outer shell; 1-12, end seat; 1-13, meltable nose component; 1-14, poppet valve; 1-15, compression spring; 1-16, inlet; 1-17, central hole; 1-18, small hole; 1-19, outlet port; 1-21, larger-diameter hole; 1-22, inclined portion; 1-24, flange;
1-100, housing;
1-110, first housing; 1-120, second housing; 1-130, chamber; 1-140, gas passage; 1-150, connecting groove;
1-111, first nozzle; 1-112, first anti-detachment portion; 1-121, second nozzle; 1-122, second anti-detachment portion;
1-30, sealing ring; 1-40, sealing member;
1-41, sealing portion; 1-42, first connector; 1-43, second connector;
1-421, first connecting hole; 1-431, connecting ring; 1-432, second connecting hole;
1-50-mounting arm;
1-51, adapter block; 1-52, mounting post; 1-53, connecting plate;
1-511, accommodating hole; 1-521, connecting post; 1-522, stop circular truncated cone; 1-523, fixing post; 1-531, recessed portion;
1-532, extension plate; 1-533, trigger post.
2-100: fire protection device; 2-20: first valve body; 2-30: second valve body; 2-40: elastic member; 2-11: fluid passage; 2-12: first limiting rib; 2-13: second limiting rib; 2-21: first mounting rod; 2-22: first mounting seat; 2-23: first mounting cavity; 2-24: first guide rod; 2-25: first positioning portion; 2-31: second mounting rod; 2-32: second mounting seat; 2-33: second mounting cavity; 2-34: second guide rod; 2-35: second positioning portion; 2-14: first positioning groove; 2-15: second positioning groove; 2-16: first mounting groove; 2-17: first sealing member; 2-18: second mounting groove; 2-19: second sealing member; 2-111: first blocking platform; 2-112: second blocking platform; 2-1111: first surface; 2-1121: second surface; 2-122: first sub-housing; 2-123: first passage; 2-124: second sub-housing; 2-125: second passage; 2-51: first joint; 2-52: second joint; 2-26: guide shaft.

### DETAILED DESCRIPTION OF THE EMBODIMENTS

The technical solutions in the embodiments of the present disclosure will be described clearly and completely in conjunction with the accompanying drawings in the embodiments of the present disclosure. Obviously, the described embodiments are part of the embodiments of the present disclosure, rather than all of the embodiments. Based on the embodiments in the present disclosure, all other embodiments obtained by a person skilled in the art without involving any inventive effort are within the scope of protection of the present disclosure.

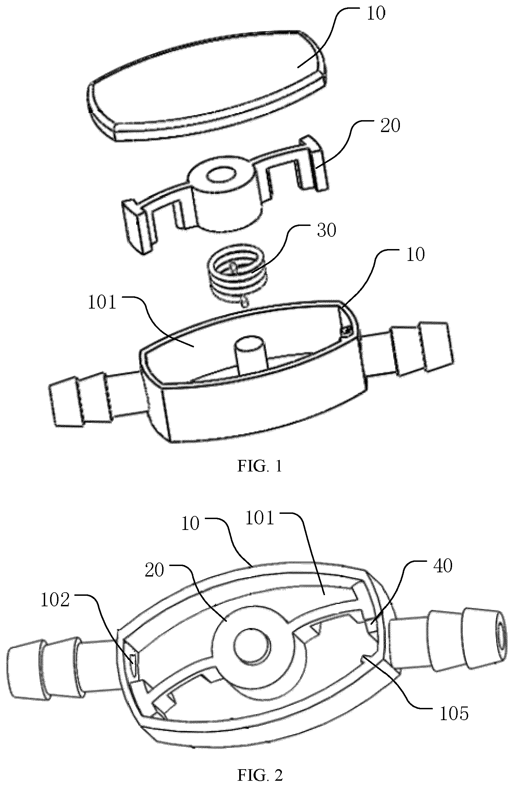

Referring to FIG. 1 to FIG. 3, the embodiment of the present disclosure discloses a first type of a fire protection device including a housing 10, a valve body 20, a torsion spring 30, and a meltable member 40. The housing 10 has a fluid passage 101 therein. The fluid passage 101 is provided with a first opening 102 and a second opening 103. The first opening 102 and the second opening 103 are respectively used for communicating with a pipeline of an oxygen therapy instrument or a patient end. The valve body 20 is located in the fluid passage 101 and is rotatably connected to the housing 10. The valve body 20 is provided with an accommodating cavity 201, and the fluid passage 101 and the accommodating cavity 201 are two spaces independent from each other. The torsion spring 30 is embedded in the accommodating cavity 201 to drive the relative rotation of the valve body 20 and the housing 10. The meltable member 40 is disposed on an inner wall of the fluid passage 101. When the meltable member is in a non-molten state, the meltable member 40 supports the valve body 20 to be in a first position, and both the first opening 102 and the second opening 103 are in an open state. When the meltable member 40 is in a molten state, the torsion spring 30 drives the valve body 20 to rotate to a second position, with at least one of the first opening 102 and the second opening 103 being in a closed state.

Specifically, as shown in FIG. 1 to FIG. 3, the first type of the fire protection device includes the housing 10, the valve body 20, the torsion spring 30, and the meltable member 40. As a main frame of the fire protection device, the housing 10 may be made of a material that is not easily chemically reacted with oxygen and is resistant to high temperatures, such as stainless steel or a ceramic material. The fluid passage 101 is provided in the housing 10. The fluid passage 101 is provided with the first opening 102 and the second opening 103, both of which may be located at two opposite sides of the fluid passage 101, with the axes of the first opening 102 and the second opening 103 being collinear. The first opening 102 and the second opening 103 may also be angularly disposed on the fluid passage 101. The first opening 102 and the second opening 103 are respectively used for communicating with the pipeline of the oxygen therapy instrument or the patient end. For example, the first opening 102 communicates with the pipeline of the oxygen therapy instrument end. The second opening 103 communicates with the pipeline of the patient end. Oxygen enters the fluid passage 101 from the first opening 102 and is transmitted to the patient end via the second opening 103. The second opening 103 may also be in communication with the pipeline of the oxygen therapy instrument end, and the first opening 102 may be in communication with the pipeline of the patient end, which is not limited by the embodiments of the present disclosure.

The valve body 20 is located in the fluid passage 101 and is rotatably connected to the housing 10. The valve body 20 and the housing 10 may be rotatably connected by a rotating shaft 104 or a roller. For example, a rotating shaft 104 is disposed on the housing 10, and the valve body 20 is sleeved on the rotating shaft 104. Alternatively, a roller is disposed on the valve body 20 to rotate relative to the inner wall of the housing 10. The valve body 20 may also be made of a material which is not easily chemically reacted with oxygen and is resistant to high temperatures. The valve body 20 is further provided with a sealing part which is engaged with the first opening 102 or the second opening 103. The sealing part may be made of a material such as silicone, rubber, etc. and may achieve a good sealing effect.

The valve body 20 is provided with the accommodating cavity 201, and the fluid passage 101 and the accommodating cavity 201 are two spaces independent from each other. The torsion spring 30 is embedded into the accommodating cavity 201 to drive the relative rotation of the valve body 20 and the housing 10. The torsion spring 30 may be a metal spring made of such as iron, copper, or an alloy, or may be a soft rubber such as silica gel or rubber that may store elastic potential energy. The torsion spring 30 may store the elastic potential energy and drive the valve body 20 to rotate in the event of a fire, so that the fluid passage 101 is closed and the oxygen passage is blocked, thereby achieving a fire prevention effect. The oxygen does not intrude into the accommodating cavity 201 while being transported in the fluid passage 101, thereby reducing the probability of the torsion spring 30 contacting oxygen, avoiding the problem of oxidation failure of the torsion spring 30, and improving the stability and durability of the fire protection device.

The meltable member 40, which may be made of a lower melting point material such as PP, PVC, or the like, is disposed on the inner wall of the fluid passage 101 and located on the rotational path of the valve body 20 to support the valve body 20. The fitting of the meltable member 40 to the inner wall of the fluid passage 101 may be accomplished by bonding, clamping, or the like.

In the absence of a fire (the temperature is low), the meltable member 40 is in a non-molten state, has a certain rigidity, and is capable of supporting the valve body 20. When the valve body 20 is in a first position, the first opening 102 and the second opening 103 are both in an open state. Oxygen may be normally transmitted in the fluid passage 101 through the first opening 102 and the second opening 103. When the valve body 20 is in the first position, the spring force exerted by the torsion spring 30 on the valve body 20 and the supporting force exerted by the meltable member 40 on the valve body 20 are in equilibrium.

In the event of a fire (the temperature is high), the meltable member 40 is in a molten state, and the meltable member 40 may not continue to support the valve body 20. The equilibrium state of the valve body 20 is broken. At this time, the valve body 20 is rotated from the first position to the second position by the elastic force of the torsion spring 30, and the sealing part of the valve body 20 is engaged with at least one of the first opening 102 and the second opening 103, so that at least one of the first opening 102 and the second opening 103 is in a closed state. At this time, the fluid passage 101 is blocked, and oxygen may not continue to be transmitted.

In the disclosed embodiment, when the meltable member 40 is in a non-molten state, the meltable member 40 supports the valve body to be in a first position, both the first opening 102 and the second opening 103 are in an open state, and the fluid passage 101 normally transmits oxygen. When a fire occurs, the meltable member 40 will be in a molten state as the temperature reaches the melting point. The meltable member 40 is insufficient to support the elastic force exerted on the valve body 20 by the torsion spring 30. The torsion spring 30 releases the stored elastic potential energy. The valve body 20 rotates from the first position to the second position under the drive of the torsion spring 30, so that at least one of the first opening 102 and the second opening 103 is in a closed state, thereby cutting off the oxygen passage and preventing the oxygen from continuously leaking out to cause the fire to spread. In addition, the accommodating cavity 201 in which the torsion spring 30 is located and the fluid passage 101 are independent from each other, so that the problem of oxidation of the torsion spring 30 is avoided, and the durability and safety factor of the device are improved.

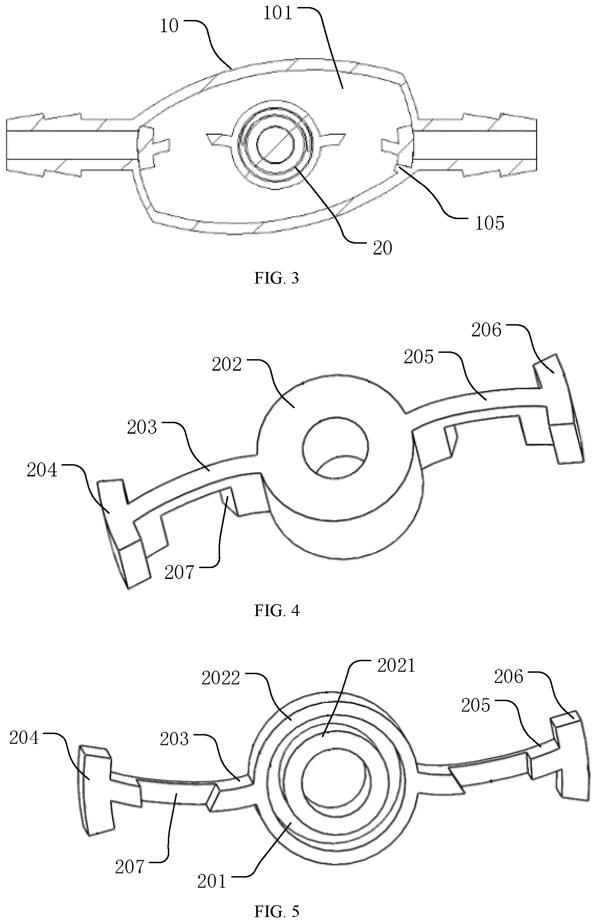

Optionally, as shown in FIG. 4 to FIG. 7, the valve body 20 includes a mounting portion 202, a first connecting portion 203, and a first sealing portion 204. The mounting portion 202 includes an inner shaft sleeve 2021 and an outer shaft sleeve 2022, and the accommodating cavity 201 is located between the inner shaft sleeve 2021 and the outer shaft sleeve 2022. A rotating shaft 104 is provided in the fluid passage 101, and the inner shaft sleeve 2021 is sleeved on the rotating shaft 104 and is rotatably connected to the rotating shaft 104. One end of the first connecting portion 203 is connected to a side wall of the outer shaft sleeve 2022, and the other end of the first connecting portion 203 is connected to the first sealing portion 204. When the valve body 20 is in the second position, the first sealing portion 204 is engaged with the first opening 102 such that the first opening 102 is in a closed state.

Specifically, as shown in FIG. 4 to FIG. 7, the fluid passage 101 has a first opening 102 and a second opening 103. The fluid passage 101 is blocked when at least one of the first opening 102 and the second opening 103 is in a closed state. In order to reduce the occupancy of space within the fluid passage 101 and the obstruction to oxygen transport, in the embodiment of the present disclosure, only the first opening 102 is controlled to switch from the open state to the closed state. Accordingly, the valve body 20 includes the mounting portion 202 through which the valve body 20 is assembled with the housing 10, the first connecting portion 203, and the first sealing portion 204. The mounting portion 202 includes an inner shaft sleeve 2021 and an outer shaft sleeve 2022. The inner shaft sleeve 2021 and the outer shaft sleeve 2022 are coaxially arranged. The bottom surfaces of one side of the inner shaft sleeve 2021 and the outer shaft sleeve 2022 are closed, and the other side thereof is in an open state. The outer side wall of the inner shaft sleeve 2021 and the inner side wall of the outer shaft sleeve 2022 together define the accommodating cavity 201, and the torsion spring 30 is embedded in the accommodating cavity 201.

The rotating shaft 104 is disposed in the fluid passage 101. The rotating shaft 104 and the inner wall of the fluid passage 101 may be made by an integral process, or may be made separately, and assembled by means of bonding or clamping. The inner shaft sleeve 2021 is sleeved on the rotating shaft 104, and the inner shaft sleeve 2021 is arranged coaxially with the rotating shaft 104 and is rotatably connected to the rotating shaft 104, thereby achieving relative rotation between the valve body 20 and the housing 10. One end of the first connecting portion 203 is connected to the side wall of the outer shaft sleeve 2022, and the other end of the first connecting portion 203 is connected to the first sealing portion 204. The first connecting portion 203, the outer shaft sleeve 2022 and the inner shaft sleeve 2021 can be made of the same material and made by integral molding.

The first sealing portion 204 is close to the first opening 102. The first sealing portion 204 may be made of silicone, rubber, or the like, and has a good sealing effect. When the valve body 20 is in the second position, the first sealing portion 204 is engaged with the first opening 102. That is, the end surface of the first sealing portion 204 abuts against the inner wall of the fluid passage 101 around the first opening 102 to cover the first opening 102. When the first opening 102 is in the closed state, the fluid passage 101 is blocked.

Alternatively, as shown in FIG. 4 to FIG. 7, the valve body 20 further includes a second connecting portion 205 and a second sealing portion 206. One end of the second connecting portion 205 is connected to a side wall of the outer shaft sleeve 2022, and the other end of the second connecting portion 205 is connected to the second sealing portion 206. When the valve body 20 is in the second position, the second sealing portion 206 is engaged with the second opening 103, so that the second opening 103 is in a closed state.

Specifically, as shown in FIG. 4 to FIG. 7, the valve body 20 further includes a second connecting portion 205 and a second sealing portion 206. The second sealing portion 206 is close to the second opening 103. The first connecting portion 203 and the second connecting portion 205 are located on both sides of the mounting portion 202, respectively. One end of the second connecting portion 205 is connected to the side wall of the outer shaft sleeve 2022, and the other end of the second connecting portion 205 is connected to the second sealing portion 206. The first connecting portion 203, the second connecting portion 205, the inner shaft sleeve 2021 and the outer shaft sleeve 2022 can be made of the same material and made by integral molding.

The second sealing portion 206 may be made of silicone or rubber, and has a good sealing effect. When the valve body 20 is in the second position, the second sealing portion 206 is engaged with the second opening 103. That is, the end surface of the second sealing portion 206 abuts against the inner wall of the fluid passage 101 around the second opening 103 to cover the second opening 103. When the second opening 103 is in the closed state, the fluid passage 101 is blocked.

By disposing a sealing portion at both the first opening 102 and the second opening 103, the first opening 102 and the second opening 103 are closed at the same time when the valve body 20 is in the second position. It ensures the blocking effect of the fluid passage 101, thereby improving the stability of the fire protection device.

Alternatively, as shown in FIG. 4 to FIG. 5, the first connecting portion 203 and/or the second connecting portion 205 is provided with a notch 207 for passage of a fluid.

Specifically, as shown in FIG. 4 to FIG. 5, oxygen is transferred in the fluid passage 101 through the first opening 102 and the second opening 103, and the first connecting portion 203 and the second connecting portion 205 are located in the fluid passage 101. In order to prevent the first connecting portion 203 and the second connecting portion 205 from obstructing the normal transfer of oxygen, a notch 207 is provided in the first connecting portion 203 and/or the second connecting portion 205, oxygen can be transferred normally through the notch 207 in the fluid passage 101 without affecting the transfer rate of oxygen. The notch 207 may be disposed only on the first connecting portion 203 or the second connecting portion 205, or both the first connecting portion 203 and the second connecting portion 205 may be provided with the notch 207 to enhance the ventilation effect. The shape and size of the notch 207 may be selected according to practical requirements, and the embodiments of the present disclosure are not limited thereto.

Alternatively, as shown in FIG. 2 to FIG. 3, the inner wall of the fluid passage 101 is provided with at least one limiting portion 105 located on the rotational path of the valve body 20. When the valve body 20 is in the second position, the valve body 20 abuts against the limiting portion 105.

Specifically, as shown in FIG. 2 to FIG. 3, the limiting portion 105 is disposed on the inner wall of the fluid passage 101, and the limiting portion 105 may specifically have a plate-like or cylindrical structure. The limiting portion 105 and the housing 10 may be formed by an integral molding process. The limiting portion 105 is located on the rotation path of the valve body 20, that is, the valve body 20 comes into contact with the limiting portion 105 when rotating relative to the housing 10. The quantity of the limiting portions 105 may be one or two. When the quantity of the limiting portions 105 is two, the two limiting portions 105 may be respectively located at both sides of the valve body 20.

In the event of a fire, the meltable member 40 is heated and melted, insufficient to counteract the rotational torque exerted by the torsion spring 30 on the valve body 20. At this moment, the torsion spring 30 releases the stored elastic potential energy and causes relative rotation between the valve body 20 and the housing 10 until the valve body 20 stops rotating when it contacts the limiting portion 105. The valve body 20 is just in the second position, and the fluid passage 101 is in the closed state. Under the combined action of the torsion spring 30 and the limiting portion 105, a stable assembly is formed between the valve body 20 and the housing 10.

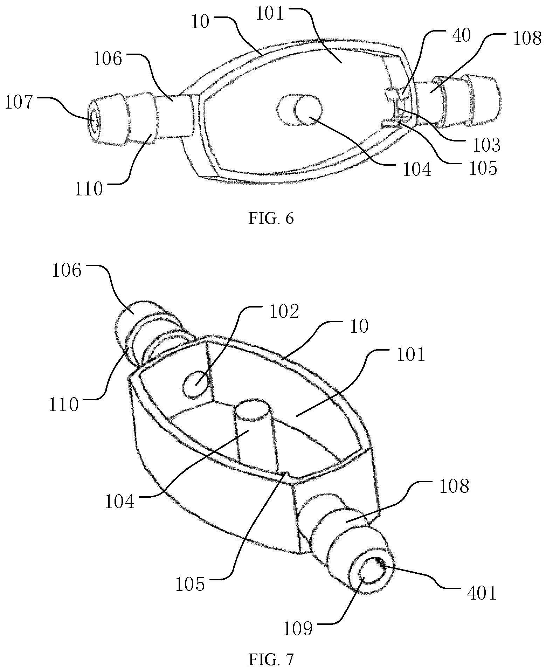

Alternatively, as shown in FIG. 6 to FIG. 7, the housing 10 is provided with a first pipeline joint 106 and a second pipeline joint 108. The first pipeline joint 106 is provided with a first through-hole 107, and the first through-hole 107 communicates with the first opening 102. The second pipeline joint 108 is provided with a second through-hole 109, and the second through-hole 109 communicates with the second opening 103. The first pipeline joint 106 and the second pipeline joint 108 are respectively used for connecting with the pipeline of the oxygen therapy instrument or the patient end.

Specifically, as shown in FIG. 6 to FIG. 7, the first pipeline joint 106 and the second pipeline joint 108 are respectively located at both sides of the fluid passage 101. The first pipeline joint 106 is provided with the first through-hole 107 communicating with the first opening 102, and the second pipeline joint 108 is provided with the second through-hole 109 communicating with the second opening 103. The first pipeline joint 106, the second pipeline joint 108, and the housing 10 may be of an integrated structure or may be made in separate pieces and then assembled.

The first pipeline joint 106 and the second pipeline joint 108 are used for communicating with the pipeline of the oxygen therapy instrument or the patient end. The pipeline of the oxygen therapy instrument or the patient end may be assembled with the first pipeline joint 106 or the second pipeline joint 108 by means of a threaded connection or an interference fit.

Alternatively, as shown in FIG. 6 to FIG. 7, the outside wall of the first pipeline joint 106 and/or the outside wall of the second pipeline joint 108 is provided with at least one clamping portion 110 for being clamped with the pipeline of the oxygen therapy instrument or the patient end.

Specifically, as shown in FIG. 6 to FIG. 7, the first pipeline joint 106 and the second pipeline joint 108 are used for communicating with the pipeline of the oxygen therapy instrument or the patient end. The pipeline is generally a catheter which is sleeved outside the first pipeline joint 106 and the second pipeline joint 108. In order to ensure the stability of the connection between the catheter and the first pipeline joint 106 and between the catheter and the second pipeline joint 108, at least one clamping portion 110 is disposed on the outside wall of the first pipeline joint 106 and/or the outside wall of the second pipeline joint 108. The shape of the clamping portion 110 is flared. When the catheter is sleeved on the outside wall of the first pipeline joint 106 and/or the outside wall of the second pipeline joint 108, the clamping portion 110 is clamped with the catheter. Air tightness is ensured between the catheter and the first pipe connection 106 and/or the second pipe connection 108, avoiding the problem of oxygen leakage. The quantity of clamping portions 110 may be selected based on the size of the first pipeline joint 106 and the second pipeline joint 108.

Optionally, as shown in FIG. 7, the meltable member 40 has an extension 401 passing through the first through-hole 107 or the second through-hole 109.

Specifically, as shown in FIG. 7, the meltable member 40 is located in the fluid passage 101, i.e., inside the housing 10, not in direct contact with the external environment, and there is a response delay. That is, when a fire occurs in the external environment, the meltable member 40 may not be rapidly heated and melted, and the oxygen path cannot be blocked in time. Thus, an extension 401 is disposed on the meltable member 40, the extension 401 passing through the first through-hole 107 or the second through-hole 109. The extension 401 is generally close to the pipeline of the patient end. the extension 401 specifically passes through the first through-hole 107 or the second through-hole 109, which can be determined according to the assembly method of the first pipeline joint 106 and the second pipeline joint 108. For example, if the first pipeline joint 106 communicates with the pipeline of the patient end, the extension 401 may be disposed in the first through-hole 107. If the second pipeline joint 108 communicates with the pipeline of the patient end, the extension 401 may be disposed in the second through-hole 109. The extension 401 and the meltable member 40 may be made of the same material, and the extension 401 may also be made of a material with better thermal conductivity such as copper and iron. When a fire occurs, if the extension 401 of the same material as the meltable member 40 is used, the extension 401 may burn at first and ignite the meltable member 40 inside the fluid passage 101, so as to melt the meltable member 40 in time. If an extension 401 made of copper, iron or the like is used, the extension 401 may quickly conduct heat to the meltable member 40 inside the fluid passage 101, so that the meltable member 40 quickly reaches the melting point and timely blocks the passage of oxygen to reduce the loss caused by fire.

Embodiments of the present disclosure also disclose a first type of an oxygen therapy instrument including the first type of the fire protection device.

Specifically, in a medical or home setting, when the patient is unable to meet his/her needs on his/her own inhaled oxygen, it is often necessary to supply oxygen through an external instrument. The method of delivering oxygen to a patient by means of an external instrument, either in an invasive manner or a non-invasive manner, is known as oxygen therapy. Instruments used to supply oxygen to a patient are collectively referred to as oxygen therapy instruments. The oxygen therapy instrument is generally composed of three parts: an oxygen generating device (e.g., an oxygen tank, an oxygen generator, etc.), an interface for delivering oxygen to a patient (e.g., a nasal oxygen tube, a mask, etc.), and a pipeline connecting the generating device and the patient interface. A fire protection device is disposed in series in the pipeline between the oxygen generating device and the patient interface. The fire protection device has a fluid passage 101 therein. The fluid passage 101 is used for communicating with the pipeline of the oxygen therapy instrument or the patient end. The fire protection device may control the opening or closing of the pipeline of the oxygen therapy instrument by controlling the opening or closing of the fluid passage 101.

In the embodiment of the present disclosure, the first type of the fire protection device is connected in series in the pipeline of the first type of the oxygen therapy instrument. When the meltable member 40 is in a non-molten state, the meltable member 40 supports the valve body 20 to be in the first position, both the first opening 102 and the second opening 103 are in the open state, and the fluid passage 101 normally transmits oxygen. When a fire occurs, the meltable member 40 will be in a molten state as the temperature reaches the melting point. The meltable member 40 is insufficient to support the elastic force exerted on the valve body 20 by the torsion spring 30. The torsion spring 30 releases the stored elastic potential energy. The valve body 20 rotates from the first position to the second position under the drive of the torsion spring 30, so that at least one of the first opening 102 and the second opening 103 is in a closed state, thereby cutting off the oxygen passage and preventing the oxygen from continuously leaking out to cause the fire to spread. In addition, the accommodating cavity 201 in which the torsion spring 30 is located and the fluid passage 101 are independent from each other, so that the problem of oxidation of the torsion spring 30 is avoided, and the durability and safety factor of the device are improved.

Embodiments of the present disclosure also disclose a first type of a ventilation treatment system including the first type of the oxygen therapy instrument.

Specifically, the first type of the ventilation treatment system includes a control device and the first type of the oxygen therapy instrument. The control device is used for controlling the oxygen supply amount, working time, etc. of the first type of the oxygen therapy instrument. The control device can be an electronic device or a component in the electronic device, such as an integrated circuit or a chip. The electronic device may be a terminal or may be a device other than a terminal. Illustratively, the electronic device may be a cell phone, a tablet computer, a notebook computer, a palmtop computer, a mobile Internet appliance, a robot, a wearable device, etc. and embodiments of the present disclosure are not particularly limited.

In the embodiment of the present disclosure, the first type of the ventilation treatment system includes the first type of the oxygen therapy instrument. The first type of the fire protection device is connected in series in the pipeline of the first type of the oxygen therapy instrument. When the meltable member 40 is in a non-molten state, the meltable member 40 supports the valve body to be in the first position, both the first opening 102 and the second opening 103 are in the open state, and the fluid passage 101 normally transmits oxygen. When a fire occurs, the meltable member 40 will be in a molten state as the temperature reaches the melting point. The meltable member 40 is insufficient to support the elastic force exerted on the valve body 20 by the torsion spring 30. The torsion spring 30 releases the stored elastic potential energy. The valve body 20 rotates from the first position to the second position under the drive of the torsion spring 30, so that at least one of the first opening 102 and the second opening 103 is in the closed state, thereby cutting off the oxygen passage and preventing the oxygen from continuously leaking out to cause the fire to spread. In addition, the accommodating cavity 201 in which the torsion spring 30 is located and the fluid passage 101 are independent from each other, so that the problem of oxidation of the torsion spring 30 is avoided, and the durability and safety factor of the device are improved.

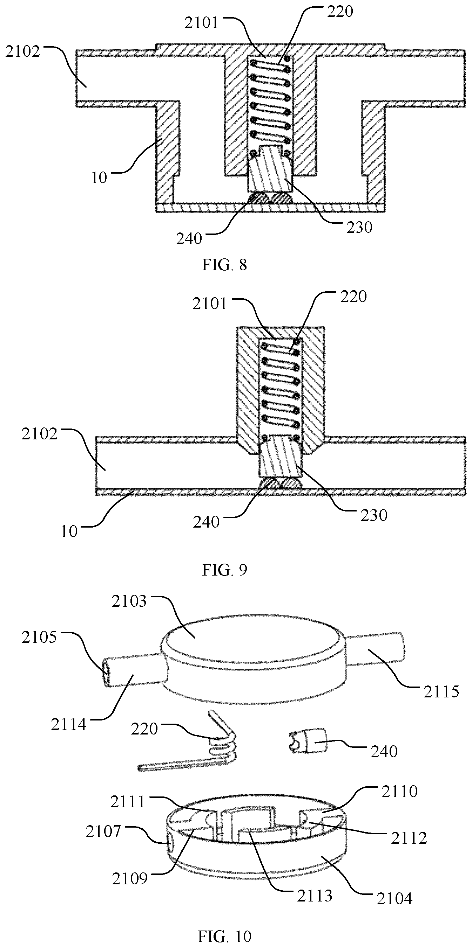

Referring to FIG. 8 to FIG. 10, a second type of a fire protection device is disclosed according to an embodiment of the present disclosure, the second type of the fire protection device including a housing 10, an elastic member 220, a valve body 230, and a meltable member 240. The housing 10 has an accommodating cavity 2101 and a gas passage 2102 therein, the gas passage 2102 is used for communicating with a pipeline of an oxygen therapy instrument or a patient end. The valve body 230 divides the accommodating cavity 2101 and the gas passage 2102 into mutually independent spaces. The elastic member 220 is disposed in the accommodating cavity 2101, the valve body 230 is at least partially located in the gas passage 2102, and the elastic member 220 respectively abuts against the housing 10 and the valve body 230. The meltable member 240 is disposed between the valve body 230 and the gas passage 2102, and a gap is provided between the meltable member 240 and the gas passage 2102. When the meltable member 240 is in a non-molten state, the meltable member 240 supports the valve body 230 to be in a first position, with the gas passage 2102 in an open state. When the meltable member 240 is in a molten state, the elastic member 220 drives the valve body 230 to be in a second position, with the gas passage 2102 in a closed state.

Specifically, as shown in FIG. 8 to FIG. 10, the second type of the fire protection device includes the housing 10, the elastic member 220, the valve body 230, and the meltable member 240. As a main frame of the second type of the fire protection device, the housing 10 may be made of plastic or the like, which is not easily chemically reacted with oxygen. The housing 10 has an accommodating cavity 2101 and a gas passage 2102 therein. A housing wall inside the housing 10 and the valve body 230 divide the accommodating cavity 2101 and the gas passage 2102 into mutually independent spaces, and oxygen in the gas passage 2102 may not enter the accommodating cavity 2101. The accommodating cavity 2101 and the housing 10 can be made by an integral molding process, for example, by means of open-die casting. The position of the accommodating cavity 2101 may be reserved on the die, and the structural strength of the housing 10 and the air tightness of the accommodating cavity 2101 may be ensured by the integral molding process. The accommodating cavity 2101 and the housing 10 may be made separately, and the accommodating cavity 2101 is divided by adding a partition plate or the like to the housing 10, which is not limited by the embodiments of the present disclosure. The valve body 230 may be made of rubber and has a good fit with the gas passage 2102 and the accommodating cavity 2101.

The elastic member 220 is disposed in the accommodating cavity 2101, and the elastic member 220 may be a metal spring such as iron, copper and alloy, or a soft rubber such as silica gel and rubber which can store the elastic potential energy. The elastic member 220 can store elastic potential energy and push the valve body 230 to close the gas passage 2102 in the event of a fire and block the passage of oxygen, thereby achieving a fire prevention effect.

The meltable member 240 is disposed between the valve body 230 and the gas passage 2102 to support the valve body 230, and a gap is provided between the meltable member 240 and the gas passage 2102. Oxygen may flow through the gap normally. The meltable member 240 is typically made of a lower-melting-point material, such as PP, PVC, or the like.

When there is no fire (the temperature is low), the meltable member 240 is in a non-molten state and has a certain rigidity to support the valve body 230. At this time, the valve body 230 is in the first position, the gas passage 2102 is in an open state, and oxygen may normally flow in the gas passage 2102. When the valve body 230 is in the first position, the elastic force exerted by the elastic member 220 on the valve body 230 and the supporting force exerted by the meltable member 240 on the valve body 230 are in equilibrium.

When a fire occurs (the temperature is high), the meltable member 240 is in a molten state, and the meltable member 240 may not continue to support the valve body 230. The equilibrium state of the valve body 230 is broken. At this time, the valve body 230 moves from the first position to the second position under the elastic force of the elastic member 220, the gas passage 2102 is in a closed state, and the oxygen passage is blocked.

Wherein, the first position and the second position of the valve body 230 are the positions of the valve body 230 relative to the gas passage 2102. When the valve body 230 is switched between the first position and the second position, the valve body 230 can be switched in a sliding manner or in a rotating manner, which can achieve the opening and closing of the gas passage 2102, which is not limited by the embodiments of the present disclosure.

In the embodiments of the present disclosure, when a fire occurs, the meltable member 240 may be in a molten state when the temperature reaches the melting point. The meltable member 240 is insufficient to support the elastic force exerted on the valve body 230 by the elastic member 220. The elastic member 220 releases the stored elastic potential energy, and the valve body 230 switches from the first position to the second position driven by the elastic member 220 to encroach on the space of the gas passage 2102, so as to cut off the oxygen passage and avoid the spread of the fire caused by the continuous leakage of oxygen. Also, the accommodating cavity 2101 where the elastic member 220 is located and the gas passage 2102 are independent from each other, so that the problem of oxidation of the elastic member 220 is avoided and the durability and safety factor of the device are improved.

Alternatively, as shown in FIG. 8 and FIG. 9, the accommodating cavity 2101 has an opening. The valve body 230 is disposed at the opening of the accommodating cavity 2101, and the valve body 230 is slidably connected to the accommodating cavity 2101. When the meltable member 240 is in the non-molten state, the meltable member 240 supports the valve body 230 to be in the first position, and the gas passage 2102 is in the open state. When the meltable member 240 is in the molten state, the elastic member 220 drives the valve body 230 to slide to the second position, with the gas passage 2102 in the closed state.

Specifically, as shown in FIG. 8 and FIG. 9, in the embodiment of the present disclosure, the accommodating cavity 2101 has an opening in a U-shape, the opening of the accommodating cavity 2101 faces towards the gas passage 2102. The valve body 230 is located at least partially in the accommodating cavity 2101 and slidably connected with the accommodating cavity 2101 via the opening. The valve body 230 may be a sphere, a cylinder or a bowl-shaped structure. The valve body 230 is made of rubber and has an interference fit with the inner wall of the accommodating cavity 2101, so that the tightness of the inside of the accommodating cavity 2101 can still be ensured during sliding. Thus, the accommodating cavity 2101 and the gas passage 2102 are separated from each other, and the chemical reaction between the elastic member 220 and oxygen during long-term use may be effectively avoided.

The elastic member 220 is located in the accommodating cavity 2101. One end of the elastic member 220 abuts against the bottom of the accommodating cavity 2101 and the other end abuts against the valve body 230. When the valve body 230 is in the first position, the elastic member 220 is in a compressed state, and accumulating the elastic potential energy. The meltable member 240 is positioned on a side of the valve body 230 close to the gas passage 2102 to support the valve body 230.

The meltable member 240 may be a series of protruding structures. It may form a gap between the protruding structures and the valve body 230 when the meltable member 240 is in contact with the valve body 230, to facilitate delivery of oxygen to a patient via the gap in the absence of a fire. Certainly, the meltable member 240 may also be provided with a hollow structure or aperture structure through which oxygen is delivered to the patient in the absence of a fire.

The gas passage 2102 may include multiple sub-channels arranged in a bent manner, as shown in FIG. 8. It may also be arranged in the form of only one passage, as shown in FIG. 9. By means of one passage, the structure is simpler and the volume is smaller. Also, the resistance to oxygen generated by the inner wall of the gas passage 2102 during oxygen transmission is also smaller.

When there is no fire (the temperature is low ), the meltable member 240 is in the non-molten state and has a certain rigidity to support the valve body 230. At this time, the valve body 230 is in the first position, the gas passage 2102 is in the open state, and oxygen may normally flow in the gas passage 2102. When the valve body 230 is in the first position, the elastic force exerted by the elastic member 220 on the valve body 230 and the supporting force exerted by the meltable member 240 on the valve body 230 are in equilibrium.

When a fire occurs (the temperature is high), the meltable member 240 is in the molten state, and the meltable member 240 may not continue to support the valve body 230. The equilibrium state of the valve body 230 is broken. At this time, the valve body 230 slides relative to the accommodating cavity 2101 under the elastic force of the elastic member 220, and slides from the first position to the second position. The valve body 230 enters the gas passage 2102, and is attached to the inner wall of the gas passage 2102, encroaches on the space of the gas passage 2102 of the gas passage 2102, so that the oxygen passage is blocked, with the gas passage 2102 in the closed state. Further leakage of oxygen is prevented, further deterioration of fire is avoided, and fire prevention is achieved.

Here, the first position and the second position of the valve body 230 are positions of the valve body 230 relative to the gas passage 2102. When the valve body 230 is in the first position, the gas passage 2102 is away from the gas passage 2102, and the gas passage 2102 is in the open state. When the valve body 230 is in the second position, the valve body 230 slides toward the side close to the gas passage 2102 and blocks the gas passage 2102, and the gas passage 2102 is in the closed state.

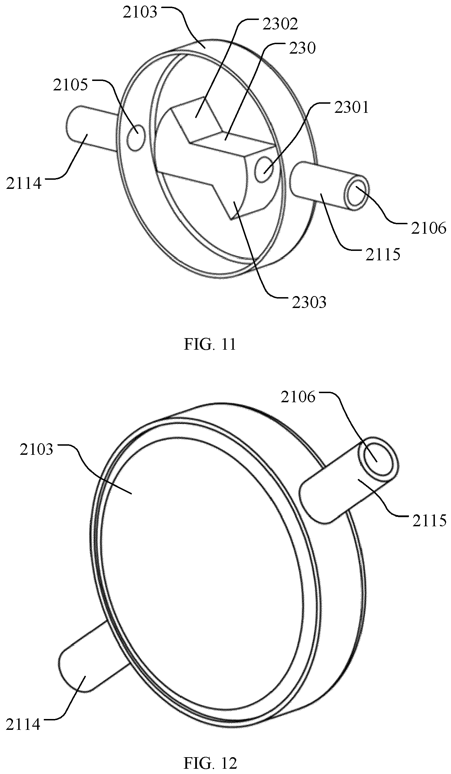

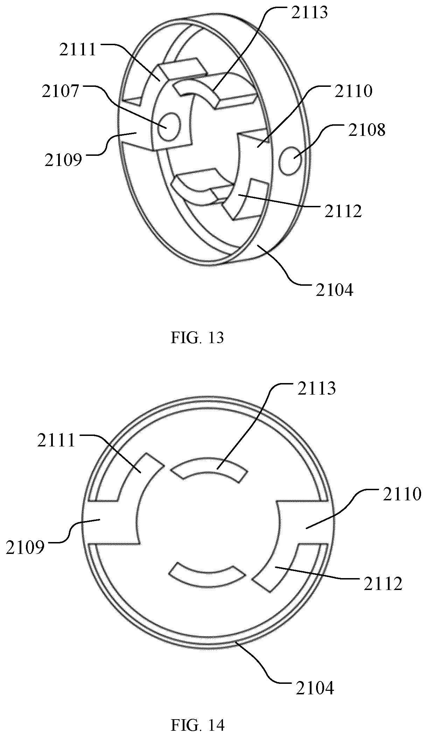

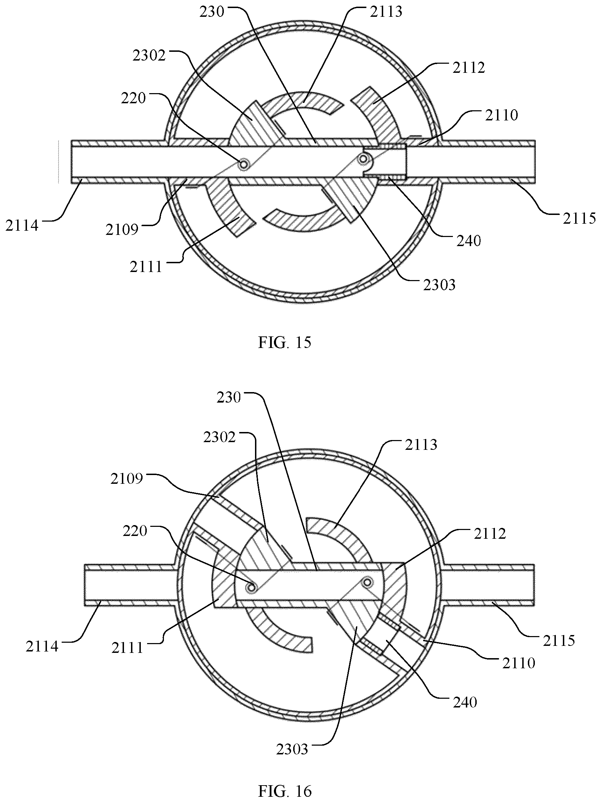

Alternatively, as shown in FIG. 10 to FIG. 16, the housing 10 includes a first housing 2103 and a second housing 2104. The first housing 2103 is rotatably connected to the second housing 2104. The first housing 2103 is provided with a first through-hole 2105 and a second through-hole 2106. The second housing 2104 is provided with a third through-hole 2107 and a fourth through-hole 2108. The valve body 230 is fixedly connected to the first housing 2103, and the valve body 230 is provided with a fifth through-hole 2301. The meltable member 240 is respectively clamped with the valve body 230 and the second housing 2104, and the meltable member 240 is provided with a sixth through-hole 2401. The elastic members 220 respectively abut against the valve body 230 and the second housing 2104. When the meltable member 240 is in the non-molten state, the meltable member 240 supports the valve body 230 to be in the first position. The first through-hole 2105, the second through-hole 2106, the third through-hole 2107, the fourth through-hole 2108, the fifth through-hole 2301 and the sixth through-hole 2401 are in communication and together form the gas passage 2102, with the gas passage 2102 in the open state. When the meltable member 240 is in the molten state, the elastic member 220 drives the first housing 2103 and the second housing 2104 to rotate relative to each other, with the valve body 230 in the second position. The first through-hole 2105, the second through-hole 2106 and the fifth through-hole 2301 are respectively attached to the wall of the second housing 2104, with the gas passage 2102 in the closed state.

Specifically, as shown in FIG. 10 to FIG. 16, in an embodiment of the present disclosure, the housing 10 includes the first housing 2103 and the second housing 2104, which may be disk-shaped, having a bottom surface and side walls. The diameter of the first housing 2103 is slightly greater than the diameter of the second housing 2104, and the first housing 2103 and the second housing 2104 are snap-fit together to form an inner space. The first housing 2103 and the second housing 2104 may also adopt a spherical structure, which is not limited by the embodiments of the present disclosure.

The first housing 2103 and the second housing 2104 are rotatably connected. In particular, a rotating shaft may be provided at the axial center of the first housing 2103 and the second housing 2104. The first housing 2103 and the second housing 2104 relatively rotate around the rotating shaft . It is also possible that the side wall of the first housing 2103 and the side wall of the second housing 2104 are attached to each other and rotate relative to each other around the attached surface. The side wall of the first housing 2103 is provided with a first through-hole 2105 and a second through-hole 2106 which are respectively used for communicating with the pipeline of the oxygen therapy instrument or the patient end. Oxygen may be input into the housing 10 from the first through-hole 2105 and then flow out through the second through-hole 2106.

The valve body 230 is fixedly connected to the first housing 2103. The valve body 230 is provided with a fifth through-hole 2301, and both ends of the fifth through-hole 2301 are opened opposite to the first through-hole 2105 and the second through-hole 2106, respectively. The side wall of the second housing 2104 is further provided with a third through-hole 2107 and a fourth through-hole 2108. The meltable member 240 has a hollow cylindrical structure, and is provided with a sixth through-hole 2401. The meltable member 240 may be disposed between the valve body 230 and the second housing 2104, and is respectively clamped with the valve body 230 and the second housing 2104. When a fire does not occur(the temperature is low), the meltable member 240 has a certain rigidity, so as to limit the relative rotation of the first housing 2103 and the second housing 2104. A quantity of the meltable members 240 may be one or two. When the quantity of the meltable members 240 is one, the meltable member 240 may be clamped with the fifth through-hole 2301 and the third through-hole 2107, respectively, and may be clamped with the fifth through-hole 2301 and the fourth through-hole 2108, respectively. When the quantity of the meltable members 240 is two, the meltable members 240 may be disposed between the fifth through-hole 2301 and the third through-hole 2107, and between the fifth through-hole 2301 and the fourth through-hole 2108.

The elastic member 220 is located in a mounting cavity formed by the first housing 2103 and the second housing 2104, and the elastic member 220 abuts against the valve body 230 and the second housing 2104, respectively, to accumulate the elastic potential energy. The elastic member 220 may be a metal torsion spring such as iron, copper, alloy, etc. a spring, etc., or a soft rubber structure such as silica gel, rubber, etc. which can store the elastic potential energy. The quantity of the elastic members 220 may be set to one or more. When the valve body 230 is in the first position, the elastic force exerted by the elastic member 220 on the second housing 2104 and the valve body 230 and the supporting force exerted by the meltable member 240 on the second housing 2104 and the valve body 230 are in equilibrium.

In the absence of a fire (the temperature is low), the meltable member 240 is in the non-molten state, has a certain rigidity, and may support the valve body 230, so that the first housing 2103 and the second housing 2104 are in a relatively stationary state. The valve body 230 is in the first position. The first through-hole 2105, the second through-hole 2106, the third through-hole 2107, the fourth through-hole 2108, the fifth through-hole 2301 and the sixth through-hole 2401 communicate with each other to form the gas passage 2102, and the gas passage 2102 is in the open state. Oxygen can enter the gas passage 2102 via the first through-hole 2105 and then flow out through the second through-hole 2106. Furthermore, the gas passage 2102 and the accommodating cavity 2101 where the elastic member 220 is located are independent from each other, thus avoiding the problem of oxidation of the elastic member 220 and improving the durability and safety factor of the device.

The first through-hole 2105, the second through-hole 2106, the third through-hole 2107, the fourth through-hole 2108, the fifth through-hole 2301 and the sixth through-hole 2401 may be located on the same straight line to form a linear gas passage 2102, or may be not located on the same straight line but bent at a certain angle, as long as the normal transmission of oxygen may be achieved.

When a fire occurs (the temperature is relatively high), the meltable member 240 is in the molten state, and the meltable member 240 is insufficient to support the rotational torque exerted by the elastic member 220 between the first housing 2103 and the second housing 2104. The equilibrium state between the first housing 2103 and the second housing 2104 is broken. At this time, the elastic member 220 releases the stored elastic potential energy. The valve body 230 and the first housing 2103 rotate relative to the second housing 2104 under the elastic force of the elastic member 220, and the valve body 230 rotates from the first position to the second position. The fifth through-hole 2301 of the valve body 230 is offset from the third through-hole 2107 and the fourth through-hole 2108 of the second housing 2104, and the first through-hole 2105 and the second through-hole 2106 are also offset from the third through-hole 2107 and the fourth through-hole 2108, respectively. After the first housing 2103 and the second housing 2104 are relatively rotated, the first through-hole 2105, the second through-hole 2106 and the fifth through-hole 2301 are respectively attached to the housing wall of the second housing 2104, so that the oxygen passage is blocked and the gas passage 2102 is in the closed state. Further leakage of oxygen is prevented, further deterioration of fire is avoided, and fire prevention is achieved.

Here, the first position and the second position of the valve body 230 are positions of the valve body 230 relative to the second housing 2104. When the valve body 230 is in the first position, the first through-hole 2105, the second through-hole 2106, the third through-hole 2107, the fourth through-hole 2108, the fifth through-hole 2301 and the sixth through-hole 2401 communicate with each other, and the gas passage 2102 is in the open state. The first housing 2103 and the second housing 2104 are relatively rotated. When the valve body 230 is rotated to the second position, the gas passage 2102 is divided into a plurality of segments and is blocked by the housing wall of the second housing 2104, and the gas passage 2102 is in a closed state.

Optionally, as shown in FIG. 10 to FIG. 16, a first protrusion 2109 and a second protrusion 2110 are disposed in the second housing 2104. The third through-hole 2107 is disposed on the first protrusion 2109, and the fourth through-hole 2108 is disposed on the second protrusion 2110. The meltable member 240 is at least partially embedded in the third through-hole 2107 or the fourth through-hole 2108.

Specifically, as shown in FIG. 10 to FIG. 16, in the second housing 2104, a first protrusion 2109 and a second protrusion 2110 are extended from the side wall, and the first protrusion 2109 and the second protrusion 2110 are oppositely disposed. The first protrusion 2109 and the second protrusion 2110 and the second housing 2104 may be formed by an integral molding process. The third through-hole 2107 is disposed in the first protrusion 2109, and the fourth through-hole 2108 is disposed in the second protrusion 2110. Two sections of the gas passages 2102 are formed in the second housing 2104. When the meltable member 240 is clamped with the valve body 230 and the second housing 2104, the meltable member 240 is at least partially nested into the third through-hole 2107 or the fourth through-hole 2108, and the specific insertion position may be selected according to the quantity of the meltable members 240 and the installation position.

The residue of the meltable member 240 after being heated and melted is stored in the third through-hole 2107 or the fourth through-hole 2108, respectively, and does not enter the oxygen supply pipeline, so that the patient does not have a risk of inhaling foreign matter.

Optionally, as shown in FIG. 8 to FIG. 16, the first protrusion 2109 is provided with a first extension 2111, and the second protrusion 2110 is provided with a second extension 2112. When the valve body 230 is in the second position, both ends of the fifth through-hole 2301 are attached to the first extension 2111 and the second extension 2112, respectively.

Specifically, as shown in FIG. 8 to FIG. 16, the first extension 2111 is provided on a side of the first protrusion 2109 close to the valve body 230, and the second extension 2112 is provided on a side of the second protrusion 2110 close to the valve body 230. The first extension 2111 and the first protrusion 2109, and the second extension 2112 and the second protrusion 2110 may be manufactured using an integral molding process, respectively. The first extension 2111 and the second extension 2112 may have an arc-shaped structure which is matched with the shape of the end of the valve body 230.

When the valve body 230 is in the second position, both ends of the fifth through-hole 2301 are attached to the first extension 2111 and the second extension 2112, respectively, to achieve sealing, so as to prevent oxygen remaining in the valve body 230 from leaking out into the accommodating cavity 2101 and also improve the fire prevention effect.

Alternatively, as shown in FIG. 8 to FIG. 16, one end of the valve body 230 is provided with a third extension 2302, and the other end of the valve body 230 is provided with a fourth extension 2303. When the valve body 230 is in the second position, the third through-hole 2107 is attached to the third extension 2302, and the fourth through-hole 2108 is attached to the fourth extension 2303.

Specifically, as shown in FIG. 8 to FIG. 16, a fifth through-hole 2301 is formed on the valve body 230. The third extension 2302 and the fourth extension 2303 are respectively extended at both ends of the fifth through-hole 2301. The third extension 2302 and the fourth extension 2303 and the valve body 230 may be made by an integral molding process. The third extension 2302 and the fourth extension 2303 may have an arc-shaped structure which is matched with the shape of the ends of the third through-hole 2107 and the fourth through-hole 2108.

When the valve body 230 is in the second position, the third through-hole 2107 is attached to the third extension 2302, and the third extension 2302 seals one end of the third through-hole 2107. The fourth through-hole 2108 is attached to the fourth extension 2303, and the fourth extension 2303 seals one end of the fourth through-hole 2108, so that oxygen cannot pass through and the fire prevention effect is more remarkable.

Alternatively, as shown in FIG. 8 to FIG. 16, the second housing 2104 is provided with at least one limiting portion 2113 located on the movement path of the valve body 230. When the valve body 230 is in the second position, the valve body 230 abuts against the limiting portion 2113.

Specifically, as shown in FIG. 8 to FIG. 16, the limiting portion 2113 is disposed in the second housing 2104, and the limiting portion 2113 may have a plate-like or cylindrical structure. The limiting portion 2113 and the second housing 2104 may be formed by an integral molding process. The limiting portion 2113 is located on the movement path of the valve body 230, that is, the valve body 230 comes into contact with the limiting portion 2113 when rotating with respect to the second housing 2104. The quantity of the limiting portions 2113 may be one or two. When the quantity of the limiting portions 2113 is two, the two limiting portions 2113 may be respectively located at both sides of the valve body 230 with a certain interval from the valve body 230.

When a fire occurs, at least a portion of the meltable member 240 is heated and melted, and it is insufficient to support the rotational torque exerted by the elastic member 220 between the first housing 2103 and the second housing 2104. At this time, the elastic member 220 releases the stored elastic potential energy and causes relative rotation between the first housing 2103 and the second housing 2104, until the valve body 230 comes into contact with the limiting portion 2113 of the second housing 2104 to stop the rotation. At this time, a stable assembly is formed between the first housing 2103 and the second housing 2104 by the cooperation of the elastic member 220 and the limiting portion 2113.

Optionally, as shown in FIG. 8 to FIG. 16, the first housing 2103 is provided with a first connecting portion 2114 and a second connecting portion 2115. The first through-hole 2105 is disposed on the first connecting portion 2114, and the second through-hole 2106 is disposed on the second connecting portion 2115. The first connecting portion 2114 and the second connecting portion 2115 are respectively used for communicating with the pipeline of the oxygen therapy instrument or the patient end.

Specifically, as shown in FIG. 10 to FIG. 15, the pipeline of the oxygen therapy instrument or the patient end is communicated with the fire protection device through the first through-hole 2105 and the second through-hole 2106. In order to improve the convenience of the connection of the pipeline, the first connecting portion 2114 and the second connecting portion 2115 are disposed on the first housing 2103. The first connecting portion 2114 and the second connecting portion 2115 extend outwards in the radial direction of the first housing 2103. The first through-hole 2105 is disposed on the first connecting portion 2114, and the second through-hole 2106 is disposed on the second connecting portion 2115. When the pipeline of the oxygen therapy instrument or the patient end is installed, the pipeline may be directly sleeved or embedded on the first connecting portion 2114 and the second connecting portion 2115 without occupying the inner space of the housing 10.

Optionally, as shown in FIG. 8 and FIG. 9, the gas passage includes a first pipeline interface, a second pipeline interface, and a communicating portion. The communicating portion has an inner cavity. The first pipeline interface and the second pipeline interface are respectively located at both ends of the communicating portion, and respectively communicate with the inner cavity to form the gas passage. The first pipeline interface and the second pipeline interface are symmetrically arranged.

Specifically, as shown in FIG. 8 and FIG. 9, the gas passage is formed by the first pipeline interface, the second pipeline interface, and the communicating portion together. The first pipeline interface, the second pipeline interface and the communicating portion may have an integral structure, or may be assembled after being made in separate bodies. The first pipeline interface and the second pipeline interface are respectively used for communicating with the pipeline of the oxygen therapy instrument or the patient end. The pipeline of the oxygen therapy instrument or the patient end can be assembled with the first pipeline interface or the second pipeline interface by means of threaded connection or interference fit. The communicating portion has a hollow cylindrical structure with an inner cavity. The first pipeline interface and the second pipeline interface are respectively located at both ends of the communicating portion. The inner cavity of the communicating portion communicates with the first pipeline interface and the second pipeline interface respectively to form a complete gas passage.

When the first pipeline interface and the second pipeline interface are assembled with the pipeline, one of the pipeline interfaces communicates with the patient end, and the other pipeline interface communicates with the oxygen therapy instrument end. Since the first pipeline interface and the second pipeline interface are symmetrically arranged, both the first pipeline interface and the second pipeline interface may be adapted to the oxygen therapy instrument end or the patient end. There is no situation of reverse installation of the fire protection device, and the assembly efficiency is greatly improved.

Optionally, as shown in FIG. 8 and FIG. 9, the meltable member is located in the inner cavity, and the meltable member is in clearance fit with a cavity wall of the inner cavity.

Specifically, as shown in FIG. 8 and FIG. 9, the meltable member is disposed in the inner cavity of the communicating portion. The blocking of the gas passage may be achieved by providing only one meltable member without providing the meltable member at both the oxygen therapy instrument end and the patient end, which greatly reduces the production cost.

Embodiments of the present disclosure also disclose a second type of an oxygen therapy instrument including the second type of the fire protection device.

Specifically, in a medical or home setting, when the patient is unable to meet his/her needs relying on his/her own inhaled oxygen, it is often necessary to supply oxygen through an external instrument. The method of delivering oxygen to a patient by means of an external instrument, either in an invasive manner or a non-invasive manner, is known as oxygen therapy. Instruments used to supply oxygen to a patient are collectively referred to as oxygen therapy instruments. The oxygen therapy instrument is generally composed of three parts: an oxygen generating device (e.g., an oxygen tank, oxygen generator, etc.), an interface for delivering oxygen to a patient (e.g., a nasal oxygen tube, a mask, etc.), and a pipeline connecting the generating device and the patient interface. The fire protection device is disposed in series in the pipeline between the oxygen generating device and the patient interface. The fire protection device has a gas passage 2102 therein. The gas passage 2102 is used for communicating with the pipeline of the oxygen therapy instrument. The fire protection device may control the opening or closing of the pipeline of the oxygen therapy instrument by controlling the opening or closing of the internal gas passage 2102.

In the embodiments of the present disclosure, the second type of the fire protection device is connected in series in the pipeline of the second type of the oxygen therapy instrument. When a fire occurs, the meltable member 240 will be in a molten state as the temperature reaches the melting point. The meltable member 240 is insufficient to support the elastic force exerted on the valve body 230 by the elastic member 220. The elastic member 220 releases the stored elastic potential energy. The valve body 230 is switched from the first position to the second position under the drive of the elastic member 220 to encroach on the space of the gas passage 2102, thereby cutting off the oxygen passage and avoiding the spread of the fire caused by the continuous leakage of oxygen. Furthermore, the accommodating cavity 2101 where the elastic member 220 is located and the gas passage 2102 are independent from each other, so that the problem of oxidation of the elastic member 220 is avoided, and the durability and safety factor of the oxygen therapy instrument are improved.

Embodiments of the present disclosure also disclose a second type of a ventilation treatment system including the second type of the oxygen therapy instrument.

Specifically, the second type of the ventilation treatment system includes a control device and the second type of the oxygen therapy instrument. The control device is used for controlling the oxygen supply amount, operation time, etc. of the oxygen therapy instrument. The control device may be an electronic device or a component in the electronic device, such as an integrated circuit or a chip. The electronic device may be a terminal or may be a device other than a terminal. Illustratively, the electronic device may be a cell phone, a tablet computer, a notebook computer, a palmtop computer, a mobile Internet appliance, a robot, a wearable device, etc. and embodiments of the present disclosure are not particularly limited.

In the embodiments of the present disclosure, the second type of the ventilation treatment system includes the second type of the oxygen therapy instrument. The second type of the fire protection device is connected in series in the pipeline of the second type of the oxygen therapy instrument. When a fire occurs, the meltable member 240 may be in a molten state when the temperature reaches the melting point. The meltable member 240 is insufficient to support the elastic force exerted on the valve body 230 by the elastic member 220. The elastic member 220 releases the stored elastic potential energy, and the valve body 230 switches from the first position to the second position driven by the elastic member 220 to encroach on the space of the gas passage 2102, so as to cut off the oxygen passage and avoid the spread of the fire caused by the continuous leakage of oxygen. Also, the accommodating cavity 2101 where the elastic member 220 is located and the gas passage 2102 are independent from each other, so that the problem of oxidation of the elastic member 220 is avoided and the durability and safety factor of the system are improved.

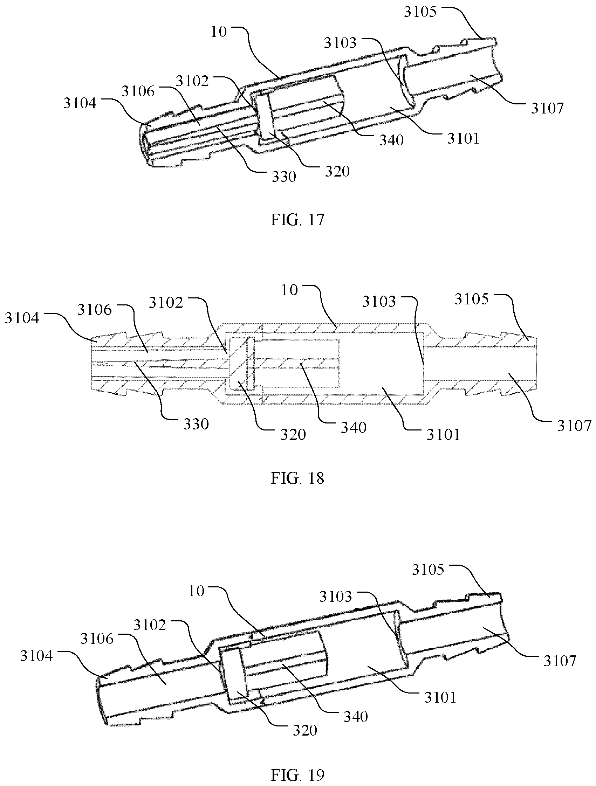

Referring to FIG. 17 to FIG. 20, a third type of a fire protection device is disclosed according to an embodiment of the present disclosure, the third type of the fire protection device including a housing 10, a first elastic valve body 320, and a first meltable member 330. An accommodating cavity 3101 is provided in the housing 10. A first opening 3102 and a second opening 3103 are formed on both sides of the accommodating cavity 3101. The first opening 3102 and the second opening 3103 are used for communicating with a pipeline of an oxygen therapy instrument or a patient end. The first elastic valve body 320 is positioned in the accommodating cavity 3101, and the first elastic valve body 320 is arranged opposite to the first opening 3102. Herein, the first elastic valve body 320 has oxidation resistance. The first meltable member 330 is located in and connected to the housing 10, and the first meltable member 330 abuts against a side of the first elastic valve body 320 close to the first opening 3102. When the first meltable member 330 is in a non-molten state, the first meltable member 330 compresses the first elastic valve body 320 with a gap between the first opening 3102 and the first elastic valve body 320 for gas flow. When the first meltable member 330 is in a molten state, the first elastic valve body 320 releases at least part of the elastic potential energy, and the first elastic valve body 320 abuts against a first end surface of the accommodating cavity 3101, so that the first opening 3102 in a closed state, where the first opening 3102 is disposed at the first end surface.

Specifically, as shown in FIG. 17 to FIG. 20, the third type of the fire protection device includes the housing 10, the first elastic valve body 320, and the first meltable member 330. As a main frame of the fire protection device, the housing 10 may be made of plastic or the like, which is not easily chemically reacted with oxygen. An accommodating cavity 3101 is disposed in the housing 10. The first opening 3102 and the second opening 3103 are formed on both sides of the accommodating cavity 3101. The first opening 3102 and the second opening 3103 may be located on two opposite sides of the accommodating cavity 3101. The axes of the first opening 3102 and the second opening 3103 are collinear, and the first opening 3102 and the second opening 3103 may also be disposed at an angle on the accommodating cavity 3101. The first opening 3102 and the second opening 3103 are used for communicating with the pipeline of the oxygen therapy instrument or the patient end. For example, the first opening 3102 communicates with the pipeline of the oxygen therapy instrument end. The second opening 3103 communicates with the patient end. Oxygen enters the accommodating cavity 3101 from the first opening 3102 and is transmitted to the patient end via the second opening 3103. The second opening 3103 may also be in communication with the pipeline at the oxygen therapy instrument end, and the first opening 3102 may be in communication with the pipeline at the patient end, which is not limited by the embodiments of the present disclosure.

The first elastic valve body 320 is positioned in the accommodating cavity 3101, and the first elastic valve body 320 is disposed opposite to the first opening 3102. A partition or positioning structure may be provided in the accommodating cavity 3101 to achieve positioning and fixing on the first elastic valve body 320. For example, a protrusion is provided in the accommodating cavity 3101, and a groove or step is provided in cooperation with the first elastic valve body 320 to achieve positioning and fixing. The opening or closing of the oxygen passage is accomplished by the cooperation of the first elastic valve body 320 with the first opening 3102. The first elastic valve body 320 has oxidation resistance characteristics. The first elastic valve body 320 may be made of a material that can be compressed and stores the elastic potential energy, such as silica gel or rubber, and is not prone to oxidation and rust when in contact with oxygen for a long time, so as to avoid affecting the therapeutic effect, and also improve the stability and durability of the fire protection device.

The first meltable member 330 is disposed within and connected to the housing 10, and the housing 10 provides a supporting and fixing function for the first meltable member 330. The side of the first meltable member 330 away from the first opening 3102 may be adhered or clamped to the housing 10. The first meltable member 330 abuts against a side of the first elastic valve body 320 close to the first opening 3102, to support the first elastic valve body 320. The first meltable member 330 is typically made of a lower-melting-point material, such as PP, PVC, or the like.

When a fire does not occur (the temperature is low), the first meltable member 330 is in a non-molten state, and the first meltable member 330 compresses the first elastic valve body 320. The thickness of the first elastic valve body 320 is reduced, and the elastic potential energy is accumulated. A gap for gas flow is provided between the first elastic valve body 320 and the first opening 3102, oxygen may normally flow through the gap. The direction of the thickness of the first elastic valve body 320 is the long axis direction of the fire protection device.

When a fire occurs(the temperature is high), the first meltable member 330 is in a molten state. The first meltable member 330 may not continue to support the first elastic valve body 320, and the equilibrium state of the first elastic valve body 320 is broken. At this time, at least part of the elastic potential energy is released by the first elastic valve body 320, the first elastic valve body 320 is increased in thickness and abuts against the first end surface of the accommodating cavity 3101. Here, the first opening 3102 is located on the first end surface. The first elastic valve body 320 covers the first opening 3102, so that the first opening 3102 is in a closed state. Oxygen may not pass through the first opening 3102, and the oxygen path is blocked.

In an embodiment of the present disclosure, when the first meltable member is in the non-molten state 330, the first meltable member 330 compresses the first elastic valve body 320, and the thickness of the first elastic valve body 320 decreases, with a gap between the first opening 3102 and the first elastic valve body 320 for gas flow. In the event of a fire, when the temperature of the meltable member reaches the melting point, the meltable member will be in a molten state. The first elastic valve body 320 releases at least part of the elastic potential energy, the first elastic valve body 320 abuts against the first end surface of the accommodating cavity 3101, so that the first opening 3102 is in a closed state, thereby cutting off the oxygen passage and preventing the oxygen from continuously leaking out to cause the fire to spread. Since the first elastic valve body 320 has oxidation resistance characteristics, the problem of oxidation of the first elastic valve body 320 is avoided, and the durability and safety factor of the device are improved.

Optionally, as shown in FIG. 17, FIG. 18 and FIG. 21, the housing 10 includes a first connecting portion 3104 and a second connecting portion 3105. The first connecting portion 3104 has a first through-hole 3106 communicating with the first opening 3102. The second connecting portion 3105 has a second through-hole 3107 communicating with the second opening 3103. The first meltable member 330 is located in the first through-hole 3106. One end of the first meltable member 330 extends to form at least two reinforcing ribs 3108, and the reinforcing ribs 3108 are connected to the hole wall of the first through-hole 3106. The other end of the first meltable member 330 abuts against a side of the first elastic valve body 320 close to the first opening 3102.

Specifically, as shown in FIG. 17, FIG. 18 and FIG. 21, the housing 10 includes a first connecting portion 3104 and a second connecting portion 3105. The first connecting portion 3104 and the second connecting portion 3105 are extension structures on the housing 10. The first connecting portion 3104 and the second connecting portion 3105 and the main body of the housing 10 may be made by an integral molding process, or may be assembled by bonding or welding after being made separately. The first connecting portion 3104 has the first through-hole 3106 communicating with the first opening 3102. The second connecting portion 3105 has the second through-hole 3107 communicating with the second opening 3103. The first connecting portion 3104 and the second connecting portion 3105 are used for communicating with the pipeline of the oxygen therapy instrument or the patient end. For example, the first connecting portion 3104 communicates with the pipeline of the oxygen therapy instrument end. The second connecting portion 3105 communicates with the pipeline of the patient end. Oxygen enters the accommodating cavity 3101 from the first connecting portion 3104 and is transmitted to the patient end via the second connecting portion 3105. The second connecting portion 3105 may also be in communication with the pipeline of the oxygen therapy instrument end and the first connecting portion 3104 may be in communication with the pipeline of the patient end, which is not limited by the embodiments of the present disclosure.

In the embodiment of the present disclosure, one end of the first meltable member 330 extends to form at least two reinforcing ribs 3108. The first meltable member 330 is located in the first through-hole 3106. The first meltable member 330 adopts a rod-shaped structure. The first meltable member 330 is made of the same material as the reinforcing rib 3108. The reinforcing rib 3108 is connected to the hole wall of the first through-hole 3106, so as to achieve the mounting and fixing of the first meltable member 330. The connecting manner of the reinforcing rib 3108 and the wall of the first through-hole 3106 may be welding or bonding, etc. The other end of the first meltable member 330 abuts against a side of the first elastic valve body 320 close to the first opening 3102, so that the first elastic valve body 320 can be compressed to have a reduced thickness, ensuring a gap between the first elastic valve body 320 and the first opening 3102 for gas flow. It facilitates oxygen transmitted to the patient through the gap in the absence of a fire.

In the event of a fire (the temperature is high), the first meltable member 330 and the reinforcing rib 3108 are in a molten state. The first meltable member 330 may not continue to support the first elastic valve body 320, and the equilibrium state of the first elastic valve body 320 is broken. At this time, at least part of the elastic potential energy is released by the first elastic valve body 320, the first elastic valve body 320 increases in thickness and abuts against the first end surface of the accommodating cavity 3101, so that the first opening 3102 is in a closed state, oxygen cannot pass through the first opening 3102, and the oxygen gas path is blocked.

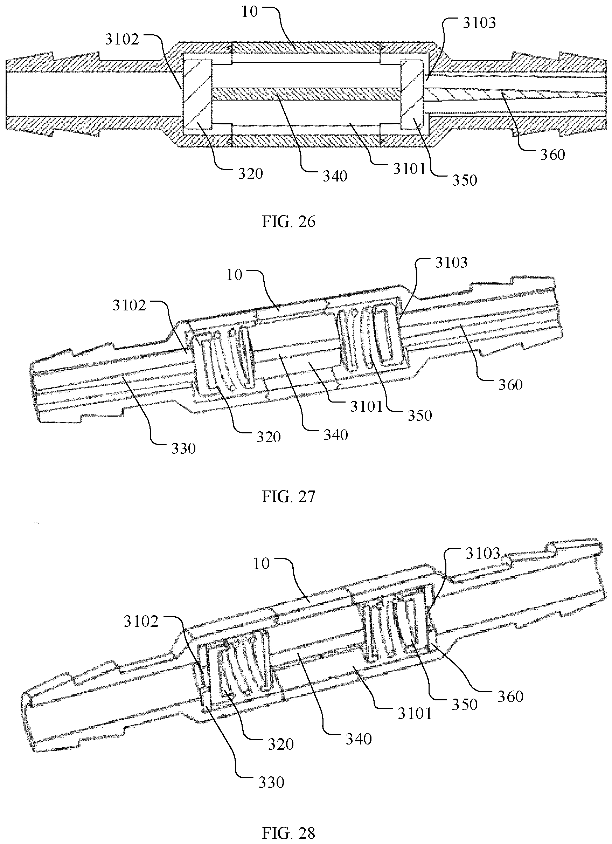

Optionally, as shown in FIG. 28, the first meltable member 330 is located between the first elastic valve body 320 and the first end surface, the first meltable member 330 abuts against a side of the first elastic valve body 320 close to the first opening 3102.

Specifically, as shown in FIG. 28, in an embodiment of the present disclosure, the first meltable member 330 is located between the first elastic valve body 320 and the first end surface. One end of the first meltable member 330 may be connected to a cavity wall or the first end surface of the accommodating cavity 3101, and the specific connection may be adhesive or clamping. The other end of the first meltable member 330 abuts against the side of the first elastic valve body 320 close to the first opening 3102. The first meltable member 330 remains flush with the first opening 3102 and does not shield the first opening 3102 to avoid affecting the oxygen transmission rate.

When a fire does not occur (the temperature is low), the first meltable member 330 is in a non-molten state, and the first meltable member 330 compresses the first elastic valve body 320. The thickness of the first elastic valve body 320 is reduced, and the elastic potential energy is accumulated. A gap for gas flow is provided between the first elastic valve body 320 and the first opening 3102, oxygen may normally circulate through the gap.

When a fire occurs (the temperature is high), the first meltable member 330 is in a molten state. The first meltable member 330 may not continue to support the first elastic valve body 320, and the equilibrium state of the first elastic valve body 320 is broken. At this time, at least part of the elastic potential energy is released by the first elastic valve body 320. The first elastic valve body 320 increases in thickness and abuts against the first end surface, so that the first opening 3102 is in a closed state. Oxygen cannot pass through the first opening 3102, and the oxygen gas path is blocked.

Optionally, as shown in FIG. 28, a quantity of the first meltable members 330 is two, and two of the first meltable members 30 are symmetrically disposed on the first end surface.

Specifically, as shown in FIG. 28, the quantity of the first meltable members 330 is two, and the two first meltable members 330 are symmetrically disposed on the first end surface of the accommodating cavity 3101. That is, on both sides of the axis of the first opening 3102, the two first meltable members 330 are symmetrically arranged, so as to achieve stable support for the first elastic valve body 320, avoid the problem of deflection of the first elastic valve body 320, and ensure the sealing effect between the first elastic valve body 320 and the first opening 3102.

Optionally, as shown in FIG. 17 to FIG. 20, the fire protection device further includes a support 340. The support 340 is located in the accommodating cavity 3101 and is connected to a cavity wall of the accommodating cavity 3101. The support 340 abuts against a side of the first elastic valve body 320 away from the first opening 3102.

Specifically, as shown in FIG. 17 to FIG. 20, the first elastic valve body 320 is positioned in the accommodating cavity 3101. A partition or positioning structure may be disposed in the accommodating cavity 3101 to position and fix the first elastic valve body 320. In the embodiment of the present disclosure, the first elastic valve body 320 may be positionally fixed by the support 340, which may be in the shape of a rod, a plate, etc. and within the accommodating cavity 3101. The support 340 is connected to the cavity wall of the accommodating cavity 3101, and the assembly can be performed by means of bonding, welding, etc. The support 340 abuts against the side of the first elastic valve body 320 away from the first opening 3102. The first meltable member 330 abuts against the side of the first elastic valve body 320 close to the first opening 3102. Under the co-pressing of the support 340 and the first meltable member 330, the first elastic valve body 320 is in a compressed state and has a reduced thickness, thereby forming a gap for gas flow with the first opening 3102.

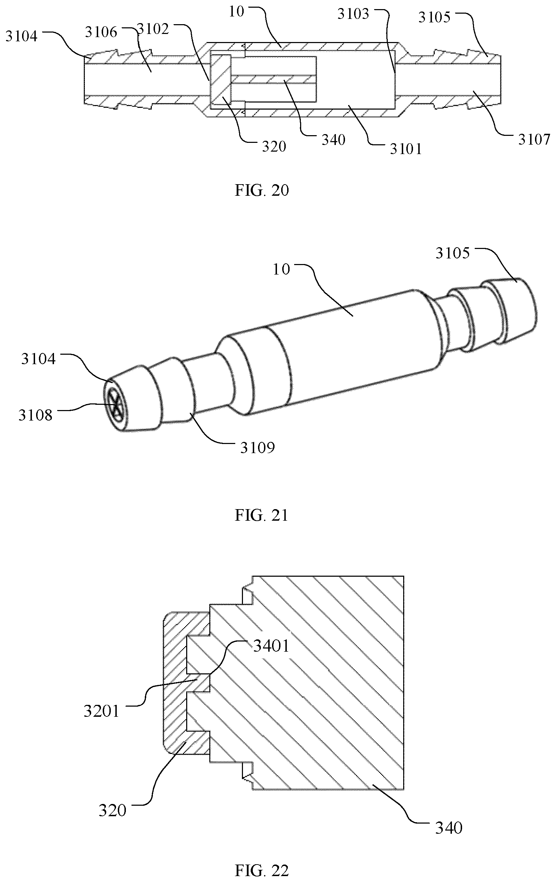

Optionally, as shown in FIG. 22, a first positioning portion 3401 is disposed at an end of the support 340 close to the first elastic valve body 320, and a second positioning portion 3201 is disposed at a side of the first elastic valve body 320 away from the first opening 3102, and the first positioning portion 3401 and the second positioning portion 3201 are in positioning fit.

Specifically, as shown in FIG. 22, the support 340 abuts against a side of the first elastic valve body 320 away from the first opening 3102. In order to ensure the stability of the assembly of the support 340 and the first elastic valve body 320, and avoid the offset phenomenon between the support 340 and the first elastic valve body 320, the first positioning portion 3401 is disposed at an end of the support 340 close to the first elastic valve body 320, and the second positioning portion 3201 is disposed at the side of the first elastic valve body 320 away from the first opening 3102. The stable assembly of the support 340 and the first elastic valve body 320 is achieved by the positioning fit of the first positioning portion 3401 and the second positioning portion 3201.

The first positioning portion 3401 may be a boss. Correspondingly, the second positioning portion 3201 may be a groove which matches with the shape of the boss. Likewise, the first positioning portion 3401 may be a groove and the second positioning portion 3201 may be a boss. The quantity of bosses and grooves may be selected according to the size of the support 340 and the first elastic valve body 320, and the embodiments of the present disclosure are not limited thereto.

Optionally, as shown in FIG. 21, the outer side wall of the first connecting portion 3104 and/or the outer side wall of the second connecting portion 3105 is provided with at least one clamping portion 3109 for being clamped with a pipeline of the oxygen therapy instrument or the patient end.

Specifically, as shown in FIG. 21, the first connecting portion 3104 and the second connecting portion 3105 are used for communicating with the pipeline of the oxygen therapy instrument or the patient end. The pipeline of the oxygen therapy instrument is generally a catheter which is sleeved outside the first connecting portion 3104 and the second connecting portion 3105. In order to ensure the stability of the connection between the catheter and the first connecting portion 3104 and the second connecting portion 3105, at least one clamping portion 3109 is disposed on the outer side wall of the first connecting portion 3104 and/or the outer side wall of the second connecting portion 3105, and the shape of the clamping portion 3109 is flared. When the catheter is sleeved on the outer side wall of the first connecting portion 3104 and/or the outer side wall of the second connecting portion 3105, the clamping portion 3109 is clamped with the catheter. The air tightness between the catheter and the first connecting portion 3104 and/or the second connecting portion 3105 is ensured, thus avoiding the problem of oxygen leakage. The quantity of the clamping portions 3109 may be selected according to the size of the first connecting portion 3104 and the second connecting portion 3105.

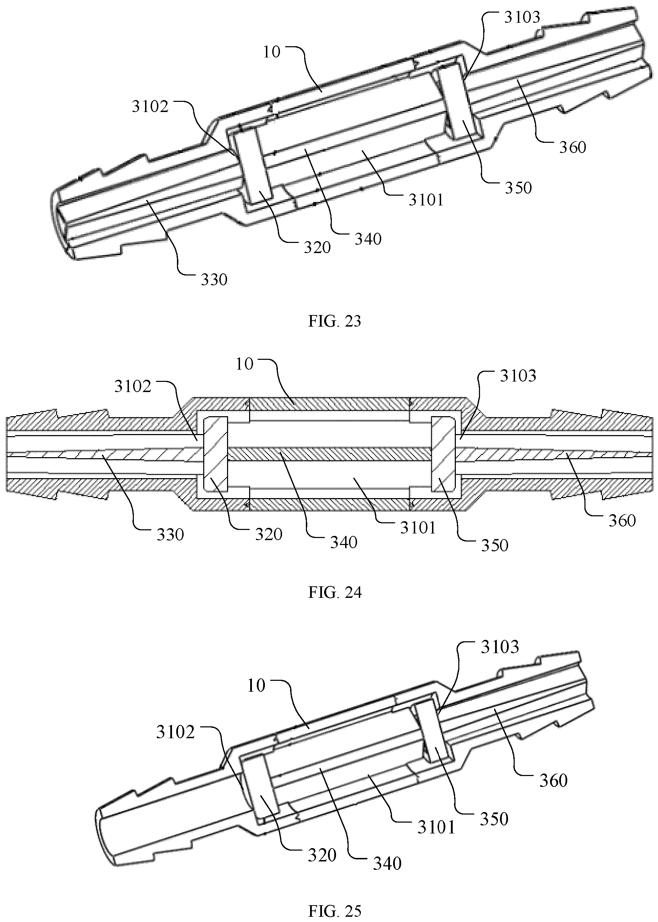

Optionally, as shown in FIG. 23 to FIG. 28, the fire protection device further includes a second elastic valve body 350 and a second meltable member 360. The second elastic valve body 350 is arranged opposite to the second opening 3103. The second meltable member 360 is located in the housing 10 and is connected to the housing 10. The second meltable member 360 abuts against a side of the second elastic valve body 350 close to the second opening 3103, where the second elastic valve body 350 has oxidation resistance characteristics. The support 340 is located between the first elastic valve body 320 and the second elastic valve body 350, and respectively abuts against the first elastic valve body 320 and the second elastic valve body 350. When the second meltable member 360 is in a non-molten state, the second meltable member 360 compresses the second elastic valve body 350, with a gap between the second opening 3103 and the second elastic valve body 350 for gas flow. When the second meltable member 360 is in the molten state, the second elastic valve body 350 releases at least part of the elastic potential energy, and the second elastic valve body 350 abuts against a second end surface of the accommodating cavity 3101, so that the second opening 3103 in a closed state, where the second opening is disposed at the second end surface.

Specifically, as shown in FIG. 23 to FIG. 28, in the embodiment of the present disclosure, not only the first elastic valve body 320 is disposed at the first opening 3102, but also the second elastic valve body 350 is disposed at the second opening 3103. The fire protection device has double protection. The second elastic valve body 350 is positioned in the accommodating cavity 3101, the second elastic valve body 350 is disposed opposite to the second opening 3103. A partition or positioning structure may be disposed in the accommodating cavity 3101 to position and fix the second elastic valve body 350. The opening or closing of the oxygen passage is accomplished by the cooperation of the second elastic valve body 350 with the second opening 3103. The second elastic valve body 350 has oxidation resistance characteristics. The second elastic valve body 350 may be made of a material that can be compressed and store the elastic potential energy, such as silica gel or rubber, and is not prone to oxidation and rust when in contact with oxygen for a long time, so as to avoid affecting the therapeutic effect, and also improve the stability and durability of the fire protection device. For example, as shown in FIG. 27 to FIG. 28, the first elastic valve body 320 and the second elastic valve body 350 each employ a silicone spring having a helical appearance of a conventional metal spring for being compressed and releasing the elastic potential energy, which has strong oxidation resistance characteristics compared to the conventional metal spring.

The second meltable member 360 is disposed within and connected to the housing 10, and the housing 10 provides a supporting and fixing function for the second meltable member 360. The second meltable member 360 may be adhered or clamped to the housing 10. The second meltable member 360 abuts against a side of the second elastic valve body 350 close to the second opening 3103 to support the second elastic valve body 350. The second meltable member 360 is typically made of a lower-melting-point material, such as PP, PVC, or the like. The supporter 340 is located between the first elastic valve body 320 and the second elastic valve body 350, and abuts against the first elastic valve body 320 and the second elastic valve body 350, respectively.

The second meltable member 360 may be positioned within the second through-hole 3107 with one end of the second meltable member 360 extending to form at least two reinforcing ribs 3108. The second meltable member 360 adopts a rod-shaped structure. The reinforcing rib 108 is connected to the hole wall of the second through-hole 3107, so as to achieve the mounting and fixing of the second meltable member 360. The connecting manner of the reinforcing rib 3108 and the hole wall of the second through-hole 3107 may be welding or bonding, etc. The other end of the second meltable member 360 abuts against a side of the second elastic valve body 350 close to the second opening 3103, so that the second elastic valve body 350 may be compressed to reduce its thickness, thus ensuring a gap between the second elastic valve body 350 and the second opening 3103 for gas flow.

The second meltable member 360 may also be located between the second elastic valve body 350 and the second end surface. One end of the second meltable member 360 may be connected to a cavity wall or the second end surface of the accommodating cavity 3101, and the specific connection may be adhesive or clamping. The other end of the second meltable member 360 abuts against a side of the second elastic valve body 350 close to the second opening 3103. The second meltable member 360 remains flush with the second opening 3103 and does not shield the second opening 3103 to avoid affecting the oxygen transmission rate.

When a fire does not occur (the temperature is low), the first meltable member 330 and the second meltable member 360 are in a non-molten state. The first meltable member 330 compresses the first elastic valve body 320, and the second meltable member 360 compresses the second elastic valve body 350. The first elastic valve body 320 and the second elastic valve body 350 both have a reduced thickness and accumulate the elastic potential energy, with a gap provided between the first elastic valve body 320 and the first opening 3102 for gas flow. The gap is also provided between the second elastic valve body 350 and the second opening 3103 for gas flow, and oxygen may normally circulate through the gap.

When a fire occurs (the temperature is relatively high), the first meltable member 330 and the second meltable member 360 are in a molten state. The first meltable member 330 may not continue to support the first elastic valve body 320. The second meltable member 360 may not continue to support the second elastic valve body 350, and the equilibrium state of the first elastic valve body 320 and the second elastic valve is broken. At this time, the first elastic valve body 320 and the second elastic valve release at least part of the elastic potential energy, and the first elastic valve body 320 and the second elastic valve increase in thickness and respectively abut against the first end surface and the second end surface. Thus, the first opening 3102 and the second opening 3103 are in the closed state, oxygen gas cannot pass through the first opening 3102 and the second opening 3103, and the oxygen gas path is blocked.

By providing an elastic valve body and a meltable member at both the first opening 3102 and the second opening 3103, at least one of the first meltable member 330 and the second meltable member 360 melts in the event of a fire (when the temperature is high), i.e., the air passage is blocked to prevent oxygen leakage, thereby improving the reliability of the fire protection device. When both the first meltable member 330 and the second meltable member 360 melts, both the first opening 3102 and the second opening 3103 are in the closed state, thus providing a double sealing safeguard, and greatly improving the safety factor of the fire protection device.

Embodiments of the present disclosure also disclose a third type of an oxygen therapy instrument, including the third type of the fire protection device.

Specifically, in a medical or home setting, when the patient is unable to meet his/her needs relying on his/her own inhaled oxygen, it is often necessary to supply oxygen through an external instrument. The method of delivering oxygen to a patient by means of an external instrument, either in an invasive manner or a non-invasive manner, is known as oxygen therapy. Instruments used to supply oxygen to a patient are collectively referred to as oxygen therapy instruments. The oxygen therapy instrument is generally composed of three parts: an oxygen generating device (e.g., an oxygen tank, oxygen generator, etc.), an interface for delivering oxygen to a patient (e.g., a nasal oxygen tube, a mask, etc.), and a pipeline connecting the generating device and the patient interface. The fire protection device is disposed in series in the pipeline between the oxygen generating device and the patient interface.

In the embodiment of the present disclosure, the third type of the fire protection device is connected in series in the pipeline of the third oxygen therapy instrument. When the first meltable member 330 is in a non-molten state, the first meltable member 330 compresses the first elastic valve body 320, and the thickness of the first elastic valve body 320 decreases, with a gap between the first opening 3102 and the first elastic valve body 320 for gas flow. In the event of a fire, when the temperature of the meltable member reaches the melting point, the meltable member will be in a molten state. The first elastic valve body 320 releases at least part of the elastic potential energy, the first elastic valve body 320 abuts against the first end surface of the accommodating cavity 3101, and the first elastic valve body 320 covers the first opening 3102, so that the first opening 3102 is in the closed state, thereby cutting off the oxygen passage and preventing the oxygen from continuously leaking out to cause the fire to spread. Since the first elastic valve body 320 has oxidation resistance characteristics, the problem of oxidation of the first elastic valve body 320 is avoided, and the durability and safety factor of the device are improved.

Embodiments of the present disclosure also disclose a third type of a ventilation treatment system including the third type of the oxygen therapy instrument.

Specifically, the third type of the ventilation treatment system includes a control device and the third type of the oxygen therapy instrument. The control device is used for controlling the oxygen supply amount, operation time, etc. of the oxygen therapy instrument. The control device may be an electronic device or a component in the electronic device, such as an integrated circuit or a chip. The electronic device may be a terminal or may be a device other than a terminal. Illustratively, the electronic device may be a cell phone, a tablet computer, a notebook computer, a palmtop computer, a mobile Internet appliance, a robot, a wearable device, etc. and embodiments of the present disclosure are not particularly limited.

In the embodiment of the present disclosure, the third type of the ventilation treatment system includes the third type of the oxygen therapy instrument. The third type of the fire protection device is connected in series in the pipeline of the third type of the oxygen therapy instrument. When the first meltable member 330 is in a non-molten state, the first meltable member 330 compresses the first elastic valve body 320, and the thickness of the first elastic valve body 320 decreases, with a gap between the first opening 3102 and the first elastic valve body 320 for gas flow. In the event of a fire, when the temperature of the meltable member reaches the melting point, the meltable member will be in a molten state. The first elastic valve body 320 releases at least part of the elastic potential energy, the first elastic valve body 320 abuts against the first end surface of the accommodating cavity 3101, and the first elastic valve body 320 covers the first opening 3102, so that the first opening 3102 is in the closed state, thereby cutting off the oxygen passage and preventing the oxygen from continuously leaking out to cause the fire to spread. Since the first elastic valve body 320 has oxidation resistance characteristics, the problem of oxidation of the first elastic valve body 320 is avoided, and the durability and safety factor of the device are improved.

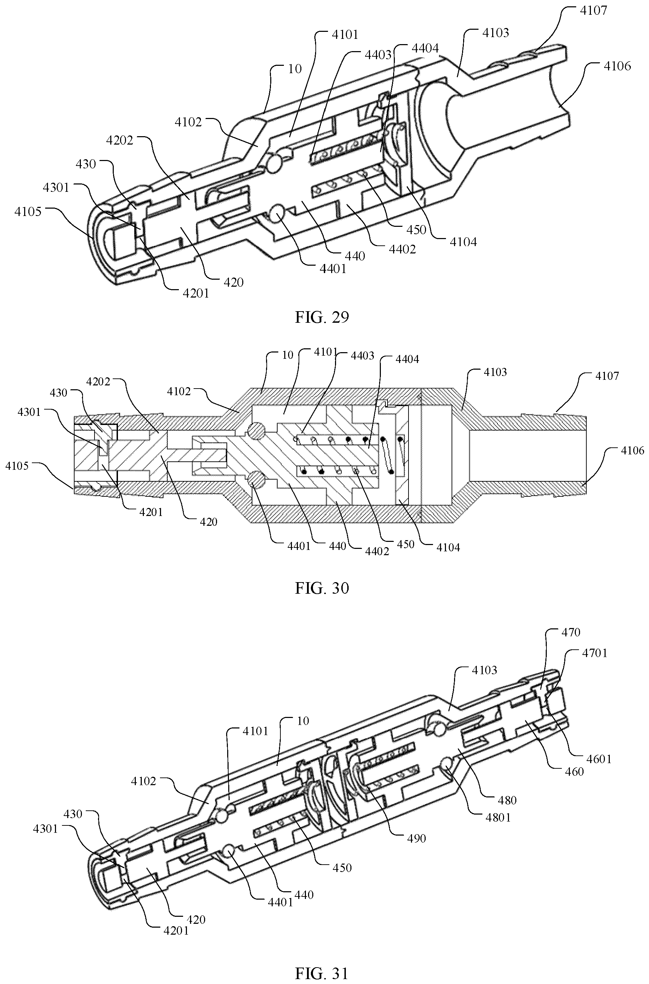

Referring to FIG. 29 and FIG. 30, a fourth type of a fire protection device is disclosed according to an embodiment of the present disclosure, the fire protection device including a housing 10, a first movable rod 420, a first meltable member 430, a first valve body 440, and a first elastic member 450. The housing 10 has a fluid passage 4101 therein which is provided with a first narrowed portion 4102 and a second narrowed portion 4103, and the first movable rod 420, the first meltable member 430, the first valve body 440 and the first elastic member 450 are all provided in the fluid passage 4101. The first meltable member 430 is disposed at one end of the fluid passage 4101 and is connected to an inner wall of the fluid passage 4101. The first movable rod 420 is slidably connected to the inner wall of the fluid passage 4101, and the first meltable member 430 is disposed on the first movable rod 420. The first valve body 440 passes through the first narrowed portion 4102 and is slidably connected to the inner wall of the fluid passage 4101. One end of the first valve body 440 is connected to the first movable rod 420, and the other end of the first valve body 440 is provided with the first elastic member 450, the first elastic member 450 abuts against the housing 10. A first sealing member 4401 is provided at a position of the first valve body 440 close to the first narrowed portion 4102. When the first valve body 440 is in an open state, the first meltable member 430 supports the first movable rod 420 and the first valve body 440 to be in a first position, the first sealing member 4401 is in clearance fit with the first narrowed portion 4102, and the fluid passage 4101 is in an open state. When the first elastic member 450 drives the first movable rod 420 and the first valve body 440 to slide to a second position, the first valve body 440 is in a closed state, the first sealing member 4401 is engaged with the first narrowed portion 4102, and the fluid passage 4101 is in a closed state.

Specifically, as shown in FIG. 29 and FIG. 30, the fourth type of the fire protection device includes the housing 10, the first movable rod 420, the first meltable member 430, the first valve body 440, and the first elastic member 450. As a main frame of the fire protection device, the housing 10 may be made of a material that is not easily chemically reacted with oxygen and is resistant to high temperatures, such as stainless steel or a ceramic material. It may also be made of fireproof and flame-retardant materials. The housing 10 has the fluid passage 4101 which has a structure which is thick in the middle and thin at the ends, so as to facilitate the connection with the pipeline of an oxygen therapy instrument end or a patient end. The fluid passage 4101 transitions from the middle to both ends and is provided with the first narrowed portion 4102 and the second narrowed portion 4103. The first movable rod 420, the first meltable member 430, the first valve body 440, and the first elastic member 450 are disposed in the fluid passage 4101.

The fluid passage 4101 includes one end and the other end that are opposite to each other. The first meltable member 430 is disposed at one end of the fluid passage 4101 and close to the opening of the fluid passage 4101. The first meltable member 430 is connected to the inner wall of the fluid passage 4101, and the specific connection method may be clamping, bonding, etc. The first meltable member 430 may be made of a lower-melting-point material, such as PP, PVC, or the like.

The first movable rod 420 is slidably connected to the inner wall of the fluid passage 4101. The axis of the first movable rod 420 coincides with the axis of the fluid passage 4101. The first movable rod 420 is made of the fireproof and flame-retardant material. The first meltable member 430 is disposed on the first movable rod 420 so as to support the first movable rod 420 in the fluid passage 4101.

The first valve body 440 is provided through the first narrowed portion 4102 and slidably connected to the inner wall of the fluid passage 4101. One end of the first valve body 440 is connected to the first movable rod 420, and the specific connection method may be clamping, bonding, etc. The first valve body 440 is also made of the fireproof and flame-retardant material. The other end of the first valve body 440 is provided with the first elastic member 450. The first elastic member 450 abuts against the housing 10, so as to be capable of providing a driving force to the first valve body 440. The first elastic member 450 may be a metal spring such as iron, copper, alloy, etc. or a soft rubber such as silica gel, rubber, etc. which can store the elastic potential energy.

The first sealing member 4401 is provided at the position of the first valve body 440 close to the first narrowed portion 4102. The first sealing member 4401 can be disposed on both sides of the first valve body 440 along its own axis. When the first valve body 440 slides relative to the housing 10, the first sealing member 4401 may block the gap between the first valve body 440 and the first narrowed portion 4102, thereby closing the fluid passage 4101. The first sealing member 4401 may be made of silicone, rubber, etc. and has a good sealing effect.

In the absence of a fire (the temperature is low), the first meltable member 430 is in the non-molten state, has a certain rigidity, and may support the first movable rod 420 and the first valve body 440. When the first movable rod 420 and the first valve body 440 are in the first position, the first valve body 440 is in the open state. The first sealing member 4401 is in clearance fit with the first narrowed portion 4102. The fluid passage 4101 is in the open state. Oxygen may be normally transmitted in the fluid passage 4101. When the first movable rod 420 and the first valve body 440 are in the first position, the elastic force applied to the first movable rod 420 and the first valve body 440 by the first elastic member 450 and the supporting force applied to the first movable rod 420 and the first valve body 440 by the first meltable member 430 are in equilibrium.

When a fire occurs (the temperature is high), the first meltable member 430 melts. The first meltable member 430 may not continue to support the first movable rod 420 and the first valve body 440, and the equilibrium state is broken. At this time, under the elastic force of the first elastic member 450, the first movable rod 420 and the first valve body 440 slide from the first position to the second position. The first valve body 440 is in the closed state. The first sealing member 4401 on the first valve body 440 is engaged with the first narrowed portion 4102, so that the fluid passage 4101 is in the closed state, and oxygen may not continue to be transmitted. At the same time, the first movable rod 420 may also be separated from the first valve body 440, and the first movable rod 420 continues to slide under the action of inertia and extends into the pipeline of the oxygen therapy instrument or the patient end, which can also play the role of cutting off the oxygen passage, further improving the safety factor of the fire protection device.

In the embodiment of the present disclosure, with the cooperation of the first meltable member 430, the first movable rod 420, the first valve body 440, and the first elastic member 450, it is possible to automatically close the fluid passage 4101 in the event of a fire, cut off the oxygen passage, and prevent the continuous leakage of the oxygen to cause the spread of the fire. The structure is simple, which reduces the manufacturing difficulty. The oxygen can be normally transmitted through the gap between the first meltable member 430 and the fluid passage 4101 without opening the first meltable member 430, thereby reducing ventilation noise and preventing the air passage from being blocked by mistake in case of non-fire. Thus, it improves the stability of the fire protection device and ensures the therapeutic effect.

Optionally, as shown in FIG. 29 and FIG. 30, the first meltable member 430 is provided with a first clamping portion 4301. The first movable rod 420 is provided with a first clamping-fitting portion 4201. The first meltable member 430 is clamped with the first movable rod 420.

Specifically, as shown in FIG. 29 and FIG. 30, the first meltable member 430 is provided with the first clamping portion 4301. The first movable rod 420 is provided with the first clamping-fitting portion 4201. The first meltable member 430 is clamped with the first movable rod 420. The first clamping portion 4301 may be a protrusion, and the first clamping-fitting portion 4201 may be a groove. Accordingly, the first clamping portion 4301 may be a groove and the first clamping-fitting portion 4201 may be a protrusion.

Optionally, as shown in FIG. 29 and FIG. 30, the side wall of the first movable rod 420 is provided with at least one first guide portion 4202, and the inner wall of the fluid passage 4101 is provided with a first chute, and the first guide portion 4202 is in sliding fit with the first chute.

Specifically, as shown in FIG. 29 and FIG. 30, the first movable rod 420 is slidably connected to the inner wall of the fluid passage 4101, and the side wall of the first movable rod 420 is opposite to the inner wall of the fluid passage 4101. At least one first guide portion 4202 is disposed on a side wall of the first movable rod 420. The shape of the first guide portion 4202 may be plate-shaped, spherical, or the like. An inner wall of the fluid passage 4101 is provided with the first chute which has an extension direction coinciding with a sliding direction of the first movable rod 420. The first guide portion 4202 is embedded in the first chute. When the first movable rod 420 slides relative to the fluid passage 4101, the smooth sliding of the first movable rod 420 may be improved by the sliding fit of the first guide portion 4202 and the first chute, so as to avoid the problems of jamming and deflection. A quantity of the first guide portions 4202 matches with the quantity of the first chutes. In the embodiment of the present disclosure, two first guide portions 4202 are symmetrically disposed on the side wall of the first movable rod 420. Correspondingly, the two first chutes are symmetrically disposed on the inner wall of the fluid passage 4101, further improving the stationarity when the first movable rod 420 slides.

Optionally, as shown in FIG. 29 and FIG. 30, the side wall of the first valve body 440 is provided with at least one second guide portion 4402. The inner wall of the fluid passage 4101 is provided with a second chute, and the second guide portion 4402 is in sliding fit with the second chute.

Specifically, as shown in FIG. 29 and FIG. 30, the first valve body 440 is slidably connected to the inner wall of the fluid passage 4101, and the side wall of the first valve body 440 is opposite to the inner wall of the fluid passage 4101. At least one second guide portion 4402 is disposed on a side wall of the first valve body 440. The shape of the second guide portion 4402 may be plate-shaped, spherical, or the like. The inner wall of the fluid passage 4101 is provided with the second chute extending in the same direction as the sliding direction of the first valve body 440. The second guide portion 4402 is embedded in the second chute. When the first valve body 440 slides relative to the fluid passage 4101, the smooth sliding of the first valve body 440 may be improved by the sliding fit of the second guide portion 4402 and the second chute, so as to avoid the problems of jamming and deflection. The quantity of the second guide portions 4402 matches with the quantity of the second chutes. In the embodiment of the present disclosure, two second guide portions 4402 are symmetrically disposed on the side wall of the first valve body 440. Correspondingly, the two second chutes are symmetrically disposed on the inner wall of the fluid passage 4101, further improving the stationarity when the first valve body 440 slides.

Optionally, as shown in FIG. 29 and FIG. 30, the other end of the first valve body 440 is provided with a groove body 4403 in which the first elastic member 450 is located at least partially. One end of the first elastic member 450 abuts against the groove bottom of the groove body 4403, and the other end of the first elastic member 450 abuts against the housing 10.

Specifically, as shown in FIG. 29 and FIG. 30, the first elastic member 450 provides a driving force for the first valve body 440 and the first movable rod 420. The groove body 4403 is provided at the other end of the first valve body 440. The groove body 4403 may be formed by an opening manner or may be integrally formed by injection molding. The first elastic member 450 is located at least partially in the groove body 4403 and partially protrudes out of the groove body 4403. One end of the first elastic member 450 abuts against the groove bottom of the groove body 4403 and the other end of the first elastic member 450 abuts against the housing 10. By providing the groove body 4403 at the other end of the first valve body 440, it is possible to provide an installation space for the first elastic member 450 and play a certain limiting role for the first elastic member 450. The first elastic member 450 is less likely to have problems of deflection and jamming, thus improving the stability of the fire protection device.

Optionally, as shown in FIG. 29 and FIG. 30, a mounting shaft 4404 is disposed in the groove body 4403. The mounting shaft 4404 is provided coaxially with the groove body 4403. The first elastic member 450 is sleeved on the mounting shaft 4404.

Specifically, as shown in FIG. 29 and FIG. 30, the first elastic member 450 is embedded in the groove body 4403. The mounting shaft 4404 is disposed in the groove body 4403. The mounting shaft 4404 is provided coaxially with the groove body 4403. One end of the mounting shaft 4404 is connected to the groove bottom, and the first elastic member 450 is sleeved on the mounting shaft 4404. Under the common limiting action of the mounting shaft 4404 and the groove body 4403, the first elastic member 450 will be directionally deformed along the direction of the common axis of the mounting shaft 4404 and the groove body 4403, so that the problems of deflection and jamming will not occur, further improving the stability of the fire protection device.

Optionally, as shown in FIG. 29 and FIG. 30, the housing 10 further includes a baffle 4104 disposed in the fluid passage 4101 and connected to the inner wall of the fluid passage 4101. The first elastic member 450 abuts against the baffle 4104.

Specifically, as shown in FIG. 29 and FIG. 30, the first elastic member 450 accumulates the elastic potential energy under the combined action of the first valve body 440 and the housing 10. A baffle 4104 is disposed on the housing 10. The baffle 4104 is connected to the inner wall of the fluid passage 4101, and the specific connection method may be bonding, clamping, etc. It may also be made by an integral molding process, and the shape of the baffle 4104 may be rectangular or semi-circular, etc. One end of the first elastic member 450 abuts against the first valve body 440, and the other end of the first elastic member 450 abuts against the baffle 4104, so that the elastic potential energy is accumulated under the combined action of the first valve body 440 and the baffle 4104. The end surface of the baffle 4104 is perpendicular to the direction of deformation of the first elastic member 450, which is advantageous for improving the stability of the fire protection device. The baffle 4104 may also be made of the fireproof and flame-retardant material.

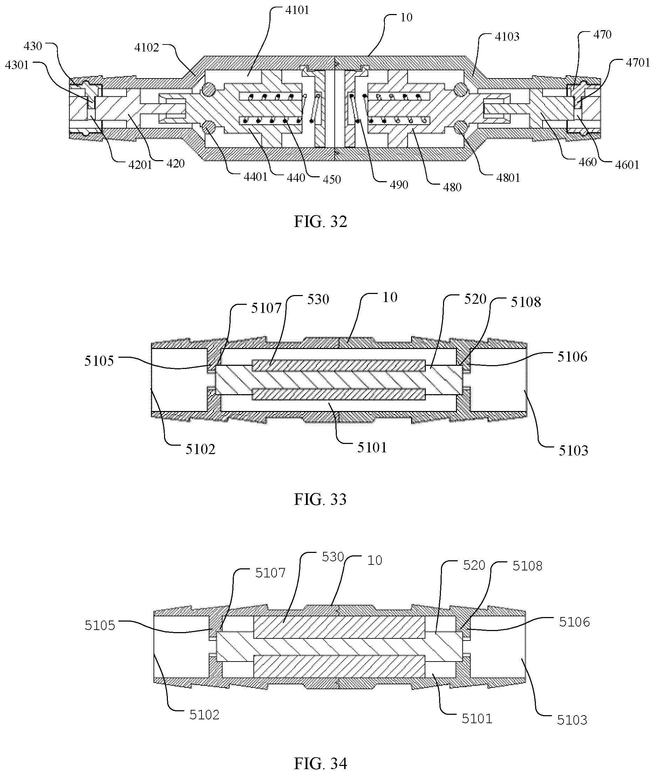

Optionally, as shown in FIG. 31 and FIG. 32, the fire protection device further includes a second movable rod 460, a second meltable member 470, a second valve body 480, and a second elastic member 490. The second movable rod 460, the second meltable member 470, the second valve body 480 and the second elastic member 490 are all disposed in the fluid passage 4101. The second meltable member 470 is disposed at the other end of the fluid passage 4101 and is connected to the inner wall of the fluid passage 4101. The second movable rod 460 is slidably connected to the inner wall of the fluid passage 4101, and the second meltable member 470 is disposed on the second movable rod 460. The second valve body 480 passes through the second narrowed portion 4103 and is slidably connected to the inner wall of the fluid passage 4101. One end of the second valve body 480 is connected to the second movable rod 460, and the other end of the second valve body 480 is provided with the second elastic member 490 which abuts against the housing 10. The second sealing member 4801 is provided at a position of the second valve body 480 close to the second narrowed portion 4103. When the second valve body 480 is in an open state, the second meltable member 470 supports the second movable rod 460 and the second valve body 480 to be in a third position. The second sealing member 4801 is in clearance fit with the second narrowed portion 4103, and the fluid passage 4101 is in the open state. When the second elastic member 490 drives the second movable rod 460 and the second valve body 480 to slide to the fourth position, the second valve body 480 is in the closed state, the second sealing member 4801 is engaged with the second narrowed portion 4103, and the fluid passage 4101 is in the closed state.

Specifically, as shown in FIG. 31 and FIG. 32, the second movable rod 460, the second meltable member 470, the second valve body 480, and the second elastic member 490 are disposed within the fluid passage 4101. The fluid passage 4101 includes one end and the other end that are opposite to each other. The second meltable member 470 is disposed at the other end of the fluid passage 4101 and close to the opening of the fluid passage 4101. The second meltable member 470 is connected to the inner wall of the fluid passage 4101, and the specific connection method may be clamping, bonding, etc. The second meltable member 470 may be made of a lower-melting-point material, such as PP, PVC, or the like.

The second movable rod 460 is slidably connected to the inner wall of the fluid passage 4101. The axis of the second movable rod 460 coincides with the axis of the fluid passage 4101. The second movable rod 460 is made of the fireproof and flame-retardant material. The second meltable member 470 is disposed on the second movable rod 460 so as to support the second movable rod 460 to be in the fluid passage 4101.

The second valve body 480 is provided through the second narrowed portion 4103 and is slidably connected to the inner wall of the fluid passage 4101. One end of the second valve body 480 is connected to the second movable rod 460, and the specific connection method may be clamping, bonding, etc. The second valve body 480 is also made of the fireproof and flame-retardant material. The other end of the second valve body 480 is provided with the second elastic member 490. The second elastic member 490 abuts against the housing 10, so as to be capable of providing a driving force for the second valve body 480. The second elastic member 490 may be a metal spring such as iron, copper, alloy, etc. or a soft rubber such as silica gel, rubber, etc. which can store the elastic potential energy.

The second sealing member 4801 is provided at a position of the second valve body 480 close to the second narrowed portion 4103. The second sealing member 4801 may be disposed on both sides of the second valve body 480 along its own axis. When the second valve body 480 slides relative to the housing 10, the second sealing member 4801 may block the gap between the second valve body 480 and the second narrowed portion 4103, thereby closing the fluid passage 4101. The second sealing member 4801 may be made of silicone, rubber, etc. to achieve a good sealing effect.

In the absence of a fire (the temperature is low), the second meltable member 470 is in the non-molten state and has a certain rigidity so as to be able to support the second movable rod 460 and the second valve body 480. When the second movable rod 460 and the second valve body 480 are in the third position, the second valve body 480 is in the open state, and the second sealing member 4801 is in clearance fit with the second narrowed portion 4103. The fluid passage 4101 is in the open state, and oxygen may be normally transmitted in the fluid passage 4101. When the second movable rod 460 and the second valve body 480 are in the third position, the elastic force exerted by the second elastic member 490 on the second movable rod 460 and the second valve body 480 and the supporting force exerted by the second meltable member 470 on the second movable rod 460 and the second valve body 480 are in equilibrium.

In the event of a fire (the temperature is high), the second meltable member 470 melts, the second meltable member 470 may not continue to support the second movable rod 460 and the second valve body 480, and the equilibrium state is broken. At this time, under the elastic force of the second elastic member 490, the second movable rod 460 and the second valve body 480 slide from the third position to the fourth position. The second valve body 480 is in the closed state. The second sealing member 4801 on the second valve body 480 is engaged with the second narrowed portion 4103, so that the fluid passage 4101 is in the closed state, and oxygen may not continue to be transmitted. At the same time, the second movable rod 460 may also be separated from the second valve body. The second movable rod 460 continues to slide under the action of inertia and extends into the pipeline of the oxygen therapy instrument or the patient end, which can also play the role of cutting off the oxygen passage, further improving the safety factor of the fire protection device.

By providing the movable rod and the valve body at both ends of the fluid passage 4101, when at least one of the first narrowed portion 4102 and the second narrowed portion 4103 is engaged with the sealing member, the fluid passage 4101 may be closed to prevent oxygen leakage, thereby improving the reliability of the fire protection device.

Optionally, as shown in FIG. 31 and FIG. 32, the second meltable member 470 is provided with a second clamping portion 4701. The second movable rod 420 is provided with a second clamping-fitting portion 4601. The second meltable member 470 is clamped with the second movable rod 460.

Specifically, as shown in FIG. 31 and FIG. 32, the second meltable member 470 is provided with the second clamping portion 4701. The second movable rod 460 is provided with the second clamping-fitting portion 4601. The second meltable member 470 is clamped with the second movable rod 460. The second clamping portion 4701 may be a protrusion, and the second clamping-fitting portion 4601 may be a groove. Correspondingly, the second clamping portion 4701 may be a groove and the second clamping-fitting portion 4601 may be a protrusion.

Optionally, as shown in FIG. 29 and FIG. 30, the housing 10 is provided with a first pipeline joint 4105 and a second pipeline joint 4106 for connecting with the pipeline of the oxygen therapy instrument or the patient side, respectively. An outer side wall of the first pipeline joint 4105 and/or the second pipeline joint 4106 is provided with at least one third clamping portion 4107 for being clamped with the pipeline of the oxygen therapy instrument or the patient end.

Specifically, as shown in FIG. 29 and FIG. 30, the first pipeline joint 4105 and the second pipeline joint 4106 are disposed on both sides of the fluid passage 4101, respectively. The first pipeline joint 4105, the second pipeline joint 4106, and the housing 10 may be of an integrated structure or may be made in separate pieces and then assembled. The first pipeline joint 4105 and the second pipeline joint 4106 are used for communicating with the pipeline of the oxygen therapy instrument or the patient end. The pipeline of the oxygen therapy instrument or the patient end may be assembled with the first pipeline joint 4105 or the second pipeline joint 4106 by means of a threaded connection or an interference fit.

The first pipeline joint 4105 and the second pipeline joint 4106 may be symmetrically disposed on the housing 10, one of which communicates with the patient end and the other of which communicates with the oxygen therapy instrument end when assembled with the pipeline of the oxygen therapy instrument or the patient end. Since the first pipeline joint 4105 and the second pipeline joint 4106 are symmetrically arranged, both the first pipeline joint 4105 and the second pipeline joint 4106 may be adapted to the oxygen therapy instrument end or the patient end. There is no reverse installation of the fire protection device, and the assembly efficiency is greatly improved.

The first pipeline joint 4105 and the second pipeline joint 4106 are used for communicating with the pipeline of the oxygen therapy instrument or the patient end. The pipeline is generally a catheter which is sleeved outside the first pipeline joint 4105 and the second pipeline joint 4106. In order to ensure the stability of the connection between the catheter and the first pipeline joint 4105 and between the catheter and the second pipeline joint 4106, at least one third clamping portion 4107 is disposed on the outside wall of the first pipeline joint 4105 and/or the outside wall of the second pipeline joint 4106. The shape of the third clamping portion 4107 is toothed. When the catheter is sleeved on the outside wall of the first pipeline joint 4105 and/or the outside wall of the second pipeline joint 4106, the third clamping portion 4107 is clamped with the catheter to ensure airtightness between the catheter and the first pipeline joint 4105 and/or the second pipeline joint 4106, so as to avoid the problem of oxygen leakage. The quantity of the third clamping portions 4107 may be selected based on the size of the first pipeline joint 4105 and the second pipeline joint 4106.

Optionally, the first movable rod 420 and the first valve body 440 form an integrated structure.

Specifically, the first movable rod 420 and the first valve body 440 may be integrally molded. A seam does not exist between the first movable rod 420 and the first valve body 440, thereby greatly improving the structural strength and aesthetic appearance of the first movable rod 420 and the first valve body 440. In addition, when the first movable rod 420 and the first valve body 440 are switched between the first position and the second position, the stability of the integrated structure of the first movable rod 420 and the first valve body 440 is stronger, and the problems of jamming and deflection do not occur easily, thus greatly improving the stability of the guard.

Optionally, the first movable rod 420 and the first valve body 440 are of a split structure.

Specifically, the first movable rod 420 and the first valve body 440 may be made separately and assembled by means of clamping or bonding, etc. Certainly, they only maintain a contact relationship under the action of the first meltable member 430 and the first elastic member 450. The first movable rod 420 is separated from the first valve body 440 once the first fusible member 430 is melted. The first movable rod 420 may also be separated from the first valve body 440, and the first movable rod 420 continues to slide under the action of inertia and extends into the pipeline of the oxygen therapy instrument or the patient end, which can also play the role of cutting off the oxygen passage, further improving the safety factor of the fire protection device.

Embodiments of the present disclosure also disclose a fourth type of an oxygen therapy instrument including the fourth type of the fire protection device.

Specifically, in a medical or home setting, when the patient is unable to meet his/her needs relying on his/her own inhaled oxygen, it is often necessary to supply oxygen through an external instrument. The method of delivering oxygen to a patient by means of an external instrument, either in an invasive manner or a non-invasive manner, is known as oxygen therapy. Instruments used to supply oxygen to a patient are collectively referred to as oxygen therapy instruments. The oxygen therapy instrument is generally composed of three parts: an oxygen generating device (e.g., an oxygen tank, an oxygen generator, etc.), an interface for delivering oxygen to a patient (e.g., a nasal oxygen tube, a mask, etc.), and a pipeline connecting the generating device and the patient interface. The fire protection device is disposed in series in the pipeline between the oxygen generating device and the patient interface. The fire protection device has the fluid passage 4101 therein. The fluid passage 4101 is used for communicating with the pipeline of the oxygen therapy instrument or the patient end. The fire protection device may control the opening or closing of the pipeline of the oxygen therapy instrument by controlling the opening or closing of the fluid passage 4101.

In an embodiment of the present disclosure, the fourth type of the fire protection device is connected in series in a pipeline of the fourth type of the oxygen therapy instrument, the fourth type of the fire protection device including the housing 10, the first movable rod 420, the first meltable member 430, the first valve body 440, and the first elastic member 450. The housing 10 has the fluid passage 4101 therein which is provided with the first narrowed portion 4102 and the second narrowed portion 4103, and the first movable rod 420, the first meltable member 430, the first valve body 440 and the first elastic member 450 are all provided in the fluid passage 4101. The first meltable member 430 is disposed at one end of the fluid passage 4101 and is connected to the inner wall of the fluid passage 4101. The first movable rod 420 is slidably connected to the inner wall of the fluid passage 4101, and the first meltable member 430 is disposed on the first movable rod 420. The first valve body 440 passes through the first narrowed portion 4102 and is slidably connected to the inner wall of the fluid passage 4101. One end of the first valve body 440 is connected to the first movable rod 420, the other end of the first valve body 440 is provided with the first elastic member 450, and the first elastic member 450 abuts against the housing 10. The first sealing member 4401 is provided at a position of the first valve body 440 close to the first narrowed portion 4102. When the first valve body 440 is in the open state, the first meltable member 430 supports the first movable rod 420 and the first valve body 440 to be in the first position, the first sealing member 4401 is in clearance fit with the first narrowed portion 4102, and the fluid passage 4101 is in the open state. When the first elastic member 450 drives the first movable rod 420 and the first valve body 440 slides to the second position, the first valve body 440 is in the closed state, the first sealing member 4401 is engaged with the first narrowed portion 4102, and the fluid passage 4101 is in the closed state. With the cooperation of the first meltable member 430, the first movable rod 420, the first valve body 440, and the first elastic member 450, it is possible to automatically close the fluid passage 4101 in the event of a fire, cut off the oxygen passage, and prevent the oxygen from continuously leaking to spread the fire. The structure is simple, which reduces the manufacturing difficulty. The oxygen can be normally transmitted through the gap between the first meltable member 430 and the fluid passage 4101 without opening the first meltable member 430, thereby reducing the ventilation noise and preventing the air passage from being blocked by mistake in case of non-fire. Thus, it improves the stability of the fire protection device and ensures the therapeutic effect.

Embodiments of the present disclosure also disclose a fourth type of a ventilation treatment system including the fourth type of the oxygen therapy instrument.

Specifically, the fourth type of the ventilation treatment system includes a control device and the fourth type of the oxygen therapy instrument. The control device is used for controlling the oxygen supply amount, operation time, etc. of the oxygen therapy instrument. The control device may be an electronic device or a component in the electronic device, such as an integrated circuit or a chip. The electronic device may be a terminal or may be a device other than a terminal. Illustratively, the electronic device may be a cell phone, a tablet computer, a notebook computer, a palmtop computer, a mobile Internet appliance, a robot, a wearable device, etc. and embodiments of the present disclosure are not particularly limited.

In an embodiment of the present disclosure, the fourth type of the ventilation treatment system includes the fourth type of the oxygen therapy instrument. The fourth type of the fire protection device is connected in series in the pipeline of the type of the fourth oxygen therapy instrument, the fire protection device including the housing 10, the first movable rod 420, the first meltable member 430, the first valve body 440, and the first elastic member 450. The housing 10 has the fluid passage 4101 therein which is provided with the first narrowed portion 4102 and the second narrowed portion 4103, and the first movable rod 420, the first meltable member 430, the first valve body 440 and the first elastic member 450 are all provided in the fluid passage 4101. The first meltable member 430 is disposed at one end of the fluid passage 4101 and is connected to the inner wall of the fluid passage 4101. The first movable rod 420 is slidably connected to the inner wall of the fluid passage 4101, and the first meltable member 430 is disposed on the first movable rod 420. The first valve body 440 passes through the first narrowed portion 4102 and is slidably connected to the inner wall of the fluid passage 4101. One end of the first valve body 440 is connected to the first movable rod 420, the other end of the first valve body 440 is provided with the first elastic member 450, and the first elastic member 450 abuts against the housing 10. The first sealing member 4401 is provided at a position of the first valve body 440 close to the first narrowed portion 4102. When the first valve body 440 is in the open state, the first meltable member 430 supports the first movable rod 420 and the first valve body 440 to be in the first position, the first sealing member 4401 is in clearance fit with the first narrowed portion 4102, and the fluid passage 4101 is in the open state. When the first elastic member 450 drives the first movable rod 420 and the first valve body 440 slides to the second position, the first valve body 440 is in the closed state, the first sealing member 4401 is engaged with the first narrowed portion 4102, and the fluid passage 4101 is in the closed state. With the cooperation of the first meltable member 430, the first movable rod 420, the first valve body 440, and the first elastic member 450, it is possible to automatically close the fluid passage 4101 in the event of a fire, cut off the oxygen passage, and prevent the oxygen from continuously leaking to spread the fire. The structure is simple, which reduces the manufacturing difficulty. The oxygen can be normally transmitted through the gap between the first meltable member 430 and the fluid passage 4101 without opening the first meltable member 430, thereby reducing the ventilation noise and preventing the air passage from being blocked by mistake in case of non-fire. Thus, it improves the stability of the fire protection device and ensures the therapeutic effect.

Referring to FIG. 33, FIG. 34 and FIG. 40, a fifth type of a fire protection device is disclosed according to an embodiment of the present disclosure, including a housing 10, a first positioning member 520, and a first sealing member 530. The fluid passage 5101 is disposed in the housing 10. A first opening 5102 and a second opening 5103 are disposed on two opposite sides of the fluid passage 5101, and the first opening 5102 and the second opening 5103 are respectively used for communicating with a pipeline of the oxygen therapy instrument or the patient end. The first positioning member 520 is disposed in the fluid passage 5101 and is connected to the housing 10. The first sealing member 530 is sleeved on the first positioning member 520, and the first sealing member 530 has oxidation resistance characteristics. At a first temperature, the first sealing member 530 is in clearance fit with the inner wall of the fluid passage 5101, and the fluid passage 5101 is in the open state. At a second temperature, the first sealing member 530 increases in volume and attaches to the inner wall of the fluid passage 5101, and the fluid passage 5101 is in the closed state. The second temperature is higher than the first temperature.

Specifically, as shown in FIG. 33, FIG. 34, and FIG. 40, the fifth type of the fire protection device includes the housing 10, the first positioning member 520, and the first sealing member 530. As a main frame of the fire protection device, the housing 10 may be made of plastic or the like, which is not easily chemically reacted with oxygen. The fluid passage 5101 is provided in the housing 10. The fluid passage 5101 is provided with a first opening 5102 and a second opening 5103, both of which may be located at two opposite sides of the fluid passage 5101, with the axes of the first opening 5102 and the second opening 5103 being collinear. The first opening 5102 and the second opening 5103 may also be angularly disposed on the fluid passage 5101. The first opening 5102 and the second opening 5103 are respectively used for communicating with a pipeline of an oxygen therapy instrument or a patient end. For example, the first opening 5102 communicates with the pipeline of the oxygen therapy instrument end. The second opening 5103 communicates with the pipeline of the patient end. Oxygen enters the fluid passage 5101 from the first opening 5102 and is transmitted to the patient end via the second opening 5103. The second opening 5103 may also be in communication with the pipeline at the oxygen therapy instrument end, and the first opening 5102 may be in communication with the pipeline at the patient end, which is not limited by the embodiments of the present disclosure.

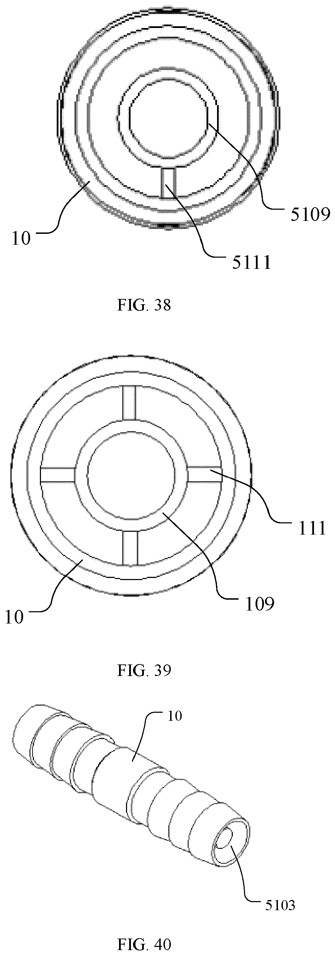

As shown in FIG. 40, the outer side wall of the housing 10 is provided with at least one toothed protrusion for being clamped with the pipeline of the oxygen therapy instrument or the patient end. The pipeline of the oxygen therapy instrument is generally a catheter which is sleeved on the outer side wall of the housing 10. In order to ensure the stability of the connection between the catheter and the housing 10, at least one toothed protrusion is disposed on the outer side wall of the housing 10. When the catheter is sleeved on the outer side wall of the housing 10, the toothed protrusion is clamped with the catheter, thus ensuring the airtightness between the catheter and the housing 10, and avoiding the problem of oxygen leakage. The quantity of the toothed protrusions may be selected according to the size of the housing 10.

The first positioning member 520 is disposed in the fluid passage 5101 and is connected to the housing 10. The connection mode of the first positioning member 520 and the housing 10 may be clamping, welding or bonding, etc. The first positioning member 520 occupies only a small portion of the space of the fluid passage 5101 and does not impede the transmission of the gas within the fluid passage 5101. The first sealing member 530 is sleeved on the first positioning member 520. The first positioning member 520 is used for fixing the first sealing member 530 in the fluid passage 5101. The first sealing member 530 is arranged coaxially with the first positioning member 520. The common axis of the first sealing member 530 and the first positioning member 520 may also coincide with the long axis of the fluid passage 5101.

The first sealing member 530 has a first state and a second state, when the first sealing member 530 is in the first state, a volume of the first sealing member 530 is small, a gap is provided between an outer surface of the first sealing member 530 and the inner wall of the fluid passage 5101, and a gas may be transmitted normally through this gap. When the first sealing member 530 is in the second state, the volume of the first sealing member 530 increases, and the outer surface of the first sealing member 530 attaches to the inner wall of the fluid passage 5101 with a certain amount of interference, so that the fluid passage 5101 is in the closed state. By switching the first sealing member 530 from the first state to the second state, the fluid passage 5101 may be controlled to switch from the open state to the closed state, thereby blocking the passage of oxygen. The first sealing member 530 has the oxidation resistance characteristics, and is not susceptible to aging and rust even when exposed to an oxygen-rich environment for a long time, thereby preventing an influence on the therapeutic effect, and improving the stability and durability of the fire protection device.

The first sealing member 530 may effect a switch from the first state to the second state based on the temperature. In the absence of a fire, the fire protection device is at the first temperature, which may be any temperature before the volume of the first sealing member 530 increases, or may be a temperature range. At the first temperature, the first sealing member 530 is in the first state as described above and has a small volume. The gap is provided between the outer surface of the first sealing member 530 and the inner wall of the fluid passage 5101, and the fluid passage 5101 is in the open state, through which gas may be normally transmitted.

In the event of a fire, the fire protection device is at a second temperature that is higher than the first temperature. The second temperature may be any temperature that is capable of increasing the volume of the first sealing member 530 or may be a temperature range. At the second temperature, the first sealing member 530 is in the second state as described above. As the volume increases, the outer surface of the first sealing member 530 attaches to the inner wall of the fluid passage 5101 with a certain amount of interference, so that the fluid passage 5101 is in the closed state.

In an embodiment of the present disclosure, the fifth type of the fire protection device includes the housing 10, the first positioning member 520, and the first sealing member 530. The fluid passage 5101 is disposed in the housing 10. The first opening 5102 and the second opening 5103 are disposed on two opposite sides of the fluid passage 5101, and the first opening 5102 and the second opening 5103 are respectively used for communicating with the pipeline of the oxygen therapy instrument or the patient end. The first positioning member 520 is disposed in the fluid passage 5101 and is connected to the housing 10. The first sealing member 530 is sleeved on the first positioning member 520, and the first sealing member 530 has oxidation resistance characteristics. When a fire does not occur, the fire protection device is at the first temperature, the first sealing member 530 is in clearance fit with the inner wall of the fluid passage 5101, and the fluid passage 5101 is in the open state. In the event of a fire, the fire protection device is at the second temperature. The first sealing member 530 increases in volume and attaches to the inner wall of the fluid passage 5101, which is in the closed state, so as to cut off the oxygen passage and prevent the oxygen from continuously leaking out and causing the fire to spread, wherein the second temperature is higher than the first temperature. Since the first sealing member 530 has oxidation resistance characteristics, the problem of oxidation of the first sealing member 530 is avoided, and durability and safety factor of the fire protection device are improved.

Optionally, as shown in FIG. 33 to FIG. 34, the first sealing member 530 is a thermally induced shape memory plastic. At the second temperature, the first sealing member 530 is thermally expanded and attaches to the inner wall of the fluid passage 5101, and the fluid passage is in the closed state.

Specifically, as shown in FIG. 33 to FIG. 34, in an embodiment of the present disclosure, the first sealing member 530 may be the thermally induced shape memory plastic, which is a thermally sensitive functional material that may be stored in a shape for a long period of time at room temperature and rapidly expand in volume when heated. The thermotropic shape memory plastic may specifically include polyurethane elastomers, polynorbornenes, trans-1,4-polyisoprene, styrene/butadiene copolymers, cross-linked polyethylene materials, etc.

In the absence of a fire, the first sealing member 530 may be stored for a long period of time in a shape. A gap is provided between the outer surface of the first sealing member 530 and the inner wall of the fluid passage 5101, and the fluid passage 5101 is in the open state, through which gas may be normally transmitted.

In the event of a fire, the first sealing member 530 is heated to rapidly expand and increase in volume, and the outer surface of the first sealing member 530 attaches to the inner wall of the fluid passage 5101 with a certain amount of interference, so that the fluid passage 5101 is in the closed state.

By using the first sealing member 530 of the thermally induced shape memory plastic, in the event of a fire, the characteristics of the thermally induced shape memory plastic can be utilized to quickly close the fluid passage 5101 and block the passage of oxygen to avoid the spread of the fire caused by the continued escape of oxygen. At the same time, the thermo-induced shape memory plastic has good oxidation resistance characteristics. Even in the oxygen-rich environment for a long time, it is not easy to cause rust and aging, so as to avoid affecting the treatment effect, and also improve the stability and durability of the fire protection device.

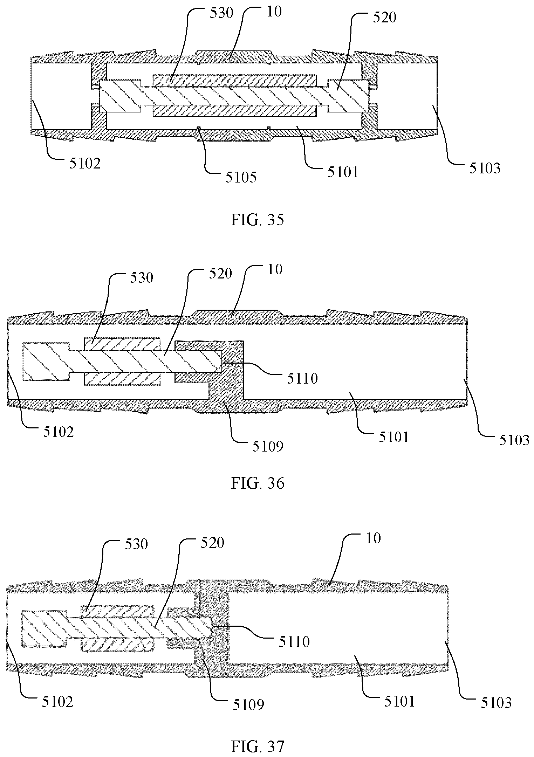

Optionally, as shown in FIG. 35, the inner wall of the fluid passage 5101 is provided with at least one annular protrusion 5104. At the second temperature, the annular protrusion 5104 abuts against the first sealing member 530, and the fluid passage 5101 is in the closed state.

Specifically, as shown in FIG. 35, the inner wall of the fluid passage 5101 is provided with at least one annular protrusion 5104 surrounding the outer surface of the first sealing member 530. In the absence of a fire, the gap is provided between the outer surface of the first sealing member 530 and the annular protrusion 5104, and the fluid passage 5101 is in the open state, through which gas may be normally transmitted. In the event of a fire, the first sealing member 530 is heated to rapidly expand and increase in volume, and the outer surface of the first sealing member 530 attaches to the annular protrusion 5104 with a certain amount of interference, so that the fluid passage 5101 is in the closed state.

By providing the annular protrusion 5104, the requirement for the expansion rate of the first sealing member 530 is reduced, without excessive expansion of the first sealing member 530, and the stability of the fire protection device is improved by closing the fluid passage 5101 in cooperation with the annular protrusion 5104.

Optionally, as shown in FIG. 33 and FIG. 34, the inner wall of the fluid passage 5101 is provided with a first protruding structure 5105 and a second protruding structure 5106. The first protruding structure 5105 and the second protruding structure 5106 are respectively located at both ends of the first positioning member 520. The first protruding structure 5105 is provided with a first positioning portion 5107. The second protruding structure 5106 is provided with a second positioning portion 5108. Both ends of the first positioning member 520 are respectively clamped with the first positioning portion 5107 and the positioning portion 5108.

Specifically, as shown in FIG. 33 to FIG. 34, the first positioning member 520 is used for positioning and fixing the first sealing member 530. The first positioning member 520 may be installed in the fluid passage 5101 in such a manner that the first protruding structure 5105 and the second protruding structure 5106 are disposed on the inner wall of the fluid passage 5101, the first protruding structure 5105 and the second protruding structure 5106 are provided at intervals along the direction of the long axis of the fluid passage 5101, and are respectively located at both ends of the first positioning member 520. The first protruding structure 5105 and the second protruding structure 5106 occupy only a small portion of the space of the fluid passage 5101 without hindering the normal transmission of oxygen. The first protrusion structure 5105 may be divided into an upper protrusion and a lower protrusion. A gap is provided between the upper protrusion and the lower protrusion. The second protruding structure 5106 is identical to the first protruding structure 5105. In the first protruding structure 5105, a step-shaped positioning portion, namely, the first positioning portion 5107, is disposed on an end surface where the upper projection and the lower projection are relatively close. Similarly, the second positioning portion 5108 is disposed in the second protruding structure 5106. The two ends of the first positioning member 520 may be respectively embedded in the first positioning portion 5107 and the second positioning portion 5108 to achieve a clamping fixation.

Optionally, as shown in FIG. 36 to FIG. 37, the inner wall of the fluid passage 5101 is provided with a mounting portion 5109 which is provided with an accommodating groove 511. One end of the first positioning member 520 is embedded into the accommodating groove 5110.

Specifically, as shown in FIG. 36 to FIG. 37, only one end of the first positioning member 520 may be fixed in order to reduce the occupation of the inner space of the fluid passage 5101 and avoid the influence on the normal transmission of oxygen. The mounting portion 5109 is disposed on the inner wall of the fluid passage 5101. The mounting portion 5109 is connected to the inner wall of the fluid passage 5101 and may be assembled by means of welding, clamping or bonding, etc. The mounting portion 5109 and the inner wall of the fluid passage 5101 may also be of an integrated structure with better structural strength. The mounting portion 5109 may also be connected to the inner wall of fluid passage 5101 using a connection structure. The mounting portion 5109 is provided with the accommodating groove 5110. One end of the first positioning member 520 is embedded into the accommodating groove 5110 to achieve mounting and fixing.

Optionally, as shown in FIG. 36, one end of the first positioning member 520 is provided with a clamping portion. The groove wall of the accommodating groove 5110 is provided with a clamping-fitting portion, and one end of the first positioning member 520 is clamped with the accommodating groove 5110.

Specifically, as shown in FIG. 36, in order to improve the stability of the mounting of the first positioning member 520, the clamping portion is disposed at one end of the first positioning member 520. The clamping-fitting portion is disposed at the groove wall of the accommodating groove 5110. The stable assembly of the first positioning member 520 and the mounting portion 5109 is achieved by the clamping action of the clamping portion and the clamping-fitting portion.

Optionally, as shown in FIG. 37, one end of the first positioning member 520 is provided with an external thread. The groove wall of the accommodating groove 5110 is provided with an internal thread. One end of the first positioning member 520 is threadedly connected with the accommodating groove 5110.

Specifically, as shown in FIG. 37, the first positioning member 520 and the mounting portion 5109 may also be assembled and disassembled by means of the threaded connection. One end of the first positioning member 520 is provided with the external thread. The groove wall of the accommodating groove 5110 is provided with the internal thread. The threaded connection of the first positioning member 520 with the mounting portion 5109 may be achieved by rotating the first positioning member 520 or the housing 10.

Optionally, one end of the first positioning member 520 is in interference fit with the accommodating groove 5110.

Specifically, one end of the first positioning member 520 may also be assembled with the accommodating groove 5110 by means of the interference fit, and the assembly is simple and stable.

Optionally, as shown in FIG. 38 and FIG. 39, the mounting portion 5109 is provided with at least one connecting rib 5111 through which the mounting portion 5109 is connected with the inner wall of the fluid passage 5101.

Specifically, as shown in FIG. 38 and FIG. 39, the mounting portion 5109 may also be connected to the inner wall of the fluid passage 5101 via the connecting rib 5111. At least one connecting rib 5111 is disposed on the mounting portion 5109. One end of the connecting rib 5111 is fixed to the inner wall of the fluid passage 5101, and the other end is fixed to the mounting portion 5109. The fixing method may be welding or bonding, etc. In order to ensure the stability of the mounting of the mounting portion 5109, the connecting ribs 5111 may be disposed on both sides of the mounting portion 5109. The quantity of the connecting ribs 5111 may be selected according to the size of the fluid passage 5101 and the mounting portion 5109. The embodiments of the present disclosure are not limited thereto.

The mounting portion 5109 is fixed in the fluid passage 5101 by the connecting rib 5111. The mounting portion 5109 does not obstruct the passage of oxygen, and oxygen may be normally transmitted through the gap between the connecting rib 5111 and the inner wall of the fluid passage 5101.

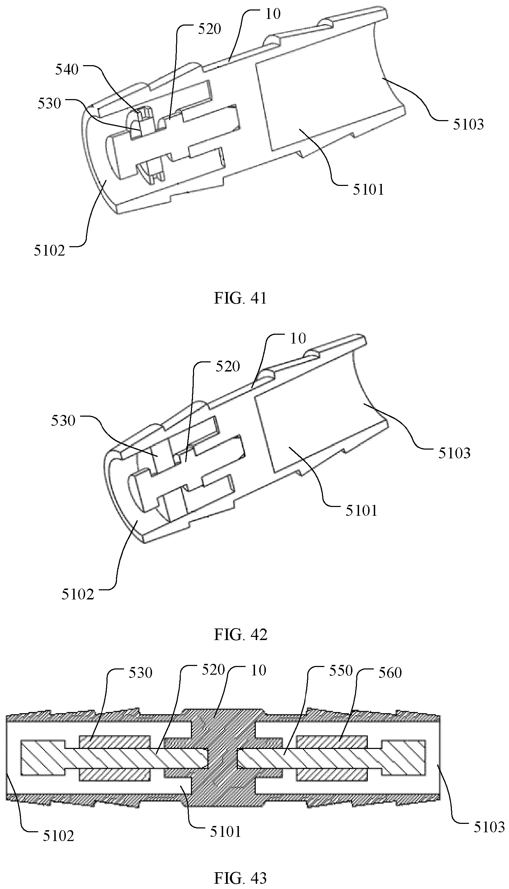

Optionally, referring to FIG. 41 to FIG. 42, the fifth type of the fire protection device further includes a meltable member 540. The first sealing member 530 is an elastomer, and the meltable member 540 wraps the first sealing member 530. When the meltable member 540 is in a non-molten state, the meltable member 540 compresses the first sealing member 530, the meltable member 540 is in clearance fit with the inner wall of the fluid passage 5101, and the fluid passage 5101 is in an open state. When the meltable member 540 is in the molten state, the first sealing member 530 releases at least part of the elastic potential energy, the first sealing member 530 increases in volume and attaches to the inner wall of the fluid passage 5101, and the fluid passage 5101 is in the closed state.

Specifically, as shown in FIG. 41 to FIG. 42, in the embodiment of the present disclosure, the first sealing member 530 may also be the elastomer, such as a material that may be compressed and store the elastic potential energy, such as silica gel or rubber. The meltable member 540, which may be a lower-melting-point material such as PP, PVC, or the like, wraps around the outer surface of the first sealing member 530. Because of the wrapping of the meltable member 540, the first sealing member 530 is not exposed to an oxygen-rich environment to avoid oxidation. At the same time, the first sealing member 530 itself is not easy to be oxidized, and oxidation and rust are not easy to occur in contact with oxygen for a long time, so as to avoid affecting the therapeutic effect. Also, the stability and durability of the fire protection device are improved.

In the absence of a fire (the temperature is low), when the meltable member 540 is in the non-molten state, the meltable member 540 compresses the first sealing member 530, the volume of the first sealing member 530 is reduced, and the elastic potential energy is accumulated. The volume of the first sealing member 530 refers to the volume perpendicular to the direction of the inner wall of the fluid passage 5101. A gap is provided between the outer surface of the first sealing member 530 and the inner wall of the fluid passage 5101 for the flow of gas, and oxygen may normally flow through the gap.

When a fire occurs (the temperature is high), the meltable member 540 is in the molten state. The meltable member 540 may not continue to wrap the first sealing member 530. The equilibrium state of the first sealing member 530 is broken. At this time, at least part of the elastic potential energy is released by the first sealing member 530, the volume of the first sealing member 530 increases, and the outer surface of the first sealing member 530 abuts against the inner wall of the fluid passage 5101, so that the fluid passage 5101 is in the closed state and the oxygen passage is blocked.

Optionally, referring to FIG. 43 to FIG. 45, the fifth type of the fire protection device further includes the second positioning member 550 and the second sealing member 560. The second positioning member 550 is disposed in the fluid passage 5101 and is connected to the housing 10. The first positioning member 520 is close to the first opening 5102. The second positioning member 550 is close to the second opening 5103. The second sealing member 560 is sleeved on the second positioning member 550, and the second sealing member 560 has oxidation resistance characteristics. At the first temperature, the first sealing member 530 and the second sealing member 560 are respectively in clearance fit with the inner wall of the fluid passage 5101, and the fluid passage 5101 is in the open state. At the second temperature, the first sealing member 530 and the second sealing member 560 increase in volume and respectively attach to the inner wall of the fluid passage 5101, which is in the closed state.

Specifically, as shown in FIG. 43 to FIG. 45, in the embodiment of the present disclosure, not only the first sealing member 530 is provided at the first opening 5102, but also the second sealing member 560 is provided at the second opening 5103. The fire protection device performs double protection. The second positioning member 550 is disposed in the fluid passage 5101 and is connected to the housing 10. The connection between the second positioning member 550 and the housing 10 may be clamping, welding or bonding, etc. The second positioning member 550 occupies only a small portion of the space of the fluid passage 5101 and does not impede the transfer of gas within the fluid passage 5101. The second sealing member 560 is sleeved on the second positioning member 550. The second positioning member 550 is used for fixing the second sealing member 560 in the fluid passage 5101. The second sealing member 560 is arranged coaxially with the second positioning member 550. The common axis of the second sealing member 560 and the second positioning member 550 may also coincide with the long axis of the fluid passage 5101.

The second sealing member 560 has the first state and the second state. When the second sealing member 560 is in the first state, the volume of the second sealing member 560 is small, the gap is provided between an outer surface of the second sealing member 560 and an inner wall of the fluid passage 5101, and the gas may be transmitted normally through this gap. When the second sealing member 560 is in the second state, the volume increases, and the outer surface of the second sealing member 560 attaches to the inner wall of the fluid passage 5101 with a certain amount of interference, so that the fluid passage 5101 is in the closed state. By switching the second sealing member 560 from the first state to the second state, the fluid passage 5101 may be controlled to switch from the open state to the closed state, thereby blocking the passage of oxygen. The second sealing member 560 has oxidation resistance characteristics, and is not susceptible to rust and aging even when exposed to an oxygen-rich environment for a long period of time, thereby preventing an influence on the therapeutic effect, and improving the stability and durability of the fire protection device.

The second sealing member 560 may effect switching of the first state to the second state based on the temperature. In the absence of a fire, the fire protection device is at the first temperature, which may be any temperature before the volume of the second sealing member 560 increases, or may be a temperature range. At the first temperature, the second sealing member 560 is in the above-mentioned first state and has a small volume. The gap is provided between the outer surface of the second sealing member 560 and the inner wall of the fluid passage 5101, and the fluid passage 5101 is in the open state, through which gas may be normally transmitted.

In the event of a fire, the fire protection device is at the second temperature that is higher than the first temperature. The second temperature may be any temperature that is capable of increasing the volume of the second sealing member 560 or may be a temperature range. At the second temperature, the second sealing member 560 is in the second state as described above. As the volume increases, the outer surface of the second sealing member 560 attaches to the inner wall of the fluid passage 5101 with a certain amount of interference, so that the fluid passage 5101 is in the closed state.

The first positioning member 520 is close to the first opening 5102, and the second positioning member 550 is close to the second opening 5103. Correspondingly, the first sealing member 530 is close to the first opening 5102 and the second sealing member 560 is close to the second opening 5103. By providing a positioning member and a sealing member at both ends of the fluid passage 5101, when at least one of the first sealing member 530 and the second sealing member 560 abuts against the inner wall of the fluid passage 5101, the fluid passage 5101 may be closed to prevent oxygen leakage, thereby improving the reliability of the fire protection device.

The first sealing member 530 and the second sealing member 560 of thermally induced shape memory plastic may be used within the fluid passage 5101, or a combination of the elastomer and the meltable member 540 may be used. Accordingly, both of the above embodiments are applicable to a solution in which they are symmetrically arranged at both ends of the fluid passage 5101.

Embodiments of the present disclosure also disclose a fifth type of a ventilation treatment apparatus including the fifth type of the fire protection device.

Specifically, the ventilation treatment apparatus includes a control device, an oxygen therapy instrument and a gas pipeline. The control device is used for controlling the oxygen supply amount, operation time, etc. of the oxygen therapy instrument. The control device may be an electronic device or a component in the electronic device, such as an integrated circuit or a chip. The electronic device may be a terminal or may be a device other than a terminal. Illustratively, the electronic device may be a cell phone, a tablet computer, a notebook computer, a palmtop computer, a mobile Internet appliance, a robot, a wearable device, etc. and embodiments of the present disclosure are not particularly limited.

In an embodiment of the present disclosure, the type of the fifth ventilation treatment apparatus includes the fifth type of the fire protection device including the housing 10, the first positioning member 520, and the first sealing member 530. The fluid passage 5101 is disposed in the housing 10. The first opening 5102 and the second opening 5103 are disposed on two opposite sides of the fluid passage 5101, and the first opening 5102 and the second opening 5103 are respectively used for communicating with the pipeline of the oxygen therapy instrument or the patient end. The first positioning member 520 is disposed in the fluid passage 5101 and is connected to the housing 10. The first sealing member 530 is sleeved on the first positioning member 520, and the first sealing member 530 has oxidation resistance characteristics. When a fire does not occur, the fire protection device is at the first temperature, the first sealing member 530 is in clearance fit with the inner wall of the fluid passage 5101, and the fluid passage 5101 is in the open state. In the event of a fire, the fire protection device is at the second temperature. The first sealing member 530 increases in volume and attaches to the inner wall of the fluid passage 5101, which is in the closed state, so as to cut off the oxygen passage and prevent the oxygen from continuously leaking out and causing the fire to spread, wherein the second temperature is higher than the first temperature. Since the first sealing member 530 has oxidation resistance characteristics, the problem of oxidation of the first sealing member 530 is avoided, and durability and safety factor of the fire protection device are improved.

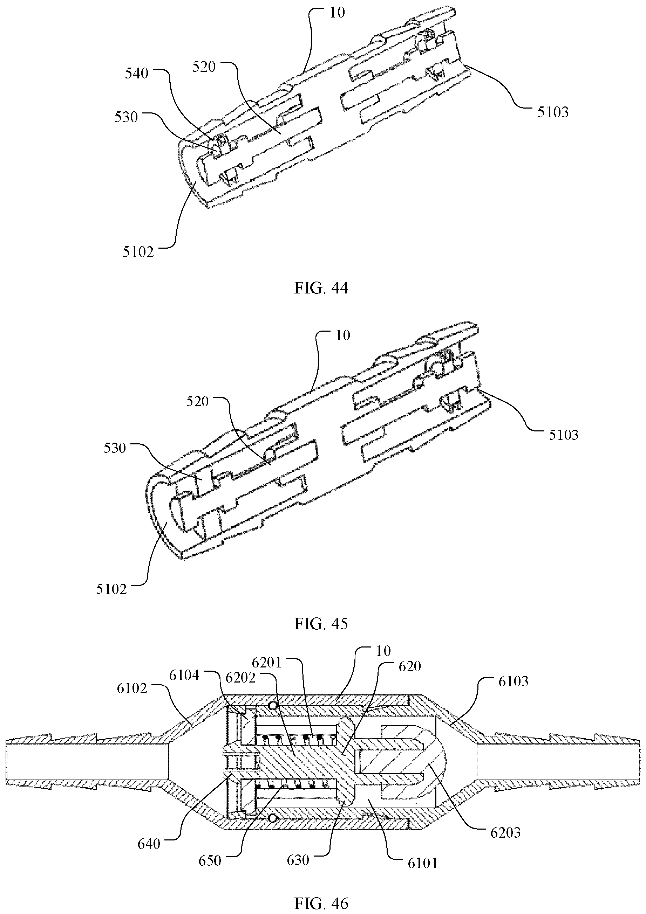

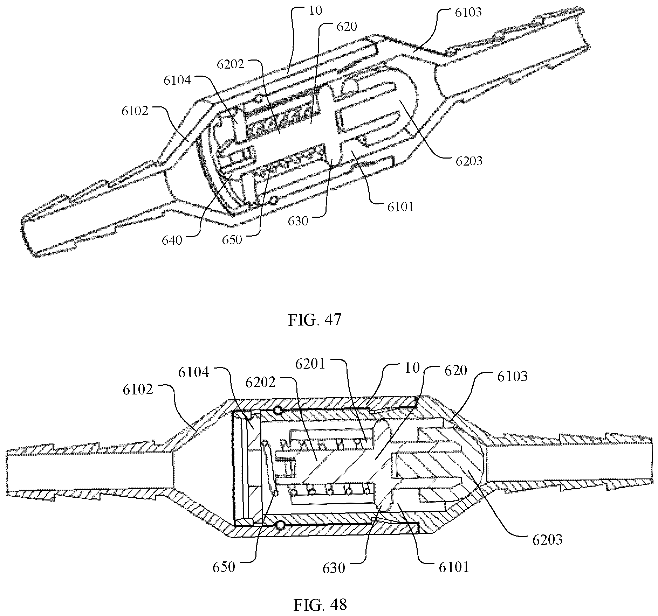

Referring to FIG. 46 to FIG. 51, a sixth type of a fire protection device is disclosed according to an embodiment of the present disclosure, the fire protection device including a housing 10 and a valve body 620. The housing 10 has a fluid passage 6101 therein, the fluid passage 6101 is provided with a first narrowed portion 6102 and a second narrowed portion 6103, and the valve body 620 is provided in the fluid passage 6101. The valve body 620 is slidably connected to the inner wall of the fluid passage 6101, the outer side wall of the valve body 620 is provided with at least one guide portion 630, and the guide portion 630 is in sliding fit with the inner wall of the fluid passage 6101. The valve body 620 has a first position and a second position relative to the fluid passage 6101. When the valve body 620 is in the first position, the valve body 620 is in clearance fit with the first narrowed portion 6102 and the second narrowed portion 6103 respectively, and the fluid passage 6101 is in an open state. When the valve body 620 is in the second position, the valve body 620 is engaged with the first narrowed portion 6102 and/or the second narrowed portion 6103, and the fluid passage 6101 is in a closed state.

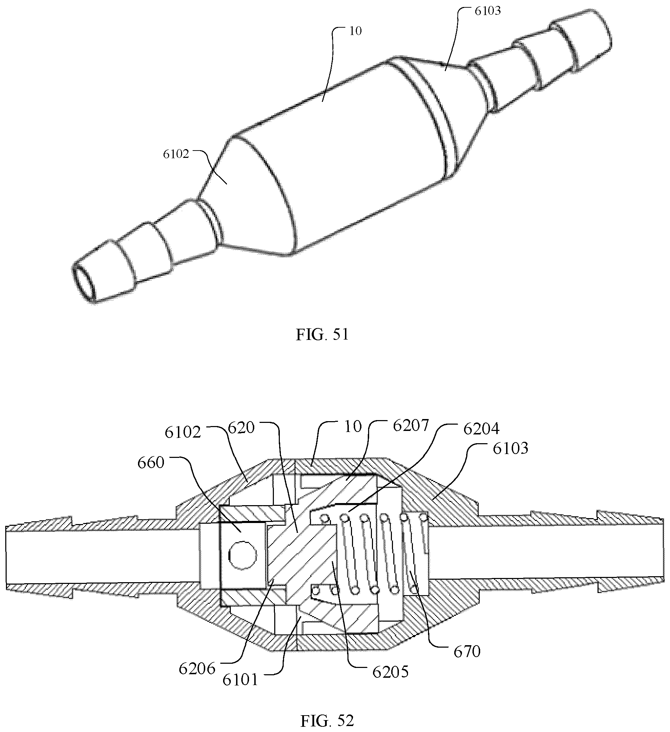

Specifically, as shown in FIG. 46 to FIG. 51, the sixth type of the fire protection device includes the housing 10 and the valve body 620. As a main frame of the fire protection device, the housing 10 may be made of a material that is not easily chemically reacted with oxygen and is resistant to high temperatures, such as stainless steel or a ceramic material. It may also be made of fireproof and flame-retardant materials. The housing 10 has the fluid passage 6101 therein. As shown in FIG. 51, the fluid passage 6101 has a structure which is thick in the middle and thin at the ends, so as to facilitate the connection with the pipeline of oxygen therapy instrument end or patient end. The fluid passage 6101 transitions from the middle to both ends and is provided with the first narrowed portion 6102 and the second narrowed portion 6103. The valve body 620 is disposed within the fluid passage 6101.

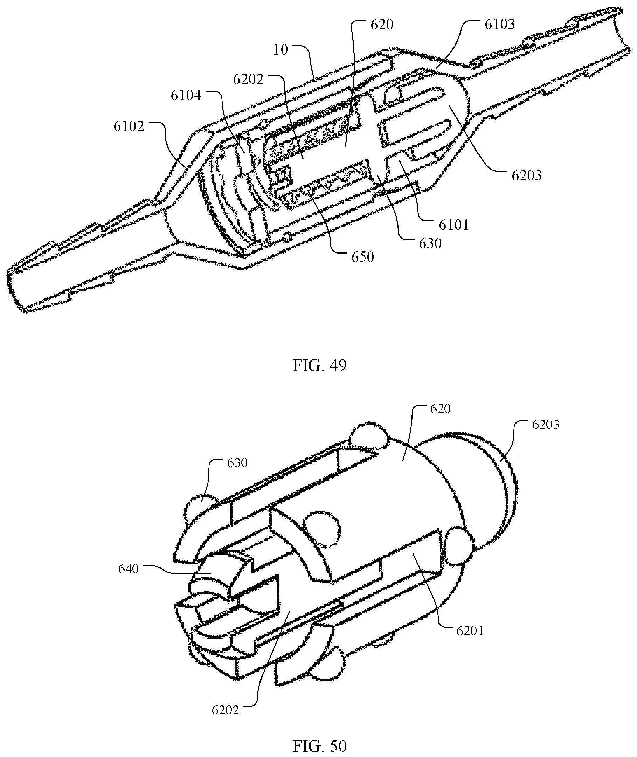

The valve body 620 is slidably connected to the inner wall of the fluid passage 6101. The axis of the valve body 620 coincides with the axis of the fluid passage 6101. The valve body 620 is made of a fire-resistant and flame-retardant material, and may stably block the fluid passage 6101 in the event of a fire. An outer side wall of the valve body 620 is opposite to an inner wall of the fluid passage 6101. The outer side wall of the valve body 620 is provided with at least one guide portion 630 which slidably cooperates with the inner wall of the fluid passage 6101 so as to serve as a limit guide when the valve body 620 slides relative to the fluid passage 6101. The guide portion 630 may be a slide, a boss, or the like. A quantity of the guide portions 630 may be selected according to practical requirements. As shown in FIG. 50, in the embodiment of the present disclosure, the outer side wall of the valve body 620 is provided with a plurality of guide portions 630. The plurality of guide portions 630 are uniformly distributed on the outer side wall of the valve body 620, and the shape of the guide portions 630 is a hemispherical boss, so as to reduce the sliding friction between the valve body 620 and the housing 10 while performing a good guide limiting function. The guide portion 630 may be made of the same material as that of the valve body 620, or may be made of a material having fire-retardant and wear-resistant characteristics. The guide portion 630 and the valve body 620 may have an integrated structure, and have good structural strength and stability. The guide portion 630 may be formed separately from the valve body 620 and assembled by bonding or welding.

The valve body 620 has a first position and a second position with respect to the fluid passage 6101. The valve body 620 is switched from the first position to the second position by sliding, and the valve body 620 slides along the direction of the long axis of the fluid passage. A driving member may be disposed on the valve body 620 so as to drive the valve body 620 to slide with respect to the fluid passage 6101. The driving member may be a metal spring, a silica gel or the like capable of storing the elastic potential energy. A driving mechanism such as a cylinder may be disposed on the valve body 620 to control the sliding of the valve body 620.

When the valve body 620 is in the first position, the valve body 620 is in clearance fit with the first narrowed portion 6102 and the second narrowed portion 6103, respectively, the fluid passage 6101 is in the open state, and oxygen may normally be transmitted in the fluid passage 6101. When the valve body 620 is in the second position, the valve body 620 is engaged with the first narrowed portion 6102 and/or the second narrowed portion 6103, the fluid passage 6101 is in the closed state, and oxygen may not continue to be transmitted. The valve body 620 is engaged with at least one of the first narrowed portion 6102 and the second narrowed portion 6103, which may block the oxygen passage. Preferably, the valve body 620 is simultaneously engaged with the first narrowed portion 6102 and the second narrowed portion 6103, so that the sealing effect is better and the performance of the fire protection device is improved.

In the embodiment of the present disclosure, by providing the guide portion 630 on the outside wall of the valve body 620, the guide portion 630 may act as a limit when the valve body 620 slides with respect to the fluid passage 6101, avoiding the problem of deflection and jamming of the valve body 620, ensuring that the valve body 620 may be accurately engaged with the first narrowed portion 6102 and/or the second narrowed portion 6103, and improving the stability of the fire protection device.

Optionally, as shown in FIG. 46 to FIG. 50, the sixth type of the fire protection device further includes a first meltable member 640 and a first elastic member 650. The valve body 620 includes first and second ends opposite to each other, the first end is close to the first narrowed portion 6102 and the second end is close to the second narrowed portion 6103. The housing 10 is provided with a fixing portion 6104 close to the first narrowed portion 6102. A first end of the valve body 620 is provided with a first groove body 6201, and the first elastic member 650 is at least partially located in the first groove body 6201. One end of the first elastic member 650 abuts against a groove bottom of the first groove body 6201, and the other end of the first elastic member 650 abuts against the end surface of the fixing portion 6104. The first meltable member 640 is provided at the first end of the valve body 620 and is clamped with the fixing portion 6104 to support the valve body 620 to be in the first position. The first meltable member 640 melts, the first elastic member 650 drives the valve body 620 to slide to the second position. The second end of the valve body 620 is engaged with the second narrowed portion 6103, and the fluid passage 6101 is in a closed state.

Specifically, as shown in FIG. 46 to FIG. 50, the sixth type of the fire protection device further includes the first meltable member 640 and the first elastic member 650. The first meltable member 640 may be made of a lower-melting-point material, such as PP, PVC, or the like. The first elastic member 650 may be a metal spring such as iron, copper, alloy, etc. or a soft rubber such as silica gel, rubber, etc. which can store the elastic potential energy.

The valve body 620 includes a first end and a second end that are opposite to each other, the first end is close to the first narrowed portion 6102 and the second end is close to the second narrowed portion 6103. A fixing portion 6104 is provided at a position of the housing 10 close to the first narrowed portion 6102. The fixing portion 6104 and the housing 10 may be of an integral structure. The fixing portion 6104 may be formed separately, and may be assembled with the housing 10 by bonding, welding, or clamping. The first elastic member 650 provides a driving force for the valve body 620. The first end of the valve body 620 is provided with a first groove body 6201, and the first elastic member 650 is located at least partially in the first groove body 6201. The first groove body 6201 may be formed at the first end of the valve body 620 by opening or may be formed integrally by injection molding. The first elastic member 650 is located at least partially in the first groove body 6201 and partially protrudes out of the first groove body 6201. One end of the first elastic member 650 abuts against the groove bottom of the first groove body 6201, and the other end of the first elastic member 650 abuts against the end surface of a side of the fixing portion 6104 close to the valve body 620. By providing the first groove body 6201 at the first end of the valve body 620, it is possible to provide an installation space for the first elastic member 650 and play a certain limiting role for the first elastic member 650. The first elastic member 650 is less likely to have problems of deflection and jamming, thus improving the stability of the fire protection device.

In the absence of a fire (the temperature is low), the first meltable member 640 is disposed at the first end of the valve body 620 and is clamped with the fixing portion 6104 to support the valve body 620 to be in a first position with the fluid passage 6101 in an open state. The first meltable member 640 may be a hook that is clamped with an end surface of a side of the fixing portion 6104 away from the valve body 620 to retain the valve body 620 in the first position.

In the event of a fire (the temperature is high), the first meltable member 640 melts. The valve body 620 loses the restraint of the first meltable member 640, and slides to the second position driven by the first elastic member 650. The second end of the valve body 620 is engaged with the second narrowed portion 6103. The fluid passage 6101 is in a closed state. Oxygen may not continue to be transmitted, so as to avoid the spread of fire caused by the continuous leakage of oxygen.

Optionally, as shown in FIG. 46 and FIG. 50, a first mounting shaft 6202 is disposed in the first groove body 6201. The first mounting shaft 6202 is provided coaxially with the first groove body 6201. The first elastic member 650 is sleeved on the first mounting shaft 6202. The first meltable member 640 is connected to the first mounting shaft 6202.

Specifically, as shown in FIG. 46 to FIG. 50, the first elastic member 650 is embedded in the first groove body 6201. The first mounting shaft 6202 is disposed in the first groove body 6201. The first mounting shaft 6202 is provided coaxially with the first groove body 6201. One end of the first mounting shaft 6202 is connected to the groove bottom of the first groove body 6201, and the other end of the first mounting shaft 6202 is connected to the first meltable member 640. The first mounting shaft 6202 and the first meltable member 640 may be connected by means of bonding, clamping, etc. The first elastic member 650 is sleeved on the first mounting shaft 6202. Under the common limiting action of the first mounting shaft 6202 and the first groove body 6201, the first elastic member 650 will be oriented and deformed along the direction of the common axis of the first mounting shaft 6202 and the first groove body 6201, so that the problems of deflection and jamming are not easy to occur, thereby further improving the stability of the fire protection device.

Optionally, as shown in FIG. 46 to FIG. 50, the second end of the valve body 620 is provided with a first sealing member 6203 that at least partially wraps the second end. When the valve body 620 is in the second position, the first sealing member 6203 is engaged with the second narrowed portion 6103 and the fluid passage 6101 is in a closed state.

Specifically, as shown in FIG. 46 to FIG. 50, the second end of the valve body 620 is configured to engage with the second narrowed portion 6103, and a first sealing member 6203 is provided at the second end of the valve body 620. The first sealing member 6203 may be made of silicone, rubber, or the like, and has a good sealing effect. The first sealing member 6203 at least partially wraps the second end, which may prevent the valve body 620 from directly contacting the second narrowed portion 6103, enhancing the sealing effect. When the valve body 620 is in the second position, the first sealing member 6203 is engaged with the second narrowed portion 6103. The fluid passage 6101 is in the closed state. Oxygen may not continue to be transmitted, so as to avoid the spread of fire caused by the continuous leakage of oxygen.

Optionally, as shown in FIG. 52 to FIG. 56, the fire protection device further includes a second meltable member 660 and a second elastic member 670. The valve body 620 includes the first end and the second end opposite to each other, the first end is close to the first narrowed portion 6102 and the second end is close to the second narrowed portion 6103. The second end of the valve body 620 is provided with a second groove body 6204, and the second elastic member 670 is at least partially located in the second groove body 6204. One end of the second elastic member 670 abuts against the groove bottom of the second groove body 6204, and the other end of the second elastic member 670 abuts against the housing 10. The second meltable member 660 is disposed at the first narrowed portion 6102 and is clamped with the first narrowed portion 6102, and the second meltable member 660 is provided with a through-hole for passing a fluid. The first end of the valve body 620 abuts against the second meltable member 660 to support the valve body 620 to be in the first position. The second meltable member 660 melts. The second elastic member 670 drives the valve body 620 to slide to the second position. The first end of the valve body 620 is engaged with the first narrowed portion 6102, and the fluid passage 6101 is in the closed state.

Specifically, as shown in FIG. 52 to FIG. 56, in an embodiment of the present disclosure, the fire protection device further includes the second meltable member 660 and the second elastic member 670. The second meltable member 660 may be made of a lower-melting-point material, such as PP, PVC, or the like. The second elastic member 670 may be a metal spring such as iron, copper, alloy, etc. or a soft rubber such as silica gel, rubber, etc. which can store the elastic potential energy.

The valve body 620 includes the first end and the second end opposite to each other, the first end is close to the first narrowed portion 6102 and the second end is close to the second narrowed portion 6103. The second elastic member 670 provides a driving force for the valve body 620, the second end of the valve body 620 is provided with the second groove body 6204, and the second elastic member 670 is located at least partially in the second groove body 6204. The second groove body 6204 may be formed by opening or may be integrally formed by injection molding. The second elastic member 670 is located at least partially in the second groove body 6204, and partially protrudes out of the second groove body 6204. One end of the second elastic member 670 abuts against the groove bottom of the second groove body 6204, and the other end of the second elastic member 670 abuts against the housing 10.

The second meltable member 660 is disposed at the first narrowed portion 6102 and is clamped with the first narrowed portion 6102. A clamping structure may be disposed on the second meltable member 660 or the first narrowed portion 6102 to realize the clamping of the second meltable member 660 with the first narrowed portion 6102. In order to prevent the second meltable member 660 from shielding the first narrowed portion 6102 to cause that the oxygen may not normally be transmitted, a through-hole for passing a fluid may be disposed in the second meltable member 660, and the size of the through-hole may be selected according to actual requirements. In the absence of a fire (the temperature is lower), the first end of the valve body 620 abuts against the second meltable member 660, with the valve body 620 in the first position and the fluid passage 6101 in the open state, supported by the second meltable member 660.

In the event of a fire (the temperature is higher), the second meltable member 660 melts. The valve body 620 loses the restraint of the second meltable member 660. The second elastic member 670 drives the valve body 620 to slide to the second position. The first end of the valve body 620 is engaged with the first narrowed portion 6102. The fluid passage 6101 is in the closed state. Oxygen may not continue to be transmitted, so as to avoid the spread of fire caused by the continuous leakage of oxygen.

Optionally, as shown in FIG. 52 to FIG. 56, a second mounting shaft 6205 is disposed in the second groove body 6204, and the second mounting shaft 6205 is provided coaxially with the second groove body 6204. The second elastic member 670 is sleeved on the second mounting shaft 6205.

Specifically, as shown in FIG. 52 to FIG. 56, the second elastic member 670 is embedded in the second groove body 6204. The second mounting shaft 6205 is disposed in the second groove body 6204. The second mounting shaft 6205 is provided coaxially with the second groove body 6204. One end of the second mounting shaft 6205 is connected to the groove bottom of the second groove body 6204. The second elastic member 670 is sleeved on the second mounting shaft 6205. Under the common limiting action of the second mounting shaft 6205 and the second groove body 6204, the second elastic member 670 may be oriented and deformed along the direction of the common axis of the second mounting shaft 6205 and the second groove body 6204, so that the problems of deflection and jamming are not easy to occur, thereby further improving the stability of the fire protection device.

Optionally, as shown in FIG. 52 to FIG. 56, the first end of the valve body 620 is provided with a second sealing member 6206 which at least partially wraps the first end. When the valve body 620 is in the second position, the second sealing member 6206 is engaged with the first narrowed portion 6102 and the fluid passage 6101 is in the closed state.

Specifically, as shown in FIG. 52 to FIG. 56, the first end of the valve body 620 is used for being engaged with the first narrowed portion 6102, and a second sealing member 6206 is disposed at the first end of the valve body 620. The second sealing member 6206 may be made of silicone, rubber, or the like, and has a good sealing effect. The second sealing member 6206 at least partially wraps the first end and prevents the valve body 620 from directly contacting the first narrowed portion 6102, thereby enhancing the sealing effect. When the valve body 620 is in the second position, the second sealing member 6206 is engaged with the first narrowed portion 6102. The fluid passage 6101 is in the closed state. Oxygen may not continue to be transmitted, so as to avoid the spread of fire caused by the continuous leakage of oxygen.

Optionally, as shown in FIG. 52 to FIG. 53, the first narrowed portion 6102 is provided with a first positioning structure, the second meltable member 660 is provided with a second positioning structure, and the first positioning structure and the second positioning structure are located for coordination.

Specifically, as shown in FIG. 52 to FIG. 53, the first narrowed portion 6102 is provided with the first positioning structure, and the second meltable member 660 is provided with the second positioning structure. The stable clamping of the second meltable member 660 with the first narrowed portion 6102 can be achieved by the interaction of the first positioning structure and the second positioning structure. The first positioning structure may be a stepped structure. Correspondingly, the second positioning structure is a stepped structure complementary to the first positioning structure. The first positioning structure may also be a groove structure or a boss structure. Correspondingly, the second positioning structure is the boss structure or the groove structure.

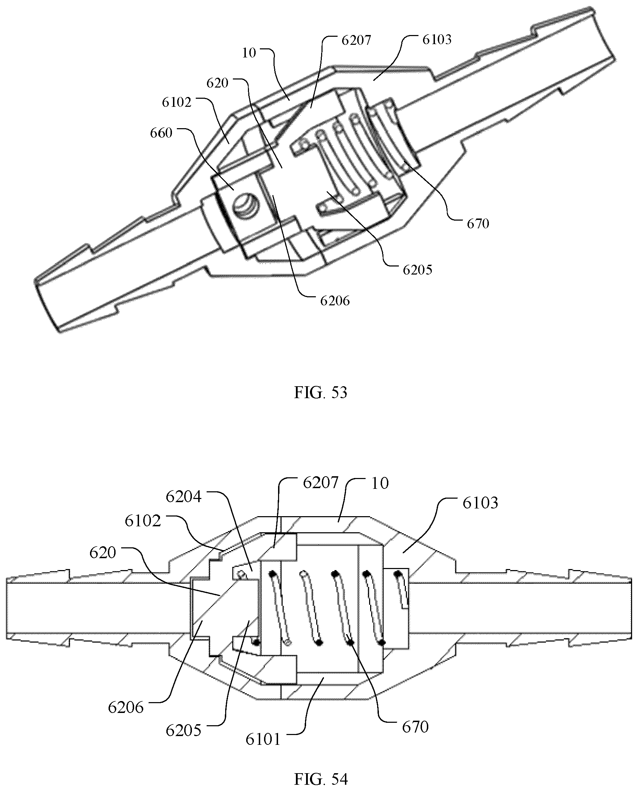

Optionally, as shown in FIG. 54 to FIG. 55, the first narrowed portion 6102 is provided with the first positioning structure and the second sealing member 6206 is provided with the third positioning structure, and the first positioning structure and the third positioning structure are in positioning fit.

Specifically, as shown in FIG. 54 to FIG. 55, the first narrowed portion 6102 is provided with the first positioning structure and the second sealing member 6206 is provided with the third positioning structure. When the valve body 620 is in the second position, the stable attachment of the second sealing member 6206 with the first narrowed portion 6102 may be achieved by the interaction of the first positioning structure and the third positioning structure to enhance the sealing effect. The first positioning structure may be a stepped structure. Correspondingly, the third positioning structure is a stepped structure complementary to the first positioning structure. The first positioning structure may also be the groove structure or the boss structure. Correspondingly, the third positioning structure is the boss structure or the groove structure.

Optionally, as shown in FIG. 52 to FIG. 56, the outer side wall of the valve body 620 is provided with at least one positioning rib 6207. The inner wall of the fluid passage 6101 is provided with at least one positioning groove 6105, and the positioning rib 6207 is in sliding fit with the positioning groove 6105.

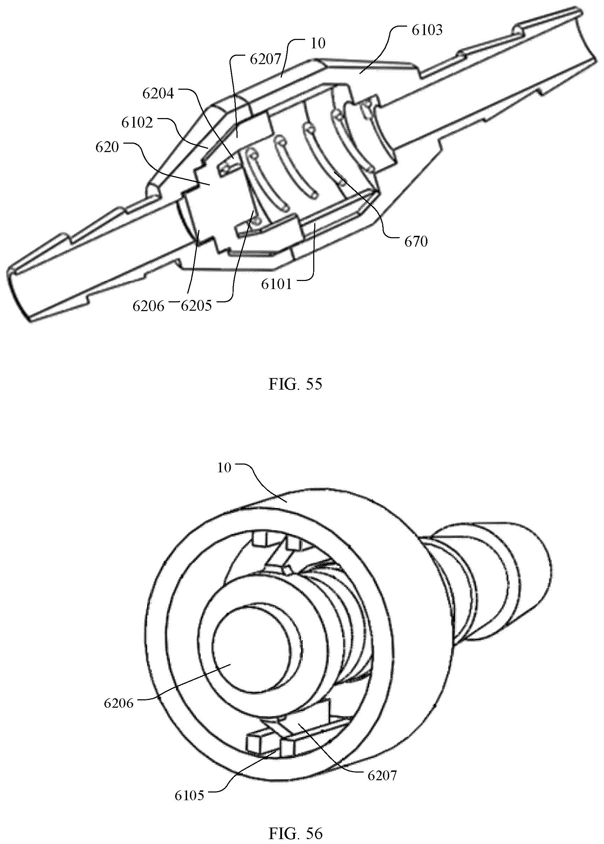

Specifically, as shown in FIG. 52 to FIG. 56, the valve body 620 is slidably connected to the inner wall of the fluid passage 6101. The outer side wall of the valve body 620 is opposed to the inner wall of the fluid passage 6101. At least one positioning rib 6207 is disposed on the outer side wall of the valve body 620. At least one positioning groove 6105 is disposed on the inner wall of the fluid passage 6101. The extension direction of the positioning groove 6105 is consistent with the sliding direction of the valve body 620. The positioning rib 6207 is embedded in the positioning groove 6105. When the valve body 620 slides relative to the fluid passage 6101, the sliding fit of the positioning rib 6207 and the positioning groove 6105 may improve the stability of the valve body 620 when it slides, so as to avoid the problems of jamming and deflection. The quantity of the positioning ribs 6207 matches with the quantity of the positioning grooves 6105. In the embodiment of the present disclosure, as shown in FIG. 56, two positioning ribs 6207 are symmetrically disposed on the outer side wall of the valve body 620. Correspondingly, two positioning grooves 6105 are symmetrically disposed on the inner wall of the fluid passage 6101, further improving the stationarity when the valve body 620 slides.

Embodiments of the present disclosure also disclose a sixth type of a ventilation treatment apparatus including the sixth type of the fire protection device.

Specifically, the sixth type of the ventilation treatment apparatus includes a control device, an oxygen therapy instrument and a gas pipeline. The control device is used for controlling the oxygen supply amount, operation time, etc. of the oxygen therapy instrument. The control device may be an electronic device or a component in the electronic device, such as an integrated circuit or a chip. The electronic device may be a terminal or may be a device other than a terminal. Illustratively, the electronic device may be a cell phone, a tablet computer, a notebook computer, a palmtop computer, a mobile Internet appliance, a robot, a wearable device, etc. and embodiments of the present disclosure are not particularly limited.

In an embodiment of the present disclosure, the sixth type of the fire protection device includes the housing 10 and the valve body 620. The housing 10 has the fluid passage 6101 therein, the fluid passage 6101 is provided with the first narrowed portion 6102 and the second narrowed portion 6103, and the valve body 620 is disposed in the fluid passage 6101. The valve body 620 is slidably connected to the inner wall of the fluid passage 6101, the outer side wall of the valve body 620 is provided with at least one guide portion 630, and the guide portion 630 is in sliding fit with the inner wall of the fluid passage 6101. The valve body 620 has the first position and the second position relative to fluid passage 6101. When the valve body 620 is in the first position, the valve body 620 is in clearance fit with the first narrowed portion 6102 and the second narrowed portion 6103 respectively, and the fluid passage 6101 is in the open state. When the valve body 620 is in the second position, the valve body 620 is engaged with the first narrowed portion 6102 and/or the second narrowed portion 6103, and the fluid passage 6101 is in the closed state, thereby cutting off the oxygen passage from continuously escaping and preventing the continued escape of oxygen resulting in the spread of fire. By providing the guide portion 630 on the outside wall of the valve body 620, the guide portion 630 may act as a limit when the valve body 620 slides with respect to the fluid passage 6101, avoiding the problem of deflection and jamming of the valve body 620, ensuring that the valve body 620 may be accurately engaged with the first narrowed portion 6102 and/or the second narrowed portion 6103, and improving the stability of the fire protection device.

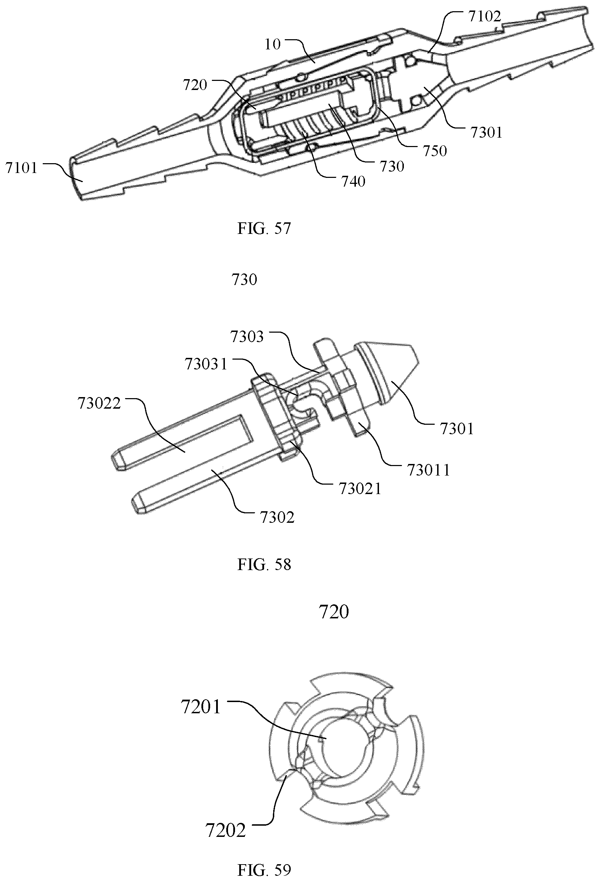

Referring to FIG. 57 to FIG. 62, a seventh type of a fire protection device is disclosed according to an embodiment of the present disclosure, including a housing 10, a base 720, a valve body 730, an elastic member 740, and a meltable collar 750. The housing 10 has a fluid passage 7101 used for communicating with a pipeline of an oxygen therapy instrument or a patient end. The base 720 is positioned in the fluid passage 7101, the valve body 730 is connected to the base 720, and the elastic member 740 is disposed between the base 720 and the valve body 730. One end of the valve body 730 away from the base 720 is provided with a sealing portion 7301, at least one narrowed portion 7102 is disposed in the fluid passage 7101, and the sealing portion 7301 is disposed opposite to the narrowed portion 7102. In the direction of elastic deformation of the elastic member 740, the meltable collar 750 is nested on the whole composed of the base 720, the elastic member 740 and at least part of the valve body 730, to compress the elastic member 740, so that there is a gap for the fluid to pass between the sealing portion 7301 and the narrowed portion 7102. After the meltable collar 750 is fused, the elastic member 740 releases at least part of the elastic potential energy and pushes against the valve body 730, thereby causing the sealing portion 7301 to be clamped with the narrowed portion 7102 to block the fluid passage 7101.

Specifically, as shown in FIG. 57 to FIG. 60, as a main frame of the fire protection device, the housing 10 may be made of a material that is not easily chemically reacted with oxygen and is resistant to high temperatures, such as stainless steel or a ceramic material. It may also be made of fireproof and flame-retardant materials. The housing 10 may be an integrated structure or a split structure. For example, the housing 10 includes a first housing and a second housing which form a complete housing 10 by means of snap-fitting. A sealing structure, such as silicone, rubber, etc. may be filled in the assembly gap between the first housing and the second housing in order to avoid gas leakage.

The housing 10 has the fluid passage 7101 therein used for communicating with a pipeline of an oxygen therapy instrument or a patient end. Since it is necessary to pass through the fluid passage 7101 during the oxygen transmission, the oxygen passage may be blocked by blocking the fluid passage 7101, so as to minimize the spread of fire caused by oxygen enrichment.

The base 720 is positioned in the fluid passage 7101, and the base 720 and the inner wall of the fluid passage 7101 may be fixed by means of bonding, clamping, etc. For example, a clamping groove is disposed on the inner wall of the fluid passage 7101, and the edge of the base 720 is embedded in the clamping groove to achieve fixation. The base 720 is fixed with respect to the housing 10. The valve body 730 is connected to the base 720. An end surface of a side of the base 720 facing towards the valve body 730 is provided with a recessed groove for positioning the valve body 730. The end of the valve body 730 is inserted into the recessed groove to achieve positioning assembly, and the valve body 730 may move in the fluid passage 7101. An elastic member 740 is provided between the valve body 730 and the base 720. Both ends of the elastic member 740 are in contact with the valve body 730 and the base 720, respectively, which may provide a driving force for the movement of the valve body 730. The elastic member 740 may be a metal spring such as iron, copper, alloy, etc. or a soft rubber such as silica gel, rubber, etc. which may store the elastic potential energy.

One end of the valve body 730 away from the base 720 is provided with a sealing portion 7301. A narrowed portion 7102 is disposed in the fluid passage 7101. The inner diameters of the fluid passage 7101 at the two ends of the narrowed portion 7102 are different. The sealing portion 7301 and the narrowed portion 7102 are oppositely arranged and matched in shape. When the valve body 730 drives the sealing portion 7301 to move to the narrowed portion 7102, the fluid passage 7101 may be blocked by means of a snap-fit sealing between the sealing portion 7301 and the narrowed portion 7102. The sealing portion 7301 may be made of a flexible sealing material, such as silicone, rubber, etc. The sealing portion 7301 may also be made of a rigid material. A flexible sealing ring is disposed only at the position where the sealing portion 7301 is close to the narrowed portion 7102. When the sealing portion 7301 abuts against the inner wall of the housing 10 at the narrowed portion 7102, the flexible sealing ring is embedded in the gap between the sealing portion 7301 and the narrowed portion 7102 to achieve sealing.

The meltable collar 750 is made of a material that easily melts or burns when heated, and may be made of an elastic material such as silicone, rubber, etc. or may be made of nylon wire, etc. When the elastic member 740 is compressed by the base 720 and the valve body 730, the elastic member 740 is elastically deformed. In the direction of the elastic deformation, the meltable collar 750 is nested in the whole composed of the base 720, the elastic member 740 and at least part of the valve body 730. The meltable collar 750 may restrain the base 720, the elastic member 740 and the valve body 730. By shortening the distance between the base 720 and the valve body 730, the elastic member 740 is in a compressed state to accumulate the elastic potential energy. Under the constraint of the meltable collar 750, in the axial direction of the fluid passage 7101, a length of the whole composed of the base 720, the elastic member 740 and the valve body 730 is relatively short, so that a gap may be formed between the sealing portion 7301 and the narrowed portion 7102 of the valve body 730. The oxygen may circulate normally, so as to ensure the normal operation of the apparatus.

In the event of a fire, the fire protection device is attacked by a high temperature. The meltable collar 750 is fused by the heating, the restriction function is lost, and at least part of the elastic potential energy is released by the elastic member 740. Since the base 720 is in a fixed state with respect to the housing 10, the elastic member 740 pushes against the valve body 730 which is displaced with respect to the base 720, so that the sealing portion 7301 moves to the narrowed portion 7102 and is engaged with the narrowed portion 7102, thereby blocking the fluid passage 7101 and cutting off the air passage.

In the embodiment of the present disclosure, the base 720, the valve body 730, the elastic member 740 and the meltable collar 750 together constitute a trigger system for the valve body 730. When the meltable collar 750 is not fused, under the constraint of the meltable collar 750, the elastic member 740 is in a compressed state, a gap is provided between the sealing portion 7301 of the valve body 730 and the narrowed portion 7102 of the fluid passage 7101 for the fluid to pass through, and oxygen may normally pass through. After the meltable collar 750 is fused, the elastic member 740 releases at least part of the elastic potential energy and pushes against the valve body 730, causing the sealing portion 7301 to snap-fit with the narrowed portion 7102 to block the fluid passage 7101. Since the meltable collar 750 itself is easily fused when heated, the fusing speed is further accelerated under the elastic force of the elastic member 740, thereby increasing the triggering speed of the valve body 730, reducing the risk of fire spreading due to the failure of triggering in time, thereby improving the safety factor of the fire protection device.

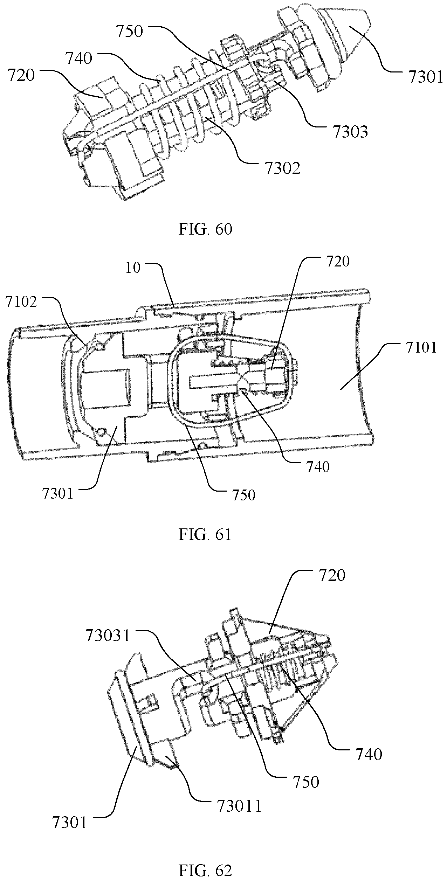

As shown in FIG. 61 to FIG. 62, in an embodiment of the present disclosure, the seventh type of the fire protection device is used for a high-flow oxygen therapy treatment mode. It has few internal parts, is simple in structure, simplifies the assembly mode, and is stable in performance, which may timely cut off the gas path in the event of a fire.

Optionally, as shown in FIG. 57 to FIG. 60, the valve body 730 further includes a bearing portion 7302 connected to the base 720. A sealing portion 7301 is disposed at one end of the bearing portion 7302 away from the base 720. A raised first support structure 73021 is disposed on a peripheral side of the bearing portion 7302. An elastic member 740 is sleeved on the bearing portion 7302. One end of the elastic member 740 abuts against the base 720, and the other end of the elastic member 740 abuts against the first support structure 73021.

Specifically, as shown in FIG. 57 to FIG. 60, the valve body 730 includes the bearing portion 7302 and the sealing portion 7301, where the bearing portion 7302 is connected to the base 720. The bearing portion 7302 adopt a plate-shaped or columnar structure. An end surface of a side of the base 720 facing towards the bearing portion 7302 is provided with a recessed groove, and the end of the bearing portion 7302 is inserted into the recessed groove to achieve positioning assembly. The sealing portion 7301 is provided at an end of the bearing portion 7302 away from the base 720, and an end of the bearing portion 7302 close to the base 720 is in contact with the base 720. A raised first support structure 73021 is disposed on the peripheral side of the bearing portion 7302. The first support structure 73021 may be a structure such as a boss, a flanging, etc. The first support structure 73021 is disposed close to the sealing portion 7301. The first support structure 73021 and the bearing portion 7302 may adopt an integral structure, with good structural strength and stability. The first support structure 73021 may also be fabricated separately from the bearing portion 7302 and assembled by bonding or welding therewith. The elastic member 740 may be a metal spring. The elastic member 740 is sleeved on the bearing portion 7302. Under the constraint of the meltable collar 750, one end of the elastic member 740 abuts against the base 720, the other end abuts against the first support structure 73021, and is in a compressed state.

By sleeving the elastic member 740 over the bearing portion 7302, on the one hand, the space inside the fluid passage 7101 is saved and the stability of assembling the elastic member 740 is ensured.

Optionally, as shown in FIG. 57 to FIG. 60, the bearing portion 7302 is provided with a hollowed-out structure 73022 along its length, and the hollowed-out structure 73022 is used for the passage of the fluid.

Specifically, as shown in FIG. 57 to FIG. 60, the bearing portion 7302 is provided with the hollowed-out structure 73022 along the length direction thereof. The hollowed-out structure 73022 may be a hollowed groove, a hollowed hole or the like. The hollowed-out structure 73022 may reduce the air resistance and avoid the influence on the oxygen flow when the oxygen passes through the fluid passage 7101.

Optionally, as shown in FIG. 57 to FIG. 60, the valve body 730 further includes a connecting portion 7303 disposed between the bearing portion 7302 and the sealing portion 7301 and connected to the bearing portion 7302 and the sealing portion 7301, respectively. The connecting portion 7303 is provided with a clamping structure 73031 for clamping and positioning the meltable collar 750.

Specifically, as shown in FIG. 57 to FIG. 60, the bearing portion 7302 and the sealing portion 7301 are connected by a connecting portion 7303. The bearing portion 7302, the sealing portion 7301, and the connecting portion 7303 may have an integrated structure or may have a split-type structure. The connecting portion 7303 is provided with a clamping structure 73031. The clamping structure 73031 may be a clamping groove, a clamping hook, etc. When the meltable collar 750 is embedded in the whole composed of the base 720, the elastic member 740 and at least a part of the valve body 730. The clamping structure 73031 provides a fixed position for mounting the meltable collar 750, so as to avoid the problem that the meltable collar 750 slips and causes the restriction effect to fail.

Optionally, as shown in FIG. 57 to FIG. 60, the peripheral side of the sealing portion 7301 extends to form a raised guide portion 73011, which is in sliding fit with the inner wall of the fluid passage 7101.

Specifically, as shown in FIG. 57 to FIG. 60, the sealing portion 7301 is provided with the raised guide portion 73011. The guide portion 73011 may be a sliding plate, a boss or the like. The guide portion 73011 may be in sliding fit with the inner wall of the fluid passage 7101, so as to play the role of guiding and positioning, avoiding the problems of jamming and deflection when the valve body 730 moves, and improving the stability of triggering of the valve body 730.

Optionally, as shown in FIG. 57 to FIG. 60, the base 720 is provided with a vent hole 7201. The edge of the base 720 is provided with at least one first positioning groove 7202 for clamping and positioning the meltable collar 750.

Specifically, as shown in FIG. 57 to FIG. 60, the base 720 is provided with a vent hole 7201. When oxygen passes through the fluid passage 7101, the vent hole 7201 may smoothly pass through the base 720, thereby preventing the base 720 from blocking the air passage, reducing the air resistance in the fluid passage 7101, and improving the oxygen flow rate. The base 720 may be a circular base 720. At least one first positioning groove 7202 is disposed on the edge of the base 720. When the meltable collar 750 is wound on the base 720, the first positioning groove 7202 provides a fixed position for mounting the meltable collar 750, and the meltable collar 750 is embedded in the first positioning groove 7202, so that the problem that the meltable collar 750 slips and causes the restraint effect to fail may be avoided.

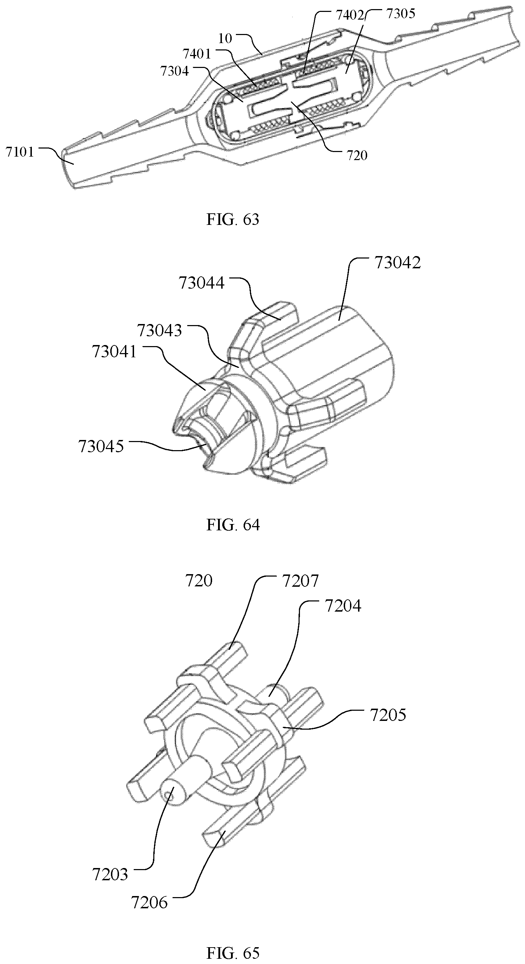

Optionally, as shown in FIG. 63 to FIG. 67, the valve body 730 includes a first valve body 7304 and a second valve body 7305, which are respectively located at both sides of the base 720 and respectively connected to the base 720. The elastic member 740 includes a first elastic member 7401 and a second elastic member 7402. The first elastic member 7401 is disposed between the base 720 and the first valve body 7304. The second elastic member 7402 is disposed between the base 720 and the second valve body 7305. The fluid passage 7101 is provided with a first narrowed portion and a second narrowed portion. One end of the first valve body 7304 away from the base 720 is provided with a first sealing portion 73041, and the first sealing portion 73041 is arranged opposite to the first narrowed portion. One end of the second valve body 7305 away from the base 720 is provided with a second sealing portion 73051, and the second sealing portion 73051 is arranged opposite to the second narrowed portion. Along the elastic deformation direction of the first elastic member 7401 and the second elastic member 7402, the meltable collar 750 is nested on the whole composed of the base 720, the first elastic member 7401, the second elastic member 7402, the first valve body 7304 and the second valve body 7305, so as to compress the first elastic member 7401 and the second elastic member 7402, and gaps are provided between the first sealing portion 73041 and the first narrowed portion and between the second sealing portion 73051 and the second narrowed portion for the passage of fluid. After the meltable collar 750 is fused, the first elastic member 7401 releases at least part of the elastic potential energy and pushes against the first valve body 7304, causing the first sealing portion 73041 to be clamped with the first narrowed portion. The second elastic member 7402 releases at least part of the elastic potential energy and pushes against the second valve body 7305, causing the second sealing portion 73051 to be clamped with the second narrowed portion to block the fluid passage 7101.

Specifically, as shown in FIG. 63 to FIG. 67, in an embodiment of the present disclosure, the valve body 730 includes the first valve body 7304 and the second valve body 7305. The structures of the first valve body 7304 and the second valve body 7305 may be the same. The base 720 is positioned at the middle position of the fluid passage 7101. The base 720 and the inner wall of the fluid passage 7101 may be fixed by means of bonding, clamping, etc. For example, the housing 10 includes a first housing and a second housing which are snap-fitted. The base 720 may be embedded into the assembly gap between the first housing and the second housing to achieve snap-fitting fixation. The first valve body 7304 and the second valve body 7305 are respectively located at two sides of the base 720 and respectively connected to the base 720, that is, the first valve body 7304 and the second valve body 7305 may move in the fluid passage 7101. The first elastic member 7401 is disposed between the base 720 and the first valve body 7304. The second elastic member 7402 is disposed between the base 720 and the second valve body 7305. The first elastic member 7401 and the second elastic member 7402 may be metal springs such as iron, copper and alloy, or soft rubber such as silica gel and rubber which can store the elastic potential energy.

The first narrowed portion and the second narrowed portion are disposed in the fluid passage 7101. The first narrowed portion and the second narrowed portion are respectively close to the openings at both ends of the fluid passage 7101. The inner diameter of the fluid passage 7101 between the first narrowed portion and the second narrowed portion is larger, and the inner diameter of the fluid passage 7101 at both sides is smaller. An end of the first valve body 7304 away from the base 720 is provided with a first sealing portion 73041, and the first sealing portion 73041 is arranged opposite to the first narrowed portion. An end of the second valve body 7305 away from the base 720 is provided with a second sealing portion 73051, and the second sealing portion 73051 is arranged opposite to the second narrowed portion. When the first valve body 7304 drives the first sealing portion 73041 to move to the first narrowed portion, the fluid passage 7101 may be blocked by snap-fit sealing of the first sealing portion 73041 with the first narrowed portion. Similarly, when the second valve body 7305 drives the second sealing portion 73051 to move to the second narrowed portion, the fluid passage 7101 may be blocked by snap-fit sealing of the second sealing portion 73051 with the second narrowed portion. With the structure of the double valve body 730, the function of blocking the air passage may be realized at both ends of the fluid passage 7101, which greatly improves the reliability of the fire protection device.

The meltable collar 750 is nested on the whole composed of the base 720, the first elastic member 7401, the second elastic member 7402, the first valve body 7304, and the second valve body 7305 to restrain the base 720, the first elastic member 7401, the second elastic member 7402, the first valve body 7304, and the second valve body 7305.

When a fire occurs, the fire protection device is attacked by a high temperature, the meltable collar 750 is heated to melt and lose the restraint function. The first elastic member 7401 and the second elastic member 7402 release at least part of the elastic potential energy. The first elastic member 7401 pushes against the first valve body 7304 to make the first sealing portion 73041 snap-fit with the first narrowed portion. The second elastic member 7402 releases at least part of the elastic potential energy and pushes against the second valve body 7305, causing the second sealing portion 73051 to snap-fit with the second narrowed portion, thereby blocking the fluid passage 7101 and cutting off the gas path.

Optionally, as shown in FIG. 63 to FIG. 67, the first valve body 7304 includes a first mounting sleeve 73042. The second valve body 7305 includes a second mounting sleeve 73052. The first sealing portion 73041 is provided at an end of the first mounting sleeve 73042 away from the base 720. The second sealing portion 73051 is provided at an end of the second mounting sleeve 73052 away from the base 720. The base 720 is provided with a raised first positioning portion 7203 towards the first valve body 7304, and the base 720 is provided with a raised second positioning portion 7204 towards the second valve body 7305. The first positioning portion 7203 is at least partially embedded in the first mounting sleeve 73042, and the second positioning portion 7204 is at least partially embedded in the second mounting sleeve 73052. A raised second support structure 73043 is disposed on a peripheral side of the first mounting sleeve 73042. The first elastic member 7401 is sleeved on an outer side wall of the first mounting sleeve 73042. One end of the first elastic member 7401 abuts against the base 720, and the other end of the first elastic member 7401 abuts against the second support structure 73043. A raised third support structure 73053 is disposed on the peripheral side of the second mounting sleeve 73052. The second elastic member 7402 is sleeved on the outer side wall of the second mounting sleeve 73052, one end of the second elastic member 7402 abuts against the base 720, and the other end of the second elastic member 7402 abuts against the third support structure 73053.

Specifically, as shown in FIG. 63 to FIG. 67, the first valve body 7304 includes a first mounting sleeve 73042 and a first sealing portion 73041, and the first sealing portion 73041 is disposed at an end of the first mounting sleeve 73042 away from the base 720. The second valve body 7305 includes a second mounting sleeve 73052 and a second sealing portion 73051, and the second sealing portion 73051 is disposed at an end of the second mounting sleeve 73052 away from the base 720. The first mounting sleeve 73042 and the second mounting sleeve 73052 are respectively located at two sides of the base 720. The openings of the first mounting sleeve 73042 and the second mounting sleeve 73052 both face towards the base 720.

A raised first positioning portion 7203 is provided at a position of the base 720 facing towards the first valve body 7304. A raised second positioning portion 7204 is provided at a position of the base 720 facing towards the second valve body 7305. The shapes of the first positioning portion 7203 and the second positioning portion 7204 may be a cylinder, a prism, etc. The first positioning portion 7203 is embedded in the first mounting sleeve 73042, and the first positioning portion 7203 is in sliding fit with the first mounting sleeve 73042. The second positioning portion 7204 is embedded in the second mounting sleeve 73052, and the second positioning portion 7204 is in sliding fit with the second mounting sleeve 73052. The stability of the assembly between the first valve body 7304, the second valve body 7305 and the base 720 may be improved by using the cooperation of the positioning portions and the mounting sleeve. When the first valve body 7304 and the second valve body 7305 slide with respect to the base 720, the problem of deflection and jamming may not easily occur.

A raised second support structure 73043 is disposed on the peripheral side of the first mounting sleeve 73042. The second support structure 73043 may be a structure such as a boss, a flanging, etc. The first elastic member 7401 may be a metal spring. The first elastic member 7401 is sleeved on an outer side wall of the first mounting sleeve 73042. One end of the first elastic member 7401 abuts against the base 720, and the other end of the first elastic member 7401 abuts against the second support structure 73043.

A raised third support structure 73053 is disposed on a peripheral side of the second mounting sleeve 73052. Similarly, the third support structure 73053 may also be a structure such as a boss, a flanging, etc. The second elastic member 7402 may be a metal spring. The second elastic member 7402 is sleeved on an outer side wall of the second mounting sleeve 73052. One end of the second elastic member 7402 abuts against the base 720, and the other end of the second elastic member 7402 abuts against the third support structure 73053.

Optionally, as shown in FIG. 63 to FIG. 67, a raised flange 7205 is disposed on the peripheral side of the base 720, and two opposite end surfaces of the flange 7205 extend towards the first valve body 7304 and the second valve body 7305 respectively to form a first limiting portion 7206 and a second limiting portion 7207. The second support structure 73043 extends towards the base 720 to form a third limiting portion 73044, and the third support structure 73053 extends towards the base 720 to form a fourth limiting portion 73054. A first limiting space is formed among the first limiting portion 7206, the third limiting portion 73044 and the outer side wall of the first mounting sleeve 73042, and the first elastic member 7401 is embedded in the first limiting space. A second limiting space is formed among the second limiting portion 7207, the fourth limiting portion 73054 and the outer side wall of the second mounting sleeve 73052, and the second elastic member 7402 is embedded in the second limiting space.

Specifically, as shown in FIG. 63 to FIG. 67, the base 720 may be positioned in the fluid passage 7101 via the flange 7205. A clamping groove cooperating with the flange 7205 is disposed on the inner wall of the fluid passage 7101. The flange 7205 is embedded in the clamping groove to fix the base 720. In addition, the flange 7205 has two opposite end surfaces which respectively extend towards the first valve body 7304 and the second valve body 7305 to form the first limiting portion 7206 and the second limiting portion 7207. The shape of the first limiting portion 7206 and the second limiting portion 7207 may be a cylinder, a prism, etc. The second support structure 73043 on the circumferential side of the first mounting sleeve 73042 extends towards the base 720 to form a third limiting portion 73044. The third support structure 73053 on the circumferential side of the second mounting sleeve 73052 extends towards the base 720 to form a fourth limiting portion 73054.

Herein, the first limiting portion 7206 and the third limiting portion 73044 are arranged opposite to each other, and form a first limiting space with the outer side wall of the first mounting sleeve 73042. The first limiting space is used for accommodating the first elastic member 7401. Under the limiting action of the first limiting portion 7206 and the third limiting portion 73044, the first elastic member 7401 may still maintain a relatively stable state when elastically deformed, so that the problems of deflection and jamming do not easily occur.

Similarly, the second limiting portion 7207 and the fourth limiting portion 73054 are arranged opposite to each other, and form a second limiting space with the outer side wall of the second mounting sleeve 73052. The second limiting space is used for accommodating the second elastic member 7402. Under the limiting action of the second limiting portion 7207 and the fourth limiting portion 73054, the second elastic member 7402 may still maintain a relatively stable state when elastically deformed, so that the problems of deflection and jamming does not easily occur.

Optionally, as shown in FIG. 63 to FIG. 67, a second positioning groove 73045 is disposed on the first sealing portion 73041. A third positioning groove 73055 is disposed on the second sealing portion 73051. The second positioning groove 73045 and the third positioning groove 73055 are used for clamping and positioning the meltable collar 750.

Specifically, as shown in FIG. 63 to FIG. 67, when the meltable collar 750 is nested on the whole composed of the base 720, the first elastic member 7401, the second elastic member 7402, the first valve body 7304 and the second valve body 7305. The second positioning groove 73045 and the third positioning groove 73055 may provide a fixed position for installation of the meltable collar 750 when the meltable collar 750 is wound around the first sealing portion 73041 and the second sealing portion 73051. The meltable collar 750 is embedded in the second positioning groove 73045 and the third positioning groove 73055, so that the problem that the meltable collar 750 slips and the restraint function fails may be avoided.

Embodiments of the present disclosure also disclose a seventh type of a ventilation treatment apparatus, including the seventh type of the fire protection device.

Specifically, the seventh type of the ventilation treatment apparatus a control device, an oxygen therapy instrument and a gas pipeline. The control device is used for controlling the oxygen supply amount, operation time, etc. of the oxygen therapy instrument. The control device may be an electronic device or a component in the electronic device, such as an integrated circuit or a chip. The electronic device may be a terminal or may be a device other than a terminal. Illustratively, the electronic device may be a cell phone, a tablet computer, a notebook computer, a palmtop computer, a mobile Internet appliance, a robot, a wearable device, etc. and embodiments of the present disclosure are not particularly limited.

In the embodiment of the present disclosure, the seventh type of the ventilation and treatment apparatus uses the seventh type of the fire protection device. In the fire protection device, the base 720, the valve body 730, the elastic member 740 and the meltable collar 750 together constitute a trigger system for the valve body 730. When the meltable collar 750 is not fused, under the constraint of the meltable collar 750, the elastic member 740 is in a compressed state, a gap is provided between the sealing portion 7301 of the valve body 730 and the narrowed portion 7102 of the fluid passage 7101 for the fluid to pass through, and oxygen may normally pass through. After the meltable collar 750 is fused, the elastic member 740 releases at least part of the elastic potential energy and pushes against the valve body 730, causing the sealing portion 7301 to snap-fit with the narrowed portion 7102 to block the fluid passage 7101. Since the meltable collar 750 itself is easily fused when heated, the fusing speed is further accelerated under the elastic force of the elastic member 740, thereby increasing the triggering speed of the valve body 730, reducing the risk of fire spreading due to delayed triggering, thereby improving the safety factor of the fire protection device.

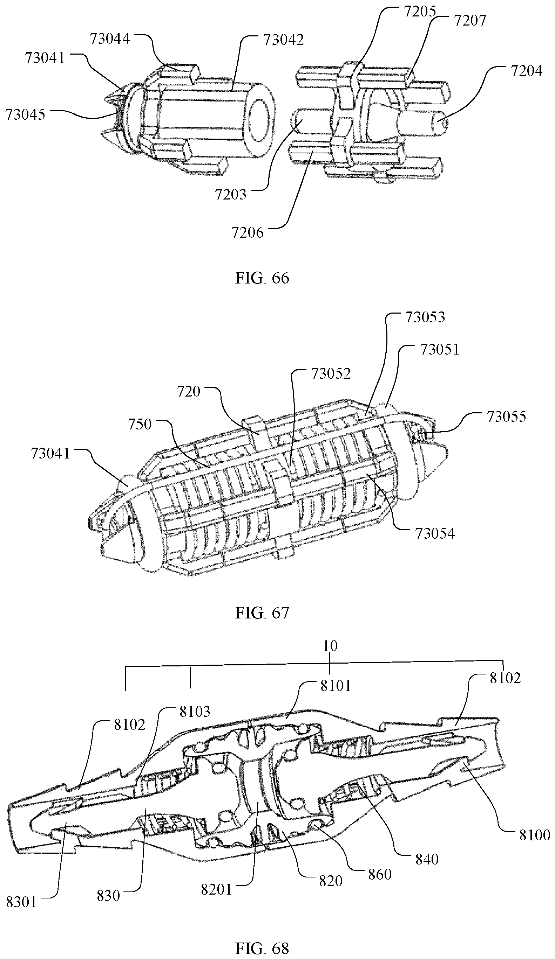

As shown in FIG. 68 to FIG. 69, the present disclosure discloses an eighth type of a fire protection device including a housing 10, an inner shell 820, a valve body 830 and an elastic member 840. The housing 10 has a fluid passage for communicating with a pipeline of an oxygen therapy instrument or a patient end. The inner shell 820 is disposed in the fluid passage and is in sealed connection with the inner wall of the fluid passage, and the inner shell 820 is provided with a through-hole 8201 for the fluid to pass through. The valve body 830 is slidably connected to the inner wall of the fluid passage and has a first position and a second position relative to the fluid passage. In the first position, there is a gap between the valve body 830 and the through-hole 8201 for the fluid to pass through. In the second position, the valve body 830 abuts against the peripheral side of the through-hole 8201 so that the fluid passage is blocked. The elastic member 840 is disposed between the valve body 830 and the inner wall of the fluid passage. The inner walls of the two ends of the fluid passage are respectively provided with at least one raised support portion 8100. One end of the valve body 830 is arranged opposite to the through-hole 8201, and the other end of the valve body 830 is used for being clamped with the support portion 8100 so as to support the valve body 830 to be in the first position, and the elastic member 840 is in a compressed state. Upon melting of at least one of the support portion 8100 and the valve body 830, the elastic member 840 releases at least part of the elastic potential energy to drive the valve body 830 from the first position to the second position.

Specifically, as a main 8101 frame of the fire protection device, the housing 10 may be made of a material that is not easily chemically reacted with oxygen and is resistant to high temperatures, such as stainless steel or a ceramic material. It may also be made of fireproof and flame-retardant materials. The fluid passage is disposed in the housing 10, and both ends of the fluid passage have openings. The fluid passage is used for communicating with a pipeline of an oxygen therapy instrument or a patient end. Since it is necessary to pass through the fluid passage during the oxygen transmission, the oxygen passage may be blocked by blocking the fluid passage, so as to minimize the spread of fire caused by oxygen enrichment.

The inner shell 820 is disposed within the fluid passage. The inner shell 820 is preferably disposed at an intermediate position of the fluid passage. The outer wall of the inner shell 820 is in a sealed connection with the inner wall of the fluid passage. The inner shell 820 and the housing 10 may be assembled by means of bonding, ultrasonic welding, etc. The inner shell 820 itself is further provided with a through-hole 8201. When oxygen is introduced into the fluid passage, the oxygen may only pass through the through-hole 8201, and cannot pass through the assembly gap between the inner shell 820 and the housing 10. Therefore, only the through-hole 8201 on the inner shell 820 needs to be closed to realize gas path blocking. In the absence of fire, oxygen may be normally transmitted through the through-hole 8201. The shape of the through-hole 8201 may be circular, elliptical, semicircular, etc.

The valve body 830 is slidably connected to the inner wall of the fluid passage. The valve body 830 has a first position and a second position relative to the fluid passage. When the valve body 830 is in the first position, a gap is provided between the valve body 830 and the through-hole 8201 of the inner shell 820 for the fluid to pass through. Oxygen may normally be transmitted through the through-hole 8201. When the valve body 830 is in the second position, the valve body 830 abuts against the peripheral side of the through-hole 8201 so that the fluid passage is blocked, and the fluid passage is blocked by the cooperation of the valve body 830 and the through-hole 8201. One end of the valve body 830 for abutting the peripheral side of the through-hole 8201 may be made of a flexible sealing material, such as silica gel, rubber, etc. The valve body 830 may also be made of a rigid material, and a flexible sealing ring is disposed only at one end of the valve body 830 close to the through-hole 8201. When the valve body 830 abuts against the peripheral side of the through-hole 8201, the sealing ring is embedded in the gap between the valve body 830 and the peripheral side of the through-hole 8201 to achieve sealing.

An elastic member 840 is disposed between the valve body 830 and the inner wall of the fluid passage for driving the valve body 830 from the first position to the second position. The elastic member 840 may be a metal spring such as iron, copper, alloy, etc. or a soft rubber such as silica gel, rubber, etc. which may store the elastic potential energy. The inner walls at both ends of the fluid passage are provided with at least one raised support portion 8100, which can be snap-fitted with the other end of the valve body 830, so that the valve body 830 is positioned in the first position, ensuring the conduction of the fluid passage in the absence of a fire. The support portion 8100 may be made of a material with a relatively low melting point, such as PP, PVC, etc.

When the valve body 830 is in the first position, the elastic member 840 will be pressed by the inner wall of the fluid passage and the valve body 830, and is in a compressed state to accumulate the elastic potential energy. When the support portion 8100 loses the clamping action on the valve body 830, the force balance is broken, and the valve body 830 moves to the second position under the elastic force of the elastic member 840, and abuts against the peripheral side of the through-hole 8201, so that the air passage is blocked.

In the event of a fire, the valve body 830 may be activated by the followings. Firstly, the support portion 8100 melts. The support portion 8100 loses the clamping action on the valve body 830. Under the elastic force of the elastic member 840, the valve body 830 moves to a second position to abut against the peripheral side of the through-hole 8201, so as to block the fluid passage. Secondly, one end of the valve body 830 close to the support portion 8100 is melted, and the end of the valve body 830 close to the support portion 8100 may be made of a material with a lower melting point, such as PP, PVC, etc. After the end of the valve body 830 close to the support portion 8100 is fused, the support portion 8100 will also lose the clamping action on the valve body 830. Under the action of the elastic force of the elastic member 840, the valve body 830 moves to the second position to abut against the peripheral side of the through-hole 8201, so as to block the fluid passage. Thirdly, the support portion 8100 and the end of the valve body 830 close to the support portion 8100 melt at the same time. The support portion 8100 loses the clamping action on the valve body 830 and may also realize fluid passage blocking.

In the embodiment of the present disclosure, the triggering system composed of the support portion 8100, the elastic member 840 and the valve body 830, when any one of the valve body 830 and the support portion 8100 melts, may move the valve body 830 to the second position in time and abut against the peripheral side of the through-hole 8201, thereby blocking the gas path, increasing the trigger speed of the valve body 830, reducing the risk of fire spreading due to the delayed triggering S, and thus improving the safety factor of the fire protection device. Also, the fire protection device has a simple structure, is easy to assemble, and also reduces production costs and installation costs.

Optionally, the support portion 8100 is disposed on the inner wall of the fluid passage close to the opening.

Specifically, the support portion 8100 and the inner wall at the opening of the fluid passage may be assembled by bonding or welding, or the support portion 8100 and the housing may be integrally formed. The support portion 8100 is disposed on the inner wall of the fluid passage close to the opening. Since a fire usually occurs outside the fire protection device, the opening of the fluid passage will be attacked by the fire. The support portion 8100 is disposed on the inner wall of the fluid passage close to the opening, so that the support portion 8100 and the end of the valve body 830 close to the support portion 8100 will be at a high temperature state and fused firstly, improving the timeliness of triggering the valve body 830.

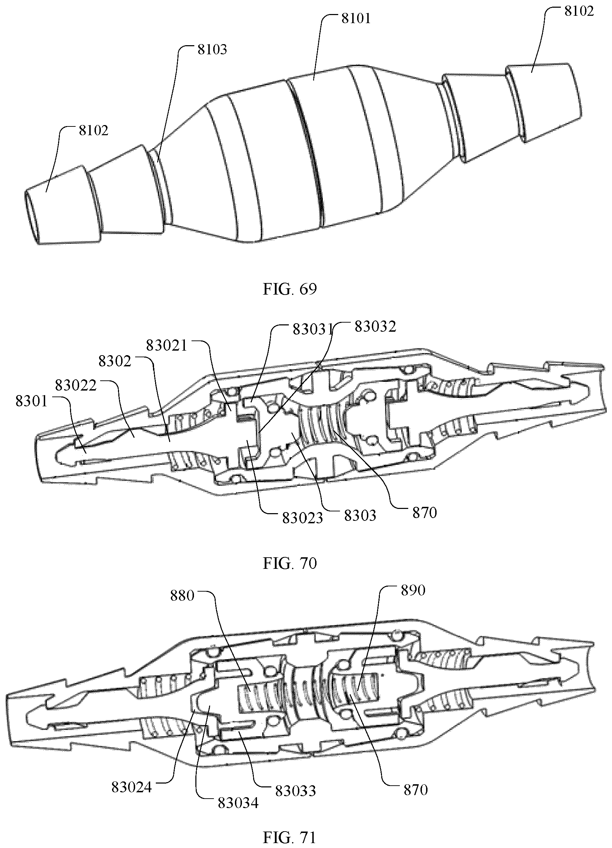

Optionally, with reference to FIG. 70 to FIG. 71, the valve body 830 includes a clamping member 8302 and a sealing member 8303. Herein, the clamping member 8302 and the sealing member 8303 are of a split-type structure, and the clamping member 8302 and the sealing member 8303 are respectively slidably connected with the inner wall of the fluid passage. The clamping member 8302 is provided with a fusible portion 8301, and the elastic member 840 is provided between the clamping member 8302 and the inner wall of the fluid passage. One end of the clamping member 8302 is used for being clamped with the support portion 8100, and the other end of the clamping member 8302 is used for pushing against the sealing member 8303, so that the sealing member 8303 abuts against the peripheral side of the through-hole 8201. An assembly gap is provided between the clamping member 8302 and the sealing member 8303 for the fluid to pass through.

Specifically, the valve body 830 includes the clamping member 8302 and the sealing member 8303. The clamping member 8302 and the sealing member 8303 are of a split-type structure and have the assembly gap through which oxygen can pass, so as to reduce the air resistance brought by the valve body 830 and improve the oxygen flux and comfortableness. The clamping member 8302 and the sealing member 8303 are respectively slidably connected to the inner wall of the fluid passage. When one end of the clamping member 8302 is clamped with the support portion 8100, the positioning and fixing of the clamping member 8302 can be achieved, so that the clamping member 8302 is in a stationary state. The clamping member 8302 may achieve clamping and fitting with the support portion 8100 through a structure such as a clamping hook and a clamping round head. The elastic member 840 is arranged between the clamping member 8302 and the inner wall of the fluid passage. Under the compression of the clamping member 8302 and the inner wall of the fluid passage, the elastic member 840 accumulates the elastic potential energy. The sealing member 8303 is in clearance fit with the through-hole 8201 of the inner shell 820. When in use, oxygen may be normally transmitted through the gap between the sealing member 8303 and the through-hole 8201 of the inner shell 820. The sealing member 8303 may be made of flexible sealing materials, such as silicone, rubber, etc. The sealing member 8303 may also be made of a rigid material. A flexible sealing ring is disposed only on one side of the sealing member 8303 close to the through-hole 8201. When the sealing member 8303 abuts against the peripheral side of the through-hole 8201, the sealing ring is embedded in the gap between the sealing member 8303 and the peripheral side of the through-hole 8201 to achieve sealing.

The above-mentioned fusible portion 8301 is disposed on the clamping member 8302. The fusible portion 8301 may be made of a material with a relatively low melting point, such as PP, PVC, etc. The fusible portion may also be realized by structural design. For example, the fusible portion 8301 adopts a structure with a relatively small diameter or thickness, and is easily fused. After the fusible portion 8301 is fused, the clamping member 8302 may be broken at the fusible portion 8301. When any one of the fusible portion 8301 and the support portion 8100 melts, the support portion 8100 loses the clamping action on the clamping member 8302. Thus, when the elastic member 840 releases at least part of the elastic potential energy, the other end of the clamping member 8302 pushes against the sealing member 8303 under the elastic force, so that the sealing member 8303 abuts against the peripheral side of the through-hole 8201, and the fluid passage is blocked.

Optionally, as shown in FIG. 70, one end of the clamping member 8302 close to the sealing member 8303 is provided with a raised first guide portion 83021. The first guide portion 83021 is in sliding fit with the inner wall of the fluid passage. The elastic member 840 is sleeved on the clamping member 8302. One end of the elastic member 840 abuts against the inner wall of the fluid passage, and the other end of the elastic member 840 abuts against the first guide portion 83021. When the clamping member 8302 is clamped with the support portion 8100, the elastic member 840 is compressed by the inner wall of the fluid passage and the first guide portion 83021. When the support portion 8100 and/or the fusible portion 8301 is in the melted state, the elastic member 840 releases at least part of the elastic potential energy to drive the sealing member 8303 to abut against the peripheral side of the through-hole 8201, so that the fluid passage is blocked.

Specifically, one end of the clamping member 8302 close to the sealing member 8303 is provided with the raised first guide portion 83021. The first guide portion 83021 may be a sliding plate, a boss or the like. A quantity of the first guide portions 83021 may be selected according to practical requirements. The material of the first guide portion 83021 may be the same as that of the clamping member 8302, and is made of a material with fire-retardant and wear-resistant characteristics. The first guide portion 83021 and the clamping member 8302 may have an integrated structure, and have good structural strength and stability. The first guide portion 83021 may be formed separately from the clamping member 8302 and assembled by bonding or welding. The first guide 83021 has two functions. Firstly, a stable support point may be provided for the elastic member 840. Secondly, the first guide portion 83021 may be in sliding fit with the inner wall of the fluid passage to play the role of guiding and positioning, so as to avoid the problems of jamming and deflection of the clamping member 8302 when moved, and improve the stability of triggering of the valve body 830.

The elastic member 840 may be a metal spring, and the metal spring is sleeved on the clamping member 8302. When the clamping member 8302 is clamped with the meltable support, both ends of the elastic member 840 respectively abut against the first guide portion 83021 and the inner wall of the fluid passage, and are in a compressed state to accumulate the elastic potential energy. When the support portion 8100 and/or the fusible portion 8301 are in the molten state, the support portion 8100 loses the clamping action on the clamping member 8302. The elastic member 840 releases at least part of the elastic potential energy to drive the sealing member 8303 into contact with the peripheral side of the through-hole 8201 so that the fluid passage is blocked.

By providing the elastic member 840, when the support portion 8100 and/or the fusible portion 8301 are melted, it may provide driving force for the clamping member 8302 and the sealing member 8303, so that the sealing member 8303 moves rapidly and abuts against the peripheral side of the through-hole 8201. At the same time, the elastic member 840 is sleeved on the clamping member 8302. It not only saves the space inside the fluid passage, but also ensures the stability of the assembly of the elastic member 840.

Optionally, as shown in FIG. 68 to FIG. 69, the fluid passage includes a main body 8101 and opening portions 8102 disposed at both ends of the main body 8101, where the inner diameter of the opening portions 8102 is less than the inner diameter of the main body 8101. The connection between the main body 8101 and the opening 8102 narrows to form a shoulder 8103. The support portion 8100 is disposed on the inner wall of the opening portion 8102. One end of the elastic member 840 abuts against the shoulder 8103, and the other end of the elastic member 840 abuts against the first guide portion 83021.

Specifically, the fluid passage includes the main body 8101 and the opening portions 8102 provided at both ends of the main body 8101. The opening portion 8102 is used for communicating with a pipeline of a patient end or an oxygen therapy instrument end. For the convenience of assembly, the inner diameter of the opening portion 8102 is less than the inner diameter of the main body 8101, forming a structure with thick in the middle and thin at the ends. The main body 8101 transitions from the middle to both ends, and narrows at the connection with the opening portion 8102 to form the shoulder 8103 used for supporting the elastic member 840.

The support portion 8100 is disposed on the inner wall of the opening portion 8102, and the sealing member 8303 is disposed inside the main body 8101. The sealing member 8303 is far away from the support portion 8100 and is not easily affected by the high temperature of combustion, which is beneficial to improve the performance of the sealing member 8303 and improve the stability of the fire protection device.

Optionally, as shown in FIG. 70, the clamping member 8302 is provided with a raised second guide portion 83022 which is in sliding fit with the inner wall of the opening portion 8102.

Specifically, the clamping member 8302 is provided with a raised second guide portion 83022. The second guide portion 83022 may have a structure such as a sliding plate and a boss. The second guide portion 83022 may slidably fit with the inner wall of the opening portion 8102 to play a guiding and positioning role, so as to avoid the problems of jamming and deflection of the clamping member 8302 when moved, and improve the triggering stability of the valve body 830.

Optionally, as shown in FIG. 70, a side of the sealing member 8303 facing towards the clamping member 8302 has an end surface. A peripheral side of the end surface extends towards the clamping member 8302 to form a third guide portion 83031, and the third guide portion 83031 is in sliding fit with the inner wall of the fluid passage.

In particular, the side of the sealing member 8303 facing towards the clamping member 8302 has an end surface, and the clamping member 8302 may push against the sealing member 8303 by contacting the end surface of the sealing member 8303. The peripheral side of the end surface extends towards the clamping member 8302 to form a third guide portion 83031. The third guide portion 83031 is capable of sliding fit with the inner wall of the fluid passage, serving as a guide and positioning function, avoiding the problems of jamming and deflection of the sealing member 8303 when moved, and improving the stability of triggering of the valve body 830.

Optionally, as shown in FIG. 70, the third guide portion 83031 is combined with the end surface to form a limiting groove 83032. The clamping member 8302 extends towards one end of the sealing member 8303 to form a limiting portion 83023 which is at least partially embedded in the limiting groove 83032.

Specifically, the third guide portion 83031 is combined with the end surface to form a limiting groove 83032. The limiting groove 83032 is U-shaped, and has an opening side facing towards the clamping member 8302. The clamping member 8302 extends towards one end of the sealing member 8303 to form a limiting portion 83023 which may have a boss structure. When the clamping member 8302 pushes against the sealing member 8303, the limiting portion 83023 is at least partially embedded in the limiting groove 83032, so as to avoid the problems of shaking and blocking when the clamping member 8302 and the sealing member 8303 are moved, and improve the triggering stability of the valve body 830.

Optionally, as shown in FIG. 71, the outer side wall of the sealing member 8303 extends to form a raised fourth guide portion 83033 which is in sliding fit with the inner wall of the fluid passage.

Specifically, as a variant, the raised fourth guide portion 83033 may also be provided by extending at the outer side wall of the sealing member 8303. The fourth guide portion 83033 may be a sliding plate, a boss or the like. The fourth guide portion 83033 may be in sliding fit with the inner wall of the fluid passage to play the role of guiding and positioning, so as to avoid the problems of jamming and deflection of the sealing member 303 when moved, and improve the triggering stability of the valve body 830.

Optionally, as shown in FIG. 71, one end of the clamping member 8302 facing towards the sealing member 8303 is provided with a groove 83024. One end of the sealing member 8303 facing towards the clamping member 8302 is provided with a boss 83034 which is at least partially embedded in the groove 83024.

Specifically, in order to ensure the stability of the assembly between the clamping member 8302 and the sealing member 8303, shaking is avoided. With regard to the problem of deflection, a groove 83024 may be disposed on the clamping member 8302. A boss 83034 may be disposed on the sealing member 8303. The groove 83024 is arranged opposite to the boss 83034 and has a matched shape.

Optionally, as shown in FIG. 68 to FIG. 71, a sealing structure 860 is disposed in the assembly gap of the housing 10 and the inner shell 820.

Specifically, the assembly gap is provided between the housing 10 and the inner shell 820. In order to prevent oxygen from passing through the assembly gap between the housing 10 and the inner shell 820 in a case that the through-hole 8201 of the inner shell 820 is closed, the sealing structure 860 is disposed in the assembly gap between the housing 10 and the inner shell 820. The sealing structure 860 may be a silicone sealing ring, a rubber sealing ring, etc. The sealing structure 860 fills the assembly gap under the compression of the housing 10 and the inner shell 820 to achieve a sealed connection between the housing 10 and the inner shell 820.

Optionally, as shown in FIG. 68 to FIG. 71, the quantity of the support portions 8100 is two or more. The support portions 8100 are provided at intervals along the circumferential direction of the inner wall of the opening portion 8102.

Specifically, the support portion 8100 is used to support the valve body 830 in the first position. The stability of the clamping between the support portion 8100 and the valve body 830 directly affects the performance of the fire protection device. In order to avoid the problem that the clamping action between the support portion 8100 and the valve body 830 fails and the fluid passage is erroneously blocked, the quantity of the support portions 8100 is two or more. The support portions 8100 are arranged at intervals along the circumferential direction of the inner wall of the opening portion 8102 to achieve multiple clamping of the clamping portion of the valve body 830, thereby improving the stability of the clamping. At the same time, the problem that the valve body 830 deflects due to uneven force may also be avoided, so as to improve the stability of triggering of the valve body 830.

Optionally, as shown in FIG. 68 to FIG. 71, the valve body 830 includes a first valve body and a second valve body. The first valve body and the second valve body are symmetrically disposed at two sides of the through-hole 8201. When at least one of the first valve body and the second valve body abuts against the peripheral side of the through-hole 8201, the fluid passage is in the blocked state.

Specifically, the fire protection device includes two first and second valve bodies symmetrically disposed in the fluid passage, the first valve body and the second valve body have the same structure and are respectively located on both sides of the through-hole 8201. The elastic members 840 are disposed between the first valve body and the inner wall of the fluid passage and between the second valve body and the inner wall of the fluid passage. The support portions 8100 are respectively disposed in the opening portions 8102 at both ends of the main body 8101 for being clamped with the first valve body and the second valve body, respectively.

The fire protection device adopts the structure of double valve body 830. Any valve body 830 may be triggered to realize the blocking of the fluid passage, which ensures the timeliness of triggering the fire protection device. When installing, it is not necessary to distinguish the port sequence of the fire protection device. Any opening end may be assembled and communicated with the pipeline of the oxygen therapy instrument or the patient end, which greatly improves the installation convenience.

Optionally, as shown in FIG. 70 and FIG. 71, an elastic support 870 is disposed between the first valve body and the second valve body, and the elastic support 870 passes through the through-hole 8201. Two ends of the elastic support 870 respectively abut against the first valve body and the second valve body. Here, the elastic force of the elastic support 870 is less than that of the elastic member 840.

Specifically, when the first valve body or the second valve body adopts a split-type structure, the clamping member 8302 and the sealing member 8303 are respectively included. When not pushed against by the clamping member 8302, a relative movement occurs between the sealing member 8303 and the through-hole 8201 of the inner shell 820. The sealing member 8303 makes an unnecessary contact with the peripheral side of the through-hole 8201 of the inner shell 820, thereby easily causing the gas path to be blocked and affecting normal ventilation. Therefore, the elastic support 870 is provided between the first valve body and the second valve body. The elastic support 870 may be a metal spring such as iron, copper, an alloy, etc. or a soft rubber such as silica gel, rubber, etc. which can store elastic potential energy. The elastic support 870 passes through the through-hole 8201 so as not to block the normal transmission of oxygen. Two ends of the elastic support 870 respectively abut against the first valve body and the second valve body so as to support the two sealing members 8303 and prevent the sealing members 8303 from contacting the peripheral side of the through-hole 8201 in a normal use state.

The elastic force of the elastic support 870 is less than the elastic force of the elastic member 840. When the support portion 8100 and/or the fusible portion 8301 are in the molten state, the support portion 8100 loses the clamping action on the clamping member 8302, the elastic member 840 releases at least part of the elastic potential energy, and the elastic support 870 does not obstruct the movement of the sealing member 8303, so that the sealing member 8303 may smoothly abut against the peripheral side of the through-hole 8201 and the fluid passage is in a blocked state.

Optionally, as shown in FIG. 71, one end of the first valve body facing towards the second valve body is provided with a first mounting groove 880. One end of the second valve body facing towards the first valve body is provided with a second mounting groove 890. The first mounting groove 880 is arranged opposite to the second mounting groove 890. The elastic support 870 is at least partially embedded into the first mounting groove 880 and the second mounting groove 890.

Specifically, by providing the first mounting groove 880 and the second mounting groove 890, the elastic support 870 may be accommodated, and also play a limiting function that the elastic support 870 is prevented from being shaken or deflected.

In a second aspect, as shown in FIG. 69 to FIG. 72, the disclosed embodiment discloses a fire protection device including a housing 10 and a valve body 830. The housing 10 has a fluid passage for communicating with a pipeline of an oxygen therapy instrument or a patient end. The valve body 830 is disposed within the fluid passage, the valve body 830 having a first position and a second position relative to the fluid passage. In the first position, a gap is provided between the valve body 830 and the fluid passage for the passage of gas. In the second position, the valve body 830 blocks the fluid passage. The inner walls at both ends of the fluid passage are respectively provided with at least one raised meltable support portion 850. When the meltable support portion 850 is in a non-molten state, the valve body 830 is clamped with the meltable support portion 850 to support the valve body 830 to be in the first position. With the meltable support portion 850 in a molten state, the valve body 830 switches from the first position to the second position.

Specifically, as a main 8101 frame of the fire protection device, the housing 10 may be made of a material that is not easily chemically reacted with oxygen and is resistant to high temperatures, such as stainless steel or a ceramic material. It may also be made of fireproof and flame-retardant materials. The fluid passage is disposed in the housing 10, and both ends of the fluid passage have openings. The fluid passage is used for communicating with the pipeline of the oxygen therapy instrument or the patient end. Since it is necessary to pass through the fluid passage during the oxygen transmission, the oxygen passage may be blocked by blocking the fluid passage, so as to minimize the spread of fire caused by oxygen enrichment.

The valve body 830 is slidably connected to the inner wall of the fluid passage. The valve body 830 has the first position and the second position relative to the fluid passage. When the valve body 830 is in the first position, a gap is provided between the valve body 830 and the fluid passage for the gas to pass through, and oxygen may normally be transmitted through the fluid passage. When the valve body 830 is in the second position, the fluid passage is in a blocked state. Specially, the blocking of the fluid passage may be achieved by the cooperation of the valve body 830 and the inner wall or opening of the fluid passage. For example, when the valve body 830 is in the second position, the valve body abuts against a shoulder of the fluid passage to block the fluid passage.

The one end of the valve body 830 used for sealing and blocking the fluid passage may be made of a flexible sealing material, such as silica gel, rubber, etc. The valve body 830 may also be made of a rigid material, with a flexible sealing ring disposed only at one end of the valve body 830 for sealing the blocked fluid passage.

The inner walls of the two ends of the fluid passage are provided with at least one raised meltable support portion 850. The meltable support portion 850 may be clamped with the other end of the valve body 830, so that the valve body 830 is positioned in the first position, ensuring that the fluid passage is unobstructed in the case of no fire. The meltable support portion 850 may be made of a material with a relatively low melting point, such as PP, PVC, etc. and is easily fused when exposed to a high temperature.

In the event of a fire, the meltable support portion 850 melts, the meltable support portion 850 loses its clamping action on the valve body 830. The valve body 830 moves to the second position to block the fluid passage.

In the embodiment of the present application, the meltable support portion 850 is used. When the meltable support portion 850 melts, the valve body 830 may be moved to the second position in time and the gas path can be blocked. Since the meltable support portion 850 is made of a fusible material, the response speed is relatively fast, the triggering speed of the valve body 830 is increased. The risk of fire spreading due to the triggering not being timely is reduced, and thus the safety factor of the fire protection device is improved. Also, the fire protection device has a simple structure, is easy to assemble, and which also reduces production costs and installation costs.

Optionally, the fire protection device further includes an elastic member 840. The elastic member 840 is provided between the valve body 830 and the inner wall of the fluid passage. The elastic member 840 is in a compressed state when the valve body 830 is in the first position. The elastic member 840 is used to provide an elastic driving force when the valve body 830 is switched from the first position to the second position.

Specifically, the elastic member 840 is provided between the valve body 830 and the inner wall of the fluid passage for driving the valve body 830 to move from the first position to the second position. The elastic member 840 may be a metal spring such as iron, copper, alloy, etc. or a soft rubber such as silica gel, rubber, etc. which may store the elastic potential energy.

When the valve body 830 is in the first position, the elastic member 840 will be pressed by the inner wall of the fluid passage and the valve body 830, and is in a compressed state to accumulate the elastic potential energy. When the support portion loses the clamping action on the valve body 830, the force balance is broken, and the valve body 830 moves to the second position under the elastic force of the elastic member 840, so that the air passage is blocked. The triggering speed of the valve body 830 is further increased under the effect of the elastic member 840.

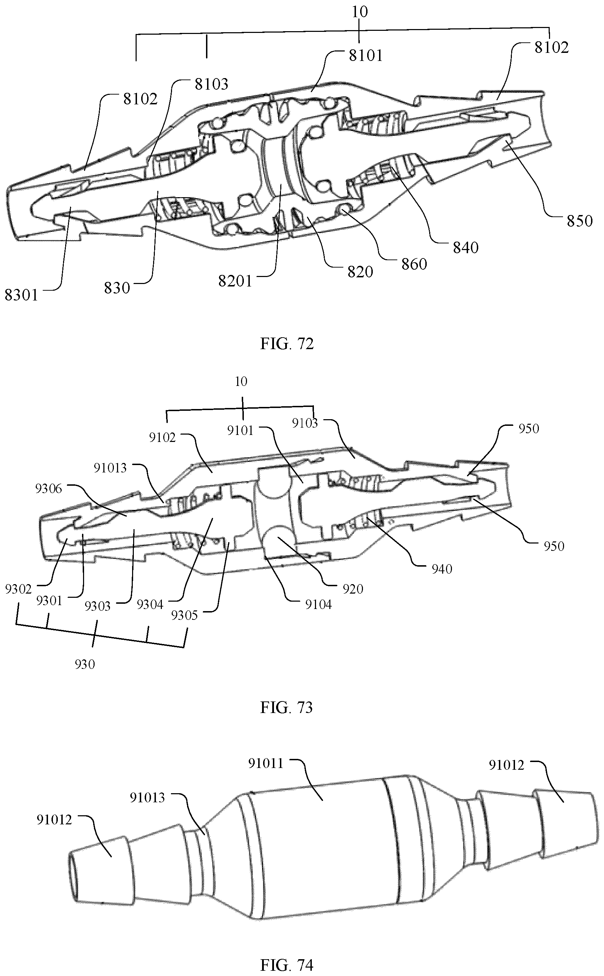

Optionally, with reference to FIG. 70 and FIG. 72, the valve body 830 includes a clamping member 8302 and a sealing member 8303. Herein, the clamping member 8302 and the sealing member 8303 are of a split-type structure, and the clamping member 8302 and the sealing member 8303 are respectively slidably connected with the inner wall of the fluid passage. The elastic member 840 is disposed between the clamping member 8302 and the inner wall of the fluid passage. One end of the clamping member 8302 is used for being clamped with the meltable support portion 850, and the other end of the clamping member 8302 is used for pushing against the sealing member 8303. An assembly gap is provided between the clamping member 8302 and the sealing member 8303 for the fluid to pass through.

Specifically, the valve body 830 includes the clamping member 8302 and the sealing member 8303. The clamping member 8302 and the sealing member 8303 are of a split-type structure and have an assembly gap through which oxygen can pass, so as to reduce the air resistance brought by the valve body 830 and improve the oxygen flux and comfortableness. The clamping member 8302 and the sealing member 8303 are respectively slidably connected to the inner wall of the fluid passage. When one end of the clamping member 8302 is clamped with the meltable support portion 850, the positioning and fixing of the clamping member 8302 can be achieved, so that the clamping member 8302 is in a stationary state. The clamping member 8302 may achieve clamping and fitting with the meltable support portion 850 through a structure such as a clamping hook and a clamping round head. The elastic member 840 is arranged between the clamping member 8302 and the inner wall of the fluid passage. Under the compression of the clamping member 8302 and the inner wall of the fluid passage, the elastic member 840 accumulates the elastic potential energy. The sealing member 8303 may be made of a flexible sealing material, such as silicone, rubber, etc. The sealing member 8303 may also be made of a rigid material, with a flexible sealing ring disposed on only one side of the sealing member 8303.

Optionally, as shown in FIG. 70, one end of the clamping member 8302 close to the sealing member 8303 is provided with a raised first guide portion 83021. The first guide portion 83021 is in sliding fit with the inner wall of the fluid passage. The elastic member 840 is sleeved on the clamping member 8302. One end of the elastic member 840 abuts against the inner wall of the fluid passage, and the other end of the elastic member 840 abuts against the first guide portion 83021. When the clamping member 8302 is clamped with the meltable support portion 850, the elastic member 840 is compressed by the inner wall of the fluid passage and the first guide portion 83021.

Specifically, one end of the clamping member 8302 close to the sealing member 8303 is provided with a raised first guide portion 83021. The first guide portion 83021 may be a sliding plate, a boss or the like. The quantity of the first guide portions 83021 may be selected according to practical requirements. The material of the first guide portion 83021 may be the same as that of the clamping member 8302, and is made of a material with fire-retardant and wear-resistant properties. The first guide portion 83021 and the clamping member 8302 may be of an integrated structure, and have good structural strength and stability. The first guide portion 83021 may be formed separately from the clamping member 8302 and assembled by bonding or welding. The first guide 83021 has two functions. Firstly, a stable support point may be provided for the elastic member 840. Secondly, the first guide portion 83021 may be in sliding fit with the inner wall of the fluid passage to play the role of guiding and positioning, so as to avoid the problems of jamming and deflection of the clamping member 8302 when moved, and improve the stability of triggering of the valve body 830.

The elastic member 840 may be a metal spring, and the metal spring is sleeved on the clamping member 8302. When the clamping member 8302 is clamped with the meltable support, both ends of the elastic member 840 respectively abut against the first guide portion 83021 and the inner wall of the fluid passage, and are in a compressed state to accumulate the elastic potential energy. When the meltable support portion 850 is in the molten state, the meltable support portion 850 loses its clamping action on the clamping member 8302 and the elastic member 840 releases at least part of the elastic potential energy to drive the valve body 830 in the second position, so that the fluid passage is in the blocked state.

By providing the elastic member 840, it is possible to provide a driving force for the clamping member 8302 and the sealing member 8303 to rapidly move the sealing member 8303 and block the gas path when the meltable support portion 850 is melted. At the same time, the elastic member 840 is sleeved on the clamping member 8302. It not only saves the space inside the fluid passage, but also ensures the stability of the assembly of the elastic member 840.

Optionally, as shown in FIG. 69 to FIG. 72, the fluid passage includes a main body 8101 and opening portions 8102 disposed at both ends of the main body 8101, where the inner diameter of the opening portions 8102 is less than the inner diameter of the main body 8101. The connection between the main body 8101 and the opening portion 8102 narrows to form a shoulder 8103. The meltable support portion 850 is disposed on the inner wall of the opening portion 8102. One end of the elastic member 840 abuts against the shoulder 8103, and the other end of the elastic member 840 abuts against the first guide portion 83021.

Specifically, the fluid passage includes the main body 8101 and the opening portions 8102 provided at both ends of the main body 8101. The opening portion 8102 is used for communicating with the pipeline of the patient end or the oxygen therapy instrument end. For the convenience of assembly, the inner diameter of the opening portion 8102 is less than the inner diameter of the main body 8101, forming a structure with thick in the middle and thin at the ends. The main body 8101 transitions from the middle to both ends, and narrows at the connection with the opening portion 8102 to form a shoulder 8103 used for supporting the elastic member 840.

The meltable support portion 850 is disposed on the inner wall of the opening portion 8102, and the sealing member 8303 is disposed inside the main body 8101. The sealing member 8303 is far away from the meltable support portion 850 and is not easily affected by the high temperature of combustion, which is beneficial to improve the performance of the sealing member 8303 and improve the stability of the fire protection device.

Optionally, as shown in FIG. 70, the clamping member 8302 is provided with a raised second guide portion 83022 which is in sliding fit with the inner wall of the opening portion 8102.

Specifically, the clamping member 8302 is provided with the raised second guide portion 83022. The second guide portion 83022 may have a structure such as a sliding plate and a boss. The second guide portion 83022 may slidably fit with the inner wall of the opening portion 8102 to play a guiding and positioning role, so as to avoid the problems of jamming and deflection of the clamping member 8302 when moved, and improve the triggering stability of the valve body 830.

Optionally, as shown in FIG. 70, a side of the sealing member 8303 facing towards the clamping member 8302 has an end surface. A peripheral side of the end surface extends towards the clamping member 8302 to form a third guide portion 83031, and the third guide portion 83031 is in sliding fit with the inner wall of the fluid passage.

In particular, the side of the sealing member 8303 facing towards the clamping member 8302 has an end surface, and the clamping member 8302 may push against the sealing member 8303 by contacting the end surface of the sealing member 8303. The peripheral side of the end surface extends towards the clamping member 302 to form the third guide portion 83031. The third guide portion 83031 is capable of sliding fit with the inner wall of the fluid passage, serving as a guide and positioning function, avoiding the problems of jamming and deflection of the sealing member 8303 when moved, and improving the stability of triggering of the valve body 830.

Optionally, as shown in FIG. 70, the third guide portion 83031 is combined with the end surface to form a limiting groove 83032. The clamping member 8302 extends towards one end of the sealing member 8303 to form the limiting portion 83023 which is at least partially embedded in the limiting groove 83032.

Specifically, the third guide portion 83031 is combined with the end surface to form the limiting groove 83032. The limiting groove 83032 is U-shaped, and has an opening side facing towards the clamping member 8302. The clamping member 8302 extends towards one end of the sealing member 8303 to form the limiting portion 83023 which may have a boss structure. When the clamping member 8302 pushes against the sealing member 8303, the limiting portion 83023 is at least partially embedded in the limiting groove 83032, so as to avoid the problems of shaking and blocking when the clamping member 8302 and the sealing member 8303 are moved, and improve the triggering stability of the valve body 830.

Optionally, as shown in FIG. 71, the outer side wall of the sealing member 8303 extends to form a raised fourth guide portion 83033 which is in sliding fit with the inner wall of the fluid passage.

Specifically, as a variant, the raised fourth guide portion 83033 may also be provided by extending at the outer side wall of the sealing member 8303. The fourth guide portion 83033 may be a sliding plate, a boss or the like. The fourth guide portion 83033 may be in sliding fit with the inner wall of the fluid passage to play the role of guiding and positioning, so as to avoid the problems of jamming and deflection of the sealing member 8303 when moved, and improve the triggering stability of the valve body 830.

Optionally, as shown in FIG. 71, one end of the clamping member 8302 facing towards the sealing member 8303 is provided with a groove 83024. One end of the sealing member 8303 facing towards the clamping member 8302 is provided with a boss 83034 which is at least partially embedded in the groove 83024.

Specifically, in order to ensure the stability of the assembly between the clamping member 8302 and the sealing member 8303, shaking is avoided. With regard to the problem of deflection, the groove 83024 may be disposed on the clamping member 8302. The boss 83034 may be disposed on the sealing member 8303. The groove 83024 is arranged opposite to the boss 83034 and has a matched shape.

As shown in FIG. 68 to FIG. 69, the present disclosure discloses an eighth type of a fire protection device including a housing 10 and a valve body 830. The housing 10 has a fluid passage for communicating with a pipeline of an oxygen therapy instrument or a patient end. The valve body 830 is disposed within the fluid passage, the valve body 830 having a first position and a second position relative to the fluid passage. In the first position, a gap is provided between the valve body 830 and the fluid passage for the passage of gas. In the second position, the valve body 830 blocks the fluid passage. The inner walls at both ends of the fluid passage are respectively provided with at least one raised meltable support portion 8100. One end of the valve body 830 close to the support portion 8100 is provided with a fusible portion 8301. When the fusible portion 8301 is in a non-molten state, the valve body 830 is clamped with the support portion 8100 to support the valve body 830 to be in the first position. When the fusible portion 8301 is in the molten state, the valve body 830 is switched from the first position to the second position.

Specifically, as a main 8101 frame of the fire protection device, the housing 10 may be made of a material that is not easily chemically reacted with oxygen and is resistant to high temperatures, such as stainless steel or a ceramic material. It may also be made of fireproof and flame-retardant materials. The fluid passage is disposed in the housing 10, and both ends of the fluid passage have openings. The fluid passage is used for communicating with a pipeline of an oxygen therapy instrument or a patient end. Since it is necessary to pass through the fluid passage during the oxygen transmission, the oxygen passage may be blocked by blocking the fluid passage, so as to minimize the spread of fire caused by oxygen enrichment.

The valve body 830 is slidably connected to the inner wall of the fluid passage. The valve body 830 has the first position and the second position relative to the fluid passage. When the valve body 830 is in the first position, the gap is provided between the valve body 830 and the fluid passage for the gas to pass through, and oxygen may normally be transmitted through the fluid passage. When the valve body 830 is in the second position, the fluid passage is in a blocked state. Specially, the blocking of the fluid passage may be achieved by the cooperation of the valve body 830 and the inner wall or opening of the fluid passage. For example, when the valve body 830 is in the second position, the valve body abuts against a shoulder of the fluid passage to block the fluid passage.

The end of the valve body 830 used for sealing and blocking the fluid passage may be made of a flexible sealing material, such as silica gel, rubber, etc. The valve body 830 may also be made of a rigid material, with a flexible sealing ring disposed only at the end of the valve body 830 for sealing and blocking the fluid passage. The inner walls at both ends of the fluid passage are provided with at least one raised support portion 8100, which can be clamped with the other end of the valve body 830, so that the valve body 830 is positioned in the first position, ensuring the conduction of the fluid passage in the absence of a fire. The support portion 8100 may be made of a material with a relatively low melting point, such as PP, PVC, etc.

The end of the valve body 830 close to the support portion 8100 is provided with the fusible portion 8301. Herein, the fusible portion 8301 may be made of a material with a relatively low melting point, such as PP, PVC, etc. The fusible portion may also be realized through structural design. For example, the fusible portion 8301 has a structure with a relatively small diameter or thickness, and is easily fused. After the fusible portion 8301 is fused, the support portion 8100 will also lose the clamping action on the valve body 830, and the valve body 830 moves to the second position to realize the blocking of the fluid passage.

In the embodiment of the present disclosure, by providing the fusible portion 8301, when the fusible portion 8301 melts, the clamping action between the support portion 8100 and the valve body 830 fails, so that the valve body 830 may move to the second position in time and block the gas path. Since the fusible portion 8301 is made of a fusible material, the response speed is relatively fast, and the triggering speed of the valve body 830 is improved. The risk of fire spreading due to the triggering not being timely is reduced, and thus the safety factor of the fire protection device is improved. Also, the fire protection device has a simple structure, is easy to assemble, and which also reduces production costs and installation costs.

Optionally, an outer diameter of the fusible portion 8301 is less than an outer diameter of the valve body in other locations.

Specifically, the fusible portion 8301 may be preferentially melted compared to other positions of the valve body 830 by structural design. The shape of the valve body 830 is a cylinder-like shape. When the valve body 830 is manufactured, the outer diameter at the fusible portion 8301 is made less than the outer diameter of the valve body 830 at other positions. When the valve body 830 is attacked by high temperature, the fusible portion 8301 is fused preferentially because the outer diameter at the fusible portion 8301 is less, thereby disabling the clamping action between the support portion 8100 and the valve body 830 and greatly improving the triggering speed of the valve body 830.

Optionally, the fire protection device further includes an elastic member. The elastic member is provided between the valve body and the inner wall of the fluid passage. The elastic member is in a compressed state when the valve body is in the first position. The elastic member serves to provide an elastic driving force when the valve body is switched from the first position to the second position.

Specifically, the elastic member 840 is provided between the valve body 830 and the inner wall of the fluid passage for driving the valve body 830 from the first position to the second position. The elastic member 840 may be a metal spring such as iron, copper, alloy, etc. or a soft rubber such as silica gel, rubber, etc. which may store the elastic potential energy.

When the valve body 830 is in the first position, the elastic member 840 will be pressed by the inner wall of the fluid passage and the valve body 830, and is in a compressed state to accumulate the elastic potential energy. When the fusible portion 8301 melts and the support portion loses the clamping action on the valve body 830, the force balance is broken, and the valve body 830 moves to the second position under the elastic force of the elastic member 840, so that the air passage is blocked. The triggering speed of the valve body 830 is further increased by the elastic member 840.

Optionally, with reference to FIG. 70 to FIG. 71, the valve body 830 includes a clamping member 8302 and a sealing member 8303. Herein, the clamping member 8302 and the sealing member 8303 are of a split-type structure, and the clamping member 8302 and the sealing member 8303 are respectively slidably connected with the inner wall of the fluid passage. The fusible portion 8301 is disposed at one end of the clamping member 8302 close to the support portion 8100. The elastic member 840 is disposed between the clamping member 8302 and the inner wall of the fluid passage. One end of the clamping member 8302 is used for being clamped with the support portion 8100, and the other end of the clamping member 8302 is used for pushing against the sealing member 8303. An assembly gap is provided between the clamping member 8302 and the sealing member 8303 for the fluid to pass through.

Specifically, the valve body 830 includes the clamping member 8302 and the sealing member 8303. The clamping member 8302 and the sealing member 8303 are of a split-type structure and have the assembly gap through which oxygen can pass, so as to reduce the air resistance brought by the valve body 830 and improve the oxygen flux and comfortableness. The clamping member 8302 and the sealing member 8303 are respectively slidably connected to the inner wall of the fluid passage. When one end of the clamping member 8302 is clamped with the support portion 8100, the positioning and fixing of the clamping member 8302 can be achieved, so that the clamping member 8302 is in a stationary state. The clamping member 8302 may achieve clamping and fitting with the support portion 8100 through a structure such as a clamping hook and a clamping round head. The elastic member 40 is arranged between the clamping member 8302 and the inner wall of the fluid passage. Under the compression of the clamping member 8302 and the inner wall of the fluid passage, the elastic member 840 accumulates the elastic potential energy. The sealing member 8303 is in clearance fit with the through-hole 8201 of the inner shell 820. When in use, oxygen may be normally transmitted through the gap between the sealing member 8303 and the through-hole 8201 of the inner shell 820. The sealing member 8303 may be made of flexible sealing materials, such as silicone, rubber, etc. The sealing member 8303 may also be made of a rigid material. A flexible sealing ring is disposed only on one side of the sealing member 8303 close to the through-hole 8201. When the sealing member 8303 abuts against the peripheral side of the through-hole 8201, the sealing ring is embedded in the gap between the sealing member 8303 and the peripheral side of the through-hole 8201 to achieve sealing.

The above-mentioned fusible portion 8301 is disposed on the clamping member 8302. The fusible portion 8301 may be made of a material with a relatively low melting point, such as PP, PVC, etc. The fusible portion may also be realized by structural design. For example, the fusible portion 8301 adopts a structure with a relatively small diameter or thickness, and is easily fused. After the fusible portion 8301 is fused, the clamping member 8302 may be broken at the fusible portion 8301. When any one of the fusible portion 8301 and the support portion 8100 melts, the support portion 8100 loses the clamping action on the clamping member 8302. Thus, when the elastic member 840 releases at least part of the elastic potential energy, the other end of the clamping member 8302 pushes against the sealing member 8303 under the elastic force, so that the sealing member 8303 abuts against the peripheral side of the through-hole 8201, and the fluid passage is blocked.

Optionally, as shown in FIG. 70, one end of the clamping member 8302 close to the sealing member 8303 is provided with a raised first guide portion 83021. The first guide portion 83021 is in sliding fit with the inner wall of the fluid passage. The elastic member 840 is sleeved on the clamping member 8302. One end of the elastic member 840 abuts against the inner wall of the fluid passage, and the other end of the elastic member 840 abuts against the first guide portion 83021. When the clamping member 8302 is clamped with the support portion 8100, the elastic member 840 is compressed by the inner wall of the fluid passage and the first guide portion 83021. When the support portion 8100 and/or the fusible portion 8301 is in the melted state, the elastic member 840 releases at least part of the elastic potential energy to drive the sealing member 8303 to abut against the peripheral side of the through-hole 8201, so that the fluid passage is blocked.

Specifically, one end of the clamping member 8302 close to the sealing member 8303 is provided with the raised first guide portion 83021. The first guide portion 83021 may be a sliding plate, a boss or the like. The quantity of the first guide portions 83021 may be selected according to practical requirements. The material of the first guide portion 83021 may be the same as that of the clamping member 8302, and is made of a material with fire-retardant and wear-resistant properties. The first guide portion 83021 and the clamping member 8302 may be of an integrated structure, and have good structural strength and stability. The first guide portion 83021 may be formed separately from the clamping member 8302 and assembled by bonding or welding. The first guide 83021 has two functions. Firstly, a stable support point may be provided for the elastic member 840. Secondly, the first guide portion 83021 may be in sliding fit with the inner wall of the fluid passage to play the role of guiding and positioning, so as to avoid the problems of jamming and deflection of the clamping member 8302 when moved, and improve the stability of triggering of the valve body 830.

The elastic member 840 may be a metal spring, and the metal spring is sleeved on the clamping member 8302. When the clamping member 8302 is clamped with the meltable support, both ends of the elastic member 840 respectively abut against the first guide portion 83021 and the inner wall of the fluid passage, and are in a compressed state to accumulate the elastic potential energy. When the support portion 8100 and/or the fusible portion 8301 are in the molten state, the support portion 8100 loses the clamping action on the clamping member 8302. The elastic member 840 releases at least part of the elastic potential energy to drive the sealing member 8303 to contact with the peripheral side of the through-hole 8201, so that the fluid passage is blocked.

By providing the elastic member 840, when the support portion 8100 and/or the fusible portion 8301 are melted, it is capable of providing driving force for the clamping member 8302 and the sealing member 8303, so that the sealing member 8303 moves rapidly and abuts against the peripheral side of the through-hole 8201. At the same time, the elastic member 840 is sleeved on the clamping member 8302. It not only saves the space inside the fluid passage, but also ensures the stability of the assembly of the elastic member 840.

Optionally, as shown in FIG. 68 to FIG. 69, the fluid passage includes a main body 8101 and opening portions 8102 disposed at both ends of the main body 8101, where the inner diameter of the opening portions 8102 is less than the inner diameter of the main body 8101. The connection between the main body 8101 and the opening 8102 narrows to form a shoulder 8103. The support portion 8100 is disposed on the inner wall of the opening portion 8102. One end of the elastic member 840 abuts against the shoulder 8103, and the other end of the elastic member 840 abuts against the first guide portion 83021.

Specifically, the fluid passage includes the main body 8101 and the opening portions 8102 provided at both ends of the main body 8101. The opening portion 8102 is used for communicating with the pipeline of the patient end or the oxygen therapy instrument end. For the convenience of assembly, the inner diameter of the opening portion 8102 is less than the inner diameter of the main body 8101, forming a structure with thick in the middle and thin at the ends. The main body 8101 transitions from the middle to both ends, and narrows at the connection with the opening portion 8102 to form the shoulder 8103 used for supporting the elastic member 840.

The support portion 8100 is disposed on the inner wall of the opening portion 8102, and the sealing member 8303 is disposed inside the main body 8101. The sealing member 8303 is far away from the support portion 8100 and is not easily affected by the high temperature of combustion, which is beneficial to improve the performance of the sealing member 8303 and improve the stability of the fire protection device.

Optionally, as shown in FIG. 70, the clamping member 8302 is provided with a raised second guide portion 83022 which is in sliding fit with the inner wall of the opening portion 8102.

Specifically, the clamping member 8302 is provided with the raised second guide portion 83022. The second guide portion 83022 may have a structure such as a sliding plate and a boss. The second guide portion 83022 may slidably fit with the inner wall of the opening portion 8102 to play a guiding and positioning role, so as to avoid the problems of jamming and deflection of the clamping member 8302 when moved, and improve the triggering stability of the valve body 830.

Optionally, as shown in FIG. 70, a side of the sealing member 8303 facing towards the clamping member 8302 has an end surface. A peripheral side of the end surface extends towards the clamping member 8302 to form a third guide portion 83031, and the third guide portion 83031 is in sliding fit with the inner wall of the fluid passage.

In particular, the side of the sealing member 8303 facing towards the clamping member 8302 has the end surface, and the clamping member 8302 may push against the sealing member 8303 by contacting the end surface of the sealing member 8303. The peripheral side of the end surface extends towards the clamping member 8302 to form the third guide portion 83031. The third guide portion 83031 is capable of sliding fit with the inner wall of the fluid passage, serving as a guide and positioning function, avoiding the problems of jamming and deflection of the sealing member 8303 when moved, and improving the stability of triggering of the valve body 830.

Optionally, as shown in FIG. 70, the third guide portion 83031 is combined with the end surface to form a limiting groove 83032. The clamping member 8302 extends towards one end of the sealing member 8303 to form a limiting portion 83023 which is at least partially embedded in the limiting groove 83032.

Specifically, the third guide portion 83031 is combined with the end surface to form a limiting groove 83032. The limiting groove 83032 is U-shaped, and has an opening side facing towards the clamping member 8302. The clamping member 8302 extends towards one end of the sealing member 8303 to form the limiting portion 83023 which may have a boss structure. When the clamping member 8302 pushes against the sealing member 8303, the limiting portion 83023 is at least partially embedded in the limiting groove 83032, so as to avoid the problems of shaking and blocking when the clamping member 8302 and the sealing member 8303 are moved, and improve the triggering stability of the valve body 30.

Optionally, as shown in FIG. 71, the outer side wall of the sealing member 8303 extends to form a raised fourth guide portion 83033 which is in sliding fit with the inner wall of the fluid passage.

Specifically, as a variant, the raised fourth guide portion 83033 may also be provided by extending at the outer side wall of the sealing member 8303. The fourth guide portion 83033 may be a sliding plate, a boss or the like. The fourth guide portion 83033 may be in sliding fit with the inner wall of the fluid passage to play the role of guiding and positioning, so as to avoid the problems of jamming and deflection of the sealing member 8303 when moved, and improve the triggering stability of the valve body 830.

Optionally, as shown in FIG. 71, one end of the snap 8302 facing towards the sealing member 8303 is provided with a groove 83024. One end of the sealing member 8303 facing towards the clamping member 8302 is provided with a boss 83034 which is at least partially embedded in the groove 83024.

Specifically, in order to ensure the stability of the assembly between the clamping member 8302 and the sealing member 8303, shaking is avoided. With regard to the problem of deflection, the groove 83024 may be disposed on the clamping member 8302. The boss 83034 may be disposed on the sealing member 8303. The groove 83024 is arranged opposite to the boss 83034 and has a matched shape.

Embodiments of the present disclosure also disclose an eighth type of a ventilation treatment apparatus including the eighth type of the fire protection device.

Specifically, the eighth type of the ventilation treatment apparatus a control device, an oxygen therapy instrument and a gas pipeline. The control device is used for controlling the oxygen supply amount, operation time, etc. of the oxygen therapy instrument. The control device may be an electronic device or a component in the electronic device, such as an integrated circuit or a chip. The electronic device may be a terminal or may be a device other than a terminal. Illustratively, the electronic device may be a cell phone, a tablet computer, a notebook computer, a palmtop computer, a mobile Internet appliance, a robot, a wearable device, etc. and embodiments of the present disclosure are not particularly limited.

Referring to FIG. 73 to FIG. 75, a ninth type of a fire protection device is disclosed according to an embodiment of the present disclosure, the fire protection device including a housing 10, a sealing member 920, a valve body 930, and an elastic member 940. The housing 10 has a fluid passage 9101 therein, and the fluid passage 9101 is used for communicating with a pipeline of an oxygen therapy instrument or a patient end. The sealing member 920 is connected to the inner wall of the fluid passage 9101, and the sealing member 920 is provided with a notch for passing a fluid. The valve body 930 is slidably connected to the inner wall of the fluid passage 9101. The elastic member 940 is provided between the valve body 930 and the inner wall of the fluid passage 9101. The valve body 930 is provided with a fusible portion 9301. The inner wall of the fluid passage 9101 is provided with at least one raised meltable support portion 950. One end of the valve body 930 is provided opposite to the notch, and the other end of the valve body 930 is used for being clamped with the meltable support portion 950. The valve body 930 has a first position and a second position relative to the fluid passage 9101. When the meltable support portion 950 and the fusible portion 9301 are in a non-melted state, the valve body 930 is clamped with the meltable support portion 950, so as to support the valve body 930 to be in a first position. A gap is provided between the valve body 930 and the sealing member 920 for the passage of the fluid, and the elastic member 940 is in a compressed state. When the meltable support portion 950 and/or the fusible portion 9301 is in the molten state, the elastic member 940 releases at least part of the elastic potential energy to drive the valve body 930 to be in the second position, the valve body 930 is snap-fitted with the notch of the sealing member 920, so that the fluid passage 9101 is in the blocked state.

Specifically, as a frame of a main body 91011 of the fire protection device, the housing 10 may be made of a material that is not easily chemically reacted with oxygen and is resistant to high temperatures, such as stainless steel or a ceramic material. It may also be made of fireproof and flame-retardant materials. The housing 10 has the fluid passage 9101 therein used for communicating with the pipeline of the oxygen therapy instrument or the patient end. Since it is necessary to pass through the fluid passage 9101 during the oxygen transmission, the oxygen passage may be blocked by blocking the fluid passage 9101, so as to minimize the spread of fire caused by oxygen enrichment.

The inner wall of the fluid passage 9101 is provided with the sealing member 920 which is preferably disposed at the middle position of the fluid passage 9101. The sealing member 920 may be made of silicone, rubber, etc. and may achieve a good sealing effect. The sealing member 920 is provided with the notch through which oxygen may normally be transmitted in the absence of a fire. The shape of the notch may be circular, elliptical, semi-circular, etc. In the embodiment of the present disclosure, the sealing member 920 takes the shape of an annular ring, the outer periphery of the annular ring is sealed connected to the inner wall of the fluid passage 9101, and the inner periphery of the annular ring is enclosed to form a notch for the passage of oxygen.

The valve body 930 is slidably connected to the inner wall of the fluid passage 9101. The valve body 930 has a first position and a second position with respect to the fluid passage 9101. A structure corresponding to the shape of the notch is disposed on the valve body 930. When the valve body 930 is in the first position, a gap is provided between the valve body 930 and the sealing member 920 for the fluid to pass through, and oxygen may normally be transmitted through the notch. When the valve body 930 is in the second position, the valve body 930 is snap-fitted with the notch of the sealing member 920 so that the fluid passage 9101 is in the blocked state. The fluid passage 9101 is blocked by the snap-fitting of the valve body 930 with the notch.

An elastic member 940 is disposed between the valve body 930 and the inner wall of the fluid passage 9101 for driving the valve body 930 from the first position to the second position. The elastic member 940 may be a metal spring such as iron, copper, alloy, etc. or a soft rubber such as silica gel, rubber, etc. which may store the elastic potential energy. The inner wall of the fluid passage 9101 is provided with at least one raised meltable support portion 950 which can be clamped with the valve body 930 to position the valve body 930 in the first position, so as to ensure that the fluid passage 9101 is unobstructed in the case of no fire. The meltable support portion 950 may be made of a lower melting point material such as PP, PVC, etc.

When the valve body 930 is in the first position, the elastic member 940 will be pressed by the inner wall of the fluid passage 9101 and the valve body 930, and is in a compressed state to accumulate the elastic potential energy. When the meltable support portion 950 loses the clamping action on the valve body 930, the force balance is broken. The valve body 930 moves to the second position under the elastic force of the elastic member 940, and is snap-fitted with the notch, so as to realize the gas path blocking.

In the event of a fire, the valve body 930 may be triggered by the followings. Firstly, the meltable support portion 950 melts, the meltable support portion 950 loses a clamping action on the valve body 930. Under the action of the elastic force of the elastic member 940, the valve body 930 moves to the second position to be snap-fitted with the notch, so as to block the fluid passage 9101. Secondly, the valve body 930 is provided with the fusible portion 9301 which can be made of a material with a relatively low melting point, such as PP, PVC, etc. The fusible portion may also be realized by structural design. For example, the fusible portion 9301 adopts a structure with a relatively small diameter or thickness, and is easily fused. After the fusible portion 9301 is fused, the meltable support portion 950 also loses the clamping action on the valve body 930. Under the action of the elastic force of the elastic member 940, the valve body 930 moves to the second position to be snap-fitted with the notch, so as to realize the blocking of the fluid passage 9101. Thirdly, the meltable support portion 950 and the fusible portion 9301 are melted at the same time, and the meltable support portion 950 loses its clamping action on the valve body 930 and also blocks the fluid passage 9101.

In the embodiment of the present disclosure, by using the combination of the fusible portion 9301 and the meltable support portion 950, when any one of the fusible portion 9301 and the meltable support portion 950 is melted, the valve body 930 may be timely moved to the second position and snap-fitted with the notch of the sealing member 920, thereby blocking the gas path, increasing the triggering speed of the valve body 930, reducing the risk of fire spreading due to the triggering not being timely, thereby improving the safety factor of the fire protection device. Also, the fire protection device has a simple structure, and is easy to assemble, which reduces production costs and installation costs.

Optionally, as shown in FIG. 73, the valve body 930 is an integrated structure.

Specifically, one end of the valve body 930 is used to be clamped with the meltable support portion 950 and the other end of the valve body 930 is used to be clamped with the notch of the sealing member 920. In an embodiment of the present disclosure, the valve body 930 is an integrated structure with greater structural strength and stability.

Optionally, as shown in FIG. 73, the notch has a first side and a second side that are opposite to each other. At least one side of the gap is provided with the valve body 930. When at least one side of the notch is snap-fit with the valve body 930, the fluid passage 9101 is in a blocked state.

Specifically, the sealing member 920, with a quantity of one, is positioned within the fluid passage 9101. The notch of the sealing member 920 has the first side and the second side that are opposite to each other. When snap-fit into the notch from either side, the fluid passage 9101 may be blocked. When the valve body 930 is provided, the above-mentioned valve body 930 may be disposed on at least one side of the notch. For example, the valve body 930 may be disposed on the first side of the notch or the second side of the notch. The valve bodies 930 may also be disposed on both the first side and the second side. When at least one side of the notch is snap-fitted with the valve body, the fluid passage 9101 is in a blocked state, and a single sealing member 920 may be used to realize the double-side blocking of the fluid passage 9101, with a simple structure and better stability.

Optionally, the valve body 930 includes a clamping portion 9302, a connecting portion 9303 and a sealing portion 9304. The clamping portion 9302 and the sealing portion 9304 are connected via the connecting portion 9303. The clamping portion 9302 is used for being clamped with the meltable support portion 950 to support the valve body 930 to be in the first position. The sealing portion 9304 is disposed opposite to the notch, and is used for being snap-fitted with the notch when the valve body 930 is in the second position. The fusible portion 9301 is disposed at the connecting portion 9303.

Specifically, the valve body 930 includes the clamping portion 9302 located at an end of the valve body 930 close to the meltable support portion 950, the connecting portion 9303 for being snap-fitted with the meltable support portion 950 to position the valve body 930 in the first position, and the sealing portion 9304. The specific structure of the clamping portion 9302 may be a structure such as a hook and a clamping round head, and may achieve a clamping fit with the meltable support portion 950. The sealing portion 9304 is used to be snap-fitted with the notch of the sealing member 920. The sealing portion 9304 and the clamping portion 9302 are connected via a connecting portion 9303. The clamping portion 9302, the connecting portion 9303 and the sealing portion 9304 may be an integral structure and are made of the same material, which can reduce the manufacturing cost. They may also be made separately according to the functional requirements of different parts, made of different materials, and assembled by welding or bonding.

In the embodiment of the present disclosure, the diameters of the clamping portion 9302 and the sealing portion 9304 are slightly greater than the diameter of the connecting portion 9303, to form the valve body 930 of the shuttle-like structure. Preferably, the valve body 930 is provided coaxially with the flow passage. The valve body 930 is more stable when sliding. The connecting portion 9303 is provided with a fusible portion 9301 which may be made of a material with a relatively low melting point, such as PP, PVC, etc. The fusible portion may also be realized through structural design. For example, the fusible portion 9301 has a structure with a relatively small diameter or thickness, and is easily fused. After the fusible portion 9301 is fused, the clamping portion 9302 and the sealing portion 9304 are separated from each other. The meltable support portion 950 loses the clamping action on the sealing portion 9304. Under the action of the elastic force of the elastic member 940, the sealing portion 9304 is snap-fitted with the notch, so as to realize the blocking of the fluid passage 9101.

Optionally, the elastic member 940 is sleeved on the connecting portion 9303. One end of the elastic member 940 abuts against the inner wall of the fluid passage 9101, and the other end of the elastic member 940 abuts against the sealing portion 9304. When the clamping portion 9302 is clamped with the meltable support portion 950, the elastic member 940 is compressed by the inner wall of the fluid passage 9101 and the sealing portion 9304.With the meltable support portion 950 and/or the fusible portion 9301 in the molten state, the elastic member 940 releases at least part of the elastic potential energy to drive the sealing portion 9304 to be snap-fitted with the notch, so that the fluid passage 9101is in the blocked state.

Specifically, the elastic member 940 may be a metal spring which is sleeved on the connecting portion 9303. When the valve body 930 is in the first position, both ends of the elastic member 940 abut against the sealing portion 9304 and the inner wall of the fluid passage 9101, respectively, and are in a compressed state to accumulate the elastic potential energy. When the meltable support portion 950 and/or the fusible portion 9301 are in the molten state, the meltable support portion 950 loses its clamping action on the sealing portion 9304, and the elastic member 940 releases at least part of its elastic potential energy, driving the sealing portion 9304 to be snap-fitted with the notch, so that the fluid passage 9101 is in the blocked state.

By providing the elastic member 940, when the meltable support portion 950 and/or the fusible portion 9301 are melted, a driving force can be provided to the sealing portion 9304 to rapidly move the sealing portion 9304 and snap-fit the same with the notch. At the same time, the elastic member 940 is sleeved on the connecting portion 9303. It not only saves the space 9101 inside the fluid passage 9101, but also ensures the stability of the assembly of the elastic member 940.

Optionally, the fluid passage 9101 includes a main body 91011 and opening portions 91012 provided at both ends of the main body 91011. The inner diameter of the opening portion 91012 is less than an inner diameter of the main body 91011. The connection between the main body 91011 and the opening portion 91012 narrows to form a shoulder 91013. The meltable support portion 950 is disposed on the inner wall of the opening portion 91012, and the sealing member 920 is connected to the inner wall of the main body 91011. One end of the elastic member 940 abuts against the shoulder 91013, and the other end of the elastic member 940 abuts against the sealing portion 9304.

Specifically, the fluid passage 9101 includes the main body 91011 and the opening portions 91012 provided at both ends of the main body 91011. The opening portion 91012 is used for communicating with a pipeline of a patient end or an oxygen therapy instrument end. For the convenience of assembly, the inner diameter of the opening portion 91012 is less than the inner diameter of the main body 91011, forming a structure with thick in the middle and thin at the ends. The main body 91011 transitions from the middle to both ends, and narrows at the connection with the opening portion 91012 to form the shoulder 91013 used for supporting the elastic member 940.

The meltable support portion 950 is disposed on the inner wall of the opening portion 91012, and the sealing member 920 is disposed on the inner wall of the main body 91011. The sealing member 920 is far away from the meltable support portion 950 and is not easily affected by the high temperature of combustion, which is beneficial to improve the performance of the sealing member 920 and improve the stability of the fire protection device.

Optionally, the sealing portion 9304 is provided with a raised first guide portion 9305, and the first guide portion 9305 is in sliding fit with the inner wall of the main body 91011. One end of the elastic member 940 abuts against the shoulder 91013, and the other end of the elastic member 940 abuts against the first guide portion 9305.

Specifically, the sealing portion 9304 is snap-fitted with the notch of the sealing member 920. The sealing portion 9304 is provided with the raised first guide portion 9305 which may be a structure such as a boss, etc. The quantity of the first guide portions 9305 may be selected according to actual requirements. The material of the first guide portion 9305 may be made of the same material as that of the valve body 930, or may be made of a material having fire-retardant and wear-resistant characteristics. The first guide portion 9305 and the valve body 930 may have an integral structure, and have good structural strength and stability. The first guide portion 9305 may be formed separately from the valve body 930 and assembled by bonding or welding. The first guide 9305 has two functions. Firstly, a stable supporting point may be provided for the elastic member 940. Secondly, the first guide portion 9305 may be in sliding fit with the inner wall of the main body 91011 to play the role of guiding and positioning, avoid the problems of jamming and deflection of the valve body 930 when moved, and improve the triggering stability of the valve body 30.

Optionally, the connecting portion 9303 is provided with a raised second guide portion 9306 which is in sliding fit with the inner wall of the opening portion 91012.

Specifically, the connecting portion 9303 is provided with the raised second guide portion 9306 which may have a structure such as a sliding plate and a boss. The second guide portion 9306 can be slidably fit with the inner wall of the opening portion 91012 to play a guiding and positioning role, so as to avoid the problems of jamming and deflection of the valve body 930 when moved, and improve the triggering stability of the valve body 930.

Optionally, the quantity of the meltable support portions 950 is two or more. The meltable support portions 950 are provided at intervals along the circumferential direction of the inner wall of the opening portion 91012.

Specifically, the meltable support portion 950 is used to support the valve body 930 to be in the first position. The stability of the clamping between the meltable support portion 950 and the valve body 930 directly affects the performance of the fire protection device. In order to avoid the problem that the clamping function between the meltable support portion 950 and the valve body 930 fails and the fluid passage 9101 is erroneously blocked, the quantity of the meltable support portions 950 is two or more. The meltable support portions 950 are arranged at intervals along the circumferential direction of the inner wall of the opening portion 91012 to achieve multiple clamping of the clamping portion 9302 of the valve body 930, thereby improving the stability of the clamping. At the same time, the problem that the valve body 930 deflects due to uneven force may also be avoided, so as to improve the stability of triggering of the valve body 930.

Optionally, the housing 10 includes a first housing 9102 and a second housing 9103. The first housing 9102 and/or the second housing 9103 is provided with a mounting engagement groove 9104. With the first housing 9102 and the second housing 9103 assembled, the sealing member 920 is in interference fit with the mounting engagement groove 9104.

Specifically, the first housing 9102 and the second housing 9103 may be detachably assembled by clamping or bolting to facilitate installation and removal of the internal components. The first housing 9102 and the second housing 9103 may also be fixedly assembled by means of welding, so as to improve the structural strength and sealability.

The first housing 9102 and the second housing 9103 each have an opening arranged opposite to each other. The opening is provided with a mounting engagement groove 9104. The sealing member 920 is embedded in the mounting engagement groove 9104 to achieve assembly. The mounting engagement groove 9104 may be separately opened on the first housing 9102 or the second housing 9103, or may be assembled into a complete mounting engagement groove 9104 when the first housing 9102 and the second housing 9103 are respectively opened and the first housing 9102 and the second housing 9103 are assembled.

When the first housing 9102 and the second housing 9103 are pressed together, the sealing member 920 is in interference fit with the mounting engagement groove 9104 to seal the assembly gap between the first housing 9102 and the second housing 9103 and prevent leakage during oxygen transmission.

Optionally, the mounting engagement groove 9104 is provided with an expanded portion 9105. The edge of the sealing member 920 extends to form an embedded portion which is at least partially embedded in the expanded portion 9105.

Specifically, the mounting engagement groove 9104 is provided with the expanded portion 9105. The expanded portion 9105 and the main body 91011 of the mounting engagement groove 9104 form a stepped groove body. The edge of the sealing member 920 extends to form the embedded portion 9201 which is at least partially embedded in the expanded portion 9105, so as to improve the security of the assembly between the sealing member 920 and the housing 10, and also improve the sealing effect of the sealing member 920.

Optionally, the fire protection device includes two valve bodies 930 symmetrically arranged in the fluid passage 9101. Two valve bodies 930 are respectively located on two sides of the sealing member 920. The elastic member 940 is provided between each valve body 930 and the inner wall of the fluid passage 9101. The meltable support portions 950 are disposed in the opening portions 91012 at both ends of the main body 91011, respectively.

Specifically, the fire protection device adopts the structure of double valve body 930. Any valve body 930 may be triggered to realize the blocking of the fluid passage 9101, which ensures the timeliness of triggering the fire protection device. When installing, it is not necessary to distinguish the port sequence of the fire protection device. Any opening end may be assembled and communicated with the pipeline of the oxygen therapy instrument or the patient end, which greatly improves the installation convenience.

Embodiments of the present disclosure also disclose a ninth type of a ventilation treatment apparatus including the ninth type of the fire protection device.

Specifically, the ninth type of the ventilation treatment apparatus includes a control device, an oxygen therapy instrument and a gas pipeline. The control device is used for controlling the oxygen supply amount, operation time, etc. of the oxygen therapy instrument. The control device may be an electronic device or a component in the electronic device, such as an integrated circuit or a chip. The electronic device may be a terminal or may be a device other than a terminal. Illustratively, the electronic device may be a cell phone, a tablet computer, a notebook computer, a palmtop computer, a mobile Internet appliance, a robot, a wearable device, etc. and embodiments of the present disclosure are not particularly limited.

In the embodiment of the present disclosure, the valve body 930 is provided with the fusible portion 9301. The inner wall of the fluid passage 9101 is provided with the meltable support portion 950. When the meltable support portion 950 and the fusible portion 9301 are in the non-molten state, the valve body 930 is clamped with the meltable support portion 950, so as to support the valve body 930 to be in the first position. The gap is provided between the valve body 930 and the sealing member 920 for the passage of the fluid, and the elastic member 940 is in a compressed state. With the meltable support portion 950 and/or the fusible portion 9301 in the molten state, the elastic member 940 releases at least part of the elastic potential energy to drive the valve body 930 to be in the second position. The valve body 930 is snap-fitted with the notch of the sealing member 920, so that the fluid passage 9101 is in the blocked state. By using the combination of the fusible portion 9301 and the meltable support portion 950, when any one of the fusible portion 9301 and the meltable support portion 950 is melted, the valve body 930 may be moved to the second position in time and snap-fitted with the notch of the sealing member 920, thereby blocking the gas path, increasing the triggering speed of the valve body 930, reducing the risk of fire spreading due to the failure of triggering in time, and thus improving the safety factor of the fire protection device.

The present disclosure provides a tenth type of a ventilation apparatus for providing ventilation treatment to a user. The ventilation apparatus may include an oxygen generating device and a gas transmission pipeline communicating the breathing airway of the user with the oxygen generating device. The oxygen generating device generates oxygen when in operation, and the oxygen is provided to the user via the gas transmission pipeline.

When a user end fires, the oxygen generated by the oxygen generating device intensifies the fire due to the combustion supporting property of oxygen. In order to avoid this, the gas transmission pipeline may be provided with a fire damper, which may be closed in the event of a fire in order to shut off the delivery of oxygen in the gas transmission pipeline.

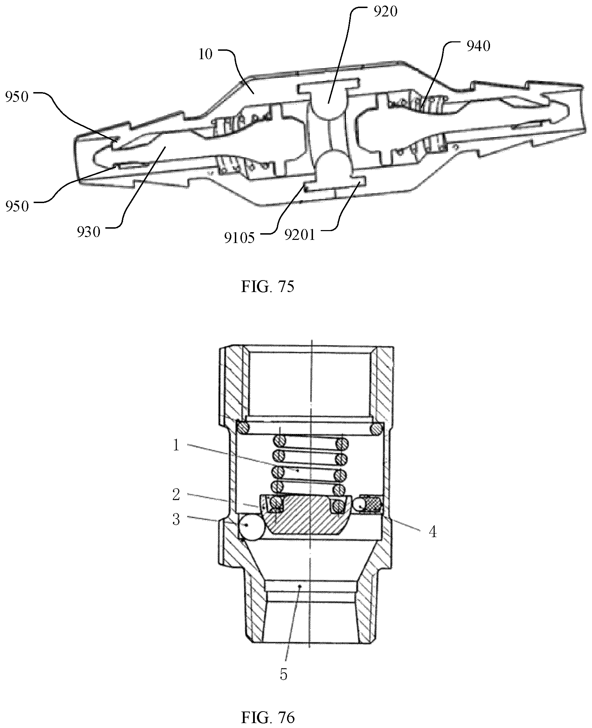

FIG. 76 is a sectional view of a fire damper in the related art. As shown in FIG. 76, an airflow passage 5 is disposed in the fire damper. A piston 2 is mounted in the airflow passage 5. An upper end of the piston 2 is elastically connected to a housing via a spring 1. A lower end of the piston 2 is limited by a first limiting ball 3 and a second limiting ball 4, thereby preventing the piston 2 from moving downwards under the elastic force of the spring 1. Wherein a stop block is disposed at a side of the second limiting ball 4 away from the piston 2 in the radial direction of the airflow passage 5. When a fire occurs, the stop block is heated and melted, the second limiting ball 4 moves in the radial direction of the airflow passage 5 under the action of the abutment of the piston 2, so that the second limiting ball 4 loses the limiting action on the piston 2. The piston 2 moves downwards under the elastic force of the spring 1 and blocks the airflow passage 5, so as to achieve the purpose of cutting off oxygen transmission.

However, in the related art, the fire damper has a complicated structure and a large quantity of parts. The piston may deviate from a predetermined trajectory when moves, resulting in an inability to completely block the airflow passage.

A fire damper in an embodiment of the present disclosure includes a housing and a sealing member mounted within the housing. The housing is provided with an airflow passage therethrough. The airflow passage is provided with a first partition for partitioning the airflow passage. The first partition is provided with a first vent hole. The sealing member is positioned within the airflow passage and a first gap is provided between the sealing member and the first vent hole before heated, to allow a gas to flow from one end of the airflow passage to the other through the first vent hole and the first gap. The sealing member includes a heat-shrinkable material layer such that the sealing member shrinks when heated and blocks the first vent hole.

A position of the housing corresponding to a first end of the airflow passage may be provided with a first joint, and a position of the housing corresponding to a second end of the airflow passage may be provided with a second joint. When the fire damper is installed in the ventilation apparatus, the first joint and the second joint may be connected to a gas transmission pipeline in the ventilation apparatus. For example, the first joint is connected to the gas transmission pipeline on the user side. The second joint is connected to the gas transmission pipeline on the oxygen generating device side. Oxygen reaches the user through the gas transmission pipeline on the oxygen generating device side, the airflow passage in the housing, and the gas transmission pipeline on the user side in this order.

The direction in which the airflow passage extends may be a straight line as a whole, or may be a broken line, a curved line, or the like. For convenience of description, the following description will be made only by taking the case where the airflow passage as a whole extends in a linear direction.

The first partition separates the airflow passage into a first passage close to the first joint and a second passage close to the second joint. Here, the "separate" does not mean that the first passage and the second passage are completely isolated, but that the first passage and the second passage communicate through the first vent hole. Once the first partition is provided, it is only necessary to block the first vent hole when it needs to close the fire damper.

The sealing member includes a heat-shrinkable material layer which may shrink when heated, so as to drive the sealing member to shrink and block the first vent hole. The heat-shrinkable material layer may be made of a heat-shrinkable material which is also called a polymer shape memory material, and is an intelligent material combining a polymer material and a radiation processing technology, namely, a functional polymer material made by mixing, shaping, cross-linking, heating, expanding and cooling and shaping using a rubber-plastic material as a base material and using the principle of "elastic memory" of a polymer. Common polymer materials, such as polyethylene and polyvinyl chloride, are generally linear structures. After being transformed into a network structure by the radiation action of radioactive sources, such as electron accelerators, these materials will have a unique "memory effect". After being heated, the expanded and cooled materials may be re-contracted to restore the original shape.

In the event of a user-side fire, the heat generated by the combustion source may pass through the gas transmission pipeline on the user-side into the fire damper to shrink the heat-shrinkable material layer by heat, thereby blocking the first vent hole. After the first vent hole is blocked, the first passage and the second passage of the airflow passage are completely isolated, thereby preventing oxygen from continuing to be supplied from the oxygen generating device side to the combustion source through the fire damper.

The present disclosure provides the fire damper. The first partition is disposed in the housing, and the first partition separates the airflow passage in the housing into two portions communicated through the first vent hole. The first gap is provided between the sealing member and the first vent hole before the sealing member is heated, so as to allow normal communication between the two portions of the airflow passage. The heat-shrinkable material layer is included in the sealing member so that the sealing member, after being heated, may block the first vent hole and prevent communication between the two portions of the airflow passage, thereby cutting off the oxygen delivery path. The present disclosure provides the fire damper that includes the housing and the sealing member, has a small quantity of parts, and has a simple structure, as compared with the fire dampers in the related art.

Here, the sealing member may be variously formed to block the first vent hole after being shrunk by heat. The fire dampers provided by the present disclosure are described in detail below by way of specific embodiments.

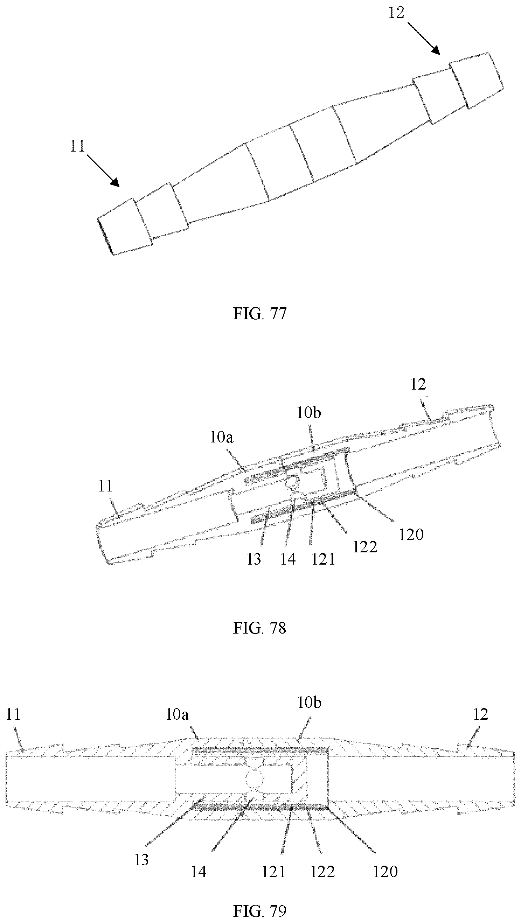

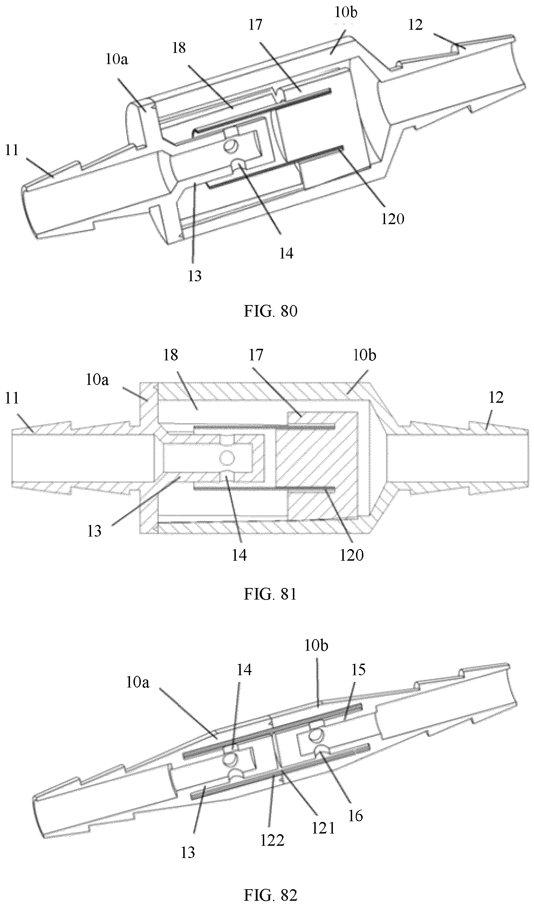

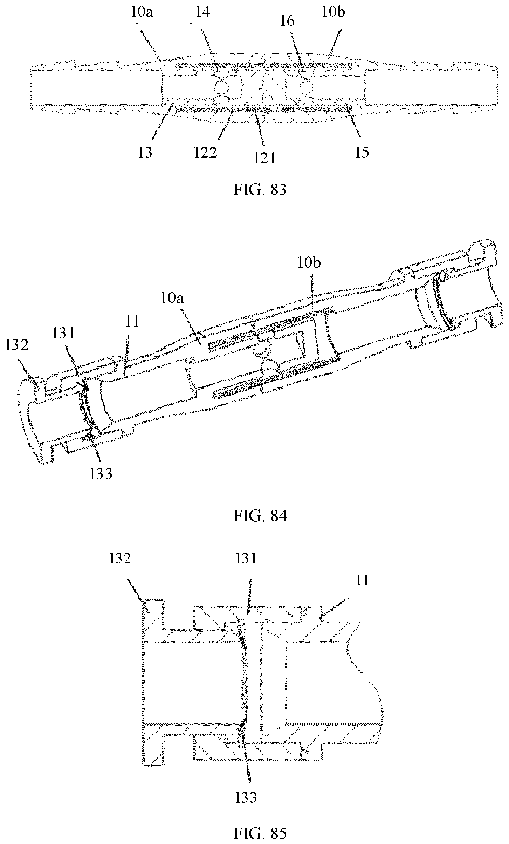

FIG. 77 is an axonometric view of a fire damper provided by an embodiment of the present disclosure, FIG. 78 is a first sectional view of a fire damper provided by an embodiment of the present disclosure, FIG. 79 is a second sectional view of a fire damper provided by an embodiment of the present disclosure, FIG. 80 is a first sectional view of another fire damper provided by an embodiment of the present disclosure, FIG. 81 is a second sectional view of another fire damper provided by an embodiment of the present disclosure, FIG. 82 is a first sectional view of another fire damper provided by an embodiment of the present disclosure, and FIG. 83 is a second sectional view of another fire damper provided by an embodiment of the present disclosure. FIG. 84 is a sectional view of another fire damper provided by an embodiment of the present disclosure, and FIG. 85 is a partial enlarged view of FIG. 84 with respect to a fast-plug joint.

As shown in FIG. 77 to FIG. 84, the fire damper includes the housing and the sealing member 120 mounted within the housing. The housing is provided with the airflow passage therethrough. The airflow passage is provided with the first partition for partitioning the airflow passage. The first partition is provided with the first vent hole 14. The sealing member 120 is positioned within the airflow passage, and the first gap is provided between the sealing member 120 and the first vent hole 14 before the sealing member 120 is heated to flow from one end of the airflow passage to the other through the first vent hole 14 and the first gap. The first vent hole 14 faces towards the sealing member 120, and the projection of the sealing member 120 on the first vent hole 14 completely covers the first vent hole 14. The sealing member 120 includes the heat-shrinkable material layer 122, such that the sealing member 120, after being shrunk by heat, fits over the first partition and covers the first vent hole 14.

The first gap is provided between the sealing member 120 and the first through-hole before the sealing member 120 is heated, and the first gap communicates with the second passage, so that the second passage, the first gap, the first vent hole 14 and the first passage communicate to form a passage for oxygen transmission.

The sealing member 120 includes the heat-shrinkable material layer 122 which gradually approaches the first vent hole 14 after being shrunk by heat, and eliminates the first gap, so as to fit and cover the first vent hole 14, thereby blocking the passage of oxygen transmission and preventing the fire from further expanding due to the combustion of oxygen. The structure of the first vent hole 14 being attached and covered by the sealing member 120 is simple and reliable.

The first partition may include a first vent pipe 13, and the first vent hole 14 is disposed on a side wall of the first vent pipe 13. The sealing member 120 has a tubular shape and is sleeved outside the first vent pipe 13. The sealing member 120 has a tubular shape, and the diameter of the tube decreases after being shrunk by heat, so that the inner wall of the sealing member 120 is attached to the side wall of the first vent pipe 13, thereby not only covering the first vent hole 14, but also closely fitting the area around the first vent hole 14, thereby improving the sealing effect. Also, the tubular sealing member 120 is simple to manufacture, and the tube diameter is reduced when contracted, i.e., the shrinking process can be expected.

By way of example, as shown in FIG. 78 and FIG. 79, the housing has an overall tubular shape. The axial direction of the first vent pipe 13 is parallel to the axial direction of the housing. A plurality of first vent holes 14 are provided at intervals along the circumferential direction of the first vent pipe 13, so as to increase the ventilation area and reduce the resistance to oxygen transmission.

Certainly, the sealing member 120 may be in the form of a sheet, which is attached to the first partition after being shrunk by heat, thereby covering the first vent hole 14.

The first vent pipe 13 divides the airflow passage into the first passage and the second passage. The sealing member 120 may be located in the second passage. A second gap 18 is provided between an outer wall of the sealing member 120 and an inner wall of the second passage. That is, before the sealing member 120 is heated, the second gap 18, the first gap, and the first vent hole 14 communicate to form a passage for oxygen transmission.

When the first passage communicates with the gas transmission pipeline on the user side, heat generated by combustion passes through the first passage and the first vent hole 14 in turn and is transferred to the sealing member 120, so that the sealing member 120 is shrunk by heat. When the second passage communicates with the gas transmission pipeline on the user side, heat generated by combustion passes through the second passage and enters the second gap 18, and is transferred to the sealing member 120, so that the sealing member 120 is shrunk by heat. That is, any one of the first passage and the second passage may block oxygen transmission when communicating with the gas transmission pipeline on the user side, i.e., the installation of the fire damper does not distinguish directions.

Illustratively, as shown in FIG. 80 and FIG. 81, a fixing portion 17 may be disposed in the second passage. One end of the sealing member 120 is connected to the fixing portion 17, such that the first gap is provided between the sealing member 120 and the first vent pipe 13 and the second gap 18 is provided between the sealing member 120 and the inner wall of the second passage.

Certainly, the sealing member 120 may be fixed by other means. For example, a plurality of projections may be disposed on the outer wall of the first vent pipe 13. The sealing member 120 may be supported by the plurality of protrusions.

In addition, in order to achieve that the installation of the fire damper does not distinguish directions, a second partition may also be disposed in the airflow passage. The second partition is provided with a second vent hole 16. The first partition and the second partition are axially provided at intervals along the airflow passage. The sealing member 120 blocks the second vent hole 16 after being shrunk by heat. The structure of the second partition and the principle of action with the sealing member 120 may be the same as the first partition.

Illustratively, as shown in FIG. 82 and FIG. 83, the housing has a symmetrical structure. The first partition is the first vent pipe 13. The second partition is a second vent pipe 15. The sealing member 120 is sleeved outside the first vent pipe 13 and the second vent pipe 15 at the same time. In order to reduce the quantity of parts and assembly difficulty, the housing may include the first housing 10a and the second housing 10b of the same structure. The first housing 10a and the second housing 10b are snap-fitted to constitute the housing.

Referring to FIG. 77 to FIG. 83, for example, the cross sections of the first joint 11 and the second joint 12 may be a zigzag structure. The zigzag structure may play a guiding role during the installation of the gas transmission pipeline sleeving on the first joint 11 and the second joint 12. The zigzag structure may play a stopping role after the installation is completed, so as to prevent the gas transmission pipeline from escaping from the first joint 11 and the second joint 12.

Referring to FIG. 84 and FIG. 85, for example, the first joint 11 and the second joint 12 may be provided with a fast-plug joint including an outer shell 131, a pressing member 132, and a reed 133, the outer shell 131 and the pressing member 132 are provided with a communicating mounting hole, and a portion of the reed 133 extends into the mounting hole. When mounted, the pressing member 132 is pressed towards the outer shell 131, and the reed 133 is bent and deformed under the abutment of the pressing member 132, so that the portion of the reed 133 extending into the mounting hole is retracted from the mounting hole. Thus, the gas transmission pipeline may be sequentially inserted into the housing through the pressing member 132 and the reed 133. When the pressing member 132 is released after the gas transmission pipeline is inserted into the housing, the reed 133 loses the abutment of the pressing member 132, so that the reed 133 grips the gas transmission pipeline. The use of a fast-plug joint makes the connection of the fire damper to the gas transmission pipeline easier, faster and more secure. The fast-plug joint and the housing may be ultrasonically welded or may be bonded or friction welded.

The structures of the first joint 11 and the second joint 12 may be the same or different. For example, the first joint 11 adopts the zigzag structure, and the second joint 12 adopts the fast-plug joint. Alternatively, the first joint 11 is the fast-plug joint and the second joint 12 is the zigzag structure. In addition, the first joint 11 and the second joint 12 may adopt other structures. The structure of the first joint 11 and the second joint 12 is not limited in the present disclosure.

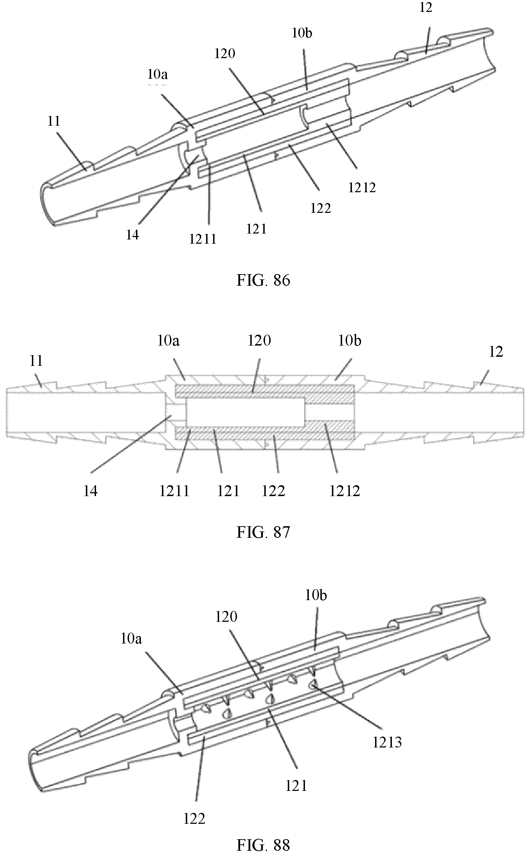

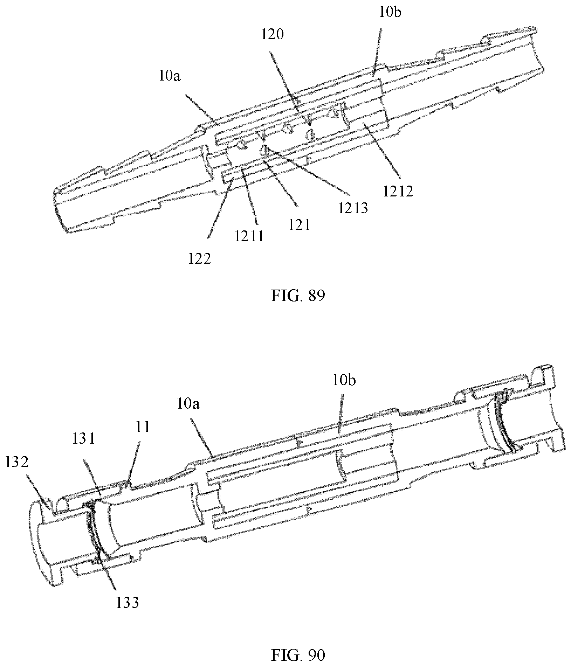

FIG. 86 is a first sectional view of a fire damper provided by an embodiment of the present disclosure. FIG. 87 is a second sectional view of a fire damper provided by an embodiment of the present disclosure. FIG. 88 is a sectional view of another fire damper provided by an embodiment of the present disclosure. FIG. 89 is a sectional view of another fire damper provided by an embodiment of the present disclosure. FIG. 90 is a sectional view of another fire damper provided by an embodiment of the present disclosure.

As shown in FIG. 86 to FIG. 90, the fire damper includes the housing and the sealing member 120 mounted within the housing. The housing is provided with the airflow passage therethrough. The airflow passage is provided with the first partition for partitioning the airflow passage. The first partition is provided with the first vent hole 14. The sealing member 120 is positioned within the airflow passage, and the first gap is provided between the sealing member 120 and the first vent hole 14 before being heated, to make the gas flow from one end of the airflow passage to the other through the first vent hole 14 and the first gap. The sealing member 120 is tubular. One end of the sealing member 120 is connected to the periphery of the first vent hole 14. The other end of the sealing member 120 is in an open state before being heated, and is in a closed state after being shrunk by heat.

The other end of the sealing member 120 is in the open state before being heated, i.e., before being heated, the second passage, the interior of the tubular sealing member 120, the first vent hole 14, and the first passage communicate to form a passage for oxygen transmission.

The other end of the sealing member 120 is in the closed state after being heated, i.e., the other end of the sealing member 120 is shrunk inwards in the radial direction of the pipe after being heated and shrunk together and then closed, so that oxygen cannot enter the inside of the sealing member 120 through the other end of the sealing member 120. At this time, the first partition and the sealing member 120 form a chamber, which is opened only at the first vent hole 14, so that the oxygen gas transmission passage is blocked and the oxygen cannot be continuously transmitted to the fire source.

Illustratively, as shown in FIG. 86 to FIG. 90, a side of the first partition facing towards the second passage is provided with a boss, and the sealing member 120 is sleeved on the boss, so as to fix one end of the sealing member 120. To make the sealing member 120 more securely, the sealing member 120 may have interference fit with the boss.

Certainly, one end of the sealing member 120 may be connected to the first partition by bonding or the like.

In order to make the other end of the sealing member 120 closed more effectively, the inner side of the shrinkable material layer 122 may be provided with a hot melt layer 121. The hot melt layer 121 may be made of a material having a relatively low melting point, such as a hot melt adhesive or the like. The hot melt layer 121 is melted by being heated, thereby improving fluidity, so that the melted hot melt layer 121 may better fill gaps that may be formed after the shrinkable material layer 122 shrinks.

Certainly, the layer structure disposed inside the shrinkable material layer 122 may be other materials, and the thermal expansion coefficients of the two layers may be different.

The wall thickness of an end of the hot melt layer 121 away from the first vent hole 14 is greater than the wall thickness of an end of the hot melt layer 121 close to the first vent hole 14. The end of the hot melt layer 121 away from the first vent hole 14 is a thick end 1212, and the end of the hot melt layer 121 close to the first vent hole 14 is a thin end 1211. The thick end 1212, away from the first vent hole 14, receives less heat and has a thicker wall thickness, the thick end 1212 does not readily melt. When the thin end 1211 melts, the hot melt material resulting from the melting is blocked by the thick end 1212 and accumulates at the thick end 1212, thereby closing the other end of the sealing member 120.

Illustratively, as shown in FIG. 86 and FIG. 87, the thick end 1212 has a uniform wall thickness, a step is formed between the thick end 1212 and the thin end 1211, which may block the melted hot melt material.

The hot melt layer 121 may be provided with a plurality of protrusions 1213 facing towards the interior of the sealing member 120. The portion of the hot melt layer 121 provided with the protrusion 1213 has a thicker wall thickness than the portion of the hot melt layer 121 not provided with the protrusion 1213, and is melted later after being heated. When the other portion of the hot melt layer 121 is melted after being heated, the melted hot melt material is blocked by the plurality of protrusions 1213, so as to gather at the protrusions 1213 to close the other end of the sealing member 120.

Illustratively, as shown in FIG. 88 and FIG. 89, the hot melt layer 121 is provided with a plurality of tapered protrusions 1213 towards the inside of the sealing member 120. Certainly, the shape of the protrusion 1213 may also be polygonal, cylindrical, frustum-shaped, etc.

In addition, in order to achieve that the installation of the fire damper does not distinguish directions, the second partition may also be disposed in the airflow passage. The second partition is provided with the second vent hole. The first partition and the second partition are axially provided at intervals along the airflow passage. The sealing member 120 blocks the second vent hole after being shrunk by heat. The structure of the second partition and the principle of action with the sealing member 120 may be the same as the first partition. This will not be repeated here.

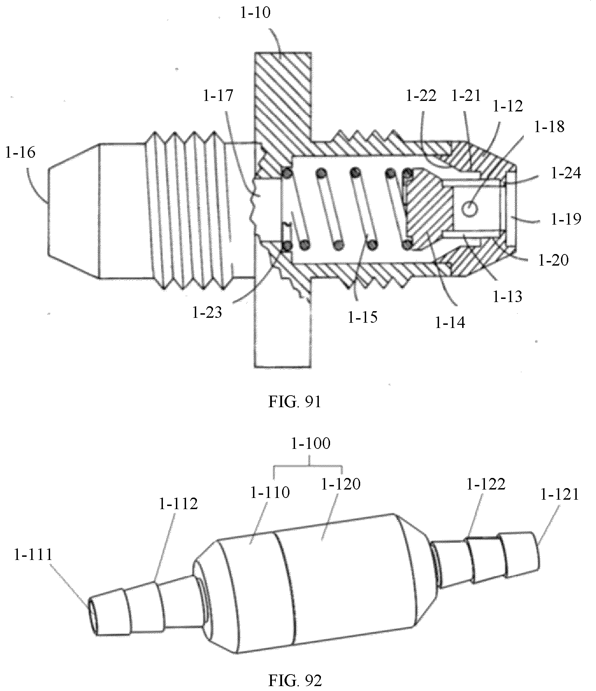

The description of the related art, for example, US4887631A, discloses a fire-protection isolation device, as shown in FIG. 91, for blocking the output path of oxygen in the event of a fire. The fire-protection isolation device includes an outer shell 1-10, an end seat 1-12, a meltable nose component 1-13, a poppet valve 1-14 and a compression spring 1-15. Here, the outer shell 1-10, the end seat 1-12, and the poppet valve 1-14 may be made of 316L stainless steel. The compression spring 1-15 may be made of a metal material. The meltable nose component 1-13 may be made of a thermoplastic, such as polyetheretherketone thermoplastic. Under normal operation, a fluid enters an inlet 1-16 via a central hole 1-17 surrounding the compression spring 1-15, passes through the poppet valve 1-14 and flows out of an outlet port 1-19 via a small hole 1-18 in the meltable nose component 1-13. The compression spring 1-15 abuts against a shoulder 1-23. The meltable nose component 1-13 is closely positioned in the inner diameter 1-20 of the outer end of the end seat 1-12, and up against a flange 1-24 on the end seat 1-12 of an end surface. The flange 1-24 is integral with the meltable nose component 1-13 and has the same inner diameter as that of the meltable nose component 1-13. The end seat 1-12 has a larger-diameter hole 1-21 at its inner end through which the fluid typically flows to the small hole 1-18 and the outlet port 1-19. This device is designed to operate at an elevated temperature of about 2000 degrees for about 15 minutes.

Under normal operation, gas (such as oxygen) flows out through the small holes 1-18 of the meltable nose component 1-13 after passing through the central hole 1-17 of the compression spring 1-15. In the event of a fire, the meltable nose component 1-13 will be melt up to 700 degrees, so that the meltable nose component 1-13 will collapse into a larger-diameter hole 1-21 and the poppet valve 1-14 will abut against an inclined portion 1-22 of the end seat 1-12. The poppet valve 1-14 loaded by the compression spring 1-15 is thereby allowed to close, preventing leakage of liquid through any additional components. Since the compression spring 1-15 is made of metallic materials which, after prolonged exposure to oxygen, particularly in humid environments, the compression spring 1-15 will inevitably appear chemical reactions such as oxidation, and thus causing failure of the compression spring 1-15, which would greatly reduce the useful life of the fire-protection isolation device while affecting the health of the patient.

In view of the above-mentioned problems in the related art, the present disclosure provides an automatic fire protection device applied to an oxygen therapy apparatus, which is capable of preventing occurrence of a failure phenomenon contacting with oxygen for a long time, thereby improving the service life of the automatic fire protection device.

As shown in FIG. 92 to FIG. 99, the present disclosure provides an automatic fire protection device applied to an oxygen therapy apparatus, including a housing 1-100 and a sealing member 1-40 disposed inside the housing 1-100. A gas passage 1-140 is disposed in the housing 1-100. One end of the gas passage 1-140 is a gas outlet end, and the gas passage 1-140 is capable of allowing a gas to pass therethrough.

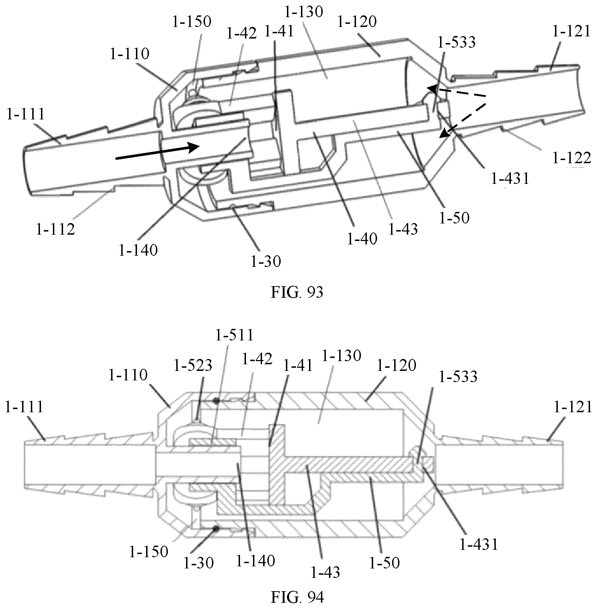

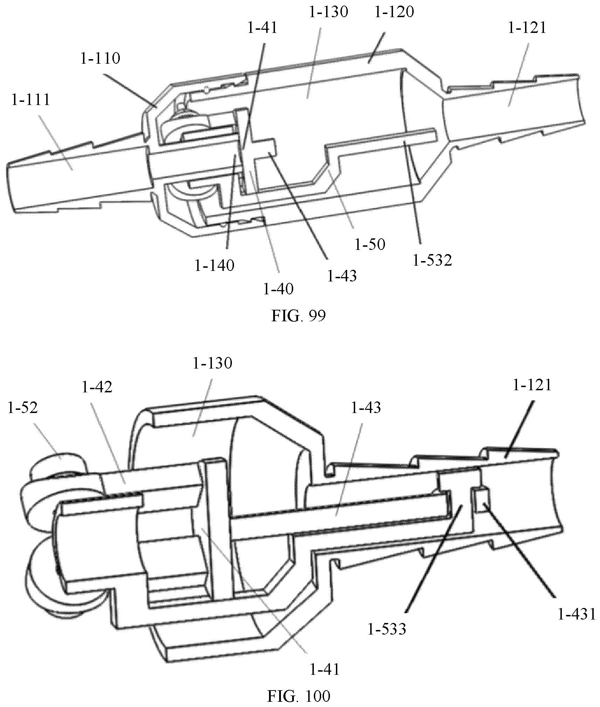

Specifically, the sealing member 1-40 includes a sealing portion 1-41. When the sealing member 1-40 is in a first state (as shown in FIG. 93), a gap is provided between the sealing portion 1-41 and the gas outlet end of the gas passage 1-140, such that the gas passage 1-140 is open. When the sealing member 1-40 is in a second state (as shown in FIG. 99), the sealing portion 1-41 attaches to and seals the gas outlet end of the gas passage 1-140 to close the gas passage 1-140.

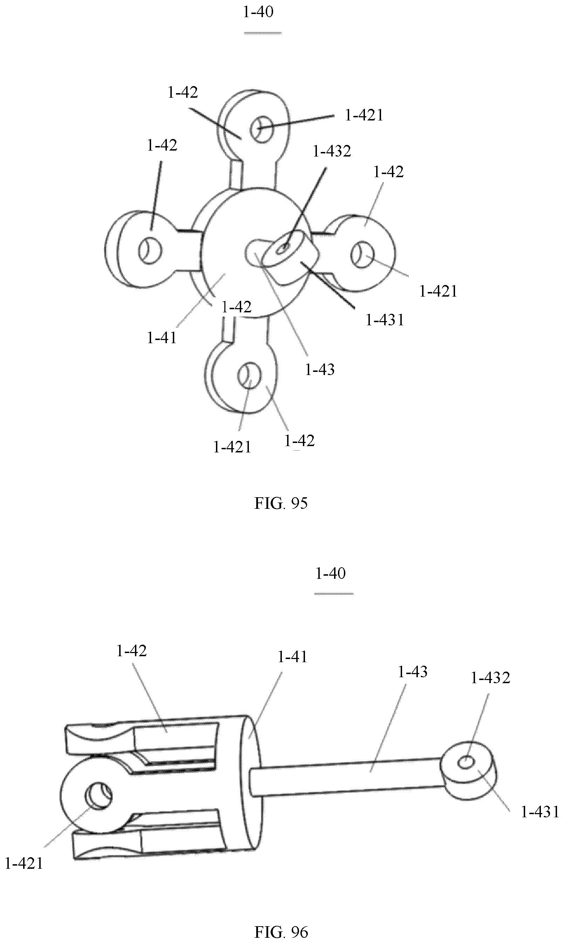

Further, referring to FIG. 95 and FIG. 96, and referring to FIG. 94, the sealing member 1-40 further includes a first connector 1-42 and a second connector 1-43 respectively disposed on the sealing portion 1-41, the first connector 1-42 and the second connector 1-43 are respectively fixed on both sides of the gas outlet end of the gas passage 1-140. In other words, the first connector 1-42 is located on an upstream side of the gas passage 1-140, and the second connector 1-43 is located on a downstream side of the gas passage 1-140. Herein, when the sealing member 1-40 is in the first state, the first connector 1-42 and the second connector 1-43 are both fixed in the housing 1-100 and are both in a stretched state (as shown in FIG. 96), so that the sealing portion 1-41 is away from the gas passage 1-140 to form a gap with the gas outlet end of the gas passage 1-140. As shown in FIG. 94, at this time, there is a certain distance between the sealing portion 1-41 and the gas outlet end of the gas passage 1-140, so that the gas in the gas passage 1-140 can flow out from the gas outlet end thereof. The state at this time corresponds to a state in which the automatic fire protection device applied to the oxygen therapy apparatus of the present disclosure is when an unexpected event such as a fire or the like does not occur.

On the contrary, when the sealing member 1-40 is in the second state, the first connector 1-42 is fixed in the housing 1-100 and is in the stretched state (as shown in FIG. 99). The second connector 1-43 is disconnected and is in a natural state, so that the sealing portion 1-41 is close to the gas passage 1-140 and attached to the gas outlet end of the gas passage 1-140. As shown in FIG. 99, at this time, the original fixed position of the second connector 1-43 is broken, so that the second connector 1-43 loses restraint. Thus, the sealing portion 1-41 is pulled into close attachment with the gas outlet end of the gas passage 1-140 by the pulling force of the first connector 1-42, so that the gas in the gas passage 1-140 cannot flow out from the gas outlet end thereof. The state at this time corresponds to a state in which the automatic fire protection device applied to the oxygen therapy apparatus of the present disclosure is when an unexpected event such as a fire or the like does occur.

Therefore, in the absence of a fire, the sealing portion 1-41 is not in contact with the gas passage 1-140. The gas can reach the mouth and nose of the user by means of the transmission of the housing 1-100 by flowing from the gas inlet end to the gas outlet end of the gas passage 1-140 and flowing out from the gas outlet end, so that the user can be normally provided with the gas such as oxygen (the flow direction of the gas is shown by a solid arrow in FIG. 3). On the contrary, in the event of a fire, the sealing portion 1-41 is closely attached to the gas outlet end of the gas passage 1-140, so that the gas passage 1-140 is closed, thereby blocking the oxygen transmission path to ensure the safety of the user.

In a preferred embodiment, the sealing member 1-40 is made of an elastic non-metallic material, preferably a silicone material, so that the sealing member 1-40 is elastic and may be stretched. Also, it may avoid problems of long contact with oxygen in the housing and oxidation reactions that affect product life and reliability. Since the gas (e.g., oxygen) flowing out through the gas outlet end of the gas passage 1-140 surrounds the sealing member 1-40 during the gas transmission, the sealing member 1-40 may be contacted with oxygen for a long period of time. The sealing member 1-40 made of the silicone material may be prevented from chemical reactions, such as oxidation reactions, thereby improving the service life of the automatic fire protection device applied to the oxygen therapy apparatus and avoiding health effects on users.

The second connector 1-43 is fixed in the housing 1-100 by a fixing structure configured such that it may be fused after being heated beyond a preset temperature. Then, the second connector 1-43 changes from the stretched state to the natural state.

In the event of a fire, the flame will gradually burn towards the direction of the oxygen source, the dashed arrow in FIG. 93 shows the ignition direction of the flame. Since the second connector 1-43 is located on the downstream side of the gas passage 1-140, the flame will first burn the fixed structure. When the fixed structure is fused after being heated beyond the preset temperature, the fixed constraint of the second connector 1-43 is lost, so that the second connector 1-43 may change from the stretched state to the natural state. At this time, since the first connector 1-42 is still fixed in the housing 1-100 and is in the stretched state, the first connector 1-42 may pull the sealing portion 1-41 to move, so that the sealing portion is closely attached to the gas outlet end of the gas passage 1-140. Thus, the gas passage 1-140 is closed, and the oxygen transmission path is blocked to ensure safe use. The specific shape and structure of the sealing portion 1-41 may be specifically set according to the shape of the ventilation cross section of the gas passage 1-140 to be attached and sealed. Therefore, the present disclosure does not define the specific shape and structure of the sealing portion 1-41. Illustratively, the present disclosure provides a preferred configuration in which, as shown in FIG. 95, the sealing portion 1-41 may be configured in the form of a disc having a sealing surface on one side thereof, the sealing surface is in contact with the gas outlet end of the gas passage 1-140, so that the area of the sealing surface is at least greater than the area of the gas outlet end of the gas passage 1-140. Thus, the sealing surface can completely cover the gas outlet end of the gas passage 1-140 when the sealing surface is in contact with the gas outlet end of the gas passage 1-140 to prevent gas leakage.

A quantity of the first connectors 1-42 is at least two. FIG. 95 shows an embodiment in which four first connectors 1-42 are provided, where the four first connectors 1-42 are equally spaced on the side wall of the sealing portion 1-41 (for example, they may be disposed on the circumferential side wall of the sealing portion 1-41). The first connector 1-42 extends in a radial direction of the sealing portion 1-41 when the first connector 1-42 is not stretched and in a natural state, i.e., a state shown in FIG. 95. The first connector 1-42 extends in an axial direction of the sealing portion 1-41 when the first connector 1-42 is stretched, i.e., a state shown in FIG. 96. Therefore, when both the first connector 1-42 and the second connector 1-43 are in the natural state, the direction in which the first connector 1-42 extends is perpendicular to the direction in which the second connector 1-43 extends.

By disposing the first connector 1-42 on the side wall of the sealing portion 1-41, mounting and forming can be facilitated. With continued reference to FIG. 5, the second connector 1-43 is disposed on one side of the end of the sealing portion 1-41, the first connector 1-42 and the second connector 1-43 are respectively disposed on two sides of the corresponding sealing portion 1-41. The first connector 1-42 is disposed on one side of the sealing portion 1-41 away from the second connector 1-43.

Herein, when the sealing member 1-40 is in the first state, the stretching and extending directions of the first connector 1-42 and the second connector 1-43 are opposite. Namely, the second connector 1-43 extends in the axial direction of the sealing portion 1-41 in a direction opposite to the extending direction of the stretched first connector 1-42. That is, the first connector 1-42 and the second connector 1-43 can be stretched in opposite directions, so that the first connector 1-42 and the second connector 1-43 may be fixed at both sides of the gas passage 1-140.

It is also conceivable to arrange the first connector 1-42 directly on one side of the sealing portion 1-41 and to arrange the second connector 1-43 on the other side of the sealing portion 1-41, so that both the first connector 1-42 and the second connector 1-43 extend in opposite directions in the axial direction of the sealing portion 1-41 when both are in a natural state.

The functions of the first connector 1-42 and the second connector 1-43 are to respectively apply a pulling force to the sealing portion 1-41 at two sides thereof. Therefore, the sealing portion 1-41 can be kept at a position at a certain distance from the gas outlet end of the gas passage 1-140 when a fire does not occur, so as to facilitate gas transmission. However, when a fire occurs, the action of the pulling force on one side of the sealing portion 1-41 disappears, and the sealing portion 1-41 attaches to the gas outlet end of the gas passage 1-140 under the action of the pulling force on the other side, thereby blocking the gas passage 1-140. Therefore, the first connector 1-42 and the second connector 1-43 of the present disclosure may also have other structural forms as long as the pulling force in opposite directions can be provided on both sides of the sealing portion 1-41, respectively.

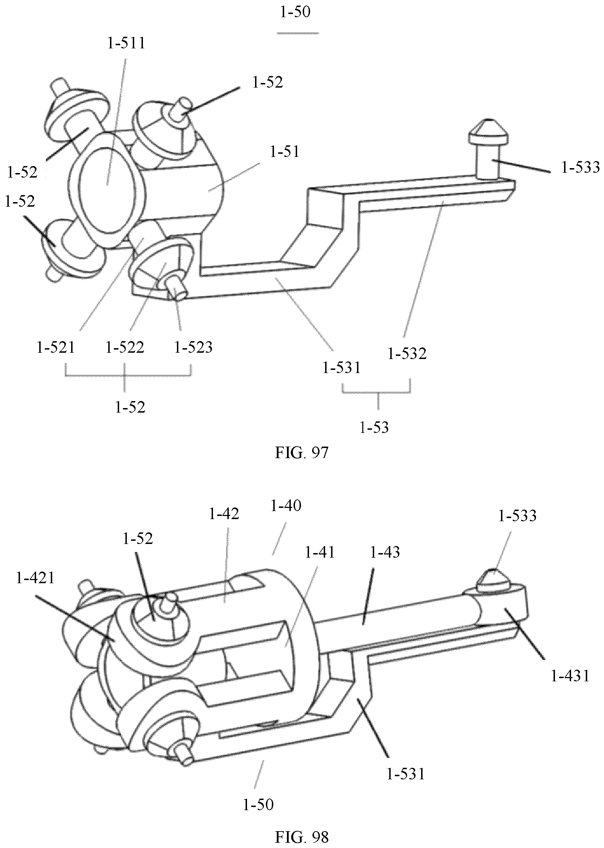

Further, referring to FIG. 94 and FIG. 97, the housing 1-100 is further provided with a mounting arm 1-50 for mounting the sealing member 1-40, and the mounting arm 1-50 includes an adapter block 1-51, a mounting post 1-52, and a connecting plate 1-53.

The adapter block 1-51 is constructed in a columnar structure. The adapter block 1-51 is provided with an accommodating hole 1-511 therethrough. The gas passage 1-140 is penetratingly disposed in the accommodating hole 1-511. Therefore, it can be understood that the gas outlet end of the gas passage 1-140 is located at an outer end of the accommodating hole 1-511 (as shown in FIG. 94 and FIG. 97).

The mounting post 1-52 is disposed on a side wall of the adapter block 1-51 (e.g., It may be disposed on a circumferential side wall of the adapter block 1-51), and the mounting post 1-52 is used to connect the first connector 1-42. The number and distribution of the first connectors 1-42 are the same as the number and distribution of the mounting posts 1-52, respectively, i.e., the two are arranged in a one-to-one correspondence. FIG. 97 shows an embodiment in which four mounting posts 1-52 are provided, where the four mounting posts 1-52 are equally spaced on the side wall of the adapter block 1-51. As shown in FIG. 98, four first connectors 1-42 are connected to corresponding four mounting posts 1-52, respectively.

The connecting plate 1-53 extends in the axial direction of the accommodating hole 1-511. A side of the connecting plate 1-53 away from the adapter block 1-51 is provided with a trigger post 1-533 for connecting with the second connector 1-43. Herein, when the first connector 1-42 is connected to the mounting post 1-52 and the second connector 1-43 is connected to the trigger post 1-533, the sealing member 1-40 is in a first state within the housing 1-100. When the first connector 1-42 is connected to the mounting post 1-52 and the second connector 1-43 is disconnected from the trigger post 1-533, the sealing member 1-40 is in the second state in the housing 1-100.

As shown in FIG. 95 and FIG. 96, the above-described fixing structure for fixing the second connector 1-43 in the housing 1-100 includes a connecting ring 1-431 provided at the end of the second connector 1-43 and the trigger post 1-533 on the connecting plate 1-53. The connecting ring 1-431 may be cooperated with the trigger post 1-533. More specifically, referring to FIG. 96 and FIG. 98, a second connecting hole 1-432 of the connecting ring 1-431 is sleeved on the trigger post 1-533, so that the second connector 1-43 is fixed to one end of the mounting arm 1-50 (as shown in FIG. 98).

As shown in FIG. 97, the end of the trigger post 1-533 is of a truncated cone structure. Therefore, the second connecting hole 1-432 may be sleeved on the trigger post 1-533 when being stretched and deformed to increase the diameter thereof. The diameter thereof is then restored so that it does not fall off the trigger posts 1-533 under the obstruction of the truncated cone structure at the ends of the trigger posts 1-533.

In addition, the second connecting hole 1-432 of the connecting ring 1-431 and the trigger post 1-533 of the truncated cone structure each have a reduced size portion, so that they are easily fused, thereby improving the triggering speed of the automatic fire protection device applied to the oxygen therapy apparatus.

Herein, the connecting ring 1-431 and/or the trigger post 1-533 may be fused (burned off) at a high temperature exceeding a preset temperature. In the event of a fire, a high-temperature gas or a flame of combustion is first propagated to a position of the connecting ring 1-431 and/or the trigger post 1-533. The connecting ring 1-431 or the trigger post 1-533 is fused, or both are fused, so that the fixing of the second connector 1-43 to the mounting arm 1-50 is broken, and the second connector 1-43 is unconstrained, thereby being retracted, as shown in FIG. 99. The sealing portion 1-41 can leave the gas outlet end of the gas passage 1-140 only under the stretching action of the second connector 1-43. When the pulling force on the second connector 1-43 disappears, the second connector 1-43 begins to retract from the original stretched state. Since the first connector 1-42 also has a stretching action on the sealing portion 1-41, the sealing portion 1-41 will be pulled into close contact with the gas outlet end of the gas passage 1-140. The gas passage 1-140 is blocked by the sealing portion 1-41, so that high-temperature gas or burned gas may not continue to pass through the gas passage 1-140, so as to ensure the safety of the user.

It is also conceivable to arrange the material of the connecting ring 1-431 to be different from the material of other parts of the second connector 1-43. For example, the connecting ring 1-431 may be made of a material with a lower high-temperature resistance, while the material of other parts of the second connector 1-43 is made of a material with a higher high-temperature resistance, so that the connecting ring 1-431 is more easily fused. In addition, the trigger post 1-533 may be similarly configured. For example, the material of the trigger post 1-533 is different from the material of other parts of the connecting plate 1-53. The material of the trigger post 533 has poor resistance to high temperature. The material of other parts of the connecting plate 1-53 is more resistant to high temperature, so that the trigger post 533 is more easily fused. It should be noted that the preset temperature described herein is generally 400°C or higher.

As shown in FIG. 97, the mounting post 1-52 includes a connecting post 1-521, a stop circular truncated cone 1-522, and a fixing post 1-523. The connecting post 1-521 extends in the radial direction of the accommodating hole 1-511 on the side wall of the adapter block 1-51. The stop circular truncated cone 1-522 is disposed on the connecting post 1-521. The maximum outer diameter of the stop circular truncated cone 1-522 is greater than the outer diameter of the connecting post 1-521, and the diameter of the stop circular truncated cone 1-522 is tapered in the direction away from the adapter block 1-51. The fixing post 1-523 is disposed at an end of the stop circular truncated cone 1-522 for connecting with an inner wall of the housing 1-100, to fix the mounting arm 1-50 in the housing 1-100. Referring to FIG. 95 and FIG. 96, the first connector 1-42 is provided with a first connecting hole 1-421 extending through the thickness thereof. The inner diameter of the first connecting hole 1-421 is less than the maximum outer diameter of the stop circular truncated cone 1-522. Therefore, when the first connector 1-42 is stretched, the diameter of the first connecting hole 1-421 is enlarged, so that the first connecting hole 1-421 may pass through the stop circular platform 1-522 corresponding thereto and is sleeved on the connecting post 1-521. Then, when the diameter thereof is restored, the first connecting hole 1-421 cannot be detached from the connecting post 1-521 due to the blocking action of the stop circular truncated cone 1-522, thereby fixing the first connector 1-42 with the other end of the mounting arm 1-50.

Further, with continued reference to FIG. 97 and FIG. 98, the connecting plate 1-53 includes a recessed portion 1-531 and an extension plate 1-532. As shown in FIG. 97, one end of the recessed portion 1-531 is connected to the side wall of the adapter block 1-51, and the recessed portion 1-531is used to receive the sealing portion 1-41. As shown in FIG. 8, when the second connector 1-43 is stretched and fixed with the mounting arm 1-50, the sealing portion 1-41 is positioned in the recessed portion 1-531 with its sealing surface positioned opposite to the end of the adapter block 1-51.

The extension plate 1-532 is connected to the other end of the recessed portion 1-531. The extension plate 1-532 extends in the axial direction of the accommodating hole 1-511. The trigger post 1-533 is disposed on the side of the extension plate 1-532 away from the recessed portion 1-531, for example, on the upper side thereof. The trigger post 1-533 may be substantially perpendicular to the extension plate 1-532. When the second connector 1-43 is stretched, it is substantially parallel to the extension plate 1-532.

As shown in FIG. 93 and FIG. 94, the housing 1-100 includes a first housing 1-110 and a second housing 1-120. The first housing 1-110 and the second housing 1-120 are connected to form a sealed chamber 1-130 therein. A gas passage 1-140 extends from one side inside the chamber 1-130. The sealing member 1-40 and the mounting arm 1-50 are both disposed in the chamber 1-130.

One side of the first housing 1-110 is provided with a first nozzle 1-111 for being connected to a catheter (not shown). The first nozzle 1-111 is connected to an oxygen source such as an oxygen generator via the catheter. A second nozzle 1-121 for connection to a catheter (not shown) is provided on the side of the second housing 1-120 opposite to the first housing 1-110, the second nozzle 1-121 is connected via the catheter to a respiratory mask worn on the face of the patient. Herein, when the sealing member 1-40 is in the first state, the first nozzle 1-111, the gas passage 1-140, the chamber 1-130, and the second nozzle 1-121 are in fluid communication. When the sealing member 1-40 is in the second state, the fluid enters the gas passage 1-140 through the first nozzle 1-111 is isolated from the chamber 1-130 outside the gas passage 1-140. Thus, in normal use, oxygen is received from the first nozzle 1-111, flows through the gas passage 1-140 and the chamber 1-130 into the second nozzle 1-121, and is supplied to the patient by the second nozzle 1-121, as shown by the solid arrows in FIG. 93.

As shown in FIG. 93, the gas passage 1-140 may extend from the inner wall side of the first housing 1-110 and communicate with the first nozzle 1-111. When the sealing member 1-40 is in the first state, there is a certain distance between the sealing portion 1-41 and the gas outlet end of the gas passage 1-140, so that the gas may flow out from the gas outlet end of the gas passage 1-140 into the chamber 1-130 and into the second nozzle 1-121. As shown in FIG. 99, when the sealing member 1-40 is in the second state, and the sealing portion 1-41 is attached to the gas outlet end of the gas passage 1-140, the gas may not flow out of the gas outlet end of the gas passage 1-140, and naturally cannot flow into the second nozzle 1-121.

Accordingly, in the embodiments of the present disclosure, the gas passage 1-140 includes the gas inlet end and the gas outlet end. The first nozzle 1-111 communicates with the gas inlet end of the gas passage 1-140. When the sealing member 1-40 is in the first state, namely, the temperature in the housing 1-100 does not exceed a preset temperature (a fusing temperature), the second connector 1-43 of the sealing member 1-40 maintains a connection state with the mounting arm 1-1250, so that a certain gap is maintained between the sealing portion 1-41 of the sealing member 1-40 and the gas outlet end of the gas passage 1-140. Therefore, the fluid may flow into the chamber 1-130 after flowing through the first nozzle 1-111, the gas inlet end of the gas passage 1-140 and the gas outlet end of the gas passage 1-140 in sequence, and output from the second nozzle 1-121. When the temperature in the housing 1-100 is higher than the preset temperature, the connecting ring 1-431 of the second connector or the trigger post 1-533 of the mounting arm 1-50 is fused. Therefore, the sealing member 1-40 is switched from the first state to the second state, namely, the end of the second connector 1-43 is disconnected from the corresponding end (the trigger post 1-533) of the mounting arm 1-50, while the end of the first connector 1-42 remains connected to the corresponding end (the fixing post 1-523) of the mounting arm. Then, the sealing portion 1-41 of the sealing member 1-40 moves in the direction close to the adapter block 1-51 in the housing 100 under the action of the pulling force of the first connector 1-42, until the sealing portion 1-41 is attached to the corresponding end (namely, the gas outlet end surface of the gas passage 1-140) of the adapter block 1-51. At this time, although the fluid may enter the gas passage 1-140 via the first nozzle 1-111 and the gas inlet end of the gas passage 1-140, the fluid cannot flow out of the gas outlet end of the gas passage 1-140 because the gas outlet end of the gas passage 1-140 is attached and sealed by the sealing portion 1-41. Meanwhile, due to the attaching and sealing function of the sealing portion 1-41, the burnt gas may not spread to the oxygen therapy device through the gas passage 1-140, so that the fire around the oxygen therapy apparatus can be prevented from being promoted and initiated or increased to cause a fire accident, thereby realizing the function of automatic fire prevention of the oxygen therapy apparatus.

As shown in FIG. 93 and FIG. 94, the inner wall of the chamber 1-130 is provided with a connecting groove 1-150 for being connected with the fixing post 1-523. The connecting groove 1-150 may be defined by an inner wall of the first housing 1-110 and an end of the second housing 1-120, so that the mounting arm 1-50 may be fixed in the chamber 1-130.

In this embodiment, the trigger post 1-533 and the connecting ring 1-431 are disposed in the chamber 1-130. More specifically, the trigger post 1-533 and the connecting ring 1-431 are disposed in the chamber 1-130 at a position close to the second nozzle 1-121, so that the burning gas propagating from the second nozzle 1-121 can first fuse the trigger post 1-533 and/or the connecting ring 1-431.

As shown in FIG. 92, the outer walls of the first nozzle 1-111 and the second nozzle 1-121 are each provided with an anti-detachment portion. Specifically, a first anti-detachment portion 1-112 is disposed on the outer wall of the first nozzle 1-111. A second anti-detachment portion 1-122 is disposed on the outer wall of the second nozzle 1-121.

In a preferred embodiment, the first anti-detachment portion 1-112 may be configured as a tapered step having a gradually increasing diameter in a direction towards the first housing 1-110, preventing the first nozzle 1-111 from escaping from the catheter after being connected to the catheter. In addition, the second anti-detachment portion 1-122 may adopt a structure similar to that of the first anti-detachment portion 1-112, i.e., it is also a tapered step having a gradually increasing diameter in a direction towards the second housing 1-120, thus preventing the second nozzle 1-121 from escaping from the catheter after being connected to the catheter.

It is contemplated that the first anti-detachment portion 1-112 may be further configured spiral protrusions spirally arranged on the outer wall of the first nozzle 1-111 in the axial direction of the first nozzle 1-111 to function as escape prevention.

Alternatively, it is also contemplated that the first anti-detachment portion 1-112 may be configured to provide a plurality of mold blocks along the circumferential direction of the first nozzle 1-111 to function as escape prevention.

The second anti-detachment portion 1-122 may adopt a structure similar to that of the first anti-detachment portion 1-112, and which will not be described in detail herein.

The first housing 1-110 and the second housing 1-120 are connected by means of a sealing snap, welding or a threaded connection. As shown in FIG. 93, the first housing 1-110 and the second housing 1-120 are connected via a snap connection, and a sealing ring 1-30 is further provided at the connection of the both, so as to ensure the tightness of the chamber 1-130.

As shown in FIG. 100, this embodiment is a modification of the above embodiment. Hereinafter, the differences between the present embodiment and the above-described embodiment will be described, and the same will not be described again.

As shown in FIG. 100, both the connecting plate 1-53 and the second connector 1-43 extend into the second nozzle 1-121, so that the connecting ring 1-431 and the trigger post 1-533 are located in the second nozzle 1-121. As described above, in the event of a fire, the flame will gradually burn from the second nozzle 1-121 in the direction towards the oxygen source, so that the arrangement in this embodiment has a faster trigger speed in the event of a fire, i.e., the connecting ring 1-431 and/or the trigger post 1-533 may be fused faster.

While the present disclosure has been described with reference to preferred embodiments, various modifications may be made and equivalents may be substituted for components thereof without departing from the scope of the present disclosure. In particular, the technical features mentioned in the various embodiments may be combined in any manner as long as there are no structural conflicts. The present disclosure is not limited to the particular embodiments disclosed herein, but includes all technical solutions falling within the scope of the claims.

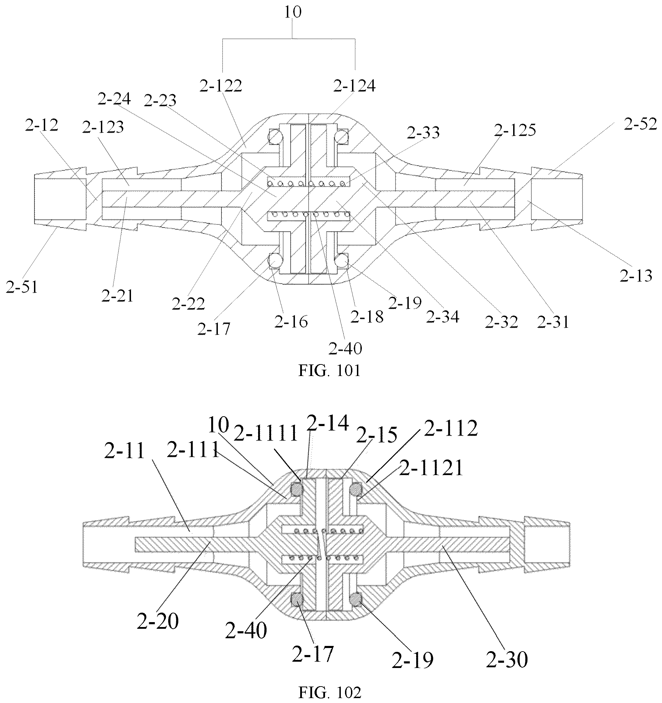

As shown in FIG. 101 to FIG. 108, the tenth type of the fire protection device 2-100 includes a housing 10, a first valve body 2-20, a second valve body 2-30 and an elastic member 2-40. The housing 10 has a fluid passage 2-11 therein, one end of the housing 10 is provided with a first opening, and a second end of the housing 10 is provided with a second opening. Both the first opening and the second opening are in communication with the fluid passage 2-11. The first opening and the second opening are used for communicating with a pipeline of an oxygen therapy instrument or a pipeline of an oxygen supply end. The first valve body 2-20 and the second valve body 2-30 are both located in the fluid passage 2-11 and spaced apart along the direction from the first opening to the second opening. A first limiting rib 2-12 is fixed on the hole wall of the first opening, and a second limiting rib 2-13 is fixed on the hole wall of the second opening. A first end of the first valve body 2-20 abuts against the first limiting rib 2-12, and a first end of the second valve body 2-30 abuts against the second limiting rib 2-13. An elastic member 2-40 is located between the first valve body 2-20 and the second valve body 2-30, and two ends of the elastic member 2-40 respectively abut against a second end of the first valve body 2-20 and a second end of the second valve body 2-30. The elastic member 2-40 is in a compressed state. Gaps for the circulation of a gas is provided between a peripheral portion of the first valve body 2-20 and a passage wall of the fluid passage 2-11 and between a peripheral portion of the second valve body 2-30 and the passage wall of the fluid passage 2-11. When the first limiting rib 2-12 is in a molten state, the elastic member 2-40 is elongated, the first valve body 2-20 is located at a first position, and the first valve body 2-20 is in sealing connection to the passage wall of the fluid passage 2-11 so as to block the fluid passage 2-11. When the second limiting rib 2-13 is in the molten state, the elastic member 2-40 is elongated, the second valve body 2-30 is in a second position, and the second valve body 2-30 is in sealing connection to the passage wall of the fluid passage 2-11 to block the fluid passage 2-11.

In the embodiment of the present disclosure, the tenth type of the fire protection device 2-100 includes the housing 10, the first valve body 2-20, the second valve body 2-30, and the elastic member 2-40. The housing 10 has the fluid passage 2-11 therein. One end of the housing 10 has the first opening, and the second end of the housing 10 has the second opening. The first opening and the second opening are in communication with the fluid passage 2-11, therefore, the first opening, the fluid passage 2-11, and the second opening may form a passage for the circulation of the gas. Herein, the first opening and the second opening are used for communicating with the pipeline of the oxygen therapy instrument or the pipeline of the oxygen supply end, so that one end of the fluid passage 2-11 may be connected to the pipeline of the oxygen therapy instrument through the first opening and the second opening, and the other end of the fluid passage 2-11 may be connected to the pipeline of the oxygen supply end. The oxygen therapy instrument may deliver oxygen to the patient through the oxygen supply end. The first limiting rib 2-12 is fixed on the hole wall of the first opening. The second limiting rib 2-13 is fixed on the hole wall of the second opening. The first end of the first valve body 2-20 abuts against the first limiting rib 2-12. The second end of the second valve body 2-30 abuts against the second limiting rib 2-13. The elastic member 2-40 is connected between the second end of the first valve body 2-20 and the second end of the second valve body 2-30, so that the first valve body 2-20 and the second valve body 2-30 may be distributed at intervals along the direction from the first opening to the second opening. Since the elastic member 2-40 is in a compressed state, and the extension and retraction direction of the elastic member 2-40 is consistent with the extension direction of the fluid passage 2-11, the elastic member 2-40 may exert a force on the first valve body 2-20 and the second valve body 2-30, so that the first valve body 2-20 has a tendency to move in a direction towards the first opening, and the second valve body 2-30 has a tendency to move towards the second opening. At this time, the first limiting rib 2-12 may block the first valve body 2-20, and the second limiting rib 2-13 may block the second valve body 2-30. Since the gaps for the circulation of the gas is provided between the peripheral portion of the first valve body 2-20 and the passage wall 2-11 of the fluid passage and between the peripheral portion of the second valve body 2-30 and the passage wall 2-11 of the fluid passage, in normal use, the gas can flow through the gaps, so that the oxygen therapy instrument can be normally used. In the case where the first limiting rib 2-12 is in the molten state when being burned by a flame, the first limiting rib 2-12 loses a blocking effect on the first valve body 2-20. At this time, the elastic member 2-40 is elongated, namely, the elastic member 2-40 may release elastic potential energy, and the first valve body 2-20 moves to the first position in the direction towards the first opening under the elastic force of the elastic member 2-40, so that the first valve body 2-20 is in sealing connection with the passage wall of the fluid passage 2-11, thereby blocking the fluid passage 2-11 and preventing oxygen from continuing to flow in the fluid passage 2-11. In the case where the second limiting rib 2-13 is in the molten state when being burned by a flame, the second limiting rib 2-13 loses a blocking effect on the second valve body 2-30. At this time, the elastic member 2-40 is elongated, namely, the elastic member 2-40 may release the elastic potential energy. The second valve body 2-30 moves to the second position in the direction towards the second opening under the elastic force of the elastic member 2-40, so that the second valve body 2-30 is in sealing connection with the passage wall of the fluid passage 2-11, thereby blocking the fluid passage 2-11 and preventing oxygen from continuing to flow in the fluid passage 2-11.

That is, in the embodiment of the present disclosure, if a flame is induced due to improper operation during the oxygen therapy, the first limiting rib 2-12 or the second limiting rib 2-13 burns in the molten state. At this time, the elastic member 2-40 disposed between the first valve body 2-20 and the second valve body 2-30 may push the first valve body 2-20 or the second valve body 2-30 to move, so that the first valve body 2-20 or the second valve body 2-30 is in sealing connection with the fluid passage 2-11, thereby blocking the fluid passage 2-11. The fire protection device 2-100 may prevent the oxygen from continuing to flow in the fluid passage 2-11, avoid the problem that the flame with the oxygen pipe gradually burns to the fuselage, which easily causes the spread of fire and finally ignites the oxygen generator to have serious fire.

Herein, in a case where the first limiting rib 2-12 is in the molten state when being burned by a flame, the elastic member 2-40 is elongated. Specifically, the elastic member 2-40 is elongated from a compressed state to a natural state. At this time, the elastic member 2-40 releases all the elastic potential energy possessed thereby, and the elastic member 2-40 is in the initial length. Alternatively, it may also be that the elastic element 2-40 releases part of the elastic potential energy. At this time, the elastic element 2-40 is elongated compared to the elastic element 2-40 in the initial state, but the elastic element 2-40 is still in the compressed state. Also, in a case where the second limiting rib 2-13 is in the molten state when burned by the flame, the elongation of the elastic member 2-40 may be in the above two states. The embodiments of the present disclosure will not be described in detail herein. In the above description, the initial state is a state in which the first limiting rib 2-12 and the second limiting rib 2-13 are both in a non-molten state.

It should be noted that the elastic member 2-40 is disposed in the fluid passage 2-11. The elastic member 2-40 may be a metal spring or a metal elastic sheet made of a metal such as iron, copper, alloy, etc., or a soft rubber such as silica gel, rubber, etc. which can store the elastic potential energy. The elastic member 2-40 may store the elastic potential energy and push the first valve body 2-20 or the second valve body 2-30 in the event of a fire to block the fluid passage and block the passage of oxygen, thereby achieving the effect of preventing the spread of a fire.

It should also be noted that the first limiting ribs 2-12 and the second limiting ribs 2-13 may both be meltable pieces, and may be made of a material with a relatively low melting point, for example, polyformaldehyde (POM), ethylene vinyl acetate (EVA), ethylene propylene diene monomer (EPDM), etc. The embodiments of the present disclosure are not specifically limited herein for the specific materials of the first limiting ribs 2-12 and the second limiting ribs 2-13. Thus, when a patient normally uses the oxygen therapy instrument, the first limiting rib 2-12 and the second limiting rib 2-13 are both in the non-molten state. At this time, the first limiting rib 2-12 and the second limiting rib 2-13 are both not burned, and are in the non-molten state. The first limiting rib 2-12 supports the first valve body 2-20, the second limiting rib 2-13 supports the second valve body 2-30, and the elastic member 2-40 is in the compression state. At this time, the fluid passage 2-11 is not blocked, and oxygen may normally circulate. When the patient uses the oxygen therapy instrument and a flame is generated, the first limiting rib 2-12 or the second limiting rib 2-13 is melted. At this time, the first limiting rib 2-12 or the second limiting rib 2-13 is in the molten state, and the elastic member 2-40 restores to the initial state. At this time, the fluid passage 2-11 is blocked, thereby preventing the circulation of oxygen and preventing a fire from occurring.

In addition, in the embodiments of the present disclosure, the first opening or the second opening is connected to the pipeline of the oxygen supply end, and is closer to the oxygen supply end used by the user. In the case where the first opening is connected to the pipeline of the oxygen supply end, since the first limiting rib 2-12 is provided at the hole wall of the first opening, if the patient accidentally ignites the pipeline of the oxygen supply end, the flame may reach the first limiting rib 2-12 faster, so that the first limiting rib 2-12 is broken faster, the fluid passage 2-11 is blocked faster, and the flow of oxygen may be blocked in time. Thus, the risk of fire spreading due to non-timely triggering is reduced, thereby improving the safety factor of the fire protection device 2-100. In the case where the second opening is connected to the pipeline of the oxygen supply end, since the second limiting rib 2-13 is provided at the hole wall of the second opening, if the patient accidentally ignites the pipeline of the oxygen supply end, the flame may reach the second limiting rib 2-13 faster, so that the second limiting rib 2-13 is broken faster, the fluid passage 2-11 is blocked faster, and the flow of oxygen may be blocked in time. Thus, the risk of fire spreading due to non-timely triggering is reduced, thereby improving the safety factor of the fire protection device 2-100.

In order to enable the fire protection device 2-100 to move normally for blocking the fluid passage 2-11 when the temperature is high and there is a tendency for a fire to occur, the first valve body 2-20, the second valve body 2-30, the first valve body 2-20, the second valve body 2-30, the elastic member 2-40 and the housing 2-10 may all be parts which have a relatively high melting point and are difficult to burn. Also, since the fire protection device 2-100 is used in an oxygen-rich environment, the first valve body 2-20, the second valve body 2-30, the elastic member 2-40, the housing 2-10, etc. may be parts that are not easily oxidized.

In addition, since the first limiting ribs 2-12 and the second limiting ribs 2-13 are distributed at both ends of the fire protection device 2-100, when the fire protection device 2-100 is installed, the first opening may be installed on the pipeline of the oxygen supply end and the second opening may also be installed on the pipeline of the oxygen supply end, thereby facilitating the installation and avoiding the problem of distinguishing the front and back of the fire protection device 2-100.

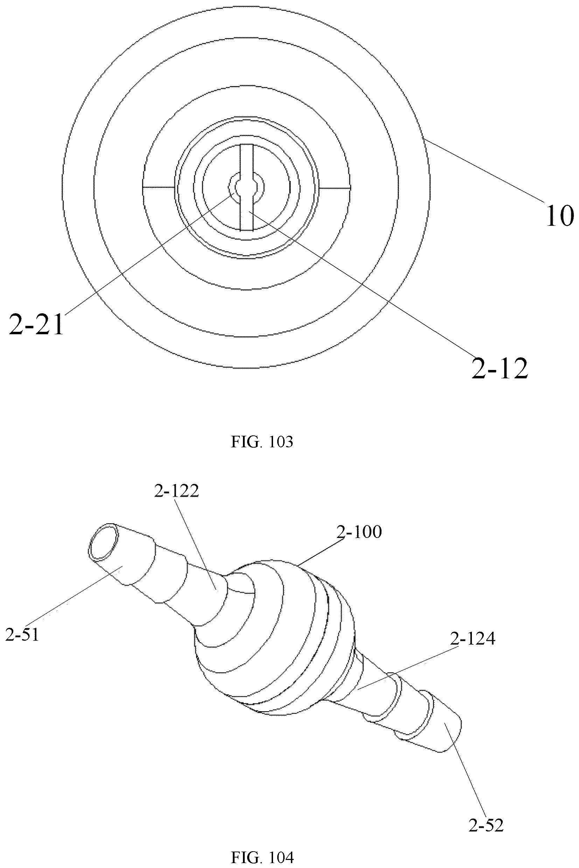

In order to avoid the influence of the first limiting ribs 2-12 on the flow of oxygen, the first limiting ribs 2-12 may be strip-shaped or have other shapes which do not influence the ventilation, for example, serrated, semi-circular, etc. The two ends of the first limiting ribs 2-12 are respectively connected to the hole wall of the first opening, and oxygen may flow into the fluid passage 2-11 through the two sides of the first limiting ribs 2-12, as shown in FIG. 103. Similarly, the second limiting ribs 2-13 are also strip-shaped. Reference may be made to the above description of the first limiting ribs 2-12. The embodiments of the present disclosure will not be described in detail herein.

In addition, in some embodiments, as shown in FIG. 101, FIG. 102 and FIG. 106, the first valve body 2-20 may include a first mounting rod 2-21 and a first mounting seat 2-22. The axial direction of the first mounting rod 2-21 is consistent with the extension and retraction direction of the elastic member 2-40. One end of the first mounting rod 2-21 is connected to the first mounting seat 2-22, and the other end of the first mounting rod 2-21 abuts against the first limiting rib 2-12. One end of the elastic member 2-40 abuts against the first mounting seat 2-22. The second valve body 2-30 includes a second mounting rod 2-31 and a second mounting seat 2-32. The axial direction of the second mounting rod 2-31 is consistent with the extension and retraction direction of the elastic member 2-40. One end of the second mounting rod 2-31 is connected to the second mounting seat 2-32, and the other end of the second mounting rod 2-31 abuts against the second limiting rib 2-13. The other end of the elastic member 2-40 abuts against the second mounting seat 2-32. When the first limiting rib 2-12 is in the molten state, the first mounting seat 2-22 is connected in a sealed manner with the passage wall of the fluid passage 2-11. When the second limiting rib 2-13 in the molten state, the second mounting seat 2-32 is connected in a sealed manner with the passage wall of the fluid passage 2-11.

The first valve body 2-20 includes the first mounting rod 2-21 and the first mounting seat 2-22. The axial direction of the first mounting rod 2-21 is consistent with the extension and retraction direction of the elastic member 2-40. One end of the first mounting rod 2-21 is connected to the first mounting seat 2-22, and the other end of the first mounting rod 2-21 abuts against the first limiting rib 2-12, so that the first valve body 2-20 may abut against the first limiting rib 2-12 via the first mounting rod 2-21. The second valve body 2-30 includes the second mounting rod 2-31 and the second mounting seat 2-32. The axial direction of the second mounting rod 2-31 is consistent with the extension and retraction direction of the elastic member 2-40. One end of the second mounting rod 2-31 is connected to the second mounting seat 2-32, and the other end of the second mounting rod 2-31 abuts against the second limiting rib 2-13, so that the second valve body 2-30 may abut against the second limiting rib 2-13 via the second mounting rod 2-31. One end of the elastic member 2-40 abuts against the first mounting seat 2-22, and the other end of the elastic member 2-40 abuts against the second mounting seat 2-32, so as to connect the first valve body 2-20 and the second valve body 2-30 via the elastic member 2-40. Thus, when the first limiting rib 2-12 is in the molten state, the first mounting rod 2-21 is separated from the first limiting rib 2-12. During the extension of the elastic member 2-40 from the compression device to the initial state, the elastic member 2-40 may push the first mounting seat 2-22, so that the first mounting seat 2-22 is in sealing connection with the passage wall to block the fluid passage 2-11. Alternatively, in the case where the second limiting rib 2-13 is in the molten state, the second mounting rod 2-31 is separated from the second limiting rib 2-13. During the extension of the elastic member 2-40 from the compression device to the initial state, the elastic member 2-40 may push the second mounting seat 2-32, so that the second mounting seat 2-32 is in sealed connection with the passage wall to block the fluid passage 2-11.

In addition, in some embodiments, as shown in FIG. 101, FIG. 102 and FIG. 106, an end of the first mounting seat 2-22 away from the first mounting rod 2-21 may be provided with a first mounting cavity 2-23. An end of the second mounting seat 2-32 away from the second mounting rod 2-31 may be provided with a second mounting cavity 2-33. The first mounting cavity 2-23 is located opposite to the second mounting cavity 2-33. One end of the elastic member 2-40 abuts against a cavity bottom of the first mounting cavity 2-23, and the other end of the elastic member 2-40 abuts against a cavity bottom of the second mounting cavity 2-33.

One end of the first mounting seat 2-22 away from the first mounting rod 2-21 is provided with the first mounting cavity 2-23, and one end of the second mounting seat 2-32 away from the second mounting rod 2-31 is provided with the second mounting cavity 2-33, so that the first mounting cavity 2-23 is opposite to the second mounting cavity 2-33. The first mounting cavity 2-23 and the second mounting cavity 2-33 may form a space for accommodating the elastic member 2-40, and the elastic member 2-40 may be provided in the first mounting cavity 2-23 and the second mounting cavity 2-33. So that one end of the elastic member 2-40 abuts against the cavity bottom of the first mounting cavity 2-23, and the other end of the elastic member 2-40 abuts against the cavity bottom of the second mounting cavity 2-33. Thus, the elastic member 2-40 disposed between the first valve body 2-20 and the second valve body 2-30 may take the first mounting cavity 2-23 and the second mounting cavity 2-33 as a mounting space, reducing the distance between the first valve body 2-20 and the second valve body 2-30, so that the distance between the first mounting rod 2-21 and the second mounting rod 2-31 is smaller, and thus distance between the first limiting rib 2-12 and the second limiting ribs 2-13 is smaller, which may reduce the size of the housing 10 from the first opening to the second opening, and reduce the size of the fire protection device 2-100 from the first opening to the second opening, so that the fire protection device 2-100 is smaller and convenient to install and carry.

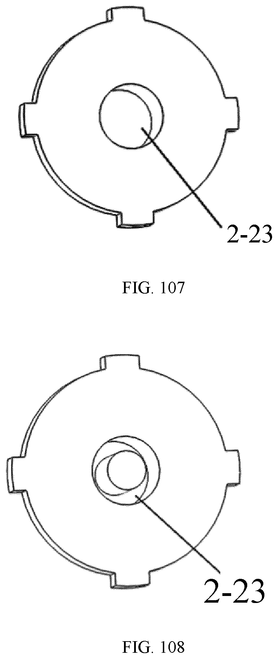

As shown in FIG. 107 and FIG. 108, the first mounting cavity 2-23 may have different forms. For example, an opening may be disposed on the first mounting seat 2-22, the opening may form the first mounting cavity 2-23, and the hole wall of the opening may be a smooth hole wall, as shown in FIG. 107. Optionally, the hole walls of the openings may not be smooth, as shown in FIG. 108, and ribs, threads, etc. may be disposed on the hole walls of the openings as long as they do not interfere with the installation and the extension and retraction of the elastic members 2-40. Also, the second mounting cavity 2-23 may have different forms. Reference may be made to the above description of the first mounting cavity 2-23. The embodiments of the present disclosure will not be described in detail herein.

In addition, in some embodiments, as shown in FIG. 101, FIG. 102 and FIG. 106, a first guide rod 2-24 may be connected to the cavity bottom of the first mounting cavity 2-23, and/or a second guide rod 2-34 may be connected to the cavity bottom of the second mounting cavity 2-33. The elastic member 2-40 is sleeved on the first guide rod 2-24 and/or the second guide rod 2-34. The first guide rod 2-24 and the second guide rod 2-34 are used for guiding the extension and retraction of the elastic member 2-40.

The first guide rod 2-24 is connected to the cavity bottom of the first mounting cavity 2-23. The elastic member 2-40 may be sleeved on the first guide rod 2-24. The first guide rod 2-24 may guide the extension and retraction of the elastic member 2-40. Thus, the elastic member 2-40 may extend and contract along the first guide rod 2-24, which may avoid the problem that the elastic member 2-40 shifts when extending and contracting, which results in that the force applied by the elastic member 2-40 to the first valve body 2-20 or the second valve body 2-30 shifts, the first valve body 2-20 or the second valve body 2-30 shifts when moving, and the sealing effect on the fluid passages 2-11 and the suppression effect on the fire is poor.

Also/optionally, the second guide rod 2-34 is connected to the cavity bottom of the second mounting cavity 2-33. The elastic member 2-40 may be sleeved on the second guide rod 2-34. The second guide rod 2-34 may guide the extension and retraction of the elastic member 2-40. Thus, the elastic member 2-40 may extend and contract along the second guide rod 2-34, which may avoid the problem that the elastic member 2-40 shifts when extending and contracting, which results in that the force applied by the elastic member 2-40 to the first valve body 2-20 or the second valve body 2-30 shifts, the first valve body 2-20 or the second valve body 2-30 shifts when moving, and the sealing effect on the fluid passages 2-11 and the suppression effect on the fire is poor.

In the embodiments of the present disclosure, the first guide rod 2-24 may be connected to the cavity bottom of the first mounting cavity 2-23, or the second guide rod 2-34 may be connected to the cavity bottom of the second mounting cavity 2-33. Optionally, the first guide rod 2-24 may be connected to the cavity bottom of the first mounting cavity 2-23, and the second guide rod 2-34 may be connected to the cavity bottom of the second mounting cavity 2-33. The elastic member 2-40 is sleeved on the first guide rod 2-24 and the second guide rod 2-34, with a better guiding effect on the extension and retraction of the elastic member 2-40.

In addition, in order to enable that when the first guide rod 2-24 is connected to the cavity bottom of the first mounting cavity 2-23, the first guide rod 2-24 has a better guide effect on the elastic member 2-40, the first guide rod 2-24 may have a longer size and may protrude into the second mounting cavity 2-33. Also, in order to enable that when the second guide rod 2-34 is connected to the cavity bottom of the second mounting cavity 2-33, the second guide rod 2-34 has a better guide effect on the elastic member 2-40, the second guide rod 2-34 may have a longer size, and may protrude into the first mounting cavity 2-23.

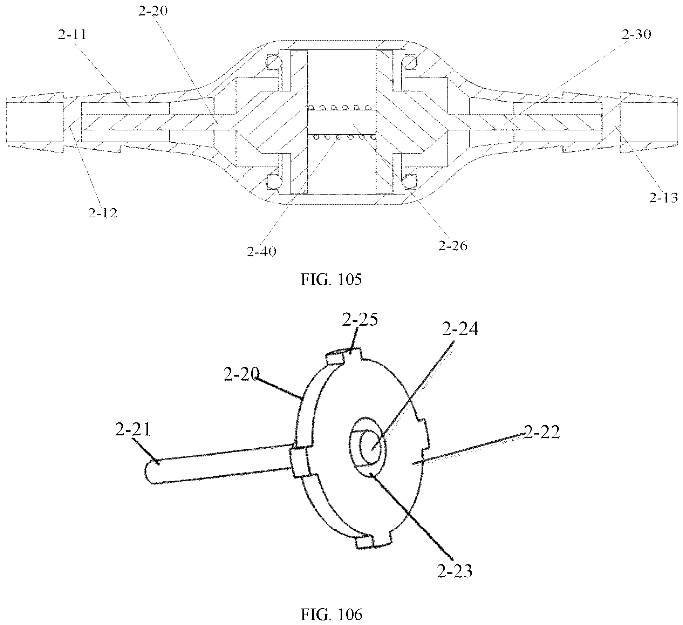

It should be noted that, the first mounting cavity 2-23 may not be disposed on the first mounting seat 2-22. The second mounting cavity 2-33 may not be disposed on the second mounting seat 2-32. A surface of the first mounting seat 2-22 facing towards the second mounting seat 2-32 and a surface of the second mounting seat 2-32 facing towards the first mounting seat 2-22 may directly abut against the elastic member 2-40, respectively. The first mounting seat 2-22 or the second mounting seat 2-32 is pushed by the elastic member 2-40. **In** order to avoid the problem that the elastic member 2-40 easily shifts when the elastic member 2-40 directly abuts against the surface of the first mounting seat 2-22 facing towards the second mounting seat 2-32 and the surface of the second mounting seat 2-32 facing towards the first mounting seat 2-22, the surface of the first mounting seat 2-22 facing towards the second mounting seat 2-32 may be provided with a guide shaft 2-26 which is allowed to extend to the surface of the second mounting seat 2-32 facing towards the first mounting seat 2-22. At this time, the extension and retraction of the elastic member 2-40 may be guided via the guide shaft 2-26. Alternatively, the surface of the second mounting seat 2-32 facing towards the first mounting seat 2-22 may be provided with the guide shaft 2-26, such that the guide shaft 2-26 extends to the surface of the first mounting seat 2-22 facing towards the second mounting seat 2-32, as shown in FIG. 105.

In addition, in some embodiments, only the first mounting cavity 2-23 may be disposed on the first mounting seat 2-22, and the first guide rod 2-24 may be provided in the first mounting cavity 2-23, so that two ends of the elastic member 2-40 respectively abut against the cavity bottom of the first mounting cavity 2-23 and the surface of the second mounting seat 2-32 facing towards the first mounting seat 2-22. Optionally, only the second mounting cavity 2-33 may be disposed on the second mounting seat 2-32, and the second guide rod 2-34 may be provided in the second mounting cavity 2-33, so that both ends of the elastic member 2-40 abut against the cavity bottom of the second mounting cavity 2-33 and the surface of the first mounting seat 2-22 facing towards the second mounting seat 2-32, respectively.

In addition, in some embodiments, as shown in FIG. 101, FIG. 102 and FIG. 106, a first positioning portion 2-25 is disposed on the peripheral portion of the first mounting seat 2-22. A first positioning groove 2-14 is disposed on the inner wall of the fluid passage 2-11. The extension direction of the first positioning groove 2-14 is consistent with the extension and retraction direction of the elastic member 2-40. The first positioning portion 2-25 may be embedded in the first positioning groove 2-14 which is used for limiting the movement direction of the first mounting seat 2-22 when the first limiting rib 2-12 is in a molten state. Also/optionally, a second positioning portion 2-35 is disposed on the peripheral portion of the second mounting seat 2-32. A second positioning groove 2-15 is disposed on the inner wall of the fluid passage 2-11. The extension direction of the second positioning groove 2-15 is consistent with the extension and retraction direction of the elastic member 2-40. The second positioning portion 2-35 may be embedded in the second positioning groove 2-15 which is used for limiting the movement direction of the second mounting seat 2-32 when the second limiting rib 2-13 is in the molten state.

The peripheral portion of the first mounting seat 2-22 is provided with the first positioning portion 2-25. The inner wall of the fluid passage 2-11 is provided with the first positioning groove 2-14. The first positioning portion 2-25 may be embedded in the first positioning groove 2-14. Since the extension direction of the first positioning groove 2-14 is consistent with the extension and retraction direction of the elastic member 2-40, in the case where the first valve body 2-20 moves, the first positioning portion 2-25 may move in the first positioning groove 2-14. In the present embodiment, the first positioning portion 2-25 may be limited by the first positioning groove 2-14 to limit the movement direction of the first mounting seat 2-22, and further to limit the movement direction of the first valve body 2-20, so as to avoid the problem that the first mounting seat 2-22 shifts during the movement and the sealing effect on the fluid passage 2-11 is poor.

Also/optionally, the peripheral portion of the second mounting seat 2-32 is provided with the second positioning portion 2-35. The inner wall of the fluid passage 2-11 is provided with the second positioning groove 2-15. The second positioning portion 2-35 may be embedded in the second positioning groove 2-15. Since the extension direction of the second positioning groove 2-15 is consistent with the extension and retraction direction of the elastic member 2-40, in the case where the second valve body 2-30 moves, the second positioning portion 2-35 may move in the second positioning groove 2-15. In the present embodiment, the second positioning portion 2-35 may be limited by the second positioning groove 2-15 to limit the movement direction of the second mounting seat 2-32 and further to limit the movement direction of the second valve body 2-30, so as to avoid the problem that the second mounting seat 2-32 shifts during the movement and the sealing effect on the fluid passage 2-11 is poor.

It should be noted that a plurality of first positioning portions 2-25 may be provided and are distributed at intervals on the peripheral portion of the first mounting seat 2-22. Correspondingly, a plurality of first positioning grooves 2-14 may also be provided. Each first positioning portion 2-25 is embedded in one positioning groove, so that the movement of the first mounting seat 2-22 may be limited at a plurality of positions, and the limiting effect on the first mounting seat 2-22 is better. Specifically, the number of the first positioning portions 2-25 may be four. At this time, four first positioning portions 2-25 are spaced apart at the peripheral portion of the first mounting seat 2-22, and the angle between each two adjacent first positioning portions 2-25 may be 90°, as shown in FIG. 106, or the number of the first positioning portions 2-25 may be another number, for example, 3, 2, 5, 6, 7, etc. The embodiment of the present disclosure is not specifically limited herein with respect to the particular number of the first positioning portions 2-25.

Certainly, the number of the second positioning portions 2-35 may be multiple. Specific reference may be made to the above description of the first positioning portions 2-25, and the embodiments of the present disclosure will not be described in detail.

In addition, in some embodiments, as shown in FIG. 101 and FIG. 102, a first mounting groove 2-16 may be disposed on the inner wall of the fluid passage 2-11. The first mounting groove 2-16 extends along the circumferential direction of the fluid passage 2-11 and surrounds the fluid passage 2-11. A first sealing member 2-17 is provided in the first mounting groove 2-16, and a portion of the first sealing member 2-17 extends outside the first mounting groove 2-16. The first sealing member 2-17 is opposite to the first valve body 2-20. The first sealing member 2-17 is used for abutting against the first valve body 2-20 when the first limiting rib 2-12 is in the molten state, so that the first valve body 2-20 is in sealing connection with the passage wall of the fluid passage 2-11. Also/optionally, the inner wall of the fluid passage 2-11 may be provided with a second mounting groove 2-18. The second mounting groove 2-18 extends along the circumferential direction of the fluid passage 2-11 and surrounds the fluid passage 2-11. A second sealing member 2-19 is provided in the second mounting groove 2-18, and a portion of the second sealing member 2-19 extends out of the second mounting groove 2-18. The second sealing member 2-19 is opposite to the second valve body 2-30. The second sealing member 2-19 is used for abutting against the second valve body 2-30 when the second limiting rib 2-13 is in the molten state, so that the second valve body 2-30 is in sealed connection with the passage wall of the fluid passage 2-11.

The first mounting groove 2-16 is disposed on the inner wall of the fluid passage 2-11. The first mounting groove 2-16 extends along the circumferential direction of the fluid passage 2-11 and surrounds the fluid passage 2-11. The first sealing member 2-17 may be provided in the first mounting groove 2-16. Since a portion of the first sealing member 2-17 extends outside the first mounting groove 2-16, the first sealing member 2-17 extends outside the passage wall of the fluid passage 2-11, so that the first mounting seat 2-22 may abut against the first sealing member 2-17 in the case where the first mounting seat 2-22 moves. It is possible to achieve a sealed connection between the first mounting seat 2-22 and the fluid passage 2-11, so that the first mounting seat 2-22 has a better blocking effect on the fluid passage 2-11, avoiding the problem of having a small amount of oxygen circulation and having a poor fire suppression effect.

The second mounting groove 2-18 is disposed on the inner wall of the fluid passage 2-11. The second mounting groove 2-18 extends along the circumferential direction of the fluid passage 2-11 and surrounds the fluid passage 2-11. The second sealing member 2-19 may be provided in the second mounting groove 2-18. Since a portion of the second sealing member 2-19 extends outside the second mounting groove 2-18, the second sealing member 2-19 extends outside the passage wall of the fluid passage 2-11, so that the second mounting seat 2-32 may abut against the second sealing member 2-19 in the case where the second mounting seat 2-32 moves. It is possible to achieve a sealed connection between the second mounting seat 2-32 and the fluid passage 2-11, so that the second mounting seat 2-32 has a better blocking effect on the fluid passage 2-11, avoiding the problem of having a small amount of oxygen circulation and having a poor fire suppression effect.

It should be noted that, the first sealing member 2-17 and the second sealing member 2-19 may be seal rings having elasticity, such that the first sealing member 2-17 and the second sealing member 2-19 may be compressed with the first mounting seat 2-22 abutting against the first sealing member 2-17 and the second mounting seat 2-32 abutting against the second sealing member 2-19. Thus, the first mounting seat 2-22 and the first sealing member 2-17 are in close contact, and the second mounting seat 2-32 and the second sealing member 2-19 are in close contact. The first sealing member 2-17 and the second sealing member 2-19 may be made of silicone rubber, ethylene-propylene rubber, neoprene rubber, nitrile rubber, etc. The embodiments of the present disclosure are not specifically limited herein for the specific materials of the first sealing member 2-17 and the second sealing member 2-19.

In addition, in some embodiments, as shown in FIG. 101 and FIG. 102, the passage wall of the fluid passage 2-11 may have a first blocking platform 2-111 and a second blocking platform 2-112 along the circumferential direction of the fluid passage 2-11. The first blocking platform 2-111 and the second blocking platform 2-112 are spaced apart along the direction from the first opening to the second opening. The first valve body 2-20 and the second valve body 2-30 are located between the first blocking platform 2-111 and the second blocking platform 2-112. In the direction from the first opening to the second opening, a projection of the first blocking platform 2-111 has an overlapping portion with a projection of the first valve body 2-20, and the projection of the second blocking platform 2-112 has an overlapping portion with the projection of the second valve body 2-30. The first blocking platform 2-111 has a first surface 2-1111 facing towards the first valve body 2-20 in the direction from the first opening to the second opening. The first mounting groove 2-16 is disposed on the first surface 2-1111 and extends along the circumferential direction of the fluid passage 2-11. The projection of the first mounting groove 2-16 is located inside the projection of the first valve body 2-20 in the direction from the first opening to the second opening. Also/optionally, the second blocking platform 2-112 has a second surface 2-1121 facing towards the second valve body 2-30 in the direction from the first opening to the second opening. The second mounting groove 2-18 is disposed on the second surface 2-1121. The second mounting groove 2-18 extends in the circumferential direction of the fluid passage 2-11. The projection of the second mounting groove 2-18 is located inside the projection of the second valve body 2-30 in the direction from the first opening to the second opening.

The passage wall of the fluid passage 2-11 may have the first blocking platform 2-111 and the second blocking platform 2-112 along the circumferential direction of the fluid passage 2-11. The first blocking platform 2-111 and the second blocking platform 2-112 are spaced apart along the direction from the first opening to the second opening. The first valve body 2-20 and the second valve body 2-30 are located between the first blocking platform 2-111 and the second blocking platform 2-112. Since the projection of the first blocking platform 2-111 has an overlapping portion with the projection of the first valve body 2-20 in the direction from the first opening to the second opening, in the case where the first limiting rib 2-12 is in the molten state, the first valve body 2-20 may move to abut against the first blocking platform 2-111, so that the first valve body 2-20 seals the fluid passage 2-11 via the first blocking platform 2-111. Since the projection of the second blocking platform 2-112 has an overlapping portion with the projection of the second valve body 2-30, in the case where the second limiting rib 2-13 is in the molten state, the second valve body 2-30 may move to abut against the second blocking platform 2-112, so that the second valve body 2-30 seals the fluid passage 2-11 via the second blocking platform 2-112.

Herein, the first blocking platform 2-111 has the first surface 2-1111 facing towards the first valve body 2-20 in the direction from the first opening to the second opening. The first mounting groove 2-16 is disposed on the first surface 2-1111, so that the first surface 2-1111 has an angle with the extension direction of the elastic member 2-40. The first valve body 2-20 may abut against the first surface 2-1111 when moving. Since the projection of the first mounting groove 2-16 is located inside the projection of the first valve body 2-20 in the direction from the first opening to the second opening, when the first valve body 2-20 moves, the first valve body 2-20 may abut against the first sealing member 2-17. In the case where the first valve body 2-20 moves to abut against the first sealing member 2-17, the first valve body 2-20 applies a force to the first sealing member 2-17, so that the first sealing member 2-17 deforms towards the first mounting groove 2-16. Thus, the first sealing member 2-17 has a good contact with the first valve body 2-20 and has a good blocking effect on the fluid passage 2-11. The first mounting groove 2-16 extends along the circumferential direction of the fluid passage 2-11. The first sealing member 2-17 also extends around the circumferential direction of the fluid passage 2-11. The first valve body 2-20 may abut against the first sealing member 2-17 in all directions along the fluid passage 2-11, with a better blocking effect on the fluid passage 2-11.

Also/optionally, the second blocking platform 2-112 has the second surface 2-1121 facing towards the second valve body 2-30 in the direction from the first opening to the second opening. The second mounting groove 2-18 is disposed on the second surface 2-1121, so that the second surface 2-1121 has an angle with the extension direction of the elastic member 2-40. The second valve body 2-30 may abut against the second surface 2-1121 when moving. Since the projection of the second mounting groove 2-18 is located inside the projection of the second valve body 2-30 in the direction from the first opening to the second opening, when the second valve body 2-30 moves, the second valve body 2-30 may abut against the second sealing member 2-19. In the case where the second valve body 2-30 moves to abut against the second sealing member 2-19, the second valve body 2-30 applies a force to the second sealing member 2-19, so that the second sealing member 2-19 deforms towards the second mounting groove 2-18. Thus, the second sealing member 2-19 has a good contact with the second valve body 2-30, and has a good blocking effect on the fluid passage 2-11. The second mounting groove 2-18 extends along the circumferential direction of the fluid passage 2-11. The second sealing member 2-19 also extends around the circumferential direction of the fluid passage 2-11. The second valve body 2-30 may abut against the second sealing member 2-19 in all directions along the fluid passage 2-11, with a better blocking effect on the fluid passage 2-11.

In the embodiment of the present disclosure, the first sealing member 2-17 is disposed on the first surface 2-1111, and is deformed towards the first surface 2-1111 when the first valve body 2-20 moves. The second sealing member 2-19 is disposed on the second surface 2-1121, and is deformed towards the second surface 2-1121 when the second valve body 2-30 moves. It may avoid the problem that when the first sealing member 2-17 is directly disposed on the passage wall of the fluid passage 2-11, when the second sealing member 2-19 is directly disposed on the passage wall of the fluid passage 2-11, and when the first valve body 2-20 or the second valve body 2-30 moves to press the first sealing member 2-17 and the second sealing member 2-19, the first sealing member 2-17 and the second sealing member 2-19 are deformed along the extension and retraction direction of the elastic member 2-40. There is a risk that the first valve body 2-20 passes over the first sealing member 2-17 and the second valve body 2-30 passes over the second sealing member 2-19, which results in that the first valve body 2-20 and the second valve body 2-30 lose the sealing effect on the fluid passage 2-11.

In addition, in some embodiments, as shown in FIG. 101 and FIG. 102, the housing 10 may include a first sub-housing 2-122 and a second sub-housing 2-124. The first sub-housing 2-122 is disposed opposite to the second sub-housing 2-124. A first passage 2-123 is disposed inside the first sub-housing 2-122. A second passage 2-125 is disposed inside the second sub-housing 2-124. The first sub-housing 2-122 is connected to the second sub-housing 2-124, and the first passage 2-123 communicates with the second passage 2-125 to form the fluid passage 2-11. The first valve body 2-20 is disposed in the first sub-housing 10, and the second valve body 2-30 is disposed in the second sub-housing 10.

The housing 10 may include the first sub-housing 2-122 and the second sub-housing 2-124. The first sub-housing 2-122 is disposed opposite to the second sub-housing 2-124. The first passage 2-123 is disposed inside the first sub-housing 2-122, and the second passage 2-125 is disposed inside the second sub-housing 2-124, so that after the first sub-housing 2-122 is connected to the second sub-housing 2-124, the first passage 2-123 communicates with the second passage 2-125 to form the fluid passage 2-11. The first valve body 2-20 is disposed in the first sub-housing 10, and the second valve body 2-30 is disposed in the second sub-housing 10.

It should be noted that since the pipeline of the oxygen therapy instrument and the pipeline of the oxygen supply end are generally small, both ends of the housing 10 as a whole are also small, and the housing 10 may be made into a structure with thick in the middle and thin at sides. Accordingly, the fluid passage 2-11 may also be made into a structure with a thick in middle and thin at sides. The middle part of the housing 10 may provide a mounting space for the first mounting seat 2-22 and the second mounting seat 2-32, as shown in FIG. 101, FIG. 102 and FIG. 104. In addition, the fluid passage 2-11 with a larger middle part may also avoid affecting the flow of oxygen. Since the pipeline to which the fire protection device 2-100 is connected is smaller, when the oxygen flows to the middle part of the fluid passage 2-11, the oxygen may flow from the gap between the first mounting seat 2-22 and the passage wall of the fluid passage 2-11 and the gap between the second mounting seat 2-32 and the passage wall of the fluid passage 2-11. Since the middle part of the fluid passage 2-11 is also larger, the amount of oxygen circulating in the gaps between the first mounting seat 2-22 and the passage wall of the fluid passage 2-11 and between the second mounting seat 2-32 and the passage wall of the fluid passage 2-11 is equal to the amount of oxygen circulating in the pipeline, so that the provision of the first valve body 2-20 and the second valve body 2-30 may be prevented from affecting the circulation of oxygen.

In the embodiment of the present disclosure, the housing 10 is provided separately, and specifically includes the first sub-housing 2-122 and the second sub-housing 2-124, so as to facilitate the installation of the first valve body 2-20 and the second valve body 2-30, and to avoid the problem that the first mounting seat 2-22 and the second mounting seat 2-32 are large in size and difficult to install from the smaller ends.

In addition, in some embodiments, as shown in FIG. 101, the first opening may have a first joint 2-51 connected thereto, and the first joint 2-51 is in communication with the fluid passage 2-11. The second opening may be connected with a second joint 2-52. The second joint 2-52 is in communication with the fluid passage 2-11. The first joint 2-51 and the second joint 2-52 are used for communicating with the pipeline of the oxygen therapy instrument or the pipeline of the oxygen supply end.

The first opening is connected with the first joint 2-51, and the second opening is connected with the second joint 2-52. Since the first joint 2-51 is in communication with the fluid passage 2-11, and the second joint 2-52 is in communication with the fluid passage 2-11, the fire protection device 2-100 may communicate with the pipeline of the oxygen therapy instrument or the pipeline of the oxygen supply end via the first joint 2-51 and the second joint 2-52, so as to apply the fire protection device 2-100 during the oxygen therapy.

It should be noted that since the pipeline of the oxygen therapy instrument and the pipeline of the oxygen supply end are generally small, both ends of the housing 10 as a whole are also small, and the housing 10 may be made into a structure with thick in the middle and thin at sides. Accordingly, the fluid passage 2-11 may also be made into a structure with a thick in middle and thin at sides. The middle part of the housing 10 may provide a mounting space for the first mounting seat 2-22 and the second mounting seat 2-32. In addition, the fluid passage 2-11 with a larger middle part may also avoid affecting the flow of oxygen. Since the pipeline to which the fire protection device 2-100 is connected is smaller, when the oxygen flows to the middle part of the fluid passage 2-11, the oxygen may flow from the gap between the first mounting seat 2-22 and the passage wall of the fluid passage 2-11 and the gap between the second mounting seat 2-32 and the passage wall of the fluid passage 2-11. Since the middle part of the fluid passage 2-11 is also larger, the amount of oxygen circulating in the gaps between the first mounting seat 2-22 and the passage wall of the fluid passage 2-11 and between the second mounting seat 2-32 and the passage wall of the fluid passage 2-11 is equal to the amount of oxygen circulating in the pipeline, so that the provision of the first valve body 2-20 and the second valve body 2-30 may be prevented from affecting the circulation of oxygen.

In the embodiment of the present disclosure, the fire protection device 2-100 includes the housing 10, the first valve body 2-20, the second valve body 2-30, and the elastic member 2-40. The housing 10 has the fluid passage 2-11 therein. One end of the housing 10 has the first opening, and the second end of the housing 10 has the second opening. The first opening and the second opening are in communication with the fluid passage 2-11, therefore, the first opening, the fluid passage 2-11, and the second opening may form a passage for the circulation of gas. Herein, the first opening and the second opening are used for communicating with the pipeline of the oxygen therapy instrument or the pipeline of the oxygen supply end, so that one end of the fluid passage 2-11 may be connected to the pipeline of the oxygen therapy instrument through the first opening and the second opening, and the other end of the fluid passage 11 may be connected to the pipeline of the oxygen supply end. The oxygen therapy instrument may deliver oxygen to the patient through the oxygen supply end. The first limiting rib 2-12 is fixed on the hole wall of the first opening. The second limiting rib 2-13 is fixed on the hole wall of the second opening. The first end of the first valve body 2-20 abuts against the first limiting rib 2-12. The second end of the second valve body 2-30 abuts against the second limiting rib 2-13. The elastic member 2-40 is connected between the second end of the first valve body 2-20 and the second end of the second valve body 2-30, so that the first valve body 2-20 and the second valve body 2-30 may be distributed at intervals along the direction from the first opening to the second opening. Since the elastic member 2-40 is in the compressed state, and the extension and retraction direction of the elastic member 2-40 is consistent with the extension direction of the fluid passage 2-11, the elastic member 2-40 may exert a force on the first valve body 2-20 and the second valve body 2-30, so that the first valve body 2-20 has a tendency to move in a direction towards the first opening, and the second valve body 2-30 has a tendency to move towards the second opening. At this time, the first limiting rib 2-12 may block the first valve body 2-20, and the second limiting rib 2-13 may block the second valve body 2-30. Since the gaps for the circulation of the gas is provided between the peripheral portion of the first valve body 2-20 and the passage wall 2-11 of the fluid passage and between the peripheral portion of the second valve body 2-30 and the passage wall 2-11 of the fluid passage, in normal use, the gas may flow through the gaps so that the oxygen therapy instrument may be normally used. In the case where the first limiting rib 2-12 is in the molten state, the first limiting rib 2-12 loses a blocking effect on the first valve body 2-20. At this time, the elastic member 2-40 is elongated, namely, the elastic member 2-40 may release the elastic potential energy, and the first valve body 2-20 moves to the first position in the direction towards the first opening under the elastic force of the elastic member 2-40, so that the first valve body 2-20 is in sealed connection with the passage wall of the fluid passage 2-11, thereby blocking the fluid passage 2-11 and preventing oxygen from continuing to flow in the fluid passage 2-11. In the case where the second limiting rib 2-13 is in the molten state, the second limiting rib 2-13 loses a blocking effect on the second valve body 2-30. At this time, the elastic member 2-40 is elongated, namely, the elastic member 2-40 may release the elastic potential energy. The second valve body 2-30 moves to the second position in the direction towards the second opening under the elastic force of the elastic member 2-40, so that the second valve body 2-30 is in sealed connection with the passage wall of the fluid passage 2-11, thereby blocking the fluid passage 2-11 and preventing oxygen from continuing to flow in the fluid passage 2-11.

That is, in the embodiment of the present disclosure, if a flame is induced due to improper operation during the oxygen therapy, the first limiting rib 2-12 or the second limiting rib 2-13 burns in a molten state. At this time, the elastic member 2-40 disposed between the first valve body 2-20 and the second valve body 2-30 may push the first valve body 2-20 or the second valve body 2-30 to move, so that the first valve body 2-20 or the second valve body 2-30 is in sealed connection with the fluid passage 2-11, thereby blocking the fluid passage 2-11. The fire protection device 2-100 may prevent the oxygen from continuing to flow in the fluid passage 2-11, avoid the problem that the flame with the oxygen pipe gradually burns to the fuselage, which easily causes the spread of fire and finally ignites the oxygen generator to have serious fire.

In addition, embodiments of the present disclosure also include a ventilation treatment apparatus including an oxygen therapy instrument, an oxygen supply end, and the fire protection device 2-100 in any one of the above-mentioned embodiments. The first opening is connected to the oxygen therapy instrument, and the second opening is connected to the oxygen supply end; or, the second opening is connected to the oxygen therapy instrument, and the first opening is connected to the oxygen supply end.

In the fire protection device 2-100 described above, when in a fire, the first valve body 2-20 or the second valve body 2-30 may be in sealed connection to the fluid passage 2-11, so as to block the fluid passage 2-11. The fire protection device 2-100 may prevent the oxygen from continuing to flow in the fluid passage 2-11, avoid the problem that the flame with the oxygen pipe gradually burns to the fuselage, which easily causes the spread of fire and finally ignites the oxygen generator to have serious fire.

It should be noted that, as used herein, the terms "comprises", "comprising", or any other variation thereof, are intended to cover a non-exclusive inclusion, such that a process, method, article, or device that includes a list of elements does not include only those elements but may include other elements not expressly listed or inherent to such process, method, article, or device. An element defined by the language "including a" does not, without more constraints, preclude the existence of additional identical elements in the process, method, article, or device that includes the element.

The embodiments of the present disclosure have been described above with reference to the accompanying drawings, but the present disclosure is not limited to the specific embodiments described above, which are intended to be illustrative only and not limiting. There are many other forms that may be made by those of ordinary skill in the art, inspired by the present disclosure, without departing from the scope protected by the purposes and claims of the present disclosure, all of which are within the protection of the present disclosure.

## Claims

1. A fire protection device, **characterized in that** the fire protection device comprises a housing, a valve body, a torsion spring, and a meltable member;
a fluid passage is provided in the housing, the fluid passage is provided with a first opening and a second opening, and the first opening and the second opening are used for communicating with a pipeline of an oxygen therapy instrument or a patient end, respectively;
the valve body is located in the fluid passage and is rotatably connected to the housing;
the valve body is provided with an accommodating cavity, and the fluid passage and the accommodating cavity are two spaces independent from each other;
the torsion spring is embedded in the accommodating cavity so as to drive the relative rotation of the valve body and the housing;
the meltable member is disposed on an inner wall of the fluid passage;
when the meltable member is in a non-molten state, the meltable member supports the valve body to be in a first position, and both the first opening and the second opening are in an open state; and
when the meltable member is in a molten state, the torsion spring drives the valve body to rotate to a second position, and at least one of the first opening and the second opening is in a closed state.

2. The fire protection device according to claim 1, **characterized in that** the valve body comprises a mounting portion, a first connecting portion and a first sealing portion;
the mounting portion comprises an inner shaft sleeve and an outer shaft sleeve, and the accommodating cavity is located between the inner shaft sleeve and the outer shaft sleeve;
a rotating shaft is provided in the fluid passage, and the inner shaft sleeve is sleeved on the rotating shaft and is rotatably connected to the rotating shaft;
one end of the first connecting portion is connected to a side wall of the outer shaft sleeve, and the other end of the first connecting portion is connected to the first sealing portion; and
when the valve body is in the second position, the first sealing portion is engaged with the first opening, so that the first opening is in the closed state.

3. The fire protection device according to claim 2, **characterized in that** the valve body further comprises a second connecting portion and a second sealing portion;
one end of the second connecting portion is connected to a side wall of the outer shaft sleeve, and the other end of the second connecting portion is connected to the second sealing portion; and
when the valve body is in the second position, the second sealing portion is engaged with the second opening, so that the second opening is in the closed state.

4. The fire protection device according to claim 3, **characterized in that** the first connecting portion and/or the second connecting portion are provided with a notch for passage of a fluid.

5. The fire protection device according to claim 1, **characterized in that** the inner wall of the fluid passage is provided with at least one limiting portion, and the limiting portion is located on a rotational path of the valve body; and
when the valve body is in the second position, the valve body abuts against the limiting portion.

6. The fire protection device according to claim 1, **characterized in that** the housing is provided with a first pipeline joint and a second pipeline joint;
the first pipeline joint is provided with a first through-hole, and the first through-hole communicates with the first opening;
the second pipeline joint is provided with a second through-hole, and the second through-hole communicates with the second opening; and
the first pipeline joint and the second pipeline joint are used for connecting with the pipeline of the oxygen therapy instrument or the patient end, respectively.

7. The fire protection device according to claim 6, **characterized in that** an outer side wall of the first pipeline joint and/or the second pipeline joint is provided with at least one clamping portion for being clamped with the pipeline of the oxygen therapy instrument or the patient end.

8. The fire protection device according to claim 6, **characterized in that** the meltable member has an extension passing through the first through-hole or the second through-hole.

9. An oxygen therapy instrument, **characterized in that** the oxygen therapy instrument comprises the fire protection device according to any one of claims 1-8.

10. A ventilation treatment system, **characterized in that** the ventilation treatment system comprises the oxygen therapy instrument according to claim 9.
